# EUROPEAN PATENT SPECIFICATION

(11) **EP 2 908 855 B1**
(45) Date of publication and mention of the grant of the patent: **28.06.2017**
(21) Application number: 13779190.1
(22) Date of filing: 15.10.2013
(51) Int. Cl.: A61K 39/09, C07K 14/315, A61K 39/00

(54) **IMMUNOGENIC COMPOSITION COMPRISING 1 OR MORE STREPTOCOCCUS PNEUMONIAE CAPSULAR SACCHARIDE CONJUGATES AND A PROTEIN COMPONENT COMPRISING PROTEIN E AND/OR PILA FROMHAEMOPHILUS INFLUENZAE .**
IMMUNOGENE ZUSAMMENSETZUNG MIT 1 ODER MEHREREN STREPTOCOCCUS PNEUMONIAE-KAPSELSACCHARIDKONJUGATEN UND PROTEINKOMPONENTE MIT PROTEIN E UND/ODER PILA FROMHAEMOPHILUS INFLUENZAE
COMPOSITION IMMUNOGÈNE COMPRENANT UN OU PLUSIEURS CONJUGUÉS SACCHARIDES CAPSULAIRES DE STREPTOCOCCUS PNEUMONIAE ET UN CONSTITUANT PROTÉIQUE COMPRENANT LA PROTÉINE E ET/OU PILA D'HAEMOPHILUS INFLUENZAE

(30) Priority: 17.10.2012 GB 201218660; 17.10.2012 US 201261714942 P; 17.10.2012 US 201261714956 P; 14.03.2013 US 201313826696; 14.03.2013 US 201313826932; 14.03.2013 US 201313827203
(43) Date of publication of application: 26.08.2015
(73) Proprietor: GlaxoSmithKline Biologicals S.A., 1330 Rixensart (BE)
(72) Inventor: CEDDIA, Francesca, B-1300 Wavre (BE); HAUSDORFF, WilliamPaul, B-1300 Wavre (BE); VERLANT, Vincent, B-1300 Wavre (BE); YSEBAERT, Carine, B-1330 Rixensart (BE)
(74) Representative: Baker, Suzanne J.
(86) International application number: PCT/EP2013/071472
(87) International publication number: WO 2014/060383

(56) References cited:
- WO-A1-2007/084053
- WO-A1-2009/000826
- WO-A1-2011/075823
- WO-A1-2012/139225
- WO-A2-2007/008527
- PRYMULA R ET AL: "Pneumococcal capsular polysaccharides conjugated to protein D for prevention of acute otitis media caused by both Streptococcus pneumoniae and non-typable Haemophilus influenzae: a randomised double-blind efficacy study", THE LANCET, LANCET LIMITED. LONDON, GB, vol. 367, no. 9512, 4 March 2006 (2006-03-04), pages 740-748, XP027948090, ISSN: 0140-6736 [retrieved on 2006-03-04]
- PRYMULA R ET AL: "Effect of vaccination with pneumococcal capsular polysaccharides conjugated to Haemophilus influenzae-derived protein D on nasopharyngeal carriage of Streptococcus pneumoniae and H. influenzae in children under 2 years of age", VACCINE, ELSEVIER LTD, GB, vol. 28, no. 1, 10 December 2009 (2009-12-10), pages 71-78, XP026789447, ISSN: 0264-410X, DOI: 10.1016/J.VACCINE.2009.09.113 [retrieved on 2009-10-08]
- CHOO S ET AL: "Immunogenicity and reactogenicity of a pneumococcal conjugate vaccine administered combined with a haemophilus influenzae type B conjugate vaccine in United Kingdom infants.", THE PEDIATRIC INFECTIOUS DISEASE JOURNAL SEP 2000, vol. 19, no. 9, September 2000 (2000-09), pages 854-862, XP002970987, ISSN: 0891-3668
- RENNELS M B ET AL: "SAFETY AND IMMUNOGENICITY OF HEPTAVALENT PNEUMOCOCCAL VACCINE CONJUGATED TO CRM197 IN UNITED STATES INFANTS", PEDIATRICS, AMERICAN ACADEMY OF PEDIATRICS, EVANSTON, IL, US, vol. 101, no. 4, PART 01, 1 April 1998 (1998-04-01), pages 604-611, XP008024884, ISSN: 0031-4005, DOI: 10.1542/PEDS.101.4.604
- BRYANT KRISTINA A ET AL: "Safety and immunogenicity of a 13-valent pneumococcal conjugate vaccine.", PEDIATRICS MAY 2010, vol. 125, no. 5, May 2010 (2010-05), pages 866-875, XP002719289, ISSN: 1098-4275
- PEETERS C C ET AL: "Effect of carrier priming on immunogenicity of saccharide-protein conjugate vaccines.", INFECTION AND IMMUNITY OCT 1991, vol. 59, no. 10, October 1991 (1991-10), pages 3504-3510, XP002719290, ISSN: 0019-9567
- SHARMA SHARAD K ET AL: "CD4+ T-cell responses among adults and young children in response to Streptococcus pneumoniae and Haemophilus influenzae vaccine candidate protein antigens.", VACCINE 26 JUN 2013, vol. 31, no. 30, 26 June 2013 (2013-06-26) , pages 3090-3097, XP002719291, ISSN: 1873-2518

## Description

### TECHNICAL FIELD

The present invention relates to immunogenic compositions comprising one or more *Streptococcus pneumoniae* capsular saccharide conjugates and a protein component comprising Protein E and/or PilA from *Haemophilus influenzae.*

### BACKGROUND

Non- typeable *Haemophilus influenzae* (NTHi) is an important and common respiratory pathogen that causes otitis media in infants and children. NTHi is, after *Streptococcus pneumoniae,* the most common cause of acute otitis media in children (J. Immunology 183: 2593-2601 (2009), Pediatrics 113:1451-1465 (2004)). It is an important cause of sinusitis in children and adults. (Current Infectious Disease Reports 11:177-182 (2009)). It has been associated with increased risk of exacerbations in chronic obstructive pulmonary disease (COPD) in adults. (Journal of Chronic Obstructive Pulmonary Disease 3:109-115 (2006)). In addition, non-typeable *H. influenzae* causes community-acquired pneumonia in adults and may cause pneumonia in children in developing countries. (Current Infectious Disease Reports 11:177-182 (2009)).

*Streptococcus pneumoniae* (*S. pneumoniae*), also known as the pneumococcus, is a Gram-positive bacterium. *S*. *pneumoniae* is a major public health problem all over the world and is responsible for considerable morbidity and mortality, especially among infants, the elderly and immunocompromised persons. *S*. *pneumoniae* causes a wide range of important human pathologies including community-acquired pneumonia, acute sinusitis, otitis media, meningitis, bacteremia, septicemia, osteomyelitis, septic arthritis, endocarditis, peritonitis, pericarditis, cellulitis, and brain abscess. *S*. *pneumoniae* is estimated to be the causal agent in 3,000 cases of meningitis, 50, 000 cases of bacteremia, 500,000 cases of pneumonia, and 7,000,000 cases of otitis media annnually in the United States alone (Reichler, M. R. et al., 1992, J. Infect. Dis. 166: 1346; Stool, S. E. and Field, M. J., 1989 Pediatr. Infect. Dis J. 8: S11). Mortality rates due to pneumococcal disease are especially high in children younger than 5 years of age from both developed and developing countries. The elderly, the immunocompromised and patients with other underlying conditions (diabetes, asthma) are also particularly susceptible to disease.

The major clinical syndromes caused by *S*. *pneumoniae* are widely recognized and discussed in standard medical textbooks (Fedson D S, Muscher D M. In: Plotkin S A, Orenstein W A, editors. Vaccines. 4th edition. Philadelphia WB Saunders Co, 2004a: 529-588). For instance, Invasive pneumococcal disease (IPD) is defined as any infection in which *S*. *pneumoniae* is isolated from the blood or another normally sterile site (Musher D M. Streptococcus pneumoniae. In Mandell G L, Bennett J E, Dolin R (eds). Principles and Practice of Infectious diseases (5th ed). New York, Churchill Livingstone, 2001, p 2128-2147).

Chronic obstructive pulmonary disease is a chronic inflammatory disease of the lungs and a major cause of morbidity and mortality worldwide. Approximately one in 20 deaths in 2005 in the US had COPD as the underlying cause. (Drugs and Aging 26:985-999 (2009)). It is projected that in 2020 COPD will rise to the fifth leading cause of disability adjusted life years, chronic invalidating diseases, and to the third most important cause of mortality (Lancet 349:1498-1504 (1997)).

Thus a need for combination vaccines against *Streptococcus pneumoniae* and *Haemophilus influenzae* exists.

Protein E (PE) is an outer membrane lipoprotein with adhesive properties. It plays a role in the adhesion/invasion of non-typeable *Haemophilus influenzae* (NTHi) to epithelial cells. (J. Immunology 183: 2593-2601 (2009); The Journal of Infectious Diseases 199:522-531 (2009), Microbes and Infection 10:87-96 (2008)). It is highly conserved in both encapsulated *Haemophilus influenzae* and non-typeable *H. influenzae* and has a conserved epithelial binding domain. (The Journal of Infectious Diseases 201:414-419 (2010)). Thirteen different point mutations have been described in different *Haemophilus* species when compared with *Haemophilus influenzae* Rd as a reference strain. Its expression is observed on both logarithmic growing and stationary phase bacteria. (WO2007/084053).

Protein E is also involved in human complement resistance through binding vitronectin. (Immunology 183: 2593-2601 (2009)). PE, by the binding domain PKRYARSVRQ YKILNCANYH LTQVR (SEQ ID NO. 1, corresponding to amino acids 84-108 of SEQ ID NO. 4), binds vitronectin which is an important inhibitor of the terminal complement pathway. (J. Immunology 183:2593-2601 (2009)).

Pilin A (PilA) is likely the major pilin subunit of *H. influenzae* Type IV Pilus (Tfp) involved in twitching motility (Infection and Immunity, 73: 1635-1643 (2005)). NTHi PilA is a conserved adhesin expressed *in vivo.* It has been shown to be involved in NTHi adherence, colonization and biofilm formation. (Molecular Microbiology 65: 1288-1299 (2007)). PRYMULA R ET AL: "Pneumococcal capsular polysaccharides conjugated to protein D for prevention of acute otitis media caused by both Streptococcus pneumoniae and non-typable Haemophilus influenzae: a randomised double-blind efficacy study", THE LANCET, LANCET LIMITED. LONDON, GB, vol. 367, no. 9512, 4 March 2006 (2006-03-04), pages 740-748, and PRYMULA R ET AL: "Effect of vaccination with pneumococcal capsular polysaccharides conjugated to Haemophilus influenzae-derived protein D on nasopharyngeal carriage of Streptococcus pneumoniae and H. influenzae in children under 2 years of age", VACCINE, ELSEVIER LTD, GB, vol. 28, no. 1, 10 December 2009 (2009-12-10), pages 71-78, disclose immunogenic compositions comprising capsular polysaccharide of pneumococcal serotypes conjugated to protein D derived from Haemophilus influenzae.

The inventors have found that PilA and PE may be beneficially present in an immunogenic composition for prevention of *H.influenzae* and furthermore that PilA and Protein E can be added to a composition comprising *S.pneumoniae* capsular saccharides in order to provide an immunogenic composition which can prevent *H.influenzae* and *S.pneumoniae* infection.The skilled person would be aware of the effect of carrier-induced epitopic suppression, and know that generally simultaneous exposure to multiple conjugate antigens can result in either enhanced or diminished immune responses (Plotkin *et al*, Vaccines fourth addition 2003).

### BRIEF SUMMARY

The inventors have found that PilA, PE (or fragments thereof) and saccharides from *Streptococcus pneumoniae* may be beneficially combined in an immunogenic composition to provide effective protection against *H.influenzae* and *S.pneumoniae.*

Accordingly in a first aspect there is provided an immunogenic composition comprising 1 or more (e.g. 7, 8, 9, 10, 11, 12, 13, 14, 15, 16, 17, 18, 19 or 20) *Streptococcus pneumoniae* capsular saccharide conjugates and a protein component comprising PilA (or an immunogenic fragment of PilA) from *Haemophilus influenzae.*

In a second aspect there is provided a vaccine comprising an immunogenic composition of the invention and a pharmaceutically acceptable excipient.

In a third aspect there is provided a method of immunising a subject against diseases caused by *Streptococcus pneumoniae* infection comprising administering to the subject a therapeutically effective dose of the immunogenic composition of the invention or the vaccine of the invention.

In a fourth aspect there is provided a method of immunising a subject against diseases caused by *Haemophilus influenzae* infection comprising administering to the subject a therapeutically effective dose of the immunogenic composition of the invention or the vaccine of the invention.

In a fifth aspect there is provided an immunogenic composition of the invention or a vaccine of the invention for use in the treatment or prevention of diseases caused by *Streptococcus pneumoniae* infection.

In a sixth aspect there is provided an immunogenic composition of the invention or a vaccine of the invention for use in the treatment or prevention of diseases caused by *Haemophilus influenzae* infection.

In a seventh aspect there is provided a use of the immunogenic composition of the invention or the vaccine of the invention in the manufacture of a medicament for the treatment or prevention of diseases caused by *Streptococcus pneumoniae* infection.

In an eighth aspect there is provided a use of the immunogenic composition of the invention or the vaccine of the invention in the manufacture of a medicament for the treatment or prevention of diseases caused by *Haemophilus influenzae* infection.

### BRIEF DESCRIPTION OF THE DRAWINGS

Figure 1. SDS-PAGE of induced bacterial extracts for fusion protein constructs LVL291, LVL268 and LVL269. Insoluble fraction (I), Soluble fraction (S) and Culture Media fraction (M) were loaded for LVL291, LVL268 and LVL269 before and after induction (ind).
Figure 2. SDS-PAGE and Western blot related to purification extracts for fusion protein constructs LVL291, LVL268 and LVL269. Flow through fraction (Ft), Wash fraction (W) and Elution fraction (E) were loaded for purification of LVL291, LVL268 and LVL269. Anti-his tag was used to probe extracts.
Figure 3. SDS-PAGE of induced bacterial and purification extracts for fusion protein constructs LVL291 and LVL315. Culture Media fraction (M), Soluble fraction (Sol), Insoluble fraction (Ins), Flow through fraction (Ft), Wash fraction #1 (W1), Wash fraction #2 (W2) and Elution fraction (E) were loaded for LVL291 and LVL315.
Figure 4. SDS-PAGE of induced bacterial and purification extracts for fusion protein construct LVL312. Culture Media fraction (M), Soluble fraction (Sol), Insoluble fraction (Ins), Flow Through fraction (Ft), Wash fraction #1 (W1), Wash fraction #2 (W2) and Elution fraction (E) were loaded for LVL312.
Figure 5. SDS-PAGE of induced (1mM and 10µM IPTG) bacterial extracts for fusion protein construct LVL317. Extracts from before (NI) and after induction (In), Soluble fraction (S), Insoluble fraction (I).
Figure 6. SDS-PAGE of induced (1mM and 10µM IPTG) bacterial extracts for fusion protein construct LVL318. Extracts from before (NI) and after induction (In), Culture Media fraction (M), Soluble fraction (S), Insoluble fraction (I).
Figure 7. CD spectra of PE, PilA and PE-PilA fusion proteins.
Figure 8. Combination of PE and PilA CD spectrum.
Figure 9. PilA thermal denaturation curve.
Figure 10. PE denaturation curve.
Figure 11. PE-PilA fusion protein thermal denaturation curve.
Figure 12. Typical SP Sepharose™ Fast Flow chromatogram.
Figure 13. Typical Q Sepharose™ Fast Flow chromatogram.
Figure 14. SDS-PAGE of In-process samples from purification process of PE-PilA fusion protein.
Figure 15. Western Blot of In-process samples of purification process from PE-PilA fusion protein. Blot using rabbit polyclonal anti-PE.
Figure 16. Western Blot of In-process samples of purification process from PE-PilA fusion protein. Blot using rabbit polyclonal anti-E.coli (BLR).
Figure 17. Thermal transition of PE-PilA fusion protein and PE and PilA proteins. Curves: PilA (1), Protein E (Prot E, PE) (2), PE-PilA Purified Bulk not diluted, 737µg/ml (3), and PE-PilA Purified Bulk diluted at Final Container concentration 60µg/ml (4).
Figure 18. Antibody responses against LVL291 PE-PilA fusion protein and against monovalent PE and PilA in the Balb/c mouse model.
Figure 19. Effect of PE-PilA fusion protein vaccination on NTHi strain 86-028NP bacterial clearance in mouse nasopharynx.
Figure 20. Effect of PE-PilA fusion protein vaccination on NTHi strain 3224A bacterial clearance in mouse nasopharynx.
Figure 21. Effect of PilA vaccination on bacterial clearance in mouse nasopharynx.
Figure 22. Effect of PE vaccination on bacterial clearance in mouse nasopharynx.
Figure 23. (a) LVL317 PE-PilA fusion protein binding to vitronectin and (b) LVL317 and LVL735 PE-PilA fusion protein bound to vitronectin.
Figure 24. Inhibition of vitronectin binding by polyclonal antibodies against PE-PilA fusion protein.
Figure 25. SDS-PAGE of soluble fractions of induced bacterial extracts for fusion protein constructs LVL291, LVL702, LVL736, LVL737, LVL738, LVL739, LVL740 and pET26b vector (negative control). (a) Experiment 1 (b) Experiment 2 (c) Experiment 3. PE-PilA fusion protein indicated by arrow.
Figure 26. The average band percentage of fusion protein in the soluble fraction from Experiments 1, 2 and 3.
Figure 27. PE and PilA antibody response to LVL317 and LVL735.
Figure 28. Effect of LVL735 and LVL317 vaccination on bacterial clearance in a mouse model of non-typeable *Haemophilus influenzae* nasopharyngeal colonization.

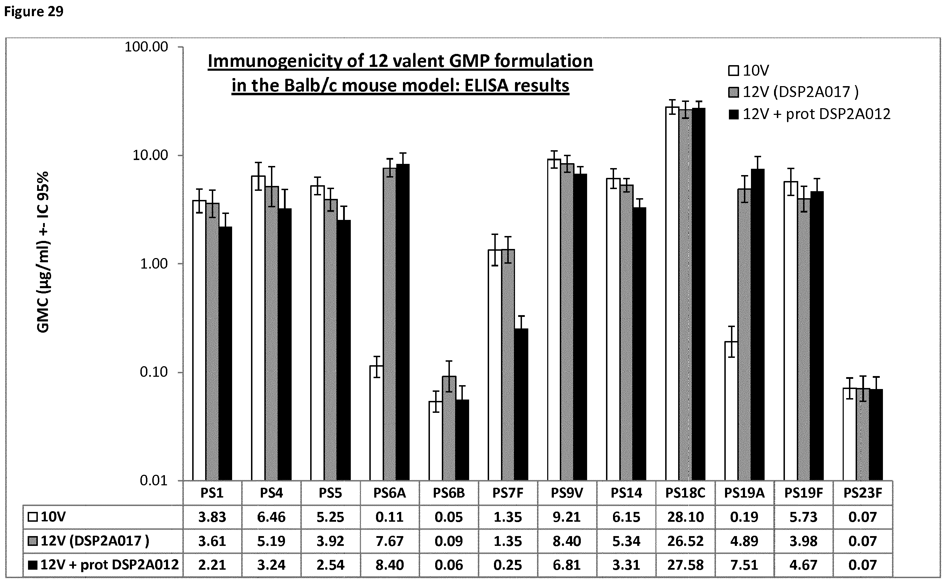

Figure 29. Graph comparing the immunogenicity of a composition comprising 12 saccharide conjugates, PhtD, dPly and PE-PilA (12V + prot) with a composition comprising 12 saccharide conjugates (12V) and a composition comprising 10 saccharide conjugates (10V) as measured after injection of mice using an anti-saccharide ELISA assay. GMC= geometric mean concentration . IC = confidence intervals.

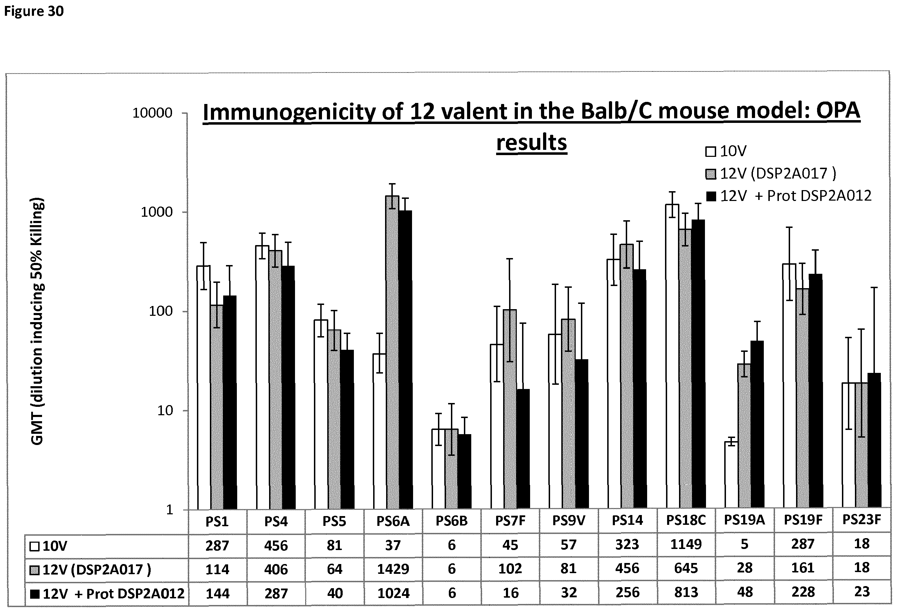

Figure 30. Graph comparing the immunogenicity of a composition comprising 12 saccharide conjugates, PhtD, dPly and PE-PilA (12V + prot) with a composition comprising 12 saccharide conjugates (12V) and a composition comprising 10 saccharide conjugates (10V) as measured after injection of mice using an opsonophagocytosis assay. GMT = geometric means titer.
Figure 31. Graph comparing the immunogenicity of a composition comprising 12 saccharide conjugates, PhtD, dPly and PE-PilA (12V + prot) with a composition comprising PhtD, dPly and PE-PilA alone (prot) as measured after injection of mice using an anti-protein ELISA assay. GMC= geometric mean concentration . IC = confidence intervals.

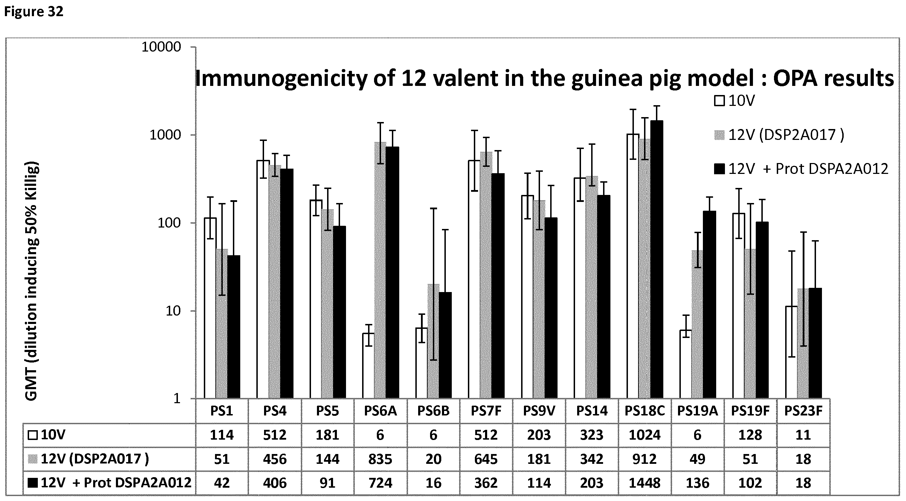

Figure 32. Graph comparing the immunogenicity of a composition comprising 12 saccharide conjugates, PhtD, dPly and PE-PilA (12V + prot) with a composition comprising 12 saccharide conjugates (12V) and a composition comprising 10 saccharide conjugates (10V) as measured after injection of guinea pigs using an opsonophagocytosis assay. GMT = geometric means titer.

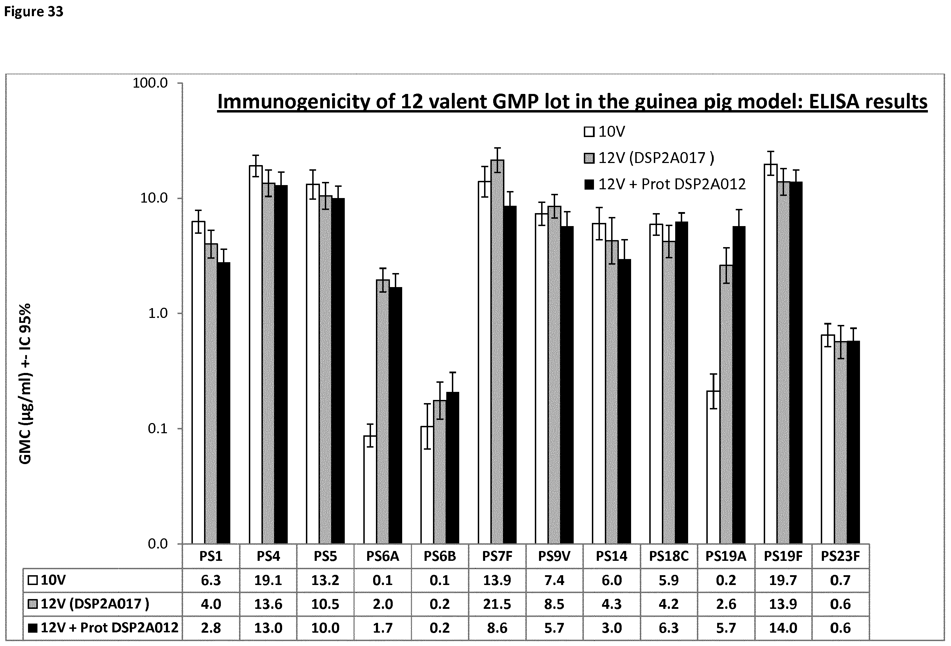

Figure 33. Graph comparing the immunogenic of a composition comprising 12 saccharide conjugates, PhtD, dPly and PE-PilA (12V + prot) with a composition comprising 12 saccharide conjugates (12V) and a composition comprising 10 saccharide conjugates (10V) as measured after injection of guinea pigs using an anti-saccharide ELISA. GMC= geometric mean concentration . IC = confidence intervals.
Figure 34. Graph comparing the immunogenic of a composition comprising 12 saccharide conjugates, PhtD, dPly and PE-PilA (12V + prot) with a composition comprising PhtD, dPly and PE-PilA alone (prot) as measured after injection of guinea pigs using an anti-protein ELISA. GMC= geometric mean concentration . ic = confidence intervals.

### DETAILED DESCRIPTION

In a first aspect, the present invention relates to an immunogenic composition comprising 1 or more (e.g. 7, 8, 9, 10, 11, 12, 13, 14, 15, 16, 17, 18, 19 or 20) *Streptococcus pneumoniae* capsular saccharide conjugates and a protein component comprising PilA (or an immunogenic fragment thereof) from *Haemophilus influenzae.* In an embodiment, the protein component comprises Protein E or an immunogenic fragment of Protein E and PilA or an immunogenic fragment of PilA from *Haemophilus influenzae.* In another embodiment the protein component comprises Protein E and PilA from *Haemophilus influenzae.*

The term "protein component" relates to a sequence of amino acids comprising Protein E (or an immunogenic fragment thereof) and/or PilA (or an immunogenic fragment thereof), the protein component may comprise Protein E alone, PilA alone, an immunogenic fragment of Protein E alone, an immunogenic fragment of PilA alone, Protein E and PilA, an immunogenic fragment of Protein E and PilA, an immunogenic fragment of Protein E and an immunogenic fragment of PilA or Protein E and an immunogenic fragment of PilA (for example as a fusion protein). The protein component may further comprise additional sequences.

### Protein E

As used herein "Protein E", "protein E", "Prot E", and "PE" mean Protein E from *H*. *influenzae.* Protein E may consist of or comprise the amino acid sequence of SEQ ID NO. 4 (MKKIILTLSL GLLTACSAQI QKAEQNDVKL APPTDVRSGY IRLVKNVNYY IDSESIWVDN QEPQIVHFDA VVNLDKGLYV YPEPKRYARS VRQYKILNCA NYHLTQVRTD FYDEFWGQGL RAAPKKQKKH TLSLTPDTTL YNAAQIICAN YGEAFSVDKK) as well as sequences with at least or exactly 75%, 77%, 80%, 85%, 90%, 95%, 97%, 99% or 100% identity, over the entire length, to SEQ ID NO. 4. Comparison of 53 sequences of Protein E from *Haemophilus influenzae* (Table 1, SEQ ID NO. 5 - SEQ ID NO. 57) demonstrated approximately 77% to approximately 100% identity to Protein E as set forth in SEQ ID NO. 4. For example, in the amino acid sequence of Protein E, amino acid #20 may be isoleucine (I) or threonine (T); amino acid #23 may be alanine (A) or valine (V); amino acid #24 may be lysine (K) or glutamic acid (E); amino acid #31 may be alanine (A) or threonine (T); amino acid #32 may be proline (P) or alanine (A); amino acid #34 may be threonine (T) or alanine (A); amino acid #37 may be arginine (R) or glutamine (Q); amino acid #47 may be valine (V) or alanine (A); amino acid #57 may be tryptophane (W) or may be absent (-); amino acid #70 may be alanine (A) or threonine (T); amino acid #93 may be glutamine (Q) or absent (-); amino acid #109 may be threonine (T) or isoleucine (I); amino acid #119 may be glycine (G) or serine (S); amino acid #153 may be glutamic acid (E) or lysine (K); amino acid #156 may be serine (S) or leucine (L); amino acid #160 may be lysine (K) or asparagine (N); amino acid #161 may be lysine (K), isoleucine (I) or absent (-); amino acids #162 - #195 may be absent, or as set forth in SEQ ID NO. 15 (with (-) indicating amino acid #166 is absent) or as set forth in SEQ ID NO. 16; or any combination thereof.

Protein E may consist of or comprise an amino acid sequence that differs from SEQ ID NO. 4 at any one or more amino acid selected from the group consisting of: amino acid #20, amino acid #23, amino acid #24, amino acid #31, amino acid #32, amino acid #34, amino acid #37, amino acid #47, amino acid #57, amino acid #70, amino acid #93, amino acid #109, amino acid #119, amino acid #153, amino acid #156, amino acid #160, amino acid #161 and amino acids #162-#195, wherein amino acid #20 is threonine (T); amino acid #23 is valine (V); amino acid #24 is lysine (K); amino acid #31 is threonine (T); amino acid #32 is alanine (A); amino acid #34 is alanine (A); amino acid #37 is glutamine (Q); amino acid #47 is alanine (A); amino acid #57 is absent (-); amino acid #70 is threonine (T); amino acid #93 is absent (-); amino acid #109 is isoleucine (I); amino acid #119 is serine (S); amino acid #153 is lysine (K); amino acid #156 is leucine (L); amino acid #160 is asparagine (N); amino acid #161 is lysine (K) or isoleucine (I); or amino acids #162 - #195 are as set forth in SEQ ID NO. 15 (with (-) indicating amino acid #166 is absent) or as set forth in SEQ ID NO. 16.

**Table 1: Protein E amino acid sequences from 53 strains of Haemophilus influenzae (SEQ ID NO. 5 - SEQ ID NO. 57). - indicates amino acid is absent.**

| **Strain Name** | **Protein E sequence** |
|---|---|
| 3224A | |
| RdKW20 | |
| 86-028NP | |
| R2846 | |
| R2866 | |
| | |
| 3655 | |
| PittAA | |
| PittEE | |
| PittHH | |
| PittII | |
| R3021 | |
| 22.4-21 | |
| 3219C | |
| 3185 | |
| 3241A | |
| 038144S1 | |
| | |
| 810956 | |
| 821246 | |
| 840645 | |
| 902550Z19 | |
| A840177 | |
| A860514 | |
| A950014 | |
| 306543X4 | |
| A930105 | |
| 901905U | |
| A920030 | |
| | |
| 3221B | |
| 27W116791 N | |
| N218 | |
| N163 | |
| N162 | |
| N107 | |
| N91 | |
| D211PG | |
| D211 PD | |
| D201PG | |
| D201PD | |
| D198PG | |
| D198PD | |
| D195PD | |
| D189PG | |
| D189PD | |
| D129CG | |
| D124PG | |
| D124PD | |
| D58PG | |
| D33OD | |
| BS433 | |
| BS432 | |
| | |
| 1714 | |
| 1128 | |
| BS430 | |

Protein E may be Protein E from *H. influenzae* strain 3224A, RdKW20, 86-028NP, R2846, R2866, 3655, PittAA, PittEE, PittHH, Pittll, R3021, 22.4-21, 3219C, 3185, 3241A, 038144S1, 810956, 821246, 840645, 902550Z19, A840177, A860514, A950014, 306543X4, A930105, 901905U, A920030, 3221B, 27W116791N, N218, N163, N162, N107, N91, D211PG, D211PD, D201PG, D201PD, D198PG, D198PD, D195PD, D189PG, D189PD, D129CG, D124PG, D124PD, D58PG, D33OD, BS433, BS432, 1714, 1128 or BS430. Protein E may be Protein E as set forth in any of SEQ ID NO. 5 - SEQ ID NO. 57.

Protein E may be a sequence with at least 95% or 98%, 99% identity, over the entire length, to any of SEQ ID NO. 4 - SEQ ID NO. 57. Protein E may be a sequence with at least 95% identity, over the entire length, to any of the sequences set forth in Table 1, SEQ ID NO. 5 - SEQ ID NO. 57.

Immunogenic fragments of Protein E comprise immunogenic fragments of at least 7, 10, 15, 20, 25, 30 or 50 contiguous amino acids of SEQ ID NO. 4. In one embodiment the fragment is less than 150, 125, 100, 75, or 60 amino acids of Protein E, for example in one embodiment the immunogenic composition of the invention comprises less than 150, 125, 100, 75 or 60 amino acids of Protein E. The immunogenic fragments may elicit antibodies which can bind SEQ ID NO. 4. The immunogenic fragment may comprise a B and/or T cell epitope of SEQ ID NO:4.

Immunogenic fragments of Protein E may comprise immunogenic fragments of at least 7, 10, 15, 20, 25, 30 or 50 contiguous amino acids of any of SEQ ID NO. 4 - SEQ ID NO. 57. The immunogenic fragments may elicit antibodies which can bind the full length sequence from which the fragment is derived. The immunogenic fragment may comprise a B and/or T cell epitope of SEQ ID NO:4 - SEQ ID NO. 57. In one embodiment the immunogenic fragment of Protein E is selected from the group consisting of amino acids 17-160 of SEQ ID NO. 4 (SEQ ID NO. 122), amino acids 18-160 of SEQ ID NO. 4 (SEQ ID NO. 123), amino acids 19-160 of SEQ ID NO. 4 (SEQ ID NO. 124), amino acids 20-160 of SEQ ID NO. 4 (SEQ ID NO. 125) and amino acids 22-160 of SEQ ID NO. 4 (SEQ ID NO. 126). In another embodiment, the immunogenic fragment of protein E is selected from the group consisting of amino acids 17-160 of SEQ ID NO. 4 (SEQ ID NO. 122), amino acids 18-160 of SEQ ID NO. 4 (SEQ ID NO. 123), amino acids 19-160 of SEQ ID NO. 4 (SEQ ID NO. 124), amino acids 20-160 of SEQ ID NO. 4 (SEQ ID NO. 125), amino acids 22-160 of SEQ ID NO. 4 (SEQ ID NO. 126), amino acids 23-160 of SEQ ID NO. 4 (SEQ ID NO. 179) and amino acids 24-160 of SEQ ID NO. 4 (SEQ ID NO. 180). In a further embodiment, the immunogenic fragment of Protein E from *H. influenzae* is selected from the group consisting of amino acids 17-160 of SEQ ID NO. 4 (SEQ ID NO. 122), amino acids 18-160 of SEQ ID NO. 4 (SEQ ID NO. 123), amino acids 20-160 of SEQ ID NO. 4 (SEQ ID NO. 125), amino acids 22-160 of SEQ ID NO. 4 (SEQ ID NO. 126), amino acids 23-160 of SEQ ID NO. 4 (SEQ ID NO. 179) and amino acids 24-160 of SEQ ID NO. 4 (SEQ ID NO. 180). More specifically, in one embodiment, the immunogenic fragment is SEQ ID NO. 124, amino acids 19-160 of SEQ ID NO. 4. In an additional embodiment, the immunogenic fragment is SEQ ID NO.125, amino acids 20-160 of SEQ ID NO. 5. In another embodiment, the immunogenic fragment is an immunogenic fragment of Protein E from *H. influenzae* selected from the group consisting of amino acids 23-160 of SEQ ID NO. 4 (SEQ ID NO. 179) and amino acids 24-160 of SEQ ID NO. 4 (SEQ ID NO. 180).

Protein E contains an epithelial cell binding region (PKRYARSVRQ YKILNCANYH LTQVR, SEQ ID NO. 128) that has been reported to be conserved among more than 100 clinical NTHi isolates, encapsulated H. influenzae, and culture collection strains analyzed (Singh et al, J. Infect. Dis. 201(3):414-9 (2010)). Singh et al. reported that Protein E was highly conserved in both NTHi and encapsulated H. influenzae (96.9% - 100% identity without the signal peptide). In one embodiment, the fragment of Protein E comprises the binding region of SEQ ID NO. 128 (PKRYARSVRQ YKILNCANYH LTQVR)
Protein E - SEQ ID NO. 4
Amino acids 17-160 of Protein E from SEQ ID NO. 4 - SEQ ID NO. 122
Amino acids 18-160 of Protein E from SEQ ID NO. 4 - SEQ ID NO. 123
Amino acids 19-160 of Protein E from SEQ ID NO. 4 - SEQ ID NO. 124
Amino acids 20-160 of Protein E from SEQ ID NO. 4 - SEQ ID NO. 125
Amino acids 22-160 of Protein E from SEQ ID NO. 4 - SEQ ID NO. 126
Amino acids 23-160 of Protein E from SEQ ID NO. 4 - SEQ ID NO. 179
Amino acids 24-160 Protein E from SEQ ID NO. 4 - SEQ ID NO. 180

In one embodiment the Protein E or an immunogenic fragment thereof is capable of eliciting an immune response which recognizes SEQ ID NO:4. Whether or not a first protein is capable of eliciting an immune response which recognizes a second protein can be determined using an ELISA assay (for example the ELISA described in example 22).

### PilA

As used herein "PilA" means Pilin A from *H*. *influenzae.* PilA may consist of or comprise the protein sequence of SEQ ID NO. 58 (MKLTTQQTLK KGFTLIELMI VIAIIAILAT IAIPSYQNYT KKAAVSELLQ ASAPYKADVE LCVYSTNETT NCTGGKNGIA ADITTAKGYV KSVTTSNGAI TVKGDGTLAN MEYILQATGN AATGVTWTTT CKGTDASLFP ANFCGSVTQ) as well as sequences with 80% to 100% identity to SEQ ID NO. 58. For example, PilA may be at least 80%, 85%, 90%, 95%, 97% or 100% identical to SEQ ID NO. 58. Full length comparison of 64 sequences of PilA from *Haemophilus influenzae* (Table 2, SEQ ID NO. 58 - SEQ ID NO. 121) demonstrated approximately 80% to 100% identity to PilA as set forth in SEQ ID NO. 58. For example, in the amino acid sequence of PilA, amino acid #6 may be glutamine (Q) or leucine (L); amino acid #7 may be glutamine (Q) or threonine (T); amino acid #37 may be glutamine (Q) or lysine (K); amino acid #44 may be alanine (A) or serine (S); amino acid #57 may be alanine (A) or serine (S); amino acid #67 may be asparagine (N) or glycine (G); amino acid #68 may be glutamic acid (E) or lysine (K); amino acid #69 may be theronine (T) or proline (P); amino acid #71 may be lysine (K), asparagine (N), serine (S) or threonine (T); amino acid #73 may be threonine (T), serine (S) or methionine (M); amino acid #76 may be lysine (K), serine (S) or asparagine (N); amino acid #84 may be threonine (T) or lysine (K); amino acid #86 may be alanine (A) or valine (V); amino acid #91 may be lysine (K) or alanine (A); amino acid #94 may be threonine (T), isoleucine (I) or lysine (K); amino acid #96 may be serine (S) or glutamine (Q); amino acid #97 may be asparagine (N) or serine (S); amino acid #99 may be alanine (A) or glycine (G); amino acid #103 may be alanine (A) or lysine (K); amino acid #109 may be aspartic acid (D), alanine (A) or threonine (T); amino acid #110 may be glycine (G), asparagine (N), or arginine (R); amino acid #112 may be serine (S) or glutamic acid (E); amino acid #114 may be threonine (T) or isoleucine (I); amino acid #116 may be threonine (T) or glutamine (Q); amino acid #118 may be glutamic acid (E), threonine (T), alanine (A), lysine (K) or serine (S); amino acid #121 may be serine (S) or alanine (A); amino acid #122 may be alanine (A) or threonine (T); amino acid #123 may be lysine (K), threonine (T) or alanine (A); amino acid #128 may be lysine (K) or threonine (T); amino acid #135 may be aspartic acid (D) or glutamic acid (E); amino acid #136 may be alanine (A) or threonine (T); amino acid #145 may be glycine (G) or arginine (R); amino acid #149 may be glutamine (Q) or lysine (K); or any combination thereof.

PilA may consist of or comprise an amino acid sequence that differs from SEQ ID NO. 58 at any one or more amino acid selected from the group consisting of amino acid #6, amino acid #7, amino acid #37, amino acid #44, amino acid #57, amino acid #67, amino acid #68, amino acid #69, amino acid #71, amino acid #73, amino acid #76, amino acid #84, amino acid #86, amino acid #91, amino acid #94, amino acid #96, amino acid #97, amino acid #99, amino acid #103, amino acid #109, amino acid #110, amino acid #112, amino acid #114, amino acid #116, amino acid #118 amino acid, #121, amino acid #122, amino acid #123, amino acid #128, amino acid #135, amino acid #136, amino acid #145 and amino acid #149, wherein amino acid #6 is leucine (L); amino acid #7 is threonine (T); amino acid #37 is lysine (K); amino acid #44 is serine (S); amino acid #57 is serine (S); amino acid #67 is glycine (G); amino acid #68 is lysine (K); amino acid #69 is proline (P); amino acid #71 is lysine (K), serine (S) or threonine (T); amino acid #73 is serine (S) or methionine (M); amino acid #76 is serine (S) or asparagine (N); amino acid #84 is lysine (K); amino acid #86 is valine (V); amino acid #91 is alanine (A); amino acid #94 is isoleucine (I) or lysine (K); amino acid #96 is glutamine (Q); amino acid #97 is serine (S); amino acid #99 is glycine (G); amino acid #103 is alanine (A); amino acid #109 is aspartic acid (D) or threonine (T); amino acid #110 is glycine (G) or arginine (R); amino acid #112 is serine (S); amino acid #114 is threonine (T); amino acid #116 is threonine (T); amino acid #118 is glutamic acid (E), alanine (A), lysine (K) or serine (S); amino acid #121 is serine (S); amino acid #122 is threonine (T); amino acid #123 is lysine (K) or alanine (A); amino acid #128 is lysine (K); amino acid #135 is glutamic acid (E); amino acid #136 is threonine (T); amino acid #145 is arginine (R); amino acid #149 is lysine (K).

**Table 2: Pilin A amino acid sequences from 64 strains of Haemophilus influenzae (SEQ ID NO. 58-SEQ ID NO. 121)**

| **Strain Name** | **PilA sequence** |
|---|---|
| 86-028NP | |
| NTHi3219C | |
| NTHi3224A | |
| NTHi12 | |
| NTHi44 | |
| NTHi67 | |
| 1054MEE | |
| 1729MEE | |
| 1728MEE | |
| 1885MEE | |
| 1060MEE | |
| RdKW20 | |
| 214NP | |
| 1236MEE | |
| 1714MEE | |
| 1128MEE | |
| R2846 | |
| R2866 | |
| | |
| 3655 | |
| PittAA | |
| PittGG | |
| PittII | |
| R3021 | |
| 22.4-21 | |
| 3185A | |
| 3221B | |
| 3241A | |
| 038144S1 | |
| 821246 | |
| 840645 | |
| 902550Z19 | |
| A840177 | |
| A920030 | |
| | |
| A950014 | |
| 901905U | |
| A920029 | |
| A930105 | |
| 306543X4 | |
| N218 | |
| N163 | |
| N162 | |
| N120 | |
| N107 | |
| N92 | |
| N91 | |
| D219PG | |
| D211PG | |
| D211 PD | |
| D204CD | |
| D198PG | |
| D198PD | |
| D195PD | |
| D195CD | |
| D189PG | |
| D189PD | |
| D124PG | |
| D124PD | |
| D124CG | |
| D58PG | |
| BS433 | |
| BS432 | |
| BS430 | |
| | |
| 1714 | |
| 1128 | |

PilA may be PilA *from H. influenzae* strain NTHi3219C, NTHi3224A, NTHi12, NTHi44, NTHi67, 1054MEE, 1729MEE, 1728MEE, 1885MEE, 1060MEE, RdKW20, 214NP, 1236MEE, 1714MEE, 1128MEE, 86-028NP, R2846, R2866, 3655, PittAA, PittGG, Pittll, R3021, 22.4-21, 3185A, 3221B, 3241A, 038144S1, 821246, 840645, 902550Z19, A840177, A920030, A950014, 901905U, A920029, A930105, 306543X4, N218, N163, N162, N120, N107, N92, N91, D219PG, D211PG, D211PD, D204CD, D198PG, D198PD, D195PD, D195CD, D189PG, D189PD, D124PG, D124PD, D124CG, D58PG, BS433, BS432, BS430, 1714 or 1128. An amino acid sequence for PilA from *H. influenzae* strain D204CD is set forth in SEQ ID NO. 106, wherein X at position #116 is either glutamine (Q) or leucine (L); ambiguity as to the amino acid at position #116 could be cleared up by technical resolution of the second nucleotide encoding amino acid #116, clarifying the PilA sequence for strain D204CD. PilA may be PilA as set forth in any of SEQ ID NO. 58 - SEQ ID NO. 121.

PilA may be a sequence with at least 95%, 98%, or 99% identity, over the entire length, to any of SEQ ID NO. 58 - SEQ ID NO. 121 (as set out in Table 2).

Immunogenic fragments of PilA comprise immunogenic fragments of at least 7, 10, 15, 20, 25, 30 or 50 contiguous amino acids of SEQ ID NO. 58 - SEQ ID NO. 121. The immunogenic fragments may elicit antibodies which can bind the full length sequence from which the fragment is derived. The immunogenic fragments may comprise a B and/or T cell epitope of SEQ ID NO. 58-SEQ ID NO:121.

For example, immunogenic fragments of PilA comprise immunogenic fragments of at least 7, 10, 15, 20, 25, 30 or 50 contiguous amino acids of SEQ ID NO. 58. In one embodiment the immunogenic fragment of PilA comprises less than 150, 125, 100, 75, or 60 amino acids of PilA, in a further embodiment the immunogenic composition comprises less than 150, 125, 100, 75 or 60 amino acids of PilA. The immunogenic fragments may elicit antibodies which can bind SEQ ID NO. 58. The immunogenic fragments may comprise a B and/or T cell epitope of SEQ ID NO:58.

In one embodiment the immunogenic fragment of PilA is a fragment from *H. influenzae* strain 86-028NP wherein PilA is SEQ ID NO. 58.
PilA from *H. influenzae* strain 86-028NP - SEQ ID NO. 58

In another embodiment, the immunogenic fragment of PilA is approximately at least 75%, 80%, 85%, 90%, 95%, 98% or 99% identical to SEQ ID NO. 127. More specifically, in one embodiment the immunogenic fragment of PilA is SEQ ID NO. 127, a fragment consisting of amino acids 40-149 of SEQ ID NO. 58.
Amino acids 40-149 of PilA from *H. influenzae* strain 86-028NP - SEQ ID NO. 127.

In another embodiment, the immunogenic fragment of PilA consists of amino acids 40-149 from any of SEQ ID NO. 58 - SEQ ID NO. 121. In an additional embodiment, the immunogenic fragment is at least 95% identical to amino acids 40-149 from any of SEQ ID NO. 58-SEQ ID NO. 121

Identity between polypeptides may be calculated by various algorithms. For example, the Needle program, from the EMBOSS package (Free software; EMBOSS: The European Molecular Biology Open Software Suite (2000). Trends in Genetics 16(6): 276-277) and the Gap program from the GCG^{®} package (Accelrys Inc.) may be used. This Gap program is an implementation of the Needleman-Wunsch algorithm described in: Needleman, S. B. and Wunsch, C. D. (1970) J. Mol. Biol. 48, 443-453. The BLOSUM62 scoring matrix has been used, and the gap open and extension penalties were respectively 8 and 2.

Looking at the computed alignment, identical residues between two compared sequences can be observed. A percentage of identity can be computed by (1) calculating the number of identities divided by the length of the alignment, multiplied by 100 (for example, for the Needle program analysis), (2) calculating the number of identities divided by the length of the longest sequence, multiplied by 100, (3) calculating the number of identities divided by the length of the shortest sequence, multiplied by 100, or (4) calculating the number of identities divided by the number of aligned residues, multiplied by 100 (a residue is aligned if it is in front of another) (for example, for the Gap program analysis).

In one embodiment the PilA is capable of eliciting an immune response which recognizes SEQ ID NO. 58.

### Protein E/PilA fusion protein

In one embodiment Protein E and PilA are present in a fusion protein. In a further embodiment the fusion protein has formula (I):

L(X)ₘ - (R₁)ₙ - A - (Y)ₒ - B - (Z)p (formula I)

wherein:
X is a signal peptide or MHHHHHH (SEQ ID NO. 2);
m is 0 or 1;
R₁ is an amino acid;
n is 0, 1, 2, 3, 4, 5 or 6;
A is Protein E from *Haemophilus influenzae* or an immunogenic fragment thereof, or PilA from *Haemophilus influenzae* or an immunogenic fragment thereof;
Y is selected from the group consisting of GG, SG, SS and (G)ₕ wherein h is 4, 5, 6, 7, 8, 9, or 10;
o is 0 or 1;
B is PilA from *Haemophilus influenzae* or an immunogenic fragment thereof, or Protein E from *Haemophilus influenzae* or an immunogenic fragment thereof;
Z is GGHHHHHH (SEQ ID NO: 3); and
p is 0 or 1.

In one embodiment, the fusion proteins of formula (I) are defined wherein X is selected from the group consisting of the signal sequence from CcmH (cytochrome c membrane protein H), DsbA (periplasmic protein disulfide isomerise I), DsbB (disulfide bond membrane protein B), Flgl (flagellar peptidoglycan ring protein), FocC (F1c Chaperone protein), MalE (maltose transporter subunit E), NadA (quinolinate synthase subunit A), NikA (nickel ABC transporter component A), NspA (Neisserial surface protein A), Omp26 (outer membrane protein 26), OmpA (outer membrane protein A), OspA (outer surface protein A), pelB (pectate lyase B), PhoA (bacterial alkaline phosphatase), PhtD (poly histidine triad protein D), PhtE (poly histidine triad protein E), SfmC (periplasmic pilin chaperone), Sip1 (surface immunogenic protein), TolB (Tol-Pal Cell Envelope Complex Component B), TorA (trimethylamine N-oxide reductase system subunit A), TorT (trimethylamine N-oxide reductase system periplasmic protein T) and Yral (putative periplasmic pilin chaperone); or any subgroup thereof. In one embodiment, X is a co-translational signal peptide or a post-translational signal peptide. In one embodiment X is the signal sequence from Flgl (*flgl sp*)*.* In another particular embodiment, X is the signal sequence from pelB (*pelB sp)*. In another embodiment, X is a post-translational signal peptide. In another embodiment, X is selected from the group consisting of the signal sequence from Flgl, NadA and pelB.

In one embodiment, the fusion proteins of formula (I) are defined wherein m is 1. In another embodiment, m is 0.

In one particular embodiment, R₁ and n are defined wherein (R₁)ₙ is 1 to 6 amino acids enriched in small, usually hydrophilic, amino acids. Hydrophilic amino acids include glutamic acid (E), aspartic acid (D) and asparagine (N).

In one embodiment, the fusion proteins of formula (I) are defined wherein n is selected from the group consisting of 0, 1, 2 and 6. In one particular embodiment, R₁ and n are defined wherein (R₁)ₙ is selected from the group consisting of D, E, ATNDDD (SEQ ID NO. 178) and MD, or any subset thereof.

In one particular embodiment, n is selected from the group consisting of 1, 2 and 6. In one particular embodiment, n is 0.

In one embodiment, the fusion proteins of formula (I) are defined wherein A is Protein E from *H. influenzae.* In another embodiment, the fusion proteins of formula (I) are defined wherein A is Protein E as encoded by an amino acid sequence selected from the group consisting of SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 18, SEQ ID NO. 19, SEQ ID NO. 20, SEQ ID NO. 21, SEQ ID NO. 22, SEQ ID NO. 23, SEQ ID NO. 24, SEQ ID NO. 25, SEQ ID NO. 26, SEQ ID NO. 27, SEQ ID NO. 28, SEQ ID NO. 29, SEQ ID NO. 30, SEQ ID NO. 31, SEQ ID NO. 32, SEQ ID NO. 33, SEQ ID NO. 34, SEQ ID NO. 35, SEQ ID NO. 36, SEQ ID NO. 37, SEQ ID NO. 38, SEQ ID NO.39, SEQ ID NO. 40, SEQ ID NO. 41, SEQ ID NO. 42, SEQ ID NO. 43 SEQ ID NO. 44, SEQ ID NO. 45, SEQ ID NO. 46, SEQ ID NO. 47, SEQ ID NO. 48, SEQ ID NO. 49, SEQ ID NO. 50, SEQ ID NO. 51, SEQ ID NO. 52, SEQ ID NO. 53, SEQ ID NO. 54, SEQ ID NO. 55, SEQ ID NO. 56 and SEQ ID NO. 57; or any subset of SEQ ID NO. 5 through SEQ ID NO. 57. In another embodiment, the fusion proteins of formula (I) are defined wherein A is Protein E, wherein Protein E is approximately at least 75%, 80%, 85%, 90%, 92%, 95%, 98% or 99% identical to the Protein E amino acid sequence set forth in SEQ ID NO: 4. In another embodiment, A is Protein E wherein Protein E is approximately 90% to 100% identical to the Protein E amino acid sequence set forth in SEQ ID NO: 4. In another embodiment, A is Protein E wherein Protein E is at least 95% identical to the Protein E amino acid sequence set forth in SEQ ID NO: 4. In additional embodiment, A is Protein E wherein Protein E is at least 95% identical to Protein E as set for in any of SEQ ID NO. 4 - SEQ ID NO. 57. In a particular embodiment, A is Protein E having the amino acid sequence set forth in SEQ ID NO. 4.

In another embodiment, the fusion proteins of formula (I) are defined wherein A is an immunogenic fragment of Protein E from *H. influenzae.* In another embodiment, A is an immunogenic fragment of Protein E wherein Protein E has an amino acid sequence selected from the group consisting of SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 18, SEQ ID NO. 19, SEQ ID NO. 20, SEQ ID NO. 21, SEQ ID NO. 22, SEQ ID NO. 23, SEQ ID NO. 24, SEQ ID NO. 25, SEQ ID NO. 26, SEQ ID NO. 27, SEQ ID NO. 28, SEQ ID NO. 29, SEQ ID NO. 30, SEQ ID NO. 31, SEQ ID NO. 32, SEQ ID NO. 33, SEQ ID NO. 34, SEQ ID NO. 35, SEQ ID NO. 36, SEQ ID NO. 37, SEQ ID NO. 38, SEQ ID NO.39, SEQ ID NO. 40, SEQ ID NO. 41, SEQ ID NO. 42, SEQ ID NO. 43 SEQ ID NO. 44, SEQ ID NO. 45, SEQ ID NO. 46, SEQ ID NO. 47, SEQ ID NO. 48, SEQ ID NO. 49, SEQ ID NO. 50, SEQ ID NO. 51, SEQ ID NO. 52, SEQ ID NO. 53, SEQ ID NO. 54, SEQ ID NO. 55, SEQ ID NO. 56 and SEQ ID NO. 57; or any subset of SEQ ID NO. 4 through SEQ ID NO. 57. In another embodiment, A is an immunogenic fragment of Protein E, wherein Protein E is approximately 75%, 80%, 85%, 90%, 92%, 95%, 98% or 99%identical to the amino acid sequence set forth in SEQ ID NO: 4. In another embodiment, A is an immunogenic fragment of Protein E, wherein Protein E is approximately 90% to 100% identical to SEQ ID NO. 4. In an additional embodiment, A is an immunogenic fragment of Protein E, wherein Protein E is at least 95% identical to any of SEQ ID NO. 4 - SEQ ID NO. 57. More specifically, in one embodiment, A is an immunogenic fragment of Protein E, wherein Protein E is at least 93%, 95%, 98%, 99% or 100% identical to SEQ ID NO. 124. In a particular embodiment, A is an immunogenic fragment of Protein E wherein Protein E is SEQ ID NO. 4.

In another embodiment, A is an immunogenic fragment of Protein E from *H. influenzae* selected from the group consisting of amino acids 17-160 of SEQ ID NO. 4 (SEQ ID NO. 122), amino acids 18-160 of SEQ ID NO. 4 (SEQ ID NO. 123), amino acids 19-160 of SEQ ID NO. 4 (SEQ ID NO. 124), amino acids 20-160 of SEQ ID NO. 4 (SEQ ID NO. 125) and amino acids 22-160 of SEQ ID NO. 4 (SEQ ID NO. 126). In another embodiment, A is an immunogenic fragment of Protein E from *H. influenzae* selected from the group consisting of amino acids 17-160 of SEQ ID NO. 4 (SEQ ID NO. 122), amino acids 18-160 of SEQ ID NO. 4 (SEQ ID NO. 123), amino acids 19-160 of SEQ ID NO. 4 (SEQ ID NO. 124), amino acids 20-160 of SEQ ID NO. 4 (SEQ ID NO. 125), amino acids 22-160 of SEQ ID NO. 4 (SEQ ID NO. 126), amino acids 23-160 of SEQ ID NO. 4 (SEQ ID NO. 179) and amino acids 24-160 of SEQ ID NO. 4 (SEQ ID NO. 180). In a further embodiment, A is an immunogenic fragment of Protein E from *H*. *influenzae* selected from the group consisting of amino acids 17-160 of SEQ ID NO. 4 (SEQ ID NO. 122), amino acids 18-160 of SEQ ID NO. 4 (SEQ ID NO. 123), amino acids 20-160 of SEQ ID NO. 4 (SEQ ID NO. 125), amino acids 22-160 of SEQ ID NO. 4 (SEQ ID NO. 126), amino acids 23-160 of SEQ ID NO. 4 (SEQ ID NO. 179) and amino acids 24-160 of SEQ ID NO. 4 (SEQ ID NO. 180). More specifically, in one embodiment, A is SEQ ID NO. 124, amino acids 19-160 of SEQ ID NO. 4. In an additional embodiment, A is SEQ ID NO.125, amino acids 20-160 of SEQ ID NO. 5. In another embodiment, A is immunogenic fragment of Protein E from *H*. *influenzae* selected from the group consisting of amino acids 23-160 of SEQ ID NO. 4 (SEQ ID NO. 179) and amino acids 24-160 of SEQ ID NO. 4 (SEQ ID NO. 180).
Protein E - SEQ ID NO. 4
Amino acids 17-160 of Protein E from SEQ ID NO. 4 - SEQ ID NO. 122
Amino acids 18-160 of Protein E from SEQ ID NO. 4 - SEQ ID NO. 123
Amino acids 19-160 of Protein E from SEQ ID NO. 4 - SEQ ID NO. 124
Amino acids 20-160 of Protein E from SEQ ID NO. 4 - SEQ ID NO. 125
Amino acids 22-160 of Protein E from SEQ ID NO. 4 - SEQ ID NO. 126
Amino acids 23-160 of Protein E from SEQ ID NO. 4 - SEQ ID NO. 179
Amino acids 24-160 Protein E from SEQ ID NO. 4 - SEQ ID NO. 180

In another embodiment, the fusion proteins of formula (I) are defined wherein A is PilA from *H. influenzae.* In another embodiment, the fusion proteins of formula (I) are defined wherein A is PilA from *H. influenzae* having an amino acid sequence selected from the group consisting of SEQ ID NO. 58, SEQ ID NO. 59, SEQ ID NO. 60, SEQ ID NO. 61, SEQ ID NO. 62, SEQ ID NO. 63, SEQ ID NO. 64, SEQ ID NO. 65, SEQ ID NO. 66, SEQ ID NO. 67, SEQ ID NO. 68, SEQ ID NO. 69, SEQ ID NO. 70, SEQ ID NO. 71, SEQ ID NO.72, SEQ ID NO. 73, SEQ ID NO. 74, SEQ ID NO. 75, SEQ ID NO. 76, SEQ ID NO. 77, SEQ ID NO. 78, SEQ ID NO. 79, SEQ ID NO. 80, SEQ ID NO. 81, SEQ ID NO. 82, SEQ ID NO. 83, SEQ ID NO. 84, SEQ ID NO. 85, SEQ ID NO. 86, SEQ ID NO. 87, SEQ ID NO. 88, SEQ ID NO. 89, SEQ ID NO. 90, SEQ ID NO. 91, SEQ ID NO. 92, SEQ ID NO. 93, SEQ ID NO. 94, SEQ ID NO. 95, SEQ ID NO. 96, SEQ ID NO. 97, SEQ ID NO. 98, SEQ ID NO. 99, SEQ ID NO. 100, SEQ ID NO. 101, SEQ ID NO. 102, SEQ ID NO. 103, SEQ ID NO. 104, SEQ ID NO. 105, SEQ ID NO. 106, SEQ ID NO. 107, SEQ ID NO. 108, SEQ ID NO. 109, SEQ ID NO. 110, SEQ ID NO. 111, SEQ ID NO. 112, SEQ ID NO. 113, SEQ ID NO. 114, SEQ ID NO. 115, SEQ ID NO. 116, SEQ ID NO. 117, SEQ ID NO. 118, SEQ ID NO. 119, SEQ ID NO. 120 and SEQ ID NO. 121; or any subset of SEQ ID NO. 58 through SEQ ID NO. 121. In another embodiment, A is PilA wherein PilA is approximately at least 75%, 80%, 85%, 90%, 92%, 95%, 98% or 99% identical to SEQ ID NO. 58. In another embodiment, A is PilA wherein PilA is at least 95% identical to any of SEQ ID NO. 58 - SEQ ID NO. 121. In a particular embodiment, A is PilA of SEQ ID NO. 58.

In another embodiment, the fusion proteins of formula (I) are defined wherein A is an immunogenic fragment of PilA from *H. influenzae.* In another embodiment, A is an immunogenic fragment of PilA wherein PilA is approximately at least 75%, 80%, 85%, 90%, 92%, 95%, 98% or 99% identical to SEQ ID NO. 58. For example, A is an immunogenic fragment of PilA wherein PilA has an amino acid sequence selected from the group consisting of SEQ ID NO. 58, SEQ ID NO. 59, SEQ ID NO. 60, SEQ ID NO. 61, SEQ ID NO. 62, SEQ ID NO. 63, SEQ ID NO. 64, SEQ ID NO. 65, SEQ ID NO. 66, SEQ ID NO. 67, SEQ ID NO. 68, SEQ ID NO. 69, SEQ ID NO. 70, SEQ ID NO. 71, SEQ ID NO.72, SEQ ID NO. 73, SEQ ID NO. 74, SEQ ID NO. 75, SEQ ID NO. 76, SEQ ID NO. 77, SEQ ID NO. 78, SEQ ID NO. 79, SEQ ID NO. 80, SEQ ID NO. 81, SEQ ID NO. 82, SEQ ID NO. 83, SEQ ID NO. 84, SEQ ID NO. 85, SEQ ID NO. 86, SEQ ID NO. 87, SEQ ID NO. 88, SEQ ID NO. 89, SEQ ID NO. 90, SEQ ID NO. 91, SEQ ID NO. 92, SEQ ID NO. 93, SEQ ID NO. 94, SEQ ID NO. 95, SEQ ID NO. 96, SEQ ID NO. 97, SEQ ID NO. 98, SEQ ID NO. 99, SEQ ID NO. 100, SEQ ID NO. 101, SEQ ID NO. 102, SEQ ID NO. 103, SEQ ID NO. 104, SEQ ID NO. 105, SEQ ID NO. 106, SEQ ID NO. 107, SEQ ID NO. 108, SEQ ID NO. 109, SEQ ID NO. 110, SEQ ID NO. 111, SEQ ID NO. 112, SEQ ID NO. 113, SEQ ID NO. 114, SEQ ID NO. 115, SEQ ID NO. 116, SEQ ID NO. 117, SEQ ID NO. 118, SEQ ID NO. 119, SEQ ID NO. 120 and SEQ ID NO. 121; or any subset SEQ ID NO. 58 through SEQ ID NO. 121. In an additional embodiment, A is an immunogenic fragment of PilA wherein PilA is at least 95% identical to any of SEQ ID NO. 58 - SEQ ID NO. 121. In a particular embodiment, A is an immunogenic fragment of PilA from *H. influenzae* strain 86-028NP wherein PilA is SEQ ID NO. 58.
PilA *from H. influenzae* strain 86-028NP - SEQ ID NO. 58

In another embodiment, A is an immunogenic fragment of PilA approximately at least 75%, 80%, 85%, 90%, 92%, 95%, 98% or 99% identical to SEQ ID NO. 127. More specifically, in one embodiment A is SEQ ID NO. 127, a fragment consisting of amino acids 40-149 of SEQ ID NO. 58.
Amino acids 40-149 of PilA from *H. influenzae* strain 86-028NP - SEQ ID NO. 127.

In another embodiment, A is an immunogenic fragment of PilA consisting of amino acids 40-149 from any of SEQ ID NO. 58 - SEQ ID NO. 121. In an additional embodiment, A is an immunogenic fragment at least 95% identical to amino acids 40-149 from any of SEQ ID NO. 58 - SEQ ID NO. 121.

In one embodiment, the fusion proteins of formula (I) are defined wherein Y is selected from the group consisting of GG, SG and SS. In another embodiment, the fusion proteins of formula (I) are defined wherein Y is GG or SG. In one particular embodiment, Y is GG.

In one embodiment, the fusion proteins of formula (I) are defined wherein o is 1. In another embodiment, o is 0.

In one embodiment, the fusion proteins of formula (I) are defined wherein B is PilA from *H. influenzae* or an immunogenic fragment of PilA from *H. influenzae* when A is Protein E from *H. influenzae* or an immunogenic fragment of Protein E from *H. influenzae.* For example, B is PilA from *H. influenzae* strain 86-028NP. In another embodiment, B is PilA from *H. influenzae* having an amino acid sequence selected from the group consisting of SEQ ID NO. 58, SEQ ID NO. 59, SEQ ID NO. 60, SEQ ID NO. 61, SEQ ID NO. 62, SEQ ID NO. 63, SEQ ID NO. 64, SEQ ID NO. 65, SEQ ID NO. 66, SEQ ID NO. 67, SEQ ID NO. 68, SEQ ID NO. 69, SEQ ID NO. 70, SEQ ID NO. 71, SEQ ID NO.72, SEQ ID NO. 73, SEQ ID NO. 74, SEQ ID NO. 75, SEQ ID NO. 76, SEQ ID NO. 77, SEQ ID NO. 78, SEQ ID NO. 79, SEQ ID NO. 80, SEQ ID NO. 81, SEQ ID NO. 82, SEQ ID NO. 83, SEQ ID NO. 84, SEQ ID NO. 85, SEQ ID NO. 86, SEQ ID NO. 87, SEQ ID NO. 88, SEQ ID NO. 89, SEQ ID NO. 90, SEQ ID NO. 91, SEQ ID NO. 92, SEQ ID NO. 93, SEQ ID NO. 94, SEQ ID NO. 95, SEQ ID NO. 96, SEQ ID NO. 97, SEQ ID NO. 98, SEQ ID NO. 99, SEQ ID NO. 100, SEQ ID NO. 101, SEQ ID NO. 102, SEQ ID NO. 103, SEQ ID NO. 104, SEQ ID NO. 105, SEQ ID NO. 106, SEQ ID NO. 107, SEQ ID NO. 108, SEQ ID NO. 109, SEQ ID NO. 110, SEQ ID NO. 111, SEQ ID NO. 112, SEQ ID NO. 113, SEQ ID NO. 114, SEQ ID NO. 115, SEQ ID NO. 116, SEQ ID NO. 117, SEQ ID NO. 118, SEQ ID NO. 119, SEQ ID NO. 120 and SEQ ID NO. 121; or any subset of SEQ ID NO. 58 through SEQ ID NO. 121. In another embodiment, B is PilA wherein PilA is approximately at least 75%, 80%, 85%, 90%, 92%, 95%, 98% or 99% identical to SEQ ID NO. 58. In another embodiment, B is PilA wherein PilA is at least 95%, 98% or 99% identical to any of SEQ ID NO. 58 - SEQ ID NO. 121. In a particular embodiment, B is PilA of SEQ ID NO. 58.

In another embodiment, B is PilA wherein PilA is at least 95%, 98% or 99% identical to any of SEQ ID NO. 58 - SEQ ID NO. 121 and A is PE wherein PE is at least 95%, 98% or 99% identical to any of SEQ ID NO. 4 - SEQ ID NO. 57.

In another embodiment, the fusion proteins of formula (I) are defined wherein B is an immunogenic fragment of PilA from *H. influenzae* when A is an immunogenic fragment of Protein E from *H. influenzae.* For example, B is an immunogenic fragment of the PilA from *H*. *influenzae* strain 86-028NP. In another embodiment, B is an immunogenic fragment of PilA wherein PilA is approximately at least 80%, 85%, 90%, 95%, 98% or 99% identical to SEQ ID NO: 58. In another embodiment, B is an immunogenic fragment of PilA wherein PilA has an amino acid selected from the group consisting of SEQ ID NO. 58, SEQ ID NO. 59, SEQ ID NO. 60, SEQ ID NO. 61, SEQ ID NO. 62, SEQ ID NO. 63, SEQ ID NO. 64, SEQ ID NO. 65, SEQ ID NO. 66, SEQ ID NO. 67, SEQ ID NO. 68, SEQ ID NO. 69, SEQ ID NO. 70, SEQ ID NO. 71, SEQ ID NO.72, SEQ ID NO. 73, SEQ ID NO. 74, SEQ ID NO. 75, SEQ ID NO. 76, SEQ ID NO. 77, SEQ ID NO. 78, SEQ ID NO. 79, SEQ ID NO. 80, SEQ ID NO. 81, SEQ ID NO. 82, SEQ ID NO. 83, SEQ ID NO. 84, SEQ ID NO. 85, SEQ ID NO. 86, SEQ ID NO. 87, SEQ ID NO. 88, SEQ ID NO. 89, SEQ ID NO. 90, SEQ ID NO. 91, SEQ ID NO. 92, SEQ ID NO. 93, SEQ ID NO. 94, SEQ ID NO. 95, SEQ ID NO. 96, SEQ ID NO. 97, SEQ ID NO. 98, SEQ ID NO. 99, SEQ ID NO. 100, SEQ ID NO. 101, SEQ ID NO. 102, SEQ ID NO. 103, SEQ ID NO. 104, SEQ ID NO. 105, SEQ ID NO. 106, SEQ ID NO. 107, SEQ ID NO. 108, SEQ ID NO. 109, SEQ ID NO. 110, SEQ ID NO. 111, SEQ ID NO. 112, SEQ ID NO. 113, SEQ ID NO. 114, SEQ ID NO. 115, SEQ ID NO. 116, SEQ ID NO. 117, SEQ ID NO. 118, SEQ ID NO. 119, SEQ ID NO. 120 and SEQ ID NO. 121; or any subset of SEQ ID NO. 58 through SEQ ID NO. 121. In another embodiment, B is an immunogenic fragment of PilA wherein PilA is at least 95%, 98% or 99% identical to any of SEQ ID NO. 58 - SEQ ID NO. 121. In a particular embodiment, B is an immunogenic fragment of PilA from *H. influenzae* wherein PilA has the amino acid sequence set forth in SEQ ID NO. 58. In another embodiment, B is an immunogenic fragment of PilA consisting of amino acids 40-149 from any of SEQ ID NO. 58 - SEQ ID NO. 121. More specifically, in one embodiment B is the fragment of PilA as set forth in SEQ ID NO. 127. In an additional embodiment, B is an immunogenic fragment at least 95%, 98% or 99% identical to amino acids 40-149 of any of SEQ ID NO. 58 - SEQ ID NO. 121.

In one particular embodiment, B is the fragment of PilA as set forth in SEQ ID NO. 127 and A is an immunogenic fragment of Protein E selected from the group consisting of SEQ ID NO. 122, SEQ ID NO. 124, SEQ ID NO. 125 and SEQ ID NO. 126. More particularly, B is the fragment of PilA as set forth in SEQ ID NO. 127 and A is the fragment of Protein E as set forth in SEQ ID NO. 124, amino acids 19-160 of Protein E from SEQ ID NO. 4. In another embodiment, B is the fragment of PilA as set forth in SEQ ID NO. 127 and A is the fragment of Protein E as set forth in SEQ ID NO. 125.

In another embodiment, B is an immunogenic fragment of PilA wherein PilA is at least 95% identical to any of SEQ ID NO. 58 - SEQ ID NO. 121 and A is an immunogenic fragment of PE wherein PE is at least 95% identical to any of SEQ ID NO. 4 - SEQ ID NO. 57.

In another embodiment, the fusion proteins of formula (I) are defined wherein B is Protein E from *H. influenzae* when A is PilA from *H. influenzae.* For example, B is Protein E having an amino acid sequence selected from the group consisting of SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 18, SEQ ID NO. 19, SEQ ID NO. 20, SEQ ID NO. 21, SEQ ID NO. 22, SEQ ID NO. 23, SEQ ID NO. 24, SEQ ID NO. 25, SEQ ID NO. 26, SEQ ID NO. 27, SEQ ID NO. 28, SEQ ID NO. 29, SEQ ID NO. 30, SEQ ID NO. 31, SEQ ID NO. 32, SEQ ID NO. 33, SEQ ID NO. 34, SEQ ID NO. 35, SEQ ID NO. 36, SEQ ID NO. 37, SEQ ID NO. 38, SEQ ID NO.39, SEQ ID NO. 40, SEQ ID NO. 41, SEQ ID NO. 42, SEQ ID NO. 43 SEQ ID NO. 44, SEQ ID NO. 45, SEQ ID NO. 46, SEQ ID NO. 47, SEQ ID NO. 48, SEQ ID NO. 49, SEQ ID NO. 50, SEQ ID NO. 51, SEQ ID NO. 52, SEQ ID NO. 53, SEQ ID NO. 54, SEQ ID NO. 55, SEQ ID NO. 56 and SEQ ID NO. 57; or any subset of SEQ ID NO. 4 through SEQ ID NO. 57. In another embodiment, the fusion proteins of formula (I) are defined wherein B is Protein E wherein Protein E is approximately at least 75%, 80%, 85%, 90%, 95%, 98% or 99% identical to the Protein E amino acid sequence set forth in SEQ ID NO: 4. In another embodiment, B is Protein E wherein Protein E is approximately 90%, 95%, 98%, or 99% identical to the Protein E amino acid sequence set forth in SEQ ID NO: 4. For example, B is Protein E wherein Protein E is at least 95% identical to Protein E as set forth in SEQ ID NO. 4. In another embodiment, B is Protein E wherein Protein E is at least 95% identical to any of SEQ ID NO. 4 - SEQ ID NO. 57. In a particular embodiment, B is Protein E having the amino acid sequence set forth in SEQ ID NO. 4.

In another embodiment, the fusion proteins of formula (I) are defined wherein B is an immunogenic fragment of Protein E from *H. influenzae* when A is an immunogenic fragment of PilA from *H. influenzae.* For example, B is an immunogenic fragment of Protein E wherein Protein E has an amino acid sequence selected from the group consisting of SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17, SEQ ID NO. 18, SEQ ID NO. 19, SEQ ID NO. 20, SEQ ID NO. 21, SEQ ID NO. 22, SEQ ID NO. 23, SEQ ID NO. 24, SEQ ID NO. 25, SEQ ID NO. 26, SEQ ID NO. 27, SEQ ID NO. 28, SEQ ID NO. 29, SEQ ID NO. 30, SEQ ID NO. 31, SEQ ID NO. 32, SEQ ID NO. 33, SEQ ID NO. 34, SEQ ID NO. 35, SEQ ID NO. 36, SEQ ID NO. 37, SEQ ID NO. 38, SEQ ID NO.39, SEQ ID NO. 40, SEQ ID NO. 41, SEQ ID NO. 42, SEQ ID NO. 43, SEQ ID NO. 44, SEQ ID NO. 45, SEQ ID NO. 46, SEQ ID NO. 47, SEQ ID NO. 48, SEQ ID NO. 49, SEQ ID NO. 50, SEQ ID NO. 51, SEQ ID NO. 52, SEQ ID NO. 53, SEQ ID NO. 54, SEQ ID NO. 55, SEQ ID NO. 56 and SEQ ID NO. 57; or any subset of SEQ ID NO. 4 through SEQ ID NO. 57. In another embodiment, the fusion proteins of formula (I) are defined wherein B is an immunogenic fragment of Protein E wherein Protein E is approximately at least 75%, 80%, 85%, 90%, 95%, 98% or 99% identical to the Protein E amino acid sequence set forth in SEQ ID NO. 4. In another embodiment, B is an immunogenic fragment of Protein E wherein Protein E is approximately 90% to 100% identical to the Protein E amino acid sequence set forth in SEQ ID NO: 4. In a particular embodiment, B is an immunogenic fragment of Protein E having the amino acid sequence set forth in SEQ ID NO. 4. In an additional embodiment, B is an immunogenic fragment of Protein E, wherein Protein E is at least 95% identical to any of SEQ ID NO. 4 - SEQ ID NO. 57.

In another embodiment, B is a fragment of Protein E from *H. influenzae* selected from the group consisting of amino acids 17-160 of SEQ ID NO. 4 (SEQ ID NO. 122), amino acids 18-160 of SEQ ID NO. 4 (SEQ ID NO. 123), amino acids 19-160 of SEQ ID NO. 4 (SEQ ID NO. 124), amino acids 20-160 of SEQ ID NO. 4 (SEQ ID NO. 125) and amino acids 22-160 of SEQ ID NO. 4 (SEQ ID NO. 126). In another embodiment, B is an immunogenic fragment of Protein E from *H. influenzae* selected from the group consisting of amino acids 17-160 of SEQ ID NO. 4 (SEQ ID NO. 122), amino acids 18-160 of SEQ ID NO. 4 (SEQ ID NO. 123), amino acids 19-160 of SEQ ID NO. 4 (SEQ ID NO. 124), amino acids 20-160 of SEQ ID NO. 4 (SEQ ID NO. 125), amino acids 22-160 of SEQ ID NO. 4 (SEQ ID NO. 126), amino acids 23-160 of SEQ ID NO. 4 (SEQ ID NO. 179) and amino acids 24-160 of SEQ ID NO. 4 (SEQ ID NO. 180). More specifically, in one embodiment, B is the fragment of Protein E as set forth in SEQ ID NO. 123, amino acids 18-160 of SEQ ID NO. 4.

In one particular embodiment B is an immunogenic fragment of Protein E as set forth in SEQ ID NO. 123, amino acids 18-160 of SEQ ID NO. 4, when A is an immunogenic fragment of PilA as set forth in SEQ ID NO. 127.

In one embodiment, the fusion proteins of formula (I) are defined wherein p is 0. In another embodiment, the fusion proteins of formula (I) are defined wherein p is 1.

In one embodiment, the fusion protein of formula (I) is selected from the group consisting of SEQ ID NO. 136, SEQ ID NO. 138, SEQ ID NO. 140, SEQ ID NO. 142, SEQ ID NO. 144, SEQ ID NO. 146, SEQ ID NO. 148, SEQ ID NO. 150, SEQ ID NO. 182, SEQ ID NO. 184, SEQ ID NO. 186, SEQ ID NO. 188, SEQ ID NO. 190, SEQ ID NO. 192, SEQ ID NO. 194, SEQ ID NO. 196, SEQ ID NO. 198, SEQ ID NO. 200, SEQ ID NO. 202 and SEQ ID NO. 204; or any subset thereof. In another embodiment, the fusion protein of formula (I) is approximately 85%, 88%, 90%, 92%, 95% or 98% identical to any of SEQ ID NO. 136, SEQ ID NO. 138, SEQ ID NO. 140, SEQ ID NO. 142, SEQ ID NO. 144, SEQ ID NO. 146, SEQ ID NO. 148, SEQ ID NO. 150, SEQ ID NO. 182, SEQ ID NO. 184, SEQ ID NO. 186, SEQ ID NO. 188, SEQ ID NO. 190, SEQ ID NO. 192, SEQ ID NO. 194, SEQ ID NO. 196, SEQ ID NO. 198, SEQ ID NO. 200, SEQ ID NO. 202 or SEQ ID NO. 204.

In one embodiment the fusion protein formula (I) is the fusion protein of SEQ ID NO.148 wherein the signal peptide has been removed SEQ ID NO. 177 (QIQKAEQN DVKLAPPTDV RSGYIRLVKN VNYYIDSESI WVDNQEPQIV HFDAVVNLDK GLYVYPEPKR YARSVRQYKI LNCANYHLTQ VRTDFYDEFW GQGLRAAPKK QKKHTLSLTP DTTLYNAAQI ICANYGEAFS VDKKGGTKKA AVSELLQASA PYKADVELCV YSTNETTNCT GGKNGIAADI TTAKGYVKSV TTSNGAITVK GDGTLANMEY ILQATGNAAT GVTWTTTCKG TDASLFPANF CGSVTQ).

In one embodiment the fusion protein of formula (I) is the fusion protein of SEQ ID NO.194 wherein the signal peptide has been removed SEQ ID NO. 219 (IQKAEQND VKLAPPTDVR SGYIRLVKNV NYYIDSESIW VDNQEPQIVH FDAVVNLDKG LYVYPEPKRY ARSVRQYKIL NCANYHLTQV RTDFYDEFWG QGLRAAPKKQ KKHTLSLTPD TTLYNAAQII CANYGEAFSV DKKGGTKKAA VSELLQASAP YKADVELCVY STNETTNCTG GKNGIAADIT TAKGYVKSVT TSNGAITVKG DGTLANMEYI LQATGNAATG VTWTTTCKGT DASLFPANFC GSVTQ).

### Streptococcus pneumoniae capsular saccharide conjugates

The term capsular saccharide includes capsular polysaccharides and oligosaccharides derivable from the capsular polysaccharide. An oligosaccharide contains at least 4 sugar residues. The terms conjugate and conjugated relate to a capsular saccharide covalently bonded to a carrier protein.

In an immunogenic composition, the total number of saccharide serotypes is optionally less than or equal to 23. In one embodiment the immunogenic composition comprises less than 23, 22, 21, 20, 19, 18, 17, 16, 15, 14, or 13 *Streptococcus pneumoniae* saccharides, optionally the immunogenic composition comprising 10-23 serotypes, 10-16 serotypes, 10-15 serotypes, 10-14 serotypes, 10-13 serotypes or 10-12 serotypes.

In one embodiment the *Streptococcus pneumoniae* capsular saccharide conjugates will be derived from the following serotypes 1, 2, 3, 4, 5, 6A, 6B, 7F, 8, 9N, 9V, 10A, 11A, 12F, 14, 15B, 17F, 18C, 19A, 19F, 20, 22F, 23F and 33F, although it is appreciated that one or two other serotypes could be substituted depending on the age of the recipient receiving the vaccine and the geographical location where the immunogenic composition will be administered. For example, a 7 valent immunogenic composition may comprise saccharides from serotypes 4, 6B, 9V, 14, 18C, 19F and 23F. A 10 valent immunogenic composition may further comprise saccharides derived from serotypes 1, 5 and 7F. A 12 valent immunogenic composition may further comprise saccharides derived from serotypes 6A, 19A. A 15 valent immunogenic composition may further comprise saccharides derived from serotypes 22F and 33F.

Further saccharide antigens, for example 23 valent (such as serotypes 1, 2, 3, 4, 5, 6B, 7F, 8, 9N, 9V, 10A, 11A, 12F, 14, 15B, 17F, 18C, 19A, 19F, 20, 22F, 23F and 33F), are also contemplated by the invention.

The term "carrier protein" is intended to cover both small peptides and large polypeptides (>10 kDa). The carrier protein may be any peptide or protein. It may comprise one or more T-helper epitopes. The carrier protein may be tetanus toxoid (TT), tetanus toxoid fragment C, non-toxic mutants of tetanus toxin [note all such variants of TT are considered to be the same type of carrier protein for the purposes of this invention], polypeptides comprising tetanus toxin T-cell epitopes such as N19 (WO2006/067632), diphtheria toxoid (DT), CRM 197 (cross reacting material 197), other non-toxic mutants of diphtheria toxin [such as CRM176, CRM 197, CRM228, CRM 45 (Uchida et al J. Biol. Chem. 218; 3838-3844, 1973); CRM 9, CRM 45, CRM102, CRM 103 and CRM107 (where CRM stands for cross reacting material) and other mutations described by Nicholls and Youle in Genetically Engineered Toxins, Ed: Frankel, Maecel Dekker Inc, 1992; deletion or mutation of Glu-148 to Asp, Gin or Ser and/or Ala 158 to Gly and other mutations disclosed in US 4709017 or US 4950740; mutation of at least one or more residues Lys 516, Lys 526, Phe 530 and/or Lys 534 and other mutations disclosed in US 5917017 or US 6455673; or fragment disclosed in US 5843711] (note all such variants of DT are considered to be the same type of carrier protein for the purposes of this invention), pneumococcal pneumolysin (Kuo et al (1995) Infect Immun 63; 2706-13), OMPC (outer membrane protein C from meningococcus usually extracted from N. meningitidis serogroup B - EP0372501), synthetic peptides (EP0378881, EP0427347), heat shock proteins (WO 93/17712, WO 94/03208), pertussis proteins (WO 98/58668, EP0471177), cytokines, lymphokines, growth factors or hormones (WO 91/01146), artificial proteins comprising multiple human CD4+ T cell epitopes from various pathogen derived antigens (Falugi et al (2001) Eur J Immunol 31; 3816-3824) such as N19 protein (Baraldoi et al (2004) Infect Immun 72; 4884-7), pneumococcal surface protein PspA (WO 02/091998), iron uptake proteins (WO 01/72337), toxin A or toxin B of Clostridium difficile (WO 00/61761), *H. influenzae* Protein D (EP594610 and WO 00/56360), pneumococcal PhtA (WO 98/18930, also referred to Sp36), pneumococcal PhtD (poly histidine triad D disclosed in WO 00/37105, and is also referred to Sp036D), pneumococcal PhtB (poly histidine triad B disclosed in WO 00/37105, and is also referred to Sp036B), or PhtE (poly histidine triad E disclosed in WO00/30299 and is referred to as BVH-3).

In one embodiment the *Streptococcus pneumoniae* capsular saccharide conjugates are conjugated to a carrier protein independently selected from the group consisting of tetanus toxoid (TT), fragment C of TT, diphtheria toxoid, CRM 197 (cross reacting material 197), detoxified pneumolysin, protein D (from *H.influenzae*), PhtD, PhtDE (a protein containing poly histidine triad protein D and poly histidine triad protein E) and N19. In a further embodiment the *Streptococcus pneumoniae* capsular saccharide conjugates are all independently conjugated to CRM197.

The term 'conjugated to' in this context means that the protein is covalently bonded to a saccharide; in this situation the protein is acting as a carrier protein.

In one embodiment the immunogenic composition comprises at least one *Streptococcus pneumoniae* capsular saccharide conjugated to protein D. In one embodiment a minority of conjugated *Streptococcus pneumoniae* saccharides are conjugated to protein D wherein the term 'minority' refers to less than half of the total number of saccharides in the composition being conjugated to protein D. In a further embodiment the immunogenic composition comprises between 1-20, 1-18, 1-16, 1-14, 1-12, 1-10, 1-9, 1-8, 1-7, 1-6, 1-5, 1-4, 1-4 or 1-2 *Streptococcus pneumoniae* capsular saccharide conjugated to protein D. In one embodiment the immunogenic composition comprises at least one *Streptococcus pneumoniae* capsular saccharides conjugated to diphtheria toxoid. In a further embodiment the immunogenic composition comprises 19F is conjugated to diphtheria toxoid. In one embodiment the immunogenic composition comprises at least one *Streptococcus pneumoniae* capsular saccharides conjugated to tetanus toxoid. In a further embodiment the immunogenic composition comprises 18C is conjugated to tetanus toxoid.

In one embodiment the immunogenic composition comprises a conjugated serotype 1 saccharide conjugated to protein D or CRM197. In one embodiment the immunogenic composition comprises a conjugated serotype 4 saccharide conjugated to protein D or CRM197. In one embodiment the immunogenic composition comprises a conjugated serotype 5 saccharide wherein the serotype 5 saccharide is conjugated to protein D or CRM197. In one embodiment the immunogenic composition comprises a conjugated serotype 6B saccharide wherein the serotype 6B saccharide is conjugated to protein D or CRM197. In one embodiment the immunogenic composition comprises a conjugated serotype 7F saccharide is wherein the serotype 7F saccharide is conjugated to protein D or CRM197. In one embodiment the immunogenic composition comprises a conjugated serotype 9V saccharide wherein the 9V saccharide is conjugated to protein D or CRM197. In one embodiment the immunogenic composition comprises a conjugated serotype 14 saccharide wherein the serotype 14 saccharide is conjugated to protein D or CRM197. In one embodiment the immunogenic composition comprises a conjugated serotype 18C saccharide wherein the serotype 18C saccharide is conjugated to tetanus toxoid or CRM 197. In one embodiment the immunogenic composition comprises a conjugated 19F saccharide wherein the serotype 19F saccharide is conjugated to diphtheria toxoid or CRM197. In one embodiment the immunogenic composition comprises a conjugated 23F saccharide wherein the serotype 23F saccharide is conjugated to protein D or CRM197. In one embodiment the immunogenic composition comprises a conjugated 6A saccharide conjugated to CRM197. In one embodiment the immunogenic composition comprises a conjugated 19A saccharide conjugated to CRM197.

In one embodiment the immunogenic composition comprises a *Streptococcus pneumoniae* serotype 1 saccharide conjugated to protein D, a *Streptococcus pneumoniae* serotype 4 saccharide conjugated to protein D, a *Streptococcus pneumoniae* serotype 5 saccharide conjugated to protein D, a *Streptococcus pneumoniae* serotype 6B saccharide conjugated to protein D, a *Streptococcus pneumoniae* serotype 7F saccharide conjugated to protein D, a *Streptococcus pneumoniae* serotype 9V saccharide conjugated to protein D, a *Streptococcus pneumoniae* serotype 14 saccharide conjugated to protein D, a *Streptococcus pneumoniae* serotype 23F saccharide conjugated to protein D, a *Streptococcus pneumoniae* serotype 18C saccharide conjugated to tetanus toxoid and a *Streptococcus pneumoniae* 19F saccharide conjugated to diphtheria toxoid. In one embodiment the immunogenic composition further comprises a *Streptococcus pneumoniae* serotype 6A conjugated to CRM197 and a *Streptococcus pneumoniae* serotype 19A conjugated to CRM197.

Optionally the ratio of carrier protein to S. *pneumoniae* saccharide is between 1:5 and 5:1; 1:2 and 2.5:1; 1:1 and 2:1 (w/w). In an embodiment, the majority of the conjugates, for example 6, 7, 8, 9 or more of the conjugates have a ratio of carrier protein to saccharide that is greater than 1:1, for example 1.1:1, 1.2:1, 1.3:1, 1.4:1, 1.5:1 or 1.6:1.

In general, the immunogenic composition of the invention may comprise a dose of each saccharide conjugate between 0.1 and 20µg, 1 and 5µg, 1 and 10µg or 1 and 3µg of saccharide.

In an embodiment, the immunogenic composition of the invention contains each S. *pneumoniae* capsular saccharide conjugate at a dose of between 0.1-20µg; 0.5-10µg; 0.5- 5µg or 1-3µg of saccharide. In an embodiment, capsular saccharides may be present at different dosages, for example some capsular saccharides may be present at a dose of exactly 1µg or some capsular saccharides may be present at a dose of exactly 3µg. In an embodiment, saccharides from serotypes 3, 18C and 19F (or 4, 18C and 19F) are present at a higher dose than other saccharides. In one aspect of this embodiment, serotypes 3, 18C and 19F (or 4, 18C and 19F) are present at a dose of around or exactly 3 µg whilst other saccharides in the immunogenic composition are present at a dose of around or exactly 1µg. In one embodiment serotypes 1, 5, 6B, 7F, 9V, 14 and 23F are present at a dose of around or exactly 1µg.

The term "saccharide" throughout this specification may indicate polysaccharide or oligosaccharide and includes both. Polysaccharides are isolated from bacteria and may be sized to some degree by known methods (see for example EP497524 and EP497525) and optionally by microfluidisation. Polysaccharides can be sized in order to reduce viscosity in polysaccharide samples and/or to improve filterability for conjugated products. Oligosaccharides have a low number of repeat units (typically 5-30 repeat units) and are typically hydrolysed polysaccharides.

Capsular polysaccharides of *Streptococcus pneumoniae* comprise repeating oligosaccharide units which may contain up to 8 sugar residues. For a review of the oligosaccharide units for the key *Streptococcus pneumoniae* serotypes see JONES, Christopher. Vaccines based on the cell surface carbohydrates of pathogenic bacteria. An. Acad. Bras. Ciênc., June 2005, vol.77, no.2, p.293-324. ISSN 0001-3765. In one embodiment, a capsular saccharide antigen may be a full length polysaccharide, however in others it may be one oligosaccharide unit, or a shorter than native length saccharide chain of repeating oligosaccharide units. In one embodiment, all of the saccharides present in the vaccine are polysaccharides. Full length polysaccharides may be "sized" i.e. their size may be reduced by various methods such as acid hydrolysis treatment, hydrogen peroxide treatment, sizing by emulsiflex® followed by a hydrogen peroxide treatment to generate oligosaccharide fragments or microfluidization.

In one embodiment the immunogenic composition further comprises unconjugated *S.penumoniae* saccharides of serotypes different from those conjugated, such that the number of conjugated and unconjugated saccharide serotypes is less than or equal to 23.

### Conjugation

The saccharide conjugates present in the immunogenic compositions of the invention may be conjugated to a carrier protein using any conjugation technique.

In an embodiment, the *Streptococcus pneumoniae* saccharide is conjugated to the carrier protein via a linker, for instance a bifunctional linker. The linker is optionally heterobifunctional or homobifunctional, having for example a reactive amino group and a reactive carboxylic acid group, two reactive amino groups or two reactive carboxylic acid groups. The linker has for example between 4 and 20, 4 and 12, 5 and 10 carbon atoms. A possible linker is adipic acid dihydrazide (ADH). Other linkers include B-propionamido (WO 00/10599), nitrophenyl-ethylamine (Gever et al (1979) Med. Microbiol. Immunol. 165; 171-288), haloalkyl halides (US4057685), glycosidic linkages (US4673574, US4808700), hexane diamine and 6-aminocaproic acid (US4459286). In an embodiment, ADH is used as a linker for conjugating saccharide from serotype 18C.

The saccharide conjugates present in the immunogenic compositions of the invention may be prepared by any known coupling technique. The conjugation method may rely on activation of the saccharide with 1-cyano-4-dimethylamino pyridinium tetrafluoroborate (CDAP) to form a cyanate ester. The activated saccharide may thus be coupled directly or via a spacer (linker) group to an amino group on the carrier protein. For example, the spacer could be cystamine or cysteamine to give a thiolated polysaccharide which could be coupled to the carrier via a thioether linkage obtained after reaction with a maleimide-activated carrier protein (for example using GMBS) or a haloacetylated carrier protein (for example using iodoacetimide [e.g. ethyl iodoacetimide HCl] or N-succinimidyl bromoacetate or SIAB, or SIA, or SBAP). Optionally, the cyanate ester (optionally made by CDAP chemistry) is coupled with hexane diamine or ADH and the amino-derivatised saccharide is conjugated to the carrier protein using carbodiimide (e.g. EDAC or EDC) chemistry via a carboxyl group on the protein carrier. Such conjugates are described in PCT published application WO 93/15760 Uniformed Services University and WO 95/08348 and WO 96/29094.

Other suitable techniques use carbodiimides, carbiinides, hydrazides, active esters, norborane, p-nitrobenzoic acid, N-hydroxysuccinimide, S-NHS, EDC, TSTU. Many are described in WO 98/42721. Conjugation may involve a carbonyl linker which may be formed by reaction of a free hydroxyl group of the saccharide with CDI (Bethell et al J. Biol. Chem. 1979, 254; 2572-4, Hearn et al J. Chromatogr. 1981. 218; 509-18) followed by reaction with a protein to form a carbamate linkage. This may involve reduction of the anomeric terminus to a primary hydroxyl group, optional protection/deprotection of the primary hydroxyl group' reaction of the primary hydroxyl group with CDI to form a CDI carbamate intermediate and coupling the CDI carbamate intermediate with an amino group on a protein.

The conjugates can also be prepared by direct reductive amination methods as described in US 4365170 (Jennings) and US 4673574 (Anderson). Other methods are described in EP-0-161-188, EP-208375 and EP-0-477508.

A further method involves the coupling of a cyanogen bromide (or CDAP) activated saccharide derivatised with adipic acid dihydrazide (ADH) to the protein carrier by Carbodiimide condensation (Chu C. et al Infect. Immunity, 1983 245 256), for example using EDAC (1-Ethyl-3-(3-dimethylaminopropyl) carbodiimide hydrochloride).

In an embodiment, a hydroxyl group (optionally an activated hydroxyl group for example a hydroxyl group activated to make a cyanate ester [e.g. using CDAP]) on a saccharide is linked to an amino or carboxylic group on a protein either directly or indirectly (through a linker). Where a linker is present, a hydroxyl group on a saccharide is optionally linked to an amino group on a linker, for example by using CDAP conjugation. A further amino group in the linker for example ADH may be conjugated to a carboxylic acid group on a protein, for example by using carbodiimide chemistry, for example by using EDAC. In an embodiment, the pneumococcal capsular saccharide(s) is conjugated to the linker first before the linker is conjugated to the carrier protein. Alternatively the linker may be conjugated to the carrier before conjugation to the saccharide.

A combination of techniques may also be used, with some saccharide-protein conjugates being prepared by CDAP, and some by reductive amination.

In general the following types of chemical groups on a protein carrier can be used for coupling / conjugation:
A) Carboxyl (for instance via aspartic acid or glutamic acid). In one embodiment this group is linked to amino groups on saccharides directly or to an amino group on a linker with carbodiimide chemistry e.g. with EDAC.
B) Amino group (for instance via lysine). In one embodiment this group is linked to carboxyl groups on saccharides directly or to a carboxyl group on a linker with carbodiimide chemistry e.g. with EDAC. In another embodiment this group is linked to hydroxyl groups activated with CDAP or CNBr on saccharides directly or to such groups on a linker; to saccharides or linkers having an aldehyde group; to saccharides or linkers having a succinimide ester group.
C) Sulphydryl (for instance via cysteine). In one embodiment this group is linked to a bromo or chloro acetylated saccharide or linker with maleimide chemistry. In one embodiment this group is activated/modified with bis diazobenzidine.
D) Hydroxyl group (for instance via tyrosine). In one embodiment this group is activated/modified with bis diazobenzidine.
E) Imidazolyl group (for instance via histidine). In one embodiment this group is activated/modified with bis diazobenzidine.
F) Guanidyl group (for instance via arginine).
G) Indolyl group (for instance via tryptophan).

On a saccharide, in general the following groups can be used for a coupling: OH, COOH or NH2. Aldehyde groups can be generated after different treatments known in the art such as: periodate, acid hydrolysis, hydrogen peroxide, etc.

Direct coupling approaches:

Saccharide-OH + CNBr or CDAP -----> cyanate ester + NH2-Prot ----> conjugate

Saccharide-aldehyde + NH2-Prot ----> Schiff base + NaCNBH3 ----> conjugate

Saccharide-COOH + NH2-Prot + EDAC ----> conjugate

Saccharide-NH2 + COOH-Prot + EDAC ----> conjugate

Indirect coupling via spacer (linker) approaches:

Saccharide-OH + CNBr or CDAP ---> cyanate ester + NH2----NH2 ----> saccharide----NH2 + COOH-Prot + EDAC -----> conjugate

Saccharide-OH + CNBr or CDAP ----> cyanate ester + NH2-----SH -----> saccharide----SH + SH-Prot (native Protein with an exposed cysteine or obtained after modification of amino groups of the protein by SPDP for instance) -----> saccharide-S-S-Prot

Saccharide-OH + CNBr or CDAP ---> cyanate ester + NH2----SH -------> saccharide----SH + maleimide-Prot (modification of amino groups) ----> conjugate

Saccharide-OH + CNBr or CDAP ---> cyanate ester + NH2-----SH ---> Saccharide-SH + haloacetylated-Prot ----> Conjugate

Saccharide-COOH + EDAC + NH2-----NH2 ---> saccharide------NH2 + EDAC + COOH-Prot ---- > conjugate

Saccharide-COOH + EDAC+ NH2----SH -----> saccharide----SH + SH-Prot (native Protein with an exposed cysteine or obtained after modification of amino groups of the protein by SPDP for instance) -----> saccharide-S-S-Prot

Saccharide-COOH + EDAC+ NH2----SH -----> saccharide----SH + maleimide-Prot (modification of amino groups) ----> conjugate

Saccharide-COOH + EDAC + NH2----SH ---> Saccharide-SH + haloacetylated-Prot ----> Conjugate

Saccharide-Aldehyde + NH2-----NH2 ----> saccharide---NH2 + EDAC + COOH-Prot ----> conjugate

Note: instead of EDAC above, any suitable carbodiimide may be used.

In summary, the types of protein carrier chemical group that may be generally used for coupling with a saccharide are amino groups (for instance on lysine residues), COOH groups (for instance on aspartic and glutamic acid residues) and SH groups (if accessible) (for instance on cysteine residues.

In one embodiment at least 1, 2, 3, 4, 5, 6, 7, 8, 9, 10, 11, 12, 13, 14, 15, 16, 17, 18, 19, 20, 21, 22, or 23 *S.pneumoniae* saccharides are conjugated to a carrier protein through reductive amination. In one embodiment less than 23, 22, 21, 20, 19, 18, 17, 16, 15, 14, 13, 12, 11, 10, 9, 8, 7, 6, 5, 4, 3, 2 or 1 *S.pneumoniae* saccharides are conjugated to a carrier protein through reductive amination. In one embodiment between 1 and 23, 2 and 22, 3 and 21, 4 and 20, 5 and 19, 6 and 18, 7 and 17, 8 and 16, 9 and 15, 10 and 14, 11 and 13, 1 and 23, and 22, 1 and 21, 1 and 20, 1 and 19, 1 and 18, 1 and 17, 1 and 16, 1 and 15, 1 and 14, 1 and 13, 1 and 12, 1 and 11, 1 and 10, 1 and 9, 1 and 8, 1 and 7, 1 and 6, 1 and 5, 1 and 4, 1 and 3, or 1 or 2 *S.pneumoniae* saccharides are conjugated to a carrier protein through reductive amination. In a further embodiment all of the *S.pneumoniae* capsular saccharides are conjugated to a carrier protein through reductive amination.

In one embodiment at least 1, 2, 3, 4, 5, 6, 7, 8, 9, 10, 11, 12, 13, 14, 15, 16, 17, 18, 19, 20, 21, 22, or 23 *S.pneumoniae* saccharides are conjugated to a carrier protein through CDAP chemistry. In one embodiment less than 23, 22, 21, 20, 19, 18, 17, 16, 15, 14, 13, 12, 11, 10, 9, 8, 7, 6, 5, 4, 3, 2 or 1 *S.pneumoniae* saccharides are conjugated to a carrier protein through CDAP chemistry. In one embodiment between 1 and 23, 2 and 22, 3 and 21, 4 and 20, 5 and 19, 6 and 18, 7 and 17, 8 and 16, 9 and 15, 10 and 14, 11 and 13, 1 and 23, and 22, 1 and 21, 10 and 23, 10 and 22, 10 and 21, 10 and 20, 10 and 19, 10 and 18, 10 and 17, 10 and 16, 10 and 15, 10 and 14, 10 and 13, 10 and 12, 10 and 11, 1 and 20, 1 and 19, 1 and 18, 1 and 17, 1 and 16, 1 and 15, 1 and 14, 1 and 13, 1 and 12, 1 and 11, 1 and 10, 1 and 9, 1 and 8, 1 and 7, 1 and 6, 1 and 5, 1 and 4, 1 and 3, or 1 or 2 *S.pneumoniae* saccharides are conjugated to a carrier protein through CDAP chemistry. In a further embodiment all of the *S.pneumoniae* capsular saccharides are conjugated to a carrier protein through CDAP chemistry.

In one embodiment the immunogenic composition of the invention comprises at least 1, 2, 3, 4, 5, 6, 7, 8, 9, 10, 11, 12, 13, 14, 15, 16, 17, 18, 19, 20, 21 or 22 saccharides conjugated to a carrier protein through reductive amination and comprises at least 1, 2, 3, 4, 5, 6, 7, 8, 9, 10, 11, 12, 13, 14, 15, 16, 17, 18, 19, 20, 21, or 22 saccharides conjugated to a carrier protein through a chemistry other than reductive amination for example CDAP chemistry.

In an embodiment capsular saccharides from at least one of the serotypes selected from the group consisting of serotypes 1, 3, 19A and 19F are conjugated through a chemistry other than reductive amination and at least one of the serotypes selected from the group consisting of serotypes 4, 5, 6A, 6B, 6C, 7F, 9V, 14, 18C and 23F are conjugated through reductive amination. In an embodiment, the immunogenic composition of the invention comprises S. *pneumoniae* capsular saccharide(s) from serotype 1 or 3 or 19A or 19F; 1 and 3; 1 and 19A; 1 and 19F; 3 and 19A; 3 and 19F; 19A and 19F; 1,3 and 19A; 1, 3 and 19F, 1, 19A and 19F; 3, 19A and 19F or 1, 3, 19A and 19F conjugated to a protein carrier through a chemistry other than reductive animation. In an embodiment, 19F is conjugated to a carrier protein through a chemistry other than reductive amination. In an embodiment, the immunogenic composition of the invention comprises *S*. *pneumoniae* capsular saccharide from serotype 1 or 3 or 19A or 19F; 1 and 3; 1 and 19A; 1 and 19F; 3 and 19A; 3 and 19F; 19A and 19F; 1, 3 and 19A; 1, 3 and 19F, 1, 19A and 19F; 3, 19A and 19F or 1, 3, 19A and 19F conjugated to a protein carrier through cyanylation chemistry such as CDAP chemistry. In an embodiment, 19F is conjugated to a carrier protein by CDAP chemistry. In an embodiment of the invention, the following *S*. *pneumoniae* capsular saccharide or saccharides is/are conjugated to a carrier protein by reductive amination; serotype 4, 5, 6A, 6B, 7F, 9V, 14, 18C or 23F, 4 and 5, 4 and 6A, 4 and 6B, 4 and 7F, 4 and 9V, 4 and 14, 4 and 18C, 4 and 23F, 5 and 6A, 5 and 6B, 5 and 7F, 5 and 9V, 5 and 14, 5 and 18C, 5 and 23F, 6A and 6B, 6A and 7F, 6A and 9V, 6A and 14, 6A and 18C, 6A and 23F, 6B and 7F, 6B and 9V, 6B and 14, 6B and 18C, 6B and 23F, 7F and 9V, 7F and 14, 7F and 18C, 7F and 23F, 9V and 14, 9V and 18C, 9V and 23F, 14 and 18C, 14 and 23F or 18C and 23F. In an embodiment, serotype 23F is conjugated to a carrier protein by reductive amination chemistry.

### Unconjugated or conjugated Streptococcus pneumoniae protein

The immunogenic compositions of the invention may comprise at least one unconjugated or conjugated *S.pneumoniae* protein. In one embodiment the at least one unconjugated or conjugated *S.pneumoniae* protein is selected from the group consisting of Poly Histidine Triad family (PhtX), detoxified pneumolysin (dPly), Choline Binding Protein Family (CbpX), CbpX truncates, LytX (autolytic enzymes) family, LytX truncates, CbpX truncate-LytX truncate chimeric proteins, PcpA (Pneumococcal Choline Binding protein A), PspA (Pneumococcal Surface Protein A), PsaA (Pneumococcal Surface Adhesin Protein A), Sp128, Sp101 *(Streptococcus pneumoniae* 101), Sp130 *(Streptococcus pneumoniae* 130), SP125 *(Streptococcus pneumoniae* 125) and SP133 *(Streptococcus pneumoniae* 133).

The Pht (Poly Histidine Triad) family comprises proteins PhtA, PhtB, PhtD, and PhtE. Note that the Pht family can be referred to as either the Poly Histidine Triad family or the Pneumococcal Histidine Triad family, thus the terms 'Poly Histidine Triad' and 'Pneumococcal Histidine Triad' can be considered to be interchangeable. The family is characterized by a lipidation sequence, two domains separated by a proline-rich region and several histidine triads, possibly involved in metal or nucleoside binding or enzymatic activity, (3-5) coiled-coil regions, a conserved N-terminus and a heterogeneous C terminus. It is present in all strains of pneumococci tested. Homologous proteins have also been found in other Streptococci and Neisseria. In one embodiment of the invention, the Pht protein of the invention is PhtD. It is understood, however, that the terms PhtA, PhtB, PhtD and PhtE refer to proteins having sequences disclosed in the citations below as well as naturally-occurring (and man-made) variants thereof that have a sequence homology that is at least 90% identical to the referenced proteins. Optionally it is at least 95% identical or at least 97% identical.

With regards to the PhtX proteins, PhtA is disclosed in WO 98/18930, and is also referred to Sp36. As noted above, it is a protein from the Pht family and has the type II signal motif of LXXC. PhtD is disclosed in WO 00/37105, and is also referred to Sp036D. As noted above, it also is a protein from the Pht family and has the type II LXXC signal motif. In one embodiment the term 'PhtD' refers to SEQ ID NO:220. PhtB is disclosed in WO 00/37105, and is also referred to Sp036B. Another member of the PhtB family is the C3-Degrading Polypeptide, as disclosed in WO 00/17370. This protein also is from the Pht family and has the type II LXXC signal motif. For example, an immunologically functional equivalent is the protein Sp42 disclosed in WO 98/18930. A PhtB truncate (approximately 79kD) is disclosed in WO99/15675 which is also considered a member of the Pht family. PhtE is disclosed in WO00/39299 and is referred to as BVH-3. Where any Pht protein is referred to herein, it is meant that immunogenic fragments or fusions thereof of the Pht protein can be used. For example, a reference to PhtX includes immunogenic fragments or fusions thereof from any Pht protein. A reference to PhtD or PhtB does not exclude PhtDE (a fusion protein comprising PhtD and PhtE) or PhtBE (a fusion protein comprising PhtB and PhtE), respectively, as found, for example, in WO0198334.

In one embodiment the at least one unconjugated to conjugated *Streptococccus pneumoniae* protein comprises at least one protein from the Poly histidine Triad family (for example, the protein may be selected from the group consisting of PhtD , PhtBD and PhtDE fusion protein). In a further embodiment the at least one unconjugated or conjugated *Streptococcus pneumoniae* protein is a PhtD protein. In a further embodiment the PhtD protein comprises an amino acid sequence at least 85%, 90%, 95%, 98%, 99% or 100% identical to the sequence at amino acids 21-838 of Sequence ID No.4 of WO00/37105.

In one embodiment the at least one unconjugated or conjugated *Streptococcus pneumoniae* protein is detoxified pneumolysin (dPly). In one embodiment the pneumolysin has been chemically detoxified. In a further embodiment the pneumolysin has been chemically detoxified. In a yet further embodiment the pneumolysin has been both chemically and genetically detoxified.

In a further embodiment the immunogenic composition of the invention comprises detoxified pneumolysin (dPly) and PhtD. In a further embodiment the immunogenic composition of the invention comprises unconjugated detoxified pneumolysin (dPly) and unconjugated PhtD.

Concerning the Choline Binding Protein family (CbpX), members of that family were originally identified as pneumococcal proteins that could be purified by choline-affinity chromatography. The choline binding proteins are non-covalently bound to phosphorylcholine moieties of cell wall teichoic acid and membrane-associated lipoteichoic acid. Structurally, they have several regions in common over the entire family, although the exact nature of the proteins (amino acid sequence, length, etc.) can vary. In general, choline binding proteins comprise an N terminal region (N), conserved repeat regions (R1 and/or R2), a proline rich region (P) and a conserved choline binding region (C), made up of multiple repeats, that comprises approximately one half of the protein. As used in this application, the term "Choline Binding Protein family (CbpX)" includes proteins from the group consisting of Choline Binding Proteins as identified in WO97/41151, PbcA, SpsA, PspC, CbpA, CbpD and CbpG. CbpA is disclosed in WO97/41151. CbpD and CbpG are disclosed in WO00/29434. PspC is disclosed in WO97/09994. PbcA is disclosed in WO98/21337.SpsA is a choline binding protein disclosed in WO 98/39450. Optionally the Choline Binding Proteins are selected from the group consisting of CbpA, PbcA, SpsA and PspC.

An embodiment of the invention comprises CbpX truncates wherein "CbpX" is defined above and "truncates" refers to CbpX proteins lacking 50% or more of the Choline binding region (C). Optionally such proteins lack the entire choline binding region. Optionally, the protein truncates lack (i) the choline binding region and (ii) a portion of the N-terminal half of the protein as well, yet retain at least one repeat region (R1 or R2). Optionally, the truncate retains 2 repeat regions (R1 and R2). Examples of such embodiments are NR1xR2 and R1xR2 as illustrated in WO99/51266 or WO99/51188, however, other choline binding proteins lacking a similar choline binding region are also contemplated within the scope of this invention.

The LytX family is membrane associated proteins associated with cell lysis. The N-terminal domain comprises choline binding domain(s). However the LytX family does not have all the features found in the CbpX family noted above. For the present invention, the LytX family is considered distinct from the CbpX family. In contrast with the CbpX family, the LytX family C-terminal domain contains the catalytic domain of the LytX protein. The family comprises LytA, LytB and LytC. With regards to the LytX family, LytA is disclosed in Ronda et al., Eur J Biochem, 164:621-624 (1987). LytB is disclosed in WO 98/18930, and is also referred to as Sp46. LytC is also disclosed in WO 98/18930, and is also referred to as Sp91. An embodiment of the invention comprises LytC.

Another embodiment comprises LytX truncates wherein "LytX" is defined above and "truncates" refers to LytX proteins lacking 50% or more of the Choline binding region. Optionally such proteins lack the entire choline binding region. Yet another embodiment of this invention comprises CbpX truncate-LytX truncate chimeric proteins or fusionproteins. Optionally the fusion protein comprises NR1xR2 (or R1xR2) of CbpX and the C-terminal portion (Cterm, i.e., the protein without the choline binding domains) of LytX (e.g., LytCCterm or Sp91Cterm). Optionally CbpX is selected from the group consisting of CbpA, PbcA, SpsA and PspC. Optionally, it is CbpA. Optionally, LytX is LytC (also referred to as Sp91). Another embodiment of the present invention is a PspA or PsaA truncate lacking the choline binding domain (C) and expressed as a fusion protein with LytX. Optionally, LytX is LytC.

With regards to PsaA and PspA, both are discussed in the art. For example, PsaA and transmembrane deletion variants thereof have been described by Berry & Paton, Infect Immun 1996 Dec;64(12):5255-62. PspA and transmembrane deletion variants thereof have been described in, for example, US 5804193, WO 92/14488, and WO 99/53940.

With regards to PcpA, this protein has been described in the art, for example PcpA has been described in WO2011/075823. The term 'PcpA' refers to a protein comprising at least 80%, 85%, 90%, 95%, 98%, 99% or 100% identical to SEQ ID NO:2 or 7 from WO2011/075823 or fragments of at least 100, 150, 200, 25 or more consecutive amino acids of SEQ ID NO:2 or 7 from WO2011/075823.

Sp128 and Sp130 are disclosed in WO00/76540. Sp125 is an example of a pneumococcal surface protein with the Cell Wall Anchored motif of LPXTG (where X is any amino acid). Proteins within this class of pneumococcal surface protein with this motif has been found to be useful within the context of this invention, and is therefore considered a further protein of the invention. Sp125 itself is disclosed in WO 98/18930, and is also known as ZmpB - a zinc metalloproteinase. Sp101 is disclosed in WO 98/06734 (where it has the reference # y85993). It is characterized by a Type I signal sequence. Sp133 is disclosed in WO 98/06734 (where it has the reference # y85992). It is also characterized by a Type I signal sequence.

Any of these further *S.pneumoniae* proteins may be present is an unconjugated or conjugated form. One or more *S.pneumoniae* proteins is optionally conjugated to an *S.pneumoniae* saccharide (described in the section entitled *Streptococcus pneumoniae* capsular saccharide conjugates above). Optionally one or more *S.pneumoniae* proteins is conjugated to a saccharide from a different bacterium.

The term 'conjugated to' in this context means that the protein is covalently bonded to a saccharide, in this situation the protein is acting as a carrier protein.

In an embodiment the at least one further unconjugated or conjugated *S.pneumoniae* protein comprises a Poly Histidine family (PhtX) protein selected from the group consisting of PhtB, PhtE, PhtA, PhtBD and PhtDE.

### Adjuvants

In one embodiment the immunogenic composition further comprises an adjuvant.

Suitable adjuvants include, but are not limited to, aluminium salts (for example, aluminium phosphate or aluminium hydroxide), monophosphoryl lipid A (for example 3D-MPL), saponins (for example QS21), oil in water emulsions, blebs or outer membrane vesicle preparations from Gram negative bacterial strains (such as those taught by WO02/09746), lipid A or derivatives thereof, alkyl glucosamide phosphates or combinations of two or more of these adjuvants. In one embodiment the adjuvant is aluminium phosphate. In a further embodiment the adjuvant comprises 100-750, 150-600, 200-500, 250-450, 300-400, or around 350µg aluminium as aluminium phosphate per human dose.

### Vaccines

The present invention provides a vaccine comprising the immunogenic compositions of the invention. Embodiments herein relating to "immunogenic compositions" of the invention are also applicable to embodiments relating to "vaccines" of the invention, and vice versa. In an embodiment, the vaccine comprises the immunogenic composition of the invention and a pharmaceutically acceptable excipient.

The vaccines of the invention may be administered by any suitable delivery route, such as intradermal, mucosal e.g. intranasal, oral, intramuscular or subcutaneous. Other delivery routes are well known in the art. Vaccine preparation is generally described in Vaccine Design ("The subunit and adjuvant approach" (eds Powell M.F. & Newman M.J.) (1995) Plenum Press New York).

In one aspect, the immunogenic composition of the invention is administered by the intramuscular delivery route. Intramuscular administration may be to the thigh or the upper arm. Injection is typically via a needle (e.g. a hypodermic needle), but needle-free injection may alternatively be used. A typical intramuscular dose is 0.5 ml.

A further aspect of the invention is a method of making a vaccine of the invention comprising the steps of mixing the unconjugated *S*. *pneumoniae* protein with the adjuvant composition.

In one aspect of the invention there is provided a method of immunizing a subject against diseases caused by *Streptococcus pneumoniae* infection comprising administering to the subject a therapeutically effective dose of the immunogenic composition or vaccine of the invention. In a further aspect of the invention there is provided a method of immunizing a subject against diseases caused by *Haemophilus influenzae* infection comprising administering to the subject a therapeutically effective dose of the immunogenic composition or vaccine of the invention. In a further embodiment there is provided a method of immunizing a subject against diseases caused by *Streptococcus pneumoniae* and *Haemophilus influenzae* infection comprising administering to the subject a therapeutically effective dose of the immunogenic composition or vaccine of the invention. In one embodiment the diseases comprise at least one disease selected from the group consisting of pneumonia, invasive pneumococcal disease (IPD), exacerbations of chronic obstructive pulmonary disease (COPD), otitis media, meningitis, bacteraemia, and conjunctivitis. In one embodiment the subject is a mammalian subject. In a further embodiment the mammalian subject is selected from the group consisting of mouse, guinea pig and human. In one embodiment the subject is an adult human, optionally an elderly human. In a further embodiment the subject is an infant human.

In a further aspect of the invention is provided an immunogenic composition or vaccine of the invention for use in the treatment or prevention of disease caused by *Streptococcus pneumoniae* infection. In a further aspect of the invention there is provided an immunogenic composition or vaccine of the invention for use in the treatment or prevention of disease caused by *Haemophilus influenzae* infection. In a further embodiment there is provided an immunogenic composition or vaccine of the invention for use in the treatment or prevention of disease caused by *Streptococcus pneumoniae* and *Haemophilus influenzae* infection. In one embodiment the use comprises administration of the immunogenic composition to an adult human host, optionally an elderly human host. In a further embodiment the use comprises administration of the immunogenic composition to an infant host. In a further embodiment the diseases comprise at least one disease selected from the group consisting of pneumonia, invasive pneumococcal disease (IPD), exacerbations of chronic obstructive pulmonary disease (COPD), otitis media, meningitis, bacteraemia, and conjunctivitis.

In a further aspect of the invention there is provided a use of the immunogenic composition or vaccine of the invention in the manufacture of a medicament for the treatment or prevention of diseases caused by *Streptococcus pneumoniae* infection. In a further aspect of the invention there is provided of use of the immunogenic composition or vaccine of the invention in the manufacture of a medicament for the treatment or prevention of diseases caused by *Haemophilus influenzae* infection. In a further embodiment there is provided a use of the immunogenic composition or vaccine of the invention in the manufacture of a medicament for the treatment or prevention of diseases caused by *Haemophilus influenzae* and *Streptococcus pneumoniae* infection. In one embodiment the diseases comprise at least one disease selected from the group consisting of pneumonia, invasive pneumococcal disease (IPD), exacerbations of chronic obstructive pulmonary disease (COPD), otitis media, meningitis, bacteraemia, and conjunctivitis,

In one embodiment the use comprises administration of the immunogenic composition or vaccine of the invention to a host selected from the group consisting of an adult human host, an elderly human host and an infant human host.

Fusion proteins of formula (I) and *Streptococcus pneumoniae* capsular saccharide conjugates are useful as immunogens in subjects such as mammals, particularly humans. In particular, the fusion proteins of formula (I) and *Streptococcus pneumoniae* capsular saccharide conjugates are useful in inducing an immune response against *H. influenzae* in subjects, particularly humans. Additionally, the fusion proteins of formula (I) and *Streptococcus pneumoniae* capsular saccharide conjugates are useful in inducing an immune response against *Streptococcus pneumoniae* in subjects, particularly humans. More specifically, the fusion proteins of formula (I) are useful in the treatment or prevention of otitis media and/or AECOPD and/or pneumonia.

In one embodiment, the invention further provides a method for the treatment or prevention of otitis media in a subject in need thereof comprising administering to said subject a therapeutically effective amount of an immunogenic composition comprising the fusion proteins of formula (I) and *Streptococcus pneumoniae* capsular saccharide conjugates as described herein. In another embodiment, the invention provides a method for the treatment or prevention of acute exacerbations of chronic obstructive pulmonary disease (AECOPD) in a subject in need thereof comprising administering to said subject a therapeutically effective amount of an immunogenic composition comprising the fusion proteins of formula (I) and *Streptococcus pneumoniae* capsular saccharide conjugates as described herein.

In another embodiment, the invention provides a method for the treatment or prevention of pneumonia in a subject in need thereof comprising administering to said subject a therapeutically effective amount of an immunogenic composition comprising the fusion proteins of formula (I) and *Streptococcus pneumoniae* capsular saccharide conjugates as described herein.

In another embodiment, the invention provides a method for the treatment or prevention of a *H. influenzae* infection or disease in a subject in need thereof, said method comprising administering to said subject a therapeutically effective amount of an immunogenic composition comprising the fusion proteins of formula (I) and *Streptococcus pneumoniae* capsular saccharide conjugates as described herein.

In another embodiment, the invention provides a method for the treatment or prevention of a *S.pneumoniae* infection or disease in a subject in need thereof, said method comprising administering to said subject a therapeutically effective amount of an immunogenic composition comprising the fusion proteins of formula (I) and *Streptococcus pneumoniae* capsular saccharide conjugates as described herein.

### Further definitions

Unless otherwise explained or defined herein, all technical and scientific terms used herein have the same meaning as commonly understood by one of ordinary skill in the art to which this disclosure belongs. For example, definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

The singular terms "a," "an," and "the" include plural referents unless context clearly indicates otherwise. Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description. Additionally, numerical limitations given with respect to concentrations or levels of a substance, such as an antigen may be approximate. Thus, where a concentration is indicated to be (for example) approximately 200 pg, it is intended that the concentration includes values slightly more or slightly less than ("about" or "∼") 200 pg

Although methods and materials similar or equivalent to those described herein can be used in the practice or testing of this disclosure, suitable methods and materials are described below

The term "comprises" means "includes". Thus, unless the context requires otherwise, the word "comprises," and variations such as "comprise" and "comprising" will be understood to imply the inclusion of a stated compound or composition (*e.g*., nucleic acid, polypeptide, antigen) or step, or group of compounds or steps, but not to the exclusion of any other compounds, composition, steps, or groups thereof. The abbreviation, "*e.g*." is derived from the Latin exempli gratia, and is used herein to indicate a non-limiting example. Thus, the abbreviation "*e.g*." is synonymous with the term "for example."

A "subject" as used herein is a mammal, including humans, non-human primates, and non-primate mammals such as members of the rodent genus (including but not limited to mice and rats) and members of the order Lagomorpha (including but not limited to rabbits).

As used herein, "adjuvant" means a compound or substance that, when administered to a subject in conjunction with a vaccine, immunotherapeutic, or other antigen- or immunogen-containing composition, increases or enhances the subject's immune response to the administered antigen or immunogen (as compared to the immune response that would be obtained in the absence of adjuvant). This is to be distinguished from "adjuvant therapy", defined by the National Cancer Institute of the United States Institutes of Health in the context of cancer treatment as additional treatment given after the primary treatment, to lower the risk that the cancer will recur.

Conservative substitutions are well known and are generally set up as the default scoring matrices in sequence alignment computer programs. These programs include PAM250 (Dayhoft M.O. et al., (1978), "A model of evolutionary changes in proteins", In "Atlas of Protein sequence and structure" 5(3) M.O. Dayhoft (ed.), 345-352), National Biomedical Research Foundation, Washington, and Blosum 62 (Steven Henikoft and Jorja G. Henikoft (1992), "Amino acid substitution matrices from protein blocks"), Proc. Natl. Acad. Sci. USA 89 (Biochemistry): 10915-10919. The invention further provides fusion proteins of formula (I) containing conservative amino acid substitutions. For example, the fusion proteins of formula (I) may contain a conservative substitution of any amino acid from PE or PilA of *H. influenzae* as described in any of the sequences set forth herein (for example, any PE sequence set forth in SEQ ID NO. 4 - SEQ ID NO. 57 and/or any PilA sequence set forth in SEQ ID NO. 58 - SEQ ID NO. 121)

As used herein "signal peptide" refers to a short (less than 60 amino acids, for example, 3 to 60 amino acids) polypeptide present on precursor proteins (typically at the N terminus), and which is typically absent from the mature protein. The signal peptide (sp) is typically rich in hydrophobic amino acids. The signal peptide directs the transport and/or secretion of the translated protein through the membrane. Signal peptides may also be called targeting signals, transit peptides, localization signals, or signal sequences. For example, the signal sequence may be a co-translational or post-translational signal peptide.

A heterologous signal peptide may be cleaved from a fusion protein construct by signal peptide peptidases during or after protein transportation or secretion. For example, the signal peptide peptidase is signal peptide peptidase I. A "heterologous" signal peptide is one which is not associated with the protein as it exists in nature.

As used herein "treatment" means the prevention of occurrence of symptoms of the condition or disease in a subject, the prevention of recurrence of symptoms of the condition or disease in a subject, the delay of recurrence of symptoms of the condition or disease in a subject, the decrease in severity or frequency of symptoms of the condition or disease in a subject, slowing or eliminating the progression of the condition and the partial or total elimination of symptoms of the disease or condition in a subject.

As used herein, "optionally" means that the subsequently described event(s) may or may not occur, and includes both event(s) that occur and events that do not occur.

All references or patent applications cited within this patent specification are incorporated by reference herein.

As used herein `infant' refers to a human 0-2 years old.

As used herein 'adult' refers to a human more than 18 years old.

As used herein 'elderly adult' refers to a human more than 60 years old, optionally more than 65 years old.

### Examples

In the examples, the following terms have the designated meaning:
6xhis = six histidines;
xg = centrifugal force (number gravities)
ATP = adenosine triphosphate;
BCA = bicinchoninic acid;
BSA = bovine serum albumin;
°C = degrees Celsius;
CaCl₂ = calcium chloride;
CV = column volume;
DNA = deoxyribonucleic acid;
DSC = differential scanning calorimetry;
DTT = dithiothreitol;
dNTP = deoxynucleoside triphosphate;
EDTA = ethylenediaminetetraacetic acid;
FT = flow through;
HCl = hydrogen chloride;
His = his = histidine;
HEPES = 4-(2-hydroxyethyl)-1-piperazineethanesulfonic acid;
IMAC = immobilized metal affinity chromatography;
IPTG = isopropyl β-D-1-thiogalactopyranoside;
KCl = potassium chloride;
K₂HPO₄ = dibasic potassium phosphate;
KH₂PO₄ = monobasic potassium phosphate;
LDS = lithium dodecyl sulfate;
L = liter;
MES = 2-(*N*-morpholino)ethanesulfonic acid;
MgCl₂ = magnesium chloride;
ml = milliliter;
RPM = revolutions per minute;
min = minute;
mM = millimolar;
µL = microliter;
NaCl = sodium chloride;
Na₂HPO₄ = dibasic sodium phosphate;
NaH₂PO₄ = monobasic sodium phosphate;
ng = nanogram;
nm = nanometer;
O/N = overnight;
PBS = phosphate buffered saline;
PCR = polymerase chain reaction;
SB = sample buffer;
sec = second;
w/v = weight/volume
PS=polysaccharide and may be used interchangeably with the term 'saccharide'.

### 1. EXAMPLES

### Example 1: Fusion proteins

Fusion proteins were produced with different signal peptides and amino acid linker sequences. These fusion proteins allowed for secretion of both Protein E and PilA (or fragments thereof) without being restricted to a single bacterial strain. The fusion protein is released into the periplasm after removal of the heterologous signal peptide by a signal peptide peptidase. Fusion protein purified from the bacteria does not contain the heterologous signal peptide. "Purified" proteins are removed from the bacteria and lack the signal peptide.

The following table describes fusion protein constructs made.

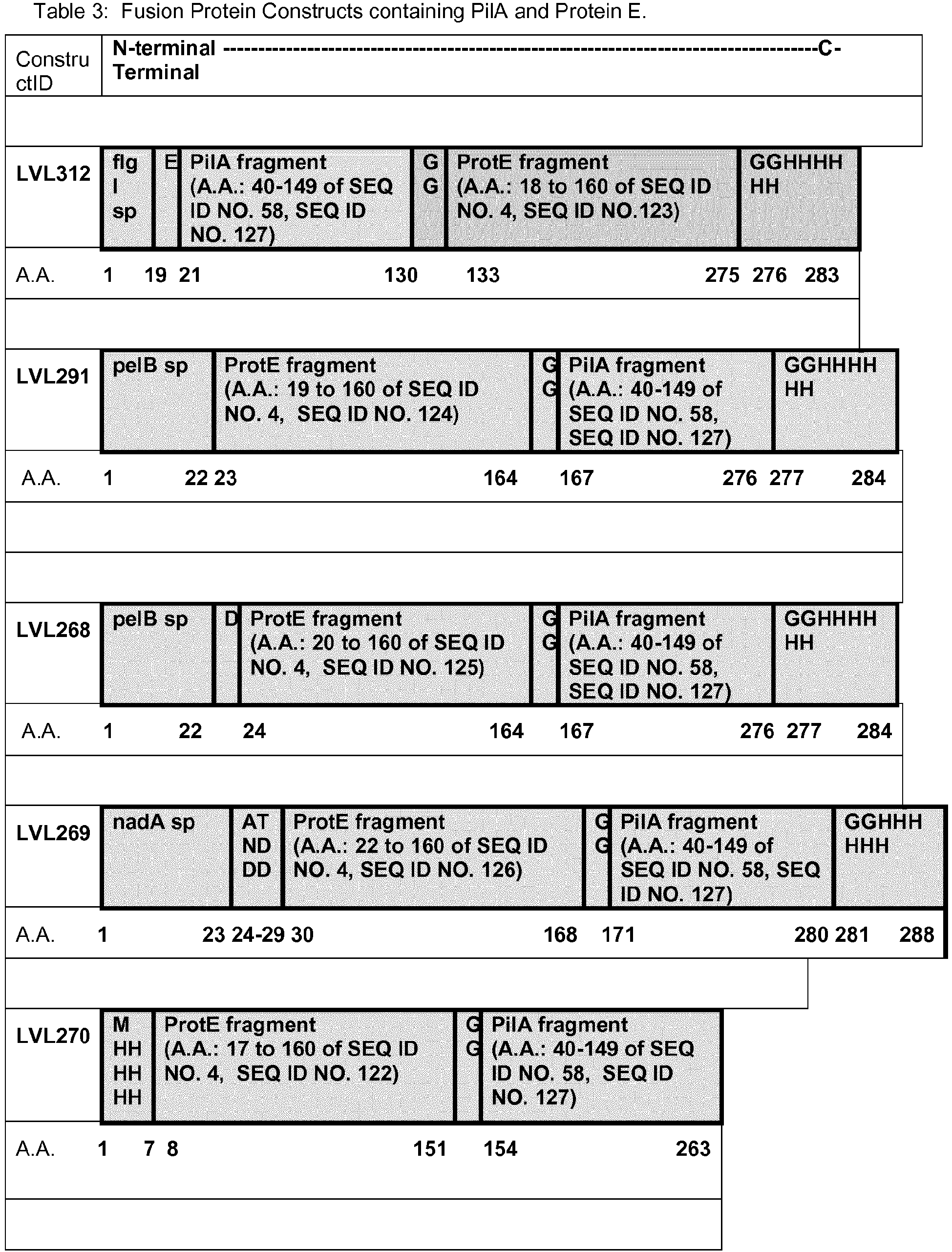

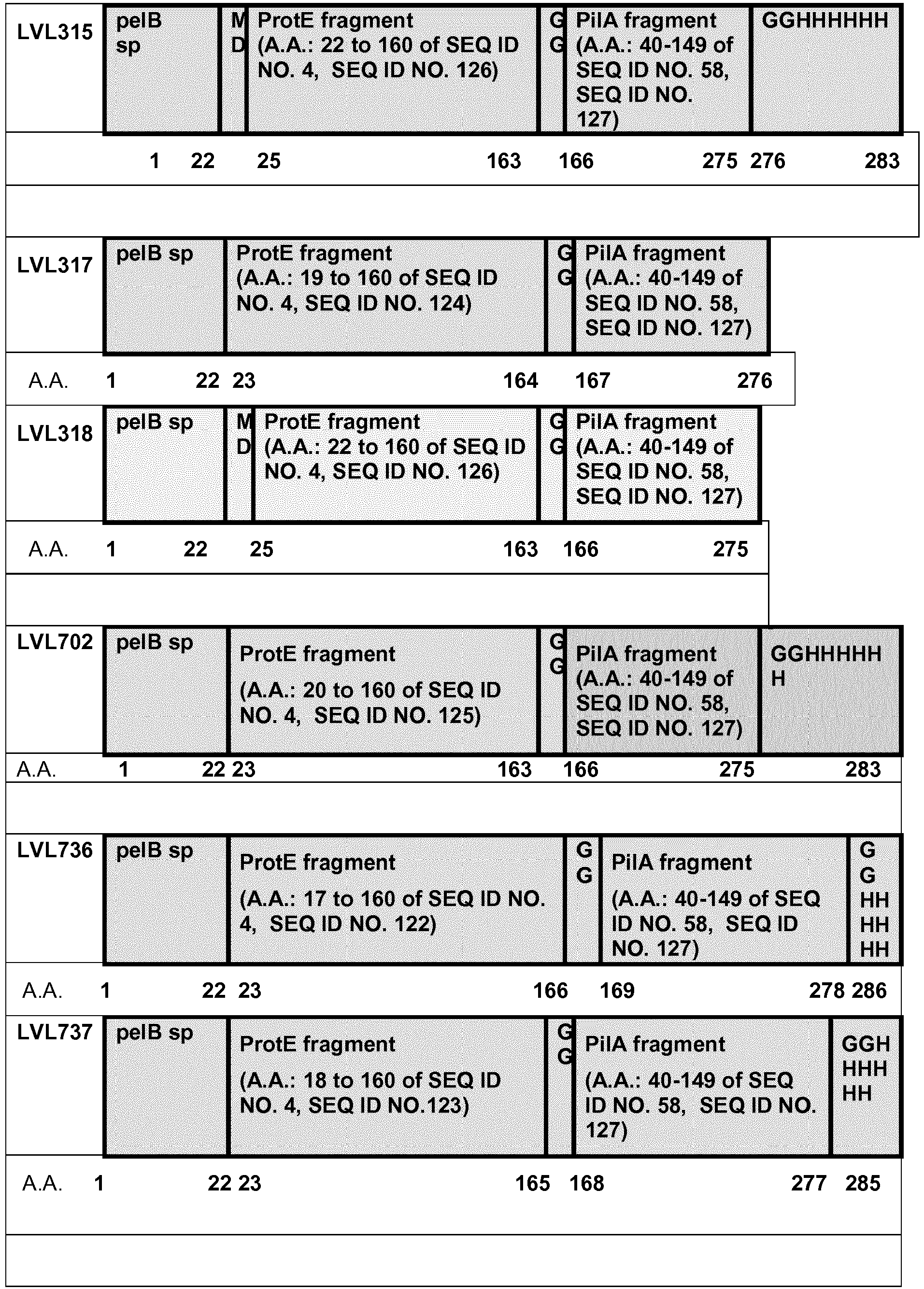

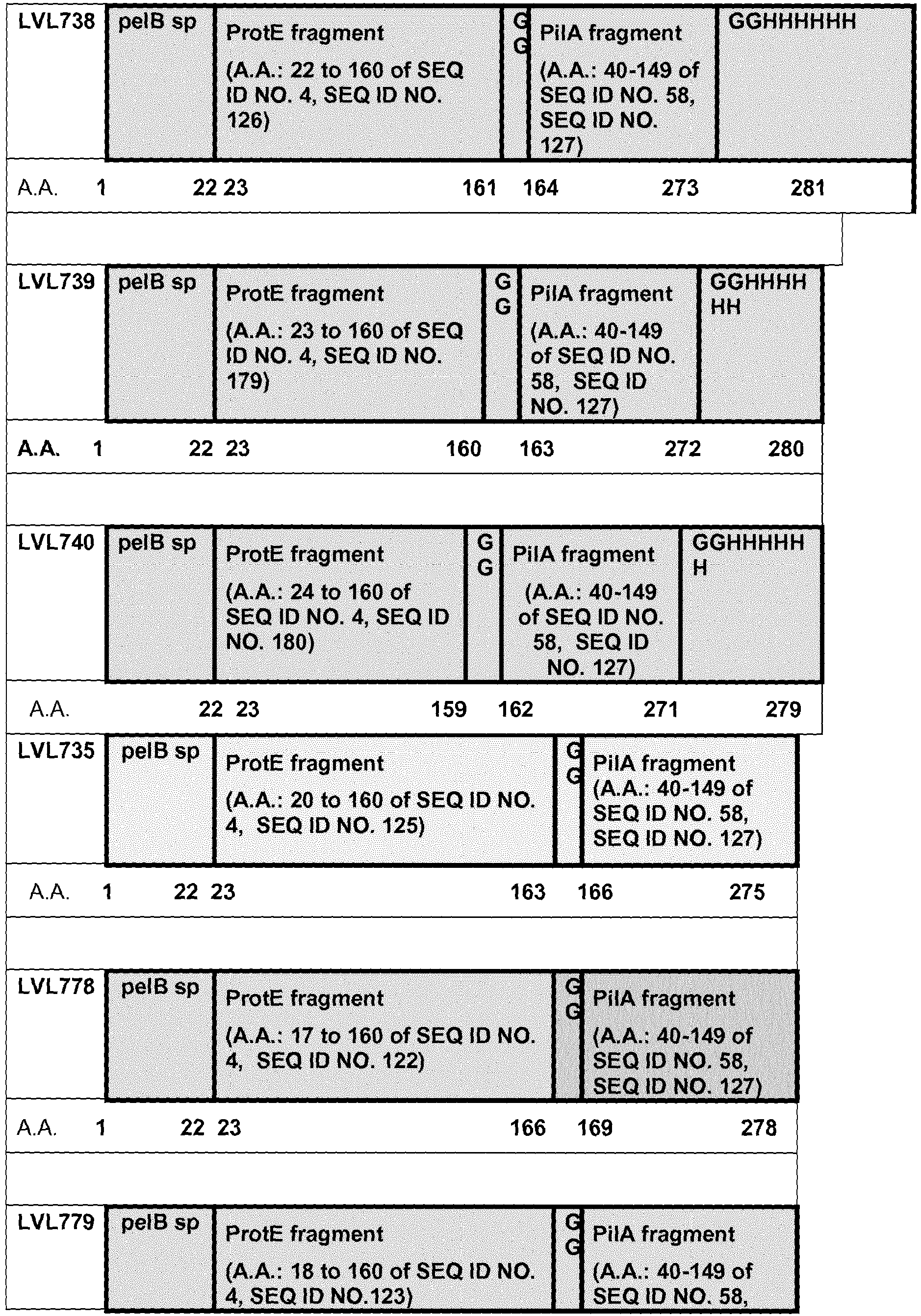

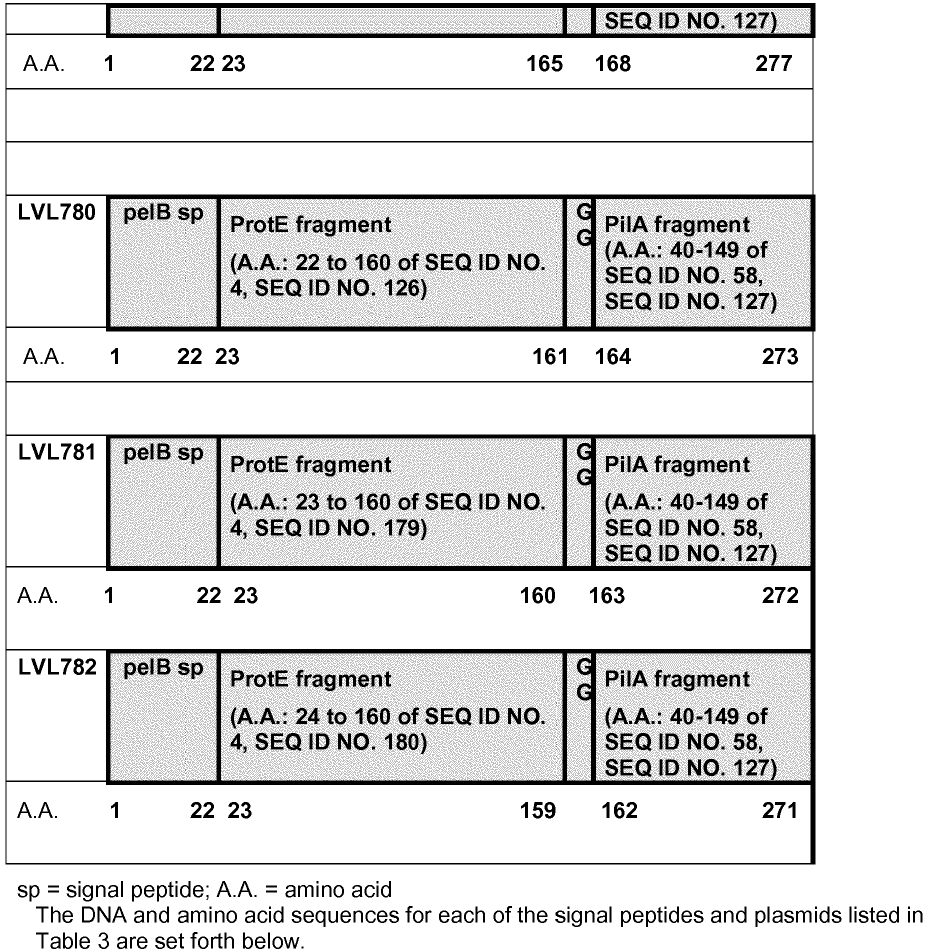

### SIGNAL SEQUENCES:

*pelB signal peptide (DNA) - SEQ ID NO. 129:*
   atgaaatacctgctgccgaccgctgctgctggtctgctgctcctcgctgcccagccggcgatggcc
*pelB signal peptide (Amino Acid) - SEQ ID NO. 130:*
   MKYLLPTAAA GLLLLAAQPA MA
*Flgl signal peptide (DNA) - SEQ ID NO. 131:*
   atgattaaatttctctctgcattaattcttctactggtcacgacggcggctcaggct
*Flgl signal peptide (Amino Acid) - SEQ ID NO. 132:*
   MIKFLSALIL LLVTTAAQA
*NadA signal peptide (DNA) - SEQ ID NO. 133:*
   atgaaacactttccatccaaagtactgaccacagccatccttgccactttctgtagcggcgcactggca
*NadA signal peptide (Amino Acid) - SEQ ID NO. 134:*
   MKHFPSKVLT TAILATFCSG ALA

### FUSION PROTEIN CONSTRUCT SEQUENCES:

The single underlined portion of the amino acid sequences is from PiIA from *Haemophilus influenzae* strain 86-028NP. The embolded underlined portion of the amino acid sequences was derived from Protein E from *Haemophilus influenza* strain 772.
*L VL312 (DNA) - SEQ ID NO. 135:*
*LVL312 (protein):* (flgl sp)(E)(PilA aa 40-149)(GG)(ProtE aa 18-160)(GGHHHHHH) - *SEQ ID NO. 136*
*LVL291 (DNA) - SEQ ID NO. 137:*
*LVL291 (Protein)(pelB* sp)(ProtE aa 19-160)(GG)(PilA aa40-149)(GGHHHHHH) - *SEQ ID NO.* 138
*L VL268 (DNA) - SEQ ID NO. 139:*
*LVL268 (protein):* (pelB sp)(D)(ProtE aa 20-160)(GG)(PiIA aa40-149)(GGHHHHHH) - *SEQ ID NO. 140:*
*LVL269 (DNA) - SEQ ID NO. 141:*
*LVL269 (protein):* (nadA sp)(ATNDDD)(ProtE aa 22-160)(GG)(PilA aa 40-149)(GGHHHHHH) *- SEQ ID NO. 142*
*L VL270 (DNA) - SEQ ID NO. 143:*
*LVL270 (protein):* (MHHHHHH)(ProtE aa 17-160)(GG)(PilA aa40-149) - *SEQ ID NO. 144:*
*L VL315 (DNA) - SEQ ID NO. 145:*
*LVL315 (protein):* (pelB sp)(MD)(ProtE aa 22-160)(GG)(PilA aa40-149)(GGHHHHHH) - *SEQ ID NO. 146:*
*LVL317 (DNA) - SEQ ID NO. 147:*
*LVL317 (protein):* (pelB sp)(ProtE aa 19-160)(GG)(PilA aa40-149) - *SEQ ID NO. 148:*
*L VL318 (DNA) - SEQ ID NO. 149:*
*LVL318 (protein):* (pelB sp)(MD)(ProtE aa 22-160)(GG)(PilA aa40-149) - *SEQ ID NO. 150:*
*LVL702 (DNA) - SEQ ID NO. 181:*
*LVL702 (protein):* (pelB sp)(ProtE aa 20-160)(GG)(PilA aa40-149)(GGHHHHHH) - *SEQ ID NO. 182:*
*LVL736 (DNA) - SEQ ID NO. 183:*
*LVL736 (protein):* (pelB sp)(ProtE aa 17-160)(GG)(PilA aa40-149)(GGHHHHHH) - *SEQ ID NO. 184:*
*LVL737 (DNA) - SEQ ID NO. 185:*
*LVL737 (protein): (pelB sp)(ProtE aa 18-160)(GG)(PilA aa40-149)(GGHHHHHH) - SEQ ID NO. 186:*
*LVL738 (DNA) - SEQ ID NO. 187:*
*LVL738 (protein): (pelB sp)(ProtE aa 22-160)(GG)(PilA aa40-149)(GGHHHHHH) - SEQ ID NO. 188:*
*L VL 739 (DNA) - SEQ ID NO. 189:*
*LVL739 (protein): (pelB sp)(ProtE* aa *23-160)(GG)(PilA aa40-149)(GGHHHHHH) - SEQ ID NO. 190:*
*LVL740 (DNA) - SEQ ID NO. 191:*
*LVL740 (protein): (pelB sp)(ProtE* aa *24-160)(GG)(PilA aa40-149)(GGHHHHHH) - SEQ ID NO. 192:*
*L VL 735 (DNA) - SEQ ID NO. 193:*
*LVL735 (protein): (pelB sp)(ProtE* aa *20-160)(GG)(PilA aa40-149) - SEQ ID NO. 194:*
*L VL 778 (DNA) - SEQ ID NO. 195:*
*LVL778 (protein): (pelB sp)(ProtE aa 17-160)(GG)(PilA aa40-149) - SEQ ID NO. 196:*
*LVL779 (DNA) - SEQ ID NO. 197:*
*LVL779 (protein): (pelB sp)(ProtE* aa *18-160)(GG)(PilA aa40-149) - SEQ ID NO. 198:*
*LVL780 (DNA) - SEQ ID NO. 199:*
*LVL780 (protein): (pelB sp)(ProtE aa 22-160)(GG)(PilA aa40-149) - SEQ ID NO. 200:*
*LVL781 (DNA) - SEQ ID NO. 201:*
*LVL781 (protein): (pelB sp)(ProtE aa 23-160)(GG)(PilA aa40-149) - SEQ ID NO. 202:*
*L VL 782 (DNA) - SEQ ID NO. 203:*
*LVL782 (protein): (pelB sp)(ProtE aa 24-160)(GG)(PilA aa40-149) - SEQ ID NO. 204:*

The full length sequence for PE and PilA from which the above sequences were obtained are set forth in SEQ ID NO. 4 (PE) and SEQ ID NO. 58 (PilA), respectively.

### Example 2: Vector Construction and Transformation

Primers for amplifying PE from *H. influenzae* strain 772 were designed based on the sequence of *H. influenzae* strain Hi Rd. The 5' primer sequence contains one nucleotide difference compared to the NTHi 772 sequence, introducing an amino acid difference at position 24 when compared with the currently reported NTHi 772 genome sequence. Amino acid #24 in the fusion protein constructs is E (glutamic acid) instead of K (lysine) as found in NTHi 772.
*DNA Sequence for PE from H. influenzae strain Rd. - SEQ ID NO. 151*
*Protein Sequence for PE from H. influenzae strain Rd. - SEQ ID NO. 152*
*DNA Sequence for PE from H. influenzae strain 772 (as set forth in: Microbes & Infection, Corrigendum to "Identification of a novel Haemophilus influenzae protein important for adhesion to epithelia cells"[Microbes Infect. 10 (2008) 87-97], available online July 6, 2010, "Article in Press')) - SEQ ID NO. 153*
*Protein Sequence for PE from H. influenzae strain 772 (as set forth in:* Microbes & Infection, Corrigendum to "Identification of a novel Haemophilus influenzae protein important for adhesion to epithelia cells"[Microbes Infect. 10 (2008) 87-97], available online July 6, 2010*, "Article in Press')) - SEQ ID NO. 154*

### Vector construction:

To generate LVL312, LVL291, LVL268, LVL269, LVL270, LVL702, LVL735, LVL778, LVL779, LVL780, LVL781 and LVL782, a polymerase chain reaction (PCR) preparation of the following components was prepared (specific components are subsequently exemplified): 36.6 µl of deionized water, 5µl of buffer #1 10X, 5 µl of dNTPs 2mM, 2 µl MgCl₂ 25 mM, 0.4 µl of primer #1 (50 µM), 0.4 µl of primer #2 (50 µM), 0.5 µl of template (100 ng/µl) and 0.4 µl of KOD HiFi DNA polymerase 2.5 units/µl (NOVAGEN^{®}) was formulated. Polymerase chain reaction involved 25 cycles of 15 seconds of denaturation at 98°C, 2 seconds for annealing at 55°C and 20 seconds of primer extension at 72°C. The PCR products were purified using QIAQUICK^{®} PCR purification kit (QIAGEN^{®}). This product was used under conditions recommended by the supplier which were: the addition of 5 volumes Buffer PB, provided in the QIAQUICK^{®} PCR purification kit, to 1 volume of the PCR preparation. The PCR preparation with Buffer PB was subsequently mixed by vortex. A QIAQUICK^{®} column was placed into a 2 ml collection tube. To bind DNA in the PCR preparation to the column, the mixed sample was applied to the QIAQUICK ^{®} column and centrifuged for 30-60 seconds at 14 000 RPM. The flow-through was discarded and the QIAQUICK ^{®} column was placed back in the same tube. To wash the bound DNA 0.75 ml Buffer PE, provided in the QIAQUICK ^{®} PCR purification kit, was added to the QIAQUICK ^{®} column, and the column was centrifuged for 30-60 seconds at 14 000 RPM. The flow-through was discarded and the QIAQUICK ^{®} column was placed back in the same tube. The QIAQUICK ^{®} column was centrifuged once more in the 2 ml collection tube for 1 minute to remove residual wash buffer. Each QIAQUICK ^{®} column was placed in a clean 1.5 ml microcentrifuge tube. To elute the DNA, 33 µl water was added to the center of the QIAQUICK^{®} membrane and the column was centrifuged for 1 minute at 14 000 RPM. Restriction enzymes and buffer related were obtained from New England BioLabs. For example, approximately 5 µl of pET26b vector (100 ng/µl), 2 µl of NEBuffer 2 (New England Biolabs, 1X NEBuffer 2: 50 mM NaCl, 10 mM Tris-HCl, 10 mM MgCl₂, 1 mM dithiothreitol, pH 7.9 at 25°C), 1 µl of *Ndel* (20 000 units/ml), 1 µl of *HindIII* (20 000 units/ml) and 11 µl of deionized water were mixed and incubated for two hours at 37°C for DNA digestion. Thereafter, a second step of purification was performed using the QIAQUICK ^{®} PCR purification kit (QIAGEN^{®}) with the procedure described above.

Ligation was performed using Quick T4 DNA ligase and Quick Ligation Reaction Buffer from New England BioLabs. For example, around 10 ng of vector and 30 ng of insert in 10 µl of deionized water were mixed with 10 µl of 2X Quick Ligation Reaction Buffer (New England Biolabs, 132 mM Tris-HCl, 20 mM MgCl₂, 2mM dithiothreitol, 2 mM ATP, 15% polyethylene glycol, pH 7.6 at 25°C) and 1 µl of Quick T4 DNA ligase (New England Biolabs). The enzymatic reaction was incubated for 5 minutes at room temperature before transformation.

To generate LVL315, LVL317, LVL318, LVL736, LVL737, LVL738, LVL739 and LVL740, a PCR preparation of the following components was prepared: 40 µl of deionized water, 5 µl of reaction buffer 10X, 1 µl of dNTPs mix, 1 µl of primer #1 (10 µM), 1 µl of primer #2 (10 µM), 1 µl of template (25 ng/µl) and 1 µl of *PfuUltra* High-Fidelity DNA polymerase 2.5 units/µl (QuikChange II Site-Directed Mutagenesis Kit, Agilent Technologies, Stratagene Division) was formulated. Polymerase chain reaction involved one cycle of denaturation at 95°C for 30 sec, 18 cycles of 30 sec of denaturation at 95°C, 1 min for annealing at 55°C and 5 min 30 sec of primer extension at 68°C. The PCR products were digested using 1 µl of Dpnl restriction enzyme at 37°C for one hour before transformation.

A detailed list of PCR primer sequences used for amplifications is illustrated in Table 4.

To generate pRIT16711, the PE gene fragment coding for amino acids 22 to 160 of SEQ ID NO. 4, which excludes the sequence coding for its corresponding secretion signal, was amplified by PCR from genomic DNA of NTHi strain 772. The amplification primers were designed based on the available strain Hi Rd sequence (at that time, the 772 sequence was not known). The 5' primer sequence contains one mutation compared to the NTHi 772 sequence (sequence as now available), introducing one amino acid difference in PE coding sequence at position 24, glutamic acid (E) instead of lysine (K). After PCR amplification, the insert was cloned in the pET-26(+) expression vector (NOVAGEN^{®}) using *Bam*HI and *Xho*I restriction sites.

To generate pRIT16671, a DNA fragment coding for a *PilA* gene fragment (amino acids 40 to 149 of SEQ ID NO. 58, SEQ ID NO. 127), which excludes its leader peptide as well as a portion of the predicted hydrophobic alpha helix, was amplified from genomic DNA of NTHi strain 86-028NP and cloned into the pET15 expression vector. The vector pRIT16790 (containing amino acids 40 to 149 from NTHi strain 86-028NP) was used as a template to generate the vector pRIT16671. The *PilA* gene fragment was amplified by PCR using the vector pRIT16790 and primers MDES PILA-3 and MDES PILA-4. The *PilA* fragment was cloned into the pET-26 expression vector using *NdeI* / *XhoI* restriction sites. The DNA sequence encoding six histidine (his) amino acids was incorporated into the 5' primer to add six histidines (6xhis) at the N-terminal end of the PilA sequence (MDES PILA-3).

To generate LVL312 (*Flgl* signal peptide-E-PilA fragment-GG-PE fragment-GGHHHHHH), a polymerase chain reaction was performed to amplify the *PilA* gene (amino acids 40-149 / strain 86-028NP) using the pRIT16671 vector as a template and primers CAN534 and CAN537. DNA sequence corresponding to *Flgl* signal peptide (sp) and glutamic acid (E) amino acid was incorporated into the 5' primer (CAN534). To link the *PilA* sequence to *PE* sequence, DNA sequence corresponding to the two glycine (GG) amino acids linker and the N-terminal PE amino acids were incorporated into the 3' primer (CAN537). Another polymerase chain reaction was performed to amplify the PE gene (amino acids 18-160) using pRIT16711 vector as a template and primers CAN536 and CAN538. DNA sequence corresponding to the C-terminal PilA amino acids and GG amino acids were incorporated into the 5' primer to link pilA to PE sequence (CAN536). DNA sequence corresponding to the GG amino acids linker and 6xhis amino acids were incorporated into the 3' primer (CAN538). Finally, to generate LVL312, a third polymerase chain reaction was performed to amplify the PilA and PE genes in fusion with the *Flgl* signal peptide at the N-terminus, a glutamic acid (E) amino acid between *Flgl* and pilA, a GG linker between PilA and PE sequences and a GG linker between PE and the 6xhis amino acids at the C-terminus. To achieve this amplification, the products of the two polymerase chain reactions described above were used as a template with primers CAN534 and CAN538. DNA sequence corresponding to *NdeI* restriction site was incorporated into the 5' primer and *HindIII* restriction site was incorporated into the 3' primer. The generated PCR product was then inserted into the pET-26b(+) cloning vector (NOVAGEN^{®}).

To generate LVL291 (pelB signal peptide-PE fragment-GG-PilA fragment-GG-6xhis), a polymerase chain reaction was performed to amplify the *PE* gene (amino acids 19-160) using the pRIT16711 vector as a template and primers CAN544 and CAN546. DNA sequence corresponding to pelB signal peptide (sp) amino acids was incorporated into the 5' primer (CAN544). To link the *PilA* sequence to the *PE* sequence, DNA sequence corresponding to GG amino acids linker and the N-terminal PilA amino acids were incorporated into the 3' primer (CAN546). Another polymerase chain reaction was performed to amplify the *PilA* gene (amino acids 40-149 of SEQ ID NO. 58, SEQ ID NO. 127) using the pRIT16671 vector as a template with primers CAN545 and CAN535. DNA sequence corresponding to the C-terminal PE amino acids and GG amino acids were incorporated into the 5' primer (CAN545) to link the PilA sequence to the PE sequence. DNA sequence corresponding to linker GG amino acids and 6xhis amino acids were incorporated into the 3' primer (CAN535). Finally, to generate LVL291, a third polymerase chain reaction was performed to amplify the PE and PilA genes in fusion with the pelB signal peptide at the N-terminus, a GG linker between the PE and PilA sequences and a GG linker between PilA and 6xhis amino acids at the C-terminus. To achieve this amplification, the products of two polymerase chain reactions described above were used as a template with primers CAN544 and CAN535. DNA sequence corresponding to *NdeI* restriction site was incorporated into the 5' primer and *HindIII* restriction site was incorporated into the 3' primer. The generated PCR product was then inserted into the pET-26b(+) cloning vector (NOVAGEN^{®}).

To generate LVL268 (pelB signal peptide-D-PE fragment-GG-PilA fragment-GG-6xhis), a polymerase chain reaction was performed to amplify the *PE* gene (amino acids 20-160) using the pRIT16711 vector as a template with primers CAN547 and CAN546. DNA sequence corresponding to the pelB signal peptide (sp) amino acids and aspartic acid (D) amino acid were incorporated into the 5' primer (CAN547). To link the *PilA* sequence to the *PE* sequence, DNA sequence corresponding to GG amino acids linker and the N-terminal PilA amino acids were incorporated into the 3' primer (CAN546). Another polymerase chain reaction was performed to amplify the *PilA* gene (amino acids 40-149 / NTHi strain 86-028NP) using the pRIT16671 vector as a template with CAN545 and CAN535. DNA sequence corresponding to the C-terminal PE amino acids and GG amino acids were incorporated into the 5' primer (CAN545) to link the PilA sequence to the PE sequence. DNA sequence corresponding to linker GG amino acids and 6xhis amino acids were incorporated into the 3' primer (CAN535). Finally, to generate LVL268, a third polymerase chain reaction was performed to amplify the PE and PilA genes in fusion with the pelB signal peptide at the N-terminus, a D amino acid between pelB signal peptide and PE, a GG linker between PE and pilA sequences and a GG linker between PilA and 6xhis amino acids in C-term. To achieve this amplification, the products of the two polymerase chain reactions described above were used as a template with primers CAN547 and CAN535. DNA sequence corresponding to *NdeI* restriction site was incorporated into the 5' primer and *HindIII* restriction site was incorporated into the 3' primer. The generated PCR product was then inserted into the pET-26b(+) cloning vector (NOVAGEN^{®}).

To generate LVL269 (NadA signal peptide-ATNDDD-PE fragment-GG-PilA fragment-GG-6xhis), a polymerase chain reaction was performed to amplify the *PE* gene (amino acids 22-160 of SEQ ID NO. 4) using the pRIT16711 vector as a template with primers CAN548 and CAN546. DNA sequence corresponding to pelB signal peptide (sp) amino acids and ATNDDD amino acids were incorporated into the 5' primer (CAN548). To link the *PilA* sequence to the *PE* sequence, DNA sequence corresponding to the GG amino acids linker and the N-terminal PilA amino acids were incorporated into the 3' primer (CAN546). Another polymerase chain reaction was performed to amplify the *PilA* gene (amino acids 40-149 of SEQ ID NO. 58, SEQ ID NO. 127) using the pRIT16671 vector as a template with primers CAN545 and CAN535. DNA sequence corresponding to the C-terminal PE amino acids and GG amino acids were incorporated into the 5' primer to link the PilA sequence to the PE sequence (CAN545). DNA sequence corresponding to linker GG amino acids and 6xhis amino acids were incorporated into the 3' primer (CAN535). Finally, to generate LVL269, a third polymerase chain reaction was performed to amplify the PE and PilA gene in fusion with the NadA signal peptide at the N-terminus, ATNDDD amino acids between the pelB signal peptide and PE, a GG linker between the PE and pilA sequences and a GG linker between PilA and 6xhis amino acids at the C-terminus. To achieve this amplification, the products of the two polymerase chain reactions describe above were used as a template with primers CAN548 and CAN535. DNA sequence corresponding to *Ndel* restriction site was incorporated into the 5' primer and *HindIII* restriction site was incorporated into the 3' primer. The generated PCR product was then inserted into the pET-26b(+) cloning vector (NOVAGEN^{®}).

To generate LVL270 (M-6xHis-PE fragment-GG-PilA fragment), a polymerase chain reaction was performed to amplify the PE gene (amino acids 17-160) using the pRIT16711 vector as a template with primers CAN540 and CAN542. DNA sequence corresponding to 6xhis amino acids were incorporated into the 5' primer (CAN540). To link the *PilA* sequence to the *PE* sequence, DNA sequence corresponding to the GG amino acids linker and the N-terminal PilA amino acids were incorporated into the 3' primer (CAN542). Another polymerase chain reaction was performed to amplify the *PilA* gene (amino acids 40-149 / NTHi strain 86-028NP) using pRIT16671 vector as a template with primers CAN541 and CAN543. DNA sequence corresponding to the C-terminal PE amino acids and GG amino acids were incorporated into the 5' primer (CAN541) to link the PilA to the PE sequence. Finally, to generate LVL270, a third polymerase chain reaction was performed to amplify the 6-his-PE-GG-PilA gene in fusion. To achieve this amplification, the products of the two polymerase chain reactions describe above were used as a template with primers CAN540 and CAN543. DNA sequence corresponding to *NdeI* restriction site was incorporated into the 5' primer and *HindIII* restriction site was incorporated into the 3' primer. The generated PCR product was then inserted into the pET-26b(+) cloning vector (NOVAGEN^{®}).

To generate LVL315 (pelB signal peptide-MD-PE fragment-GG-PilA fragment-GG-6xhis), a site-directed mutagenesis was performed to change the N-terminal PE amino acid sequence from QIQ to MD using LVL291 as a template with primers CAN670 and CAN671 and the QuikChange II Site-Directed Mutagenesis Kit (Agilent Technologies, Stratagene Division).

To generate LVL317 (pelB signal peptide-PE fragment-GG-pilA fragment), a site-directed mutagenesis was performed to incorporate a stop codon between the PilA gene and the DNA sequence corresponding to GGHHHHHH amino acid residues (SEQ ID NO: 3) using LVL291 as a template with primers CAN678 and CAN679 and the QuikChange II Site-Directed Mutagenesis Kit (Agilent Technologies, Stratagene Division).

To generate LVL318 (pelB signal peptide-MD-PE-GG-PilA), a site-directed mutagenesis was performed to incorporate a stop codon between the PilA gene and the DNA sequence corresponding to GGHHHHHH amino acid residues (SEQ ID NO: 3) using LVL315 as a template with primers CAN678 and CAN679 and the QuikChange II Site-Directed Mutagenesis Kit (Agilent Technologies, Stratagene Division).

To generate LVL702 (LVL291 ΔQ), a polymerase chain reaction was performed using the LVL291 vector as template and primers CAN1517 and CAN1518. Deletion of three nucleotides corresponding to the amino acid Q at the position 23 on LVL291 sequence was incorporated to the 5' primer. The only difference between LVL702 and LVL291 is the deletion of amino acid Q at the position 23 on LVL291 sequence. *NdeI* and *HindIII* restriction sites were incorporated into the 5' and 3' primers respectively. The generated PCR product was then inserted into the pET-26b(+) cloning vector (NOVAGEN^{®}).

To generate LVL735 (LVL317 ΔQ), a polymerase chain reaction was performed using the LVL317 vector as template and primers CAN1517 and CAN1519. Deletion of three nucleotides corresponding to the amino acid Q at the position 23 on LVL317 sequence was incorporated to the 5' primer. The only difference between LVL735 and LVL317 is the deletion of amino acid Q at the position 23 on LVL317 sequence. *NdeI* and *HindIII* restriction sites were incorporated into the 5' and 3' primers respectively. The generated PCR product was then inserted into the pET-26b(+) cloning vector (NOVAGEN^{®}).

To generate LVL736 (LVL291 + SA), a site-directed mutagenesis was performed to add amino acids S and A between amino acid 22 and 23 on LVL291 sequence. LVL291 was used as template with primers CAN1531 and CAN1532 and the QuikChange II Site-Directed Mutagenesis Kit (Agilent Technologies, Stratagene Division).

To generate LVL737 (LVL291 + A), a site-directed mutagenesis was performed to add amino acid A between amino acid 22 and 23 on LVL291 sequence. LVL291 was used as template with primers CAN 1529 and CAN 1530 and the QuikChange II Site-Directed Mutagenesis Kit (Agilent Technologies, Stratagene Division).

To generate LVL738 (LVL291 AQIQ), a site-directed mutagenesis was performed to delete amino acids Q, I and Q at positions 23 to 25 on LVL291 sequence. LVL291 was used as template with primers CAN1523 and CAN1524 and the QuikChange II Site-Directed Mutagenesis Kit (Agilent Technologies, Stratagene Division).

To generate LVL739 (LVL291 AQIQK), a site-directed mutagenesis was performed to delete amino acids Q, I, Q and K at positions 23 to 26 on LVL291 sequence. LVL291 was used as template with primers CAN1525 and CAN1526 and the QuikChange II Site-Directed Mutagenesis Kit (Agilent Technologies, Stratagene Division).

To generate LVL740 (LVL291 ΔQIQKA), a site-directed mutagenesis was performed to delete amino acids Q, I, Q, K and A at positions 23 to 27 on LVL291 sequence. LVL291 was used as template with primers CAN1527 and CAN1528 and the QuikChange II Site-Directed Mutagenesis Kit (Agilent Technologies, Stratagene Division).

To generate LVL778 (LVL736 Δ6xHis tag), LVL779 (LVL737 Δ6xHis tag), LVL780 (LVL738 Δ6xHis tag), LVL781 (LVL739 Δ6xHis tag) and LVL782 (LVL740 Δ6xHis tag) a polymerase chain reaction was performed using the LVL736, LVL737, LVL738, LVL739 and LVL740 vectors as template, respectively, with primers CAN1669 and CAN543. Deletion of 6xHis tag corresponds to the amino acid sequence GGHHHHHH (SEQ ID NO. 3) at the C-terminal sequences. This deletion was incorporated to the 3' primer. *NdeI* and *HindIII* restriction sites were incorporated into the 5' and 3' primers respectively. The generated PCR product was then inserted into the pET-26b(+) cloning vector (NOVAGEN^{®}).

**Table 4: PCR primer sequences used for PE. PilA and PE-PilA amplifications**

| **Primer ID** | **DNA Sequence** |
|---|---|
| | **5' - 3'** |
| **CAN534** | |
| **CAN535** | |
| **CAN536** | |
| **CAN537** | |
| **CAN538** | |
| **CAN540** | |
| **CAN541** | CATTTTCAGTTGATAAAAAAGGCGGCACTAAAAAAGCAGCGGTATC (SEQ ID NO. 161) |
| **CAN542** | GATACCGCTGCTTTTTTAGTGCCGCCTTTTTTATCAACTGAAAATG (SEQ ID NO. 162) |
| **CAN543** | TGTGTGAAGCTTTTATTGTGTGACACTTCCGCAAA (SEQ ID NO. 163) |
| **CAN544** | |
| **CAN545** | GCATTTTCAGTTGATAAAAAAGGCGGCACTAAAAAAGCAGCGGTATCTG (SEQ ID NO. 165) |
| **CAN546** | CAGATACCGCTGCTTTTTTAGTGCCGCCTTTTTTATCAACTGAAAATGC (SEQ ID NO. 166) |
| **CAN547** | |
| **CAN548** | |
| **CAN670** | GCCGGCGATGGCCATGGATAAGGCTGAACAAAATG (SEQ ID NO. 169) |
| **CAN671** | CATTTTGTTCAGCCTTATCCATGGCCATCGCCGGC (SEQ ID NO. 170) |
| **CAN 678** | GGAAGTGTCACACAATAAGGCGGCCACCACCACC (SEQ ID NO. 171) |
| **CAN679** | GGTGGTGGTGGCCGCCTTATTGTGTGACACTTCC (SEQ ID NO. 172) |
| **CAN1517** | |
| **CAN1518** | GGCCGCAAGCTTTTAGTGGTGGTGGTGGTGGTGGCCGCC(SEQ ID NO. 206) |
| **CAN1519** | GGCCGCAAGCTTTTATTGTGTGACACTTCC(SEQ ID NO. 207) |
| **CAN1523** | GCTGCCCAGCCGGCGATGGCCAAGGCTGAACAAAATGATGTG (SEQ ID NO. 208) |
| **CAN1524** | CACATCATTTTGTTCAGCCTTGGCCATCGCCGGCTGGGCAGC (SEQ ID NO. 209) |
| **CAN1525** | GCTGCCCAGCCGGCGATGGCCGCTGAACAAAATGATGTGAAGC (SEQ ID NO. 210) |
| **CAN1526** | GCTTCACATCATTTTGTTCAGCGGCCATCGCCGGCTGGGCAGC (SEQ ID NO. 211) |
| **CAN1527** | GCTGCCCAGCCGGCGATGGCCGAACAAAATGATGTGAAGCTGG (SEQ ID NO. 212) |
| **CAN1528** | CCAGCTTCACATCATTTTGTTCGGCCATCGCCGGCTGGGCAGC (SEQ ID NO. 213) |
| **CAN1529** | GCTGCCCAGCCGGCGATGGCCGCCCAGATTCAGAAGGCTGAAC (SEQ ID NO. 214) |
| **CAN1530** | GTTCAGCCTTCTGAATCTGGGCGGCCATCGCCGGCTGGGCAGC (SEQ ID NO. 215) |
| **CAN1531** | GCTGCCCAGCCGGCGATGGCCAGCGCCCAGATTCAGAAGGCTGAAC (SEQ ID NO. 216) |
| **CAN1532** | GTTCAGCCTTCTGAATCTGGGCGCTGGCCATCGCCGGCTGGGCAGC (SEQ ID NO. 217) |
| **CAN1669** | CACACACATATGAAATACCTGCTGCCGACC (SEQ ID NO. 218) |
| **MDesPILA -3** | |
| **MDesPILA -4** | GCGCCGCTCGAGTCATTGTGTGACACTTCCGC (SEQ ID NO. 174) |
| **MnoNTHi-44** | GCCCAGCCGGCGATGGCCCAGATCCAGAAGGCTGAACAAAATG (SEQ ID NO. 175) |
| **MnoNTHi-45** | CATTTTGTTCAGCCTTCTGGATCTGGGCCATCGCCGGCTGGGC (SEQ ID NO. 176) |

### Transformation

*Escherichia coli* BLR (DE3) or *E. coli* HMS (DE3) cells were transformed with plasmid DNA according to standard methods with CaCl₂-treated cells. (Hanahan D. « Plasmid transformation by Simanis. » In Glover, D. M. (Ed), DNA cloning. IRL Press London. (1985): p. 109-135.). Briefly, BLR (DE3) or HMS174(DE3) competent cells were gently thawed on ice. Approximately 4µl of plasmid (10-100 ng) were mixed using 50-100 µl competent cells. Thereafter, this formulation was incubated on ice for 30 min. To perform the transformation reaction, the formulation was heat pulsed at 42°C for 45 seconds then incubated on ice for 2 minutes.

Approximately 0.5 ml of SOC medium (Super Optimal broth with Catabolite repression) was added to the transformed cells and the cell culture was incubated at 37°C for one hour before plating on Luria-Bertani (LB) agar with 50 ug/ml kanamycin. Around 100 µl of transformed cell culture was plated and incubated overnight at 37°C.

BLR (DE3): BLR is a *recA*⁻derivative of BL21 (F- *ompT hsdSB(rB-* mB-) *gal dcm* (DE3). This *E. coli* strain used for expression of recombinant proteins improves plasmid monomer yields and may help stabilize target plasmids containing repetitive sequences or whose products may cause the loss of the DE3 prophage. (Studier, F.W. (1991) J. Mol. Biol. 219: 37-44). The detailed genotype of *E.coli* BLR (DE3) has been published by NOVAGEN^{®}. (F- ompT hsdSB (rB- mB-) gal dcm Δ(srl-recA)306::Tn10 (TetR) (DE3).

HMS174 (DE3): HMS174 strains provide the *recA* mutation in a K-12 background. Like BLR, these strains may stabilize certain target genes whose products may cause the loss of the DE3 prophage. The detailed genotype of *E.coli* HMS174 (DE3) has been published by NOVAGEN^{®}. (F- *recA1 hsdR*(rK12- mK12+) (DE3) (Rif R).

### Production using BLR (DE3) and Characterization of His tagged constructs are described in Example 3 through Example 6

### Example 3: Protein expression using shake flask

Generally, one confluent agar plate inoculated with *Escherichia coli* BLR (DE3) transformed with recombinant plasmid was stripped, resuspended in culture media and used to inoculate 800 ml of LB broth (Becton, Dickinson and Company) ± 1% (weight/volume, w/v) glucose (Laboratoire MAT, catalogue number: GR-0101) and 50µg/ml kanamycin (Sigma) to obtain O.D.₆₀₀ₙₘ between 0.1 and 0.2. Cultures were incubated at 37°C with agitation of 250 RPM to reach an O.D.₆₀₀ₙₘ of ∼0.8.

One ml of each culture was then collected, centrifuged at 14 000 RPM for 5 minutes and supernatants and pellets were frozen at -20°C separately.

At an O.D.₆₀₀ₙₘ ∼0.8, the BLR (DE3) cultures were cooled down (-20°C, 20 minutes or 4°C, 1 hour, preferably at 4°C for 1 hour) before inducing the expression of the recombinant protein by addition of 1 mM isopropyl β-D-1-thiogalactopyranoside (IPTG; EMD Chemicals Inc., catalogue number: 5815) and incubation overnight at 16, 22 and 30°C, or 3 hours at 37°C with agitation of 250 RPM, preferably overnight at 22°C. After the induction period the cultures were centrifuged at 14 000 RPM for 5 minutes or 6 000 RPM for 15 minutes and supernatant (media fraction sample) and pellets (containing soluble and insoluble fractions) were frozen at -20°C separately.

These conditions are used for periplasmic protein expression.

### Example 4: Protein purification using shake flask, cell pastes, His tagged constructs

Each bacterial pellet obtained after induction was resuspended in 20 mM 4-(2-hydroxyethyl)-1-piperazineethanesulfonic acid (HEPES) buffer (pH 8.0) containing 500 mM NaCl 10 mM imidazole and Roche COMPLETE^{®} Protease Inhibitor Cocktail (1 tablet/50 ml of HEPES buffer containing 500 mM NaCl, Roche COMPLETE® ULTRA tablets, Roche Diagnostics Corporation).

Alternatively, 20 to 50 mM bicine buffer may be used instead of HEPES buffer containing NaCl. For example, 20 mM bicine buffer may be used. Bacteria were lysed using a Constant System 1.1 KW 2 X 30 000 PSI (pounds per square inch). Soluble (supernatant) and insoluble (pellet) components were separated by centrifugation at 20 000g for 20 min at 4°C.

6-His tagged-proteins were purified under native conditions on immobilized metal affinity chromatography (IMAC) using PROFINIA™ protein purification protocol (Bio-Rad Laboratories, Inc.). The soluble components were loaded on a 5ml His Trap column (Bio-Rad Laboratories, Inc.) preequilibrated with the same buffer used for bacterial resuspension; the soluble components were added at up to 5 ml/min (producing a "flow through fraction") After loading on the column, the column was washed with 10 column volumes of the same buffer at a rate of 10 ml/min (producing a "wash fraction #1). A second wash using 20 mM bicine buffer or 20 mM HEPES buffer (pH 8.0) containing 500 mM NaCl and 20 mM imidazole was performed, producing a "wash fraction #2). Elution was performed using 2 column volumes of 20mM HEPES buffer or 50mM bicine buffer (pH 8.0) containing 500 mM NaCl and 250 mM imidazole at a rate of 10 ml/min, producing an "elution fraction".

To improve the purity of the protein, positive elution fractions from IMAC were pooled and loaded on a size exclusion chromatography (SEC) column (HILOAD™ SUPERDEX™ 200 26/60 from GE Healthcare) preequilibrated in phosphate buffered saline without calcium or magnesium (NaCl 137 mM, KCl 2.7 mM, Na₂HPO₄ 8.1 mM, KH₂PO₄ 1.47 mM, pH 7.4). Samples from elution fractions were analyzed by sodium dodecyl sulfate polyacrylamide gel electrophoresis (SDS-PAGE). Samples were concentrated using Centricon 10 000 MW (Millipore).

Protein concentration was determined using spectrometer.

### Example 5: SDS-PAGE and Western Blot Analysis of His tagged constructs & SDS-PAGE Analysis of non-his tagged LVL317 & LVL318 constructs

### Soluble and insoluble fraction preparation

For example, 1 ml of culture after induction (see, for example, Example 3 above) was centrifuged at 14 000 RPM for 2 min. The pellet was resolubilized using 40 µl of BUGBUSTER^{®} Protein Extraction Reagent (NOVAGEN^{®}, EMD4 Biosciences, Merck), creating a cell suspension. The cell suspension was incubated on a rotating platform for 10 min at room temperature. The cell suspension was then centrifuged at 14 000 RPM for 2 min to separate the soluble fraction. The resulting pellet (insoluble fraction) was resolubilized using 70 µl of deionized water, 5 µl of dithiothreitol (DTT) 1 M and 25 µl of NUPAGE^{®} LDS (Lithium Dodecyl Sulphate) Sample Buffer 4X (INVITROGEN™). The soluble fraction (supernatant from the cell suspension of the resolubilized pellet) was added to 30 µl of deionized water, 5 µl of DTT 1 M and 25 µl of LDS Sample Buffer 4X.

### Media fraction preparation

For example, to prepare the media fraction, 100 µl of the supernatant from the induced whole cell culture following centrifugation (see, for example, Example 3 above) was concentrated by adding 500 µl of RC reagent I (Bio-Rad Laboratories, Inc.); the sample was mixed and incubated for 1 min at room temperature. Then, 500 µl of Reagent II (Bio-Rad Laboratories, Inc.) was added to the sample and mixed. This formulation was centrifuged at 14 000 RPM for 10 min. The pellet was resolubilized using 28 µl of deionized water, 2 µl of DTT 1 M and 10 µl of LDS SB 4X.

### Purification fraction preparation

For example, purified proteins (for example, obtained as described in Example 4) were prepared for SDS-PAGE analysis by adding 70 µl of sample, 5 µl of DTT 1M and 25 µl of LDS Sample Buffer 4X.

### SDS-PAGE analysis and transfer to nitrocellulose membrane

SDS-PAGE analysis and transfer to nitrocellulose membrane were performed according to manufacturer's recommendations (Invitrogen) using NUPAGE^{®} Bis-Tris 4-12% gels. Preparations of samples, buffers and migration conditions were done under conditions recommended by the suppliers.

In one example, the gel was loaded with a 20 ul sample from a master mix comprising 70 µl of a purified protein fraction, 5 µl of DTT 1M and 25 µl of LDS SB 4X.

After samples were run on NUPAGE^{®} Bis-Tris 4-12% gels, the proteins were transferred to nitrocellulose membranes.

Nitrocellulose membranes were blocked for 30 minutes at 37°C, 60 RPM using 3 % milk / PBS 1X fresh solution. After the blocking incubation, Primary Antibodies were added (6X His Tag^{®} antibody, Abcam PLC, catalogue number: ab9108) at a dilution of: 1:1000 in 3 % milk / PBS 1X fresh solution for 1 hour at 37°C, 60 RPM. After that, membranes were washed three times, for 5 minutes each, at room temperature using 0.02% polsorbate 20 (for example, TWEEN™ 20) / PBS 1X. Secondary Antibodies (alkaline phosphatase (AP) Rabbit anti-IgG (H+L) rabbit, Jackson ImmunoResearch Laboratories, Inc.) were added at dilution 1:14 000 using 3 % milk / PBS 1X fresh solution. Membranes were incubated for 1 hour at 37°C, 60 RPM. After that, membranes were washed three times for 5 minutes at room temperature using 0.02% polysorbate 20 (for example, TWEEN™ 20) / PBS 1X before the membrane expositions to 5-bromo-4-chloro-3-indolyl phosphate/nitro blue tetrazolium (for example, BCIP^{®}/NBT from Sigma-Aldrich^{®}, 1 tablet / 10 ml water).

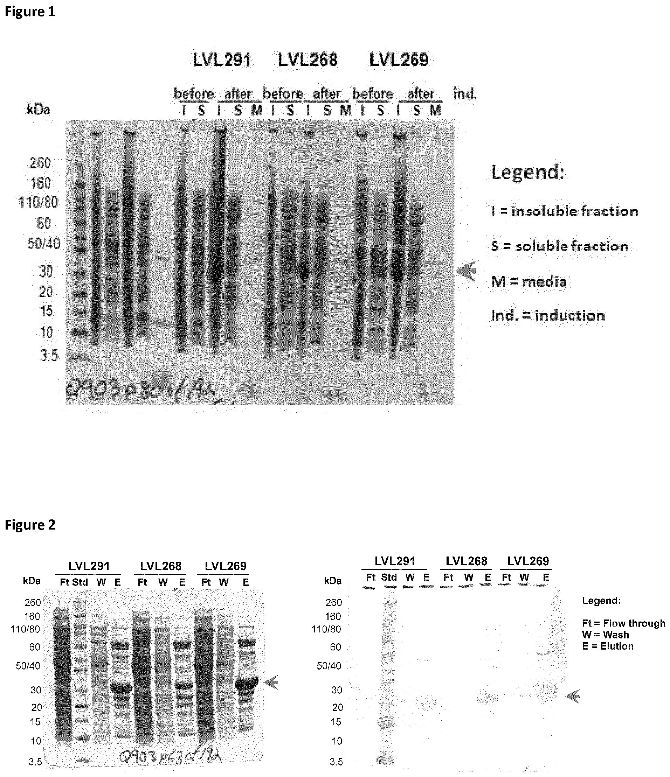

See Figure 1 for SDS-PAGE of induced bacterial extracts for fusion protein constructs LVL291, LVL268 and LVL269. Insoluble fraction (I), Soluble fraction (S) and Culture Media fraction (M) were loaded for LVL291, LVL268 and LVL269 before and after induction (ind).

See Figure 2 for SDS-PAGE and Western blot related to purification extracts for fusion protein constructs LVL291, LVL268 and LVL269. Flow through fraction (Ft), Wash fraction (W) and Elution fraction (E) were loaded for purification of LVL291, LVL268 and LVL269. Anti-his tag was used to probe extracts.

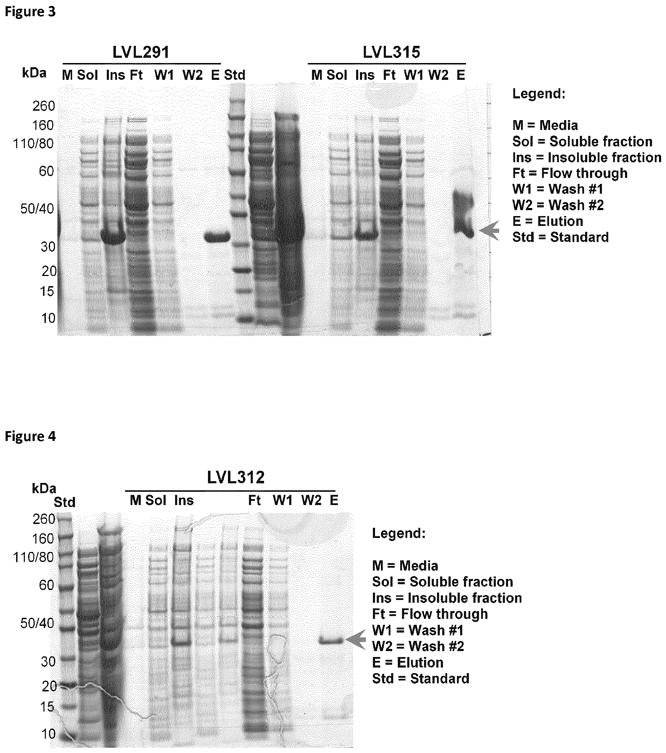

See Figure 3 for SDS-PAGE of induced bacterial and purification extracts for fusion protein constructs LVL291 and LVL315. Culture Media fraction (M), Soluble fraction (Sol), Insoluble fraction (Ins), Flow through fraction (Ft), Wash fraction #1 (W1), Wash fraction #2 (W2) and Elution fraction (E) were loaded for LVL291 and LVL315.

See Figure 4 for SDS-PAGE of induced bacterial and purification extracts for fusion protein construct LVL312. Culture Media fraction (M), Soluble fraction (Sol), Insoluble fraction (Ins), Flow Through fraction (Ft), Wash fraction #1 (W1), Wash fraction #2 (W2) and Elution fraction (E) were loaded for LVL312.

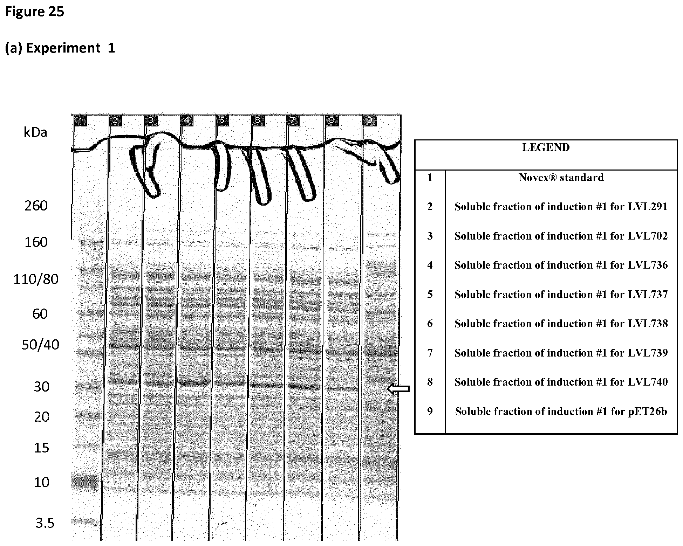

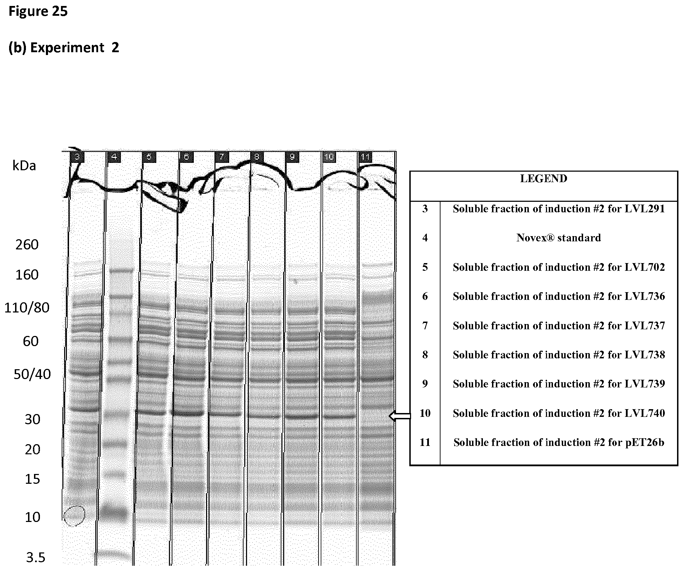

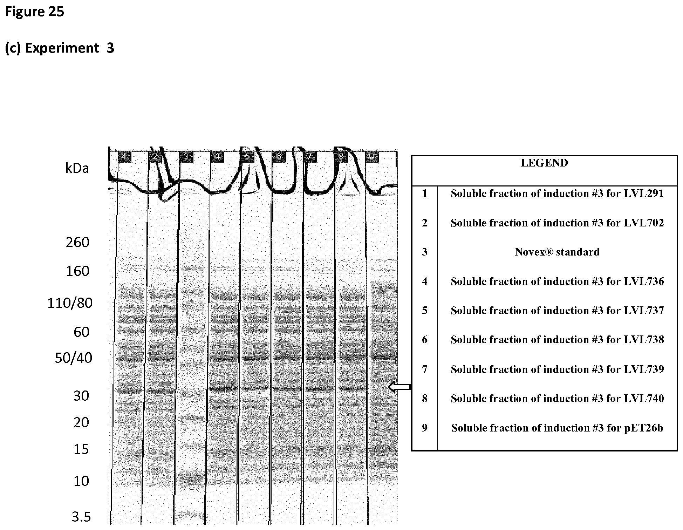

See Figure 25 for SDS-PAGE of soluble fractions from induced bacterial extracts for fusion protein constructs LVL291, LVL702, LVL736, LVL737, LVL738, LVL739, LVL740 and pET26b vector (negative control). (a) Experiment 1 (b) Experiment 2 (c) Experiment 3. PE-PilA fusion protein indicated by arrow.

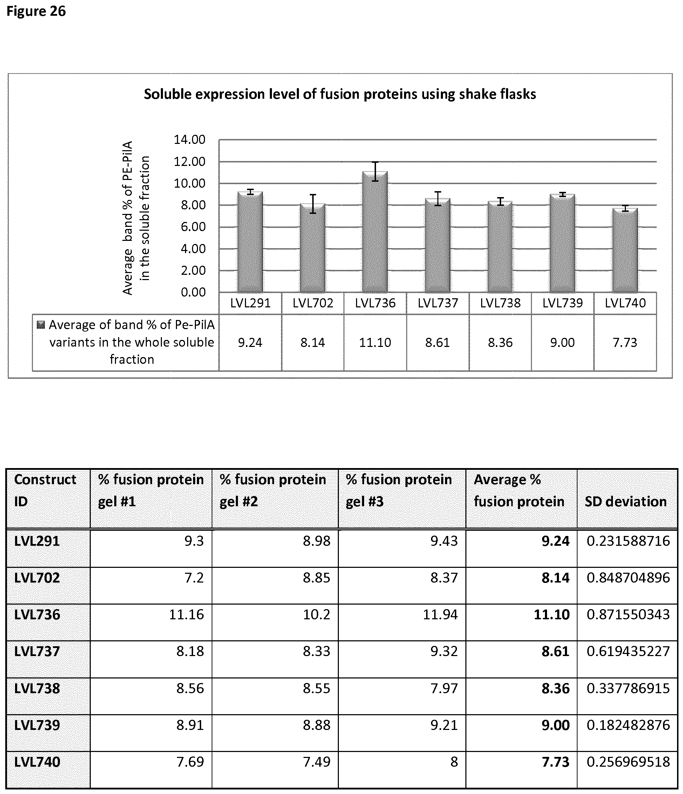

See Figure 26 for the average band percentage of fusion protein in the soluble fraction from Experiments 1, 2 and 3.

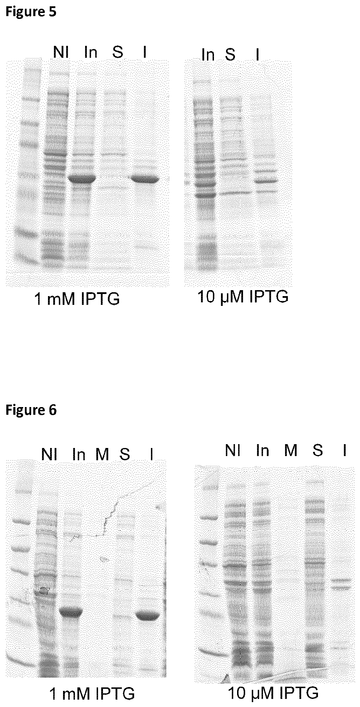

LVL317 and LVL318 bacterial extracts used in the SDS-PAGE analysis in Figure 5 and Figure 6, respectively, were prepared generally as described above.

Figure 5. SDS-PAGE of induced (1mM and 10µM IPTG) bacterial extracts for fusion protein construct LVL317. Extracts from before (NI) and after induction (In), Soluble fraction (S), Insoluble fraction (I).

Figure 6. SDS-PAGE of induced (1mM and 10µM IPTG) bacterial extracts for fusion protein construct LVL318. Extracts from before (NI) and after induction (In), Culture Media fraction (M), Soluble fraction (S), Insoluble fraction (I).

Proteins separate by SDS-PAGE were transferred to an Immobilon-P membrane. The Coomassie Blue stained protein bands were cut and placed in a sequenator reactor. Sequencing was carried out according to manufacturer's protocol using an Applied Biosystems PROCISE® Protein Sequencer, model 494-cLC.

**Table 5: Shake flask protein expression profiles and signal peptide cleavage for fusion protein constructs.**

| **Fusion Protein Construct ID** | **Description** | **Protein Expression profile** | **Signal peptide cleavage** |
|---|---|---|---|
| | **N-term → C-term** | | |
| LVL312 | Flgl sp - E - PilA fragment - GG - PE fragment - GGHHHHHH | In: +++ | Confirmed |
| | | So: + | |
| | | Se: + | |
| LVL291 | PelB sp - PE fragment - GG - PilA fragment - GGHHHHHH | In : +++ | Confirmed |
| | | So : ++ | |
| | | Se: + | |
| LVL268 | PelB sp - D - PE fragment - GG - PilA fragment - GGHHHHHH | In : +++ | Confirmed |
| | | So : ++ | |
| | | Se: + | |
| LVL269 | NadA sp - ATNDDD - PE fragment - GG - PilA fragment - GGHHHHHH | In : +++ | Confirmed |
| | | So : ++ | |
| | | Se: + | |
| LVL270 | MHHHHHH - PE fragment - GG - PilA fragment | In : + | Not tested |
| | | So : - | |
| | | Se: - | |
| LVL315 | PelB sp - MD - PE fragment - GG - PilA fragment - GGHHHHHH | In : +++ | Confirmed |
| | | So : ++ | |
| | | Se: + | |
| LVL317 | PelB - PE fragment - GG - PilA fragment | In : +++ | Confirmed |
| | | So : + | |
| | | Se: Nt | |
| LVL318 | PelB sp - MD - PE fragment - GG - PilA fragment | In : +++ | |
| | | So: + | |
| | | Se: - | |
| LVL702 | PelB sp - PE fragment - GG - PilA fragment - GGHHHHHH | In : +++ | Confirmed |
| | | So : ++ | |
| | | Se: Nt | |
| LVL736 | PelB sp - PE fragment - GG - PilA fragment - GGHHHHHH | In : +++ | Confirmed |
| | | So : ++ | |
| | | Se: Nt | |
| LVL737 | PelB sp - PE fragment - GG - PilA fragment - GGHHHHHH | In : +++ | Confirmed |
| | | So : ++ | |
| | | Se: Nt | |
| LVL738 | PelB sp - PE fragment - GG - PilA fragment - GGHHHHHH | In : +++ | Confirmed |
| | | So : ++ | |
| | | Se: Nt | |
| LVL739 | PelB sp - PE fragment - GG - PilA fragment - GGHHHHHH | In : +++ | Confirmed |
| | | So : ++ | |
| | | Se: Nt | |
| LVL740 | PelB sp - PE fragment - GG - PilA fragment - GGHHHHHH | In : +++ | Confirmed |
| | | So : ++ | |
| | | Se: Nt | |

| | | | |
|---|---|---|---|
| So = Soluble fraction. In = Insoluble fraction. Se = Protein Secreted in the media fraction. Nt = Not tested. The following rating were based on a visual inspection (coomassie) + : low expression; ++ : medium expression; +++ : high expression; - : no expression | | | |

### Example 6: LVL291 Fusion protein characterization

### PHYSICAL PROPERTIES OF LVL291: Folding of PE and PilA in LVL291 & Melting Point Circular Dichroism :

### Analysis of Secondary Structure

Circular dichroism (CD) is used to determine the secondary structure composition of a protein by measuring the difference in the absorption of left-handed polarized light versus right-handed polarized light which is due to structural asymmetry. The shape and the magnitude of the CD spectra in the far-UV region (190-250nm) are different whether a protein exhibits a beta-sheet, alpha-helix or random coil structure. The relative abundance of each secondary structure type in a given protein sample can be calculated by comparison to reference spectra.

Far UV spectra are measured using an optical path of 0,01 cm from 178 to 250nm, with a 1 nm resolution and bandwidth on a Jasco J-720 spectropolarimeter. Temperature of the cell is maintained at 23°C by a Peltier thermostated RTE-111 cell block. A nitrogen flow of 10L/min is maintained during the measurements.

### Results:

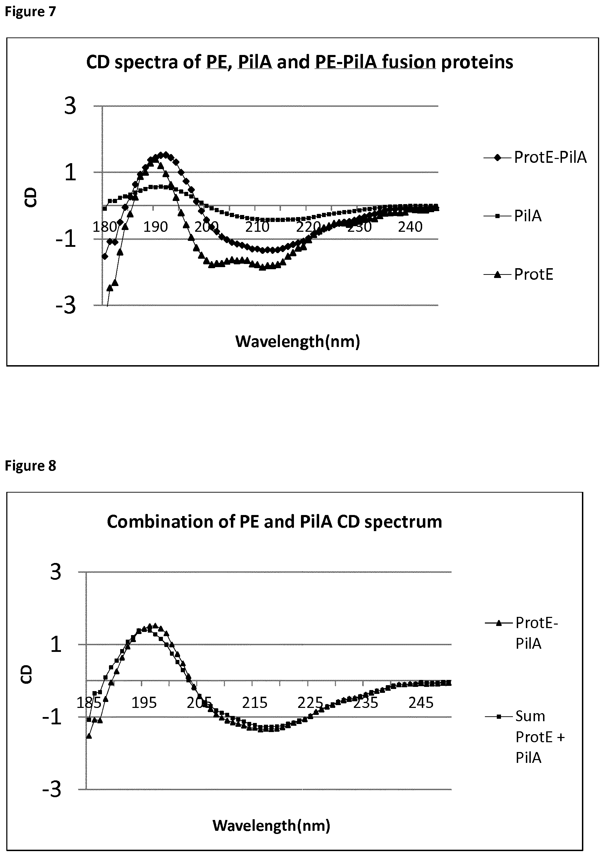

The far-UV CD spectra obtained for PE (from construct pRIT16762), PilA (from construct pRIT 16790) and PE-PilA proteins are characteristic of folded proteins containing a mix of alpha and beta structures, but PE is significantly richer in alpha helix than PilA and PE-PilA (Figure 7, CD spectra of PE, PilA and PE-PilA fusion proteins).

In order to evaluate the integrity of the folding of PE and PilA individual proteins once bound together in a chimeric protein and then verify a possible interaction between both, difference spectra were calculated.
When the PE and PilA far-UV spectra are combined, the resulting spectrum superposes to the spectrum of PE-PilA chimer (Figure 8, Combination of PE and PilA CD spectrum). This result suggests that the PE-PilA chimer contains all the secondary structures that are detected in the individual components. It also suggests that the fusion of the proteins has no major impact on the secondary structures of the individual components and consequently that the folding of PE and PilA is not significantly different whether the proteins are separate or in fusion.

### Melting Point Evaluation:

In order to evaluate if the expression in fusion has an impact on the thermodynamic properties of the individual proteins, the melting points of PE, PilA and PE-PilA have been evaluated by monitoring the defolding of the alpha helix with temperatue by circular dichroism.

The presence of alpha helix is characterized by a minimum in the Circular dichroism signal at 222nm, so a significant increase in CD signal at 222nm during temperature increase is an indication of protein denaturation. The determination of the temperature at which the protein undergoes loss in secondary structure allows the determination of the melting point (Tm), which corresponds to the temperature at which half of the proteins have lost their structure.

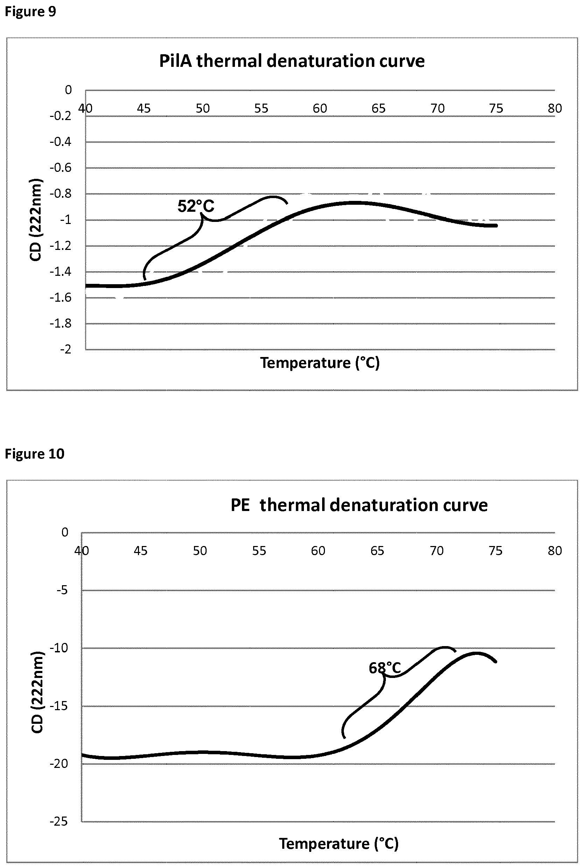

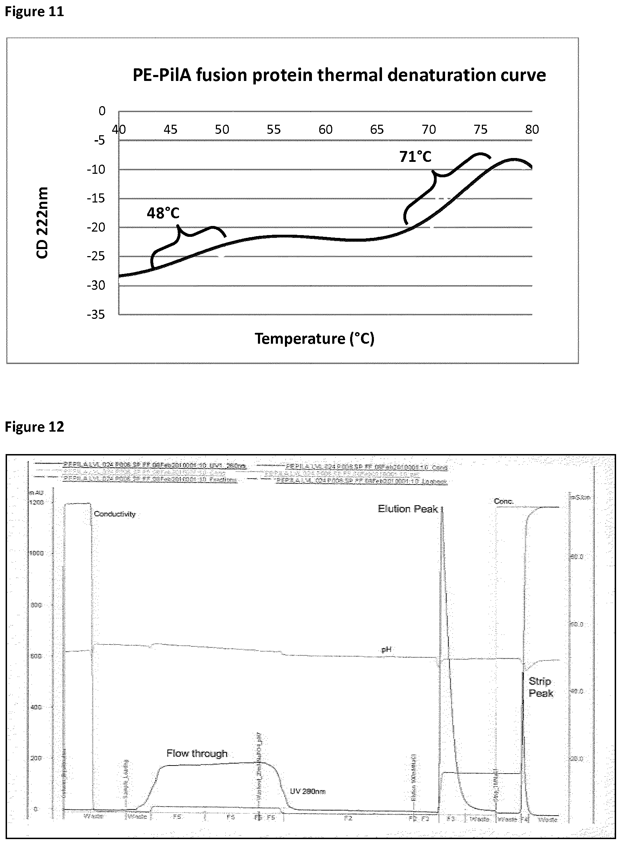

Melting point can be determined by identification of the inflexion point on the thermal denaturation curve obtained from a temperature versus CD 222nm plot.
Melting point of PilA and PE as determined by far-UV CD are respectively of 52°C and 68°C (Figure 9, PilA thermal denaturation curve; Figure 10, PE thermal denaturation curve).
The PE-PilA fusion protein exhibits two distinct Tm's at 48°C and 71°C (Figure 11, PE-PilA fusion protein thermal denaturation curve). Those values indicate that the PE and PilA proteins are still independently folded when bound into a chimer and that they defold at a similar temperature whether they are separate or in fusion. The observation that the defolding of the PilA portion at 48°C doesn't cause precipitation or impact the Tm of the PE portion at 71°C is a strong indication that the interaction between PE and PilA within the fusion is minimal and that they don't have a major observable impact on each other. The melting points of proteins are sensitive to various external conditions, including buffer composition or presence of interacting molecules; that no major variation is observed upon fusion of PE and PilA is a strong indication of the preservation of most of the structure and of the properties of both PE and PilA when they are bound together.

### Example 7: Fermentation process

Fusion proteins of the invention may be prepared by methods known by those skilled in the art.

### Example 8: Protein Purification of PE, PilA, and LVL317

### PE protein purification from pRIT16762:

To generate the pRIT16762 expression vector, the pRIT16711 vector was digested using BamHl and Ncol restriction enzymes in order to delete 6 amino acid residues between the signal sequence (pelB) and PE. The vector obtained was named pRIT16712. In this vector, there are 3 amino acids between the signal sequence pelB and PE: MDP. In a second step, a site directed mutagenesis was performed to change amino acid sequence from MDP to QIQ using pRIT16712 as template with primers MnoNTHi-44 and MnoNTHi-45 (described in Table 4) and the QuikChange II Site-Directed Mutagenesis Kit (Agilent Technologies, Stratagene Division).

Working seed of *E*. *coli* BLR(DE3) containing PE QIQ (from the pRIT16762 construct) was thawed from -80°C and used to prepare 100 ml of pre-culture in LB broth by overnight incubation at 37°C under agitation at 215 RPM. After overnight incubation, eight flasks containing 800 ml of LB APS were inoculated with 12.5 ml of pre-culture and OD₆₀₀ measured at around 0.06. The cultures were incubated 3h at 37°C with shaking. At a OD₆₀₀ of around 0.9, 1mM IPTG was added to start the induction. During the induction, the cultures were incubated 19h at 22°C with shaking. After induction, OD₆₀₀ was at around 2.2. The cell cultures were transferred into 1L centrifuge bags placed inside 1L bottles and centrifuged at 4°C for 30 minutes at 6,000xg and supernatant discarded. 1ml aliquots of culture pre- and post-induction and supernatant were kept for future analysis.

### Lysis of the BLR(DE3) induced with PE QIQ

The centrifuge bags were removed from the centrifugation bottles, opened and the pellet was expulsed from the bag into a beaker. The eight pellets were pulled together and resuspended in 100ml of binding buffer (20mM Hepes, 10mM imidazole, 500mM NaCl, pH 8.01). The *E.coli* BLR (DE3) containing the PE QIQ contruct were disrupted with the TS Series Bench Top cell disrupter from Constant Systems Ltd. (1x30 kPsi; 1x15kPsi). The lysate was centrifuged 30 minutes, 6000RPM, 4°C. The supernatant was kept and loaded on an IMAC column.

### IMAC purification of PE QIQ

IMAC column (BioRad, Bio-Scale Mini Profinity IMAC cartridge 5ml) was equilibrated with 5CV of Binding buffer (20mM HEPES, 10mM imidazole, 500mM NaCl, pH 8.01) at 5ml/min. 100ml of lysate supernatant was loaded on the IMAC at 2.5mL/min. Flow-through was collected in 50ml fractions for future analysis. The column was washed with 3CV of Binding buffer to remove unbound protein. Sample containing unbound proteins was collected in one aliquot of 15 ml in a 50 ml tube. The column was washed with 2CV of Wash buffer (20mM HEPES, 20mM imidazole, 500mM NaCl, pH 8.01) collected in 2 ml fractions in a 96 well plate. The bound protein was then eluted with 6CV of 100% Elution buffer (20mM HEPES, 250mM imidazole, 500mM NaCl, pH 8.01). The eluted protein was collected in 2 ml fractions in 96-well plates. Wash and elution were performed at 5ml/min.

### Size exclusion chromatography (SEC) on the IMAC pool of PE QIQ

SEC column (GE healthcare, HILOAD™ 26/60 SUPERDEX™ 75 prep grade, 60cm height approx 319ml volume) was equilibrated with 3CV of SEC buffer (20mM HEPES, 150mM NaCl, pH8.49). 11 ml of IMAC eluate was loaded onto the column at a flow rate of 2.5 ml/min. 2ml fractions were collected from 0.3CV to 0.9CV. Two runs were performed then fractions were analyzed by SDS-PAGE. Fractions from the two runs containing Prot E protein were pooled together ("SEC pool" , 48ml approx total volume). 500mM of Arginine was added to the SEC pool.

### Dosage of the PE QIQ pooled samples generated in the above SEC protocol

The SEC pool was dosed with the RCDC (Reducing Agent and Detergent Compatible) method from the Bio-Rad RC DC™ kit following manufacturer's protocol:
For each tested sample and standard, 25µL was distributed in microfuge tubes in duplicate. 125µL of Bio-Rad RC Reagent I was added into each tube; each tube was vortexed and incubate for 1 minute at room temperature. 125µL of Bio-Rad RC Reagent II is added into each tube; each tube is vortexed and then centrifuged at 14,000xg for 5 minutes. Supernatants are discarded by inverting the tubes on clean, adsorbent tissue paper allowing the liquid to drain completely from the tubes. 25.4µL of Reagent A (already prepared by mixing 20µL of Reagent S per 1ml of Reagent A) is added to each tube; each tube is vortexed and incubated at room temperature for 5 minutes, or until precipitate is completely dissolved. Vortex before proceeding to next step. Add 200µL of DC reagent B to each tube and vortex immediately. Incubate at room temperature for 15 minutes. Transfer all samples to a 96-well plate and read the adsorbance at 750nm to determine the protein concentration for each unknown protein sample.

### The ProtE concentration was 1.069 mg/ml

### PilA His-taaaed protein purification:

PilA was purified following the general procedure below:
*E. coli* cells containing a construct encoding PilA or a fragment thereof are suspended in BUGBUSTER® and BENZONASE® nuclease (NOVAGEN®), for example 10 ml BUGBUSTER® and 10 ul BENZONASE® nuclease. The cell lysate is mixed at room temperature on a rotating platform, for example, for 15 minutes. The cell lysate is centrifuged at 4°C, for example at 16,000g for 20 minutes. The supernatant containing the protein is added to a Ni NTA column containing Ni NTA HIS BIND® resin and mixed at 4°C, for example for 1 hour. The column may consist of 2 ml of Ni NTA HIS ·BIND® resin (NOVAGEN®) and 10 ml 1X Binding Buffer (from NOVAGEN®'s Ni-NTA Buffer Kit). The column flow through is then collected. The resin is washed two times with 1X wash buffer, for example, containing 300 mM NaCl, 50mM NaH₂PO₄, 25 mM imidazone, pH 8.0). The wash is collected by gravity flow. The protein is eluted from the column with 1X elution buffer, for example, 300 mM NaCl, 50mM NaH₂PO₄, 250 mM imidazone, pH 8.0. The protein may be further purified by dialysis with the Binding Buffer and rerun over a Ni NTA column as described above.

### Thrombin cleavage of PilA.

PilA is then incubated with thrombin (diluted 1/50) at room temperature for 16h, to remove the histidine tag.

### Size exclusion chromatography (SEC) on PilA cleaved with thrombin.

SEC column (GE healthcare, HILOAD™ 26/60 SUPERDEX™ 75 prep grade, 60cm height approx 319ml volume) was equilibrated with 5CV of SEC buffer (20mM HEPES, 150mM NaCl, pH8.52). Approximately 10 ml of cleaved PilA was loaded onto the column at a flow rate of 2.5 ml/min. 2ml fractions collected from 0.3CV to 0.9CV. Two runs were performed then fractions were analyzed by SDS-PAGE. Fractions from the two runs containing cleaved PilA protein were pooled together ("SEC pool", 52ml approx total volume).

### Dosage of PilA, SEC pool.

The SEC pool was dosed with the RCDC method as described above. The cleaved PilA concentration was at 5.37 mg/ml.

### Dialysis of the PilA SEC pool with PBS 1x pH 7.4 (dialysis factor = 1600) and dosage by RCDC

The concentration post-dialysis determined by RCDC was at 3.0 mg/ml.

### Purification of LVL317

### Osmotic shock

Since LVL317 fusion protein is expressed and processed in bacterial periplasm, the protein was extracted by osmotic shock.

Frozen (-20°C) harvested *E*. *coli* B2448 cell paste containing LVL317 from 4 L of fermentor culture were pooled and resuspended in a hypertonic buffer consisting of 24 mM Tris-HCl, 16% (w/v) sucrose, 9.9% (w/v) glucose, 10 mM EDTA, pH 8.0 up to a final volume of 4L. The suspension was mixed gently for 30 min at room temperature using a 3-blade propeller installed on RW 16 basic stirrer, at medium speed. The suspension was centrifuged at 15,900 x g for 30 minutes at room temperature. Supernatant (SN1) was kept for gel analysis.

The resulting pellet was resuspended in a hypotonic solution; 38 mM MgCl₂, and mixed for 30 min at room temperature. The mixture was centrifuged at 15,900 x g for 30 minutes at room temperature and the antigen recovered in the supernatant (SN2).

A clarification of the SN2 was performed by filtration through a 0.45/0.2 µm polyethersulfone Sartorius Sartopore 2 MidiCap filter, at 600ml/min of flow rate.

The SN2 was diluted 1:3 with 20 mM NaH₂PO₄-Na₂HPO₄, pH 7.0, the pH adjusted to 7.0 if necessary and another clarification by filtration through a 0.45/0.2 µm polyethersulfone Sartorius Sartopore 2 MidiCap filter, at 600ml/min was performed.

### SP SEPHAROSE™ Fast Flow (SP FF) chromatography

The diluted/filtered SN2 was loaded and captured on a strong cationic exchanger resin (SP SEPHAROSE™ FF - GE Healthcare) in a 14 cm ID (internal diameter) x 20 cm length column (column volume 3100ml) equilibrated with 2CV of 20 mM NaH₂PO₄ / Na₂HPO₄ buffer pH 7.0. After washing the column with 5CV of 20 mM NaH₂PO₄ / Na₂HPO₄ buffer pH 7.0, the antigen (contained within LVL317) was eluted by increasing the concentration of NaCl up to 100 mM in the same washing buffer.

See Figure 12 for a typical SP SEPHAROSE™ Fast Flow chromatogram.

### Q SEPHAROSE™ Fast Flow (Q FF) chromatography

The antigen present in the SP FF Eluate was diluted 1:4 with a 20 mM Tris pH 8.5, pH adjusted to 8.5 if necessary and passed through a strong anionic exchanger resin (Q SEPHAROSE™ FF - GE Healthcare) in a 14 cm ID x 11.8 cm length column (column volume 1800ml) equilibrated with 2CV of 20 mM Tris buffer pH 8.5. The antigen was recovered in the flow-through fraction.

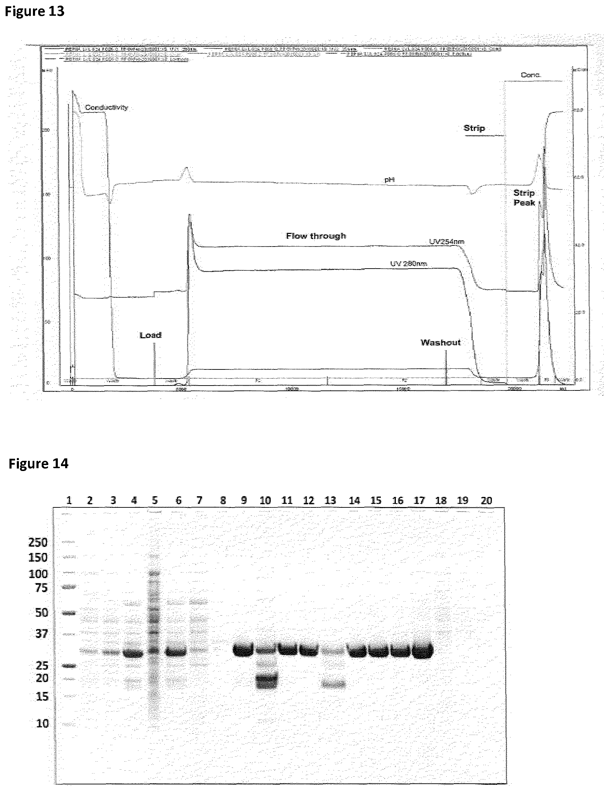

See Figure 13 for a typical Q SEPHAROSE™ Fast Flow chromatogram.

### Concentration, diaflitration, polysorbate 80 addition and sterile filtration

The Q FF flow-through containing the antigen was concentrated up to 0.7-0.8mg/ml based on chromatogram UV and diafiltered with 5DV of 10 mM KH₂PO₄ / K₂HPO₄ buffer pH 6.5 using a Pellicon-2™ 10 kDa cutoff membrane (Millipore).

Using a 5% stock solution, polysorbate 80 (for example, TWEEN™ 80) was added to the ultrafiltration retentate and agitated for 30 minutes with magnetic stirrer at 130rpm at 4°C. The final concentration of polysorbate 80 was 0.04%. Ultrafiltration retentate was sterilized by filtration through a 0.45/0.2 µm Cellulose Acetate membrane (Sartobran 300, Sartorius). The purified bulk was stored at -20°C or -80°C. Absolute protein concentration was measured by AAA (Amino Acid Analysis) at 0.737mg/ml.

### Example 9: Use of Polysorbate 80

A titration experiment indicated that the addition of polysorbate 80, specifically, TWEEN™ 80 to a final concentration of 0.04% (w/v) to the purified bulk prior to sterile filtration reduced filamentous particle formation and aggregation.

According to DSC analysis, TWEEN™ 80 reduced the degree of structural change (30-45°C) seen after freeze/thaw cycles after storage at -20°C and after storage 4 days at 4°C, -20°C and -80°C and 37°C.

### Example 10: SDS-PAGE and Western Blot Analysis of LVL317

### SDS-PAGE and Western Blot analysis:

NUPAGE^{®}, Bis-Tris 4-12% gel was loaded as described below with 10µg of sample in NUPAGE^{®} LDS sample buffer containing 50mM DTT heated 5min at 95°C (20µL of sample was loaded for samples having low concentration). Migration: 35 minutes at 200Volts at room temperature (RT) in NUPAGE^{®} MES Running Buffer. Gel Stained 2 hours in Instant blue (Novexin cat.: ISB01L) and destained overnight in water.

**Lane contents:**

| | | |
|---|---|---|
| 1: MW standard (10µL) | 2: Start (total fraction) (10µg) | 3: SN1 non filtered (10µg) |
| 4: SN2 not filtered (10µg) | 5: Not extracted (10µg) | 6: Load SP FF (10µg) |
| 7: Flow through SP FF (6.9µg) | 8: Wash SP FF (20µL) | 9: Elution SP FF (10µg) |
| 10: Strip SP FF (10µg) | 11: Load Q FF (8.9µg) | 12: Elution Q FF (9.8µg) |
| 13: Strip Q FF (4.8µg) | 14: TFF retentate before0.04% TWEEN™ 80 spiked (10µg) | |
| 15: Purified bulk Not filtered 0.04% TWEEN™ 80 spiked (10µg) | | |
| 16: Purified bulk Sterile Filtered 0.04% TWEEN™ 80 spiked (10µg) | | |
| 17: Purified bulk Sterile Filtered 0.04% TWEEN™ 80 spiked (20µg + spiked E. Coli Cell lysate Rix (1µg)) | | |
| 18: E. Coli Cell lysate Rix (2µg) | | |
| 19: E. Coli Cell lysate Rix (1µg) | | |
| 20: E. Coli Cell lysate Rix (0.5µg) | | |

See Figure 14 for a SDS-PAGE of In-process samples from purification process of PE-PilA fusion protein.

For Western Blot, proteins were transferred at 4°C overnight at 30Volts in NUPAGE^{®} transfer buffer + 20% Methanol, 0.1 % SDS on nitrocellulose membrane. Membranes were blocked 1 hour with 50mM Tris, 150mM NaCl pH 7.4 + 5% non-fat dry milk, incubated 2 hours in rabbit polyclonal primary antibody diluted in blocking buffer (anti-Prot-E 1/50 000 and anti-Ecoli (BLR) 1/1 000), washed 3x5minutes in 50mM Tris pH 7.4 + 0.05% Tween 20, incubated 1 hour in secondary antibody (goat anti-rabbit conjugated to alkaline phosphatase diluted 1/5000 in blocking buffer), washed 3x5minutes in wash buffer and developed in BCIP/NBT substrate (1 tablet per 10ml). All incubations performed in 25ml per membrane.

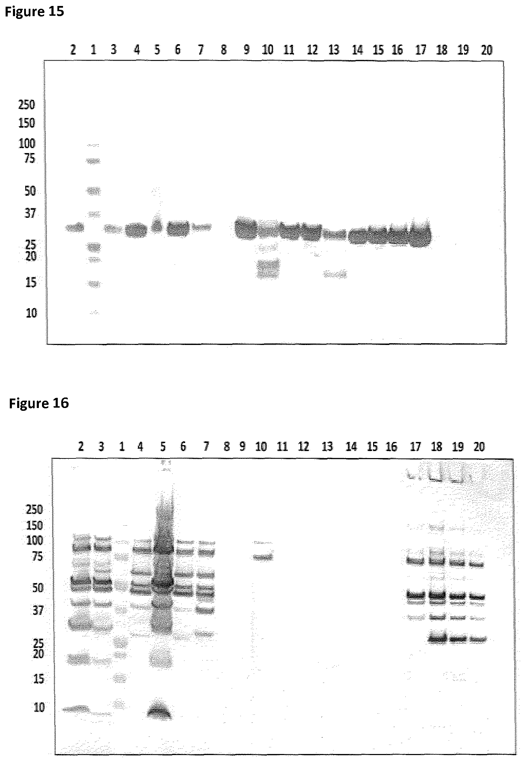

See Figure 15 for a Western Blot of In-process samples of purification process from PE-PilA fusion protein. Blot using rabbit polyclonal anti-PE.

**Lane contents:**

| | | |
|---|---|---|
| 1: MW standard (10µL) | 2: Start (total fraction) (10µg) | 3: SN1 non filtered (10µg) |
| 4: SN2 not filtered (10µg) | 5: Not extracted (10µg) | 6: Load SP FF (10µg) |
| 7: Flow through SP FF (6.9µg) | 8: Wash SP FF (20µL) | 9: Elution SP FF (10µg) |
| 10: Strip SP FF (10µg) | 11: Load Q FF (8.9µg) | 12: Elution Q FF (9.8µg) |
| 13: Strip Q FF (4.8µg) | 14: TFF retentate before0.04% TWEEN™ 80 spiked (10µg) | |
| 15: Purified bulk Not filtered 0.04% TWEEN™ 80 spiked (10µg) | | |
| 16: Purified bulk Sterile Filtered 0.04% TWEEN™ 80 spiked (10µg) | | |
| 17: Purified bulk Sterile Filtered 0.04% TWEEN™ 80 spiked (20µg + spiked E. Coli Cell lysate Rix (1µg)) | | |
| 18: E. Coli Cell lysate Rix (2µg) | | |
| 19: E. Coli Cell lysate Rix (1µg) | | |
| 20: E. Coli Cell lysate Rix (0.5µg) | | |

See Figure 16 for a Western Blot of In-process samples of purification process from PE-PilA fusion protein. Blot using rabbit polyclonal anti-*E.coli* (BLR).

**Lane contents:**

| | | |
|---|---|---|
| 1: MW standard (10µL) | 2: Start (total fraction) (10µg) | 3: SN1 non filtered (10µg) |
| 4: SN2 not filtered (10µg) | 5: Not extracted (10µg) | 6: Load SP FF (10µg) |
| 7: Flow through SP FF (6.9µg) | 8: Wash SP FF (20µL) | 9: Elution SP FF (10µg) |
| 10: Strip SP FF (10µg) | 11: Load Q FF (8.9µg) | 12: Elution Q FF (9.8µg) |
| 13: Strip Q FF (4.8µg) | 14: TFF retentate before0.04% TWEEN™ 80 spiked (10µg) | |
| 15: Purified bulk Not filtered 0.04% TWEEN™ 80 spiked (10µg) | | |
| 16: Purified bulk Sterile Filtered | 0.04% TWEEN™ 80 spiked (10µg) | |
| 17: Purified bulk Sterile Filtered 0.04% TWEEN™ 80 spiked (20µg + spiked E. Coli Cell lysate Rix (1µg)) | | |
| 18: E. Coli Cell lysate Rix (2µg) | | |
| 19: E. Coli Cell lysate Rix (1µg) | | |
| 20: E. Coli Cell lysate Rix (0.5µg) | | |

**SDS-PAGE and Western Blot figures comments**: The PE-PilA fusion protein migrates at 30kDa. The extraction by osmotic shock extracts the fusion protein expressed and processed in bacteria periplasm and reduced contamination from bacteria. Small loss of fusion protein during hypertonic treatment (lane 3). A small proportion is not extracted by hypotonic treatment and remains associated with cells (lane 5). Small loss in SP FF Flow through (lane 7) and in strip fraction of both columns (lanes 10 and 13). Since the total volume of strip fraction is low the loss of fusion protein is not significant. Degraded bands are visible in strip fractions but not in final product. No significant contamination from *E. coli* host cell proteins in purified bulk (lane 16).

Analysis of LVL735 and LVL778 yielded similar profiles as LVL317.

### Example 11: Melting Point Data for PE, PilA and LVL317

Thermal transition of PE-PilA fusion non His-tagged protein (LVL317) was compared with the thermal transition of both PE his-tagged (as described in Example 8) and cleaved PilA (as described in Example 8) proteins, purified as described above.

Before DSC, PE and PilA were dialyzed overnight in 10mM K₂HPO₄/KH₂PO₄ pH 6.5 + 0.04% Tween 80 (1:250 sample:buffer volume ratio) to have them in the same buffer as the fusion protein. After dialysis, proteins concentration was measured by BCA and adjusted to 300µg/ml (PE) and 500µg/ml (PilA).

Analysis done on VP™-DSC from MicroCal, LLC (part of GE Healthcare). The final dialysis buffer was used as reference and subtracted from the scans. DSC scan rate 90°C/hr. In order to evaluate the capacity to measure the thermal transition in the Final Container (FC) after formulation, the fusion protein was diluted to the FC concentration (60µg/ml). Final container data not shown.

### Results:

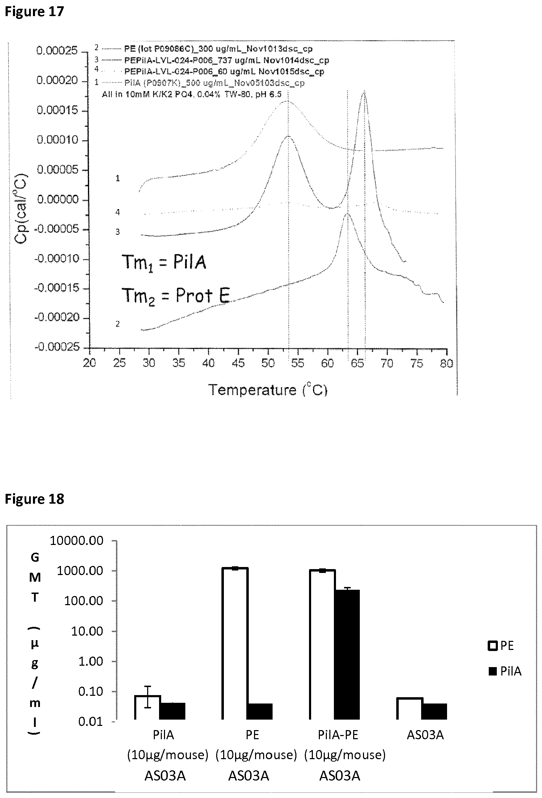

See Figure 17 for Thermal transition of PE-PilA fusion protein and PE and PilA proteins.
Curves: PilA (1), Protein E (Prot E, PE) (2), PE-PilA PB not diluted 737µg/ml (3), and PE-PilA PB diluted at FC concentration 60µg/ml (4).

| | |
|---|---|
| 1 - PilA | Tm: 53°C |
| 2 - Protein E | Tm: 63 |
| 3 - PE-PilA PB (Purified Bulk) not diluted 737µg/ml | Tm₁ : 53.7°C and Tm₂ : 66.1°C |
| 4 - PE-PilA PB diluted at FC concentration 60µg/ml | Tm1: 53.2°C and Tm2: 67.6°C |

Two transitions were detected in the purified fusion protein (LVL317) (curves 3 and 4).

The Tm₁ (53.7°C) of the PE-PilA fusion protein is similar to PilA transition (53°C).

Significant shift of Tm₂ in PE-PilA (66.1°C) as compared to PE transition (63°C). The fusion of both domains seems to stabilize the PE fragment.

The shift of Tm₂ in the diluted fusion protein as compared to undiluted is a concentration artifact arising from the steep decreasing slope typical of aggregation which is concentration dependant.

Antigen folding analysis of LVL735 and LVL778 were similar to that of LVL317.

### Example 12: PE-PilA fusion protein construct LVL291 anti-PilA immunogenicity response in Balb/c mice.

The immune response directed against purified LVL291 PE-PilA fusion protein (the LVL291 fusion protein without the heterologous signal peptide) formulated in AS03_{A} was evaluated in Balb/c mice. Animals (20 mice/group) were immunized by the intramuscular route at days 0, 14 and 28 with 10 µg of PE (from vector pRIT16762), PilA (from vector pRIT16790) or PE-PilA, each formulated in AS03_{A}. The control group was vaccinated with AS03_{A} alone. Antibody response directed against each antigen was determined in individual sera collected at day 42. No antibody response was obtained with the negative control. As shown in Figure 18, the antibody response directed against PilA was higher in mice immunized with the PE-PilA fusion compared to antibody response in mice immunized with monovalent PilA. The antibody responses directed against PE were similar in mice immunized with the fusion protein and mice immunized with monovalent PE. GMT = geometric means titer. Data were captured and analyzed with the SOFTMAX^{®} Pro Software (Molecular Devices) running under WINDOWS^{®} (Microsoft); the four parameters logistic log function was used to calculate the standard curve. The four-parameter logistic-log function describes, with a high degree of accuracy, the curve of the reference serum displaying a pronounced sigmoïdal shape when plotted on an optical density-versus-concentration (log) scale. Antibody concentrations were calculated at each dilution of mice serum samples by interpolation of the standard curve. The antibody in quality control sera and in unknown serum samples is obtained by averaging the values from all dilutions that fall within the working range (10-80 %) of the dilution curve of the reference. Results are shown in Figure 18, which graphs the antibody responses against LVL291 PE-PilA fusion protein and against monovalent PE and PilA in the Balb/c mouse model.

### Example 13: Murine nasopharyngeal colonization model. Immunization with PE-PilA. Challenge with NTHi strain 86-028NP and NTHi strain 3224A.

Balb/c female mice (20/group) were immunized intranasally at days 0 and 14 with 6µg of a purified PE-PilA fusion protein (LVL291 for challenge with 86-028NP; LVL317 for challenge with strain 3224A) formulated with LT (heat labile toxin of *Escheria coli*) and on day 28 with 6 µg of a purified PE-PilA fusion protein in phosphate buffered saline (PBS). Control mice (20/group) were vaccinated with LT alone. Mice were subsequently challenged intranasally with 5 x 10⁶ CFU (colony forming units) of homologous NTHi strain 86-028NP and heterologous NTHi strain 3224A. Homology and heterology are determined by reference to the NTHi strain with which the mice were immunized. Bacterial colonies were counted in nasal cavities removed 1 and 2 days after the challenge. D1 = day 1. D2 = day 2. PE-PilA vaccination increased the clearance of NTHi strain 86-028NP and strain 3224A in the nasopharynx at day 1 and day 2 post challenge.

For the experiment performed with NTHi strain 86-028NP: A 2-way fixed ANOVA was performed using the log10 values of the counts as response, the fixed factors being the group (4 levels) and the day (2 levels). The assumption of variance heterogeneity was rejected and a model with heterogeneous variances was fitted to the data. No significant interaction was detected between the 2 factors. The group fusion PE-PilA (6 µg per mouse) significantly reduced CFU compared with the control group (LT); the geometric mean ratio being equal to 0.06 with a 95% confidence interval of 0.01, 0.25.

For the experiment conducted with NTHi strain 3224A: A 3-way fixed ANOVA was performed using the log10 values as response, the fixed factors being the group, the day, and the experiment. The Shapiro-Wilk and Levene's test did not reject the assumptions of normality and of homogeneity of variances. No significant interaction between any of the 2 factors or between the 3 factors was detected and only main factors were kept in the analysis. PE-PilA / LT significantly reduced CFU compared with the control group; the geometric mean ratio being equal to 0.11 with a 95% confidence interval of 0.02, 0.61.

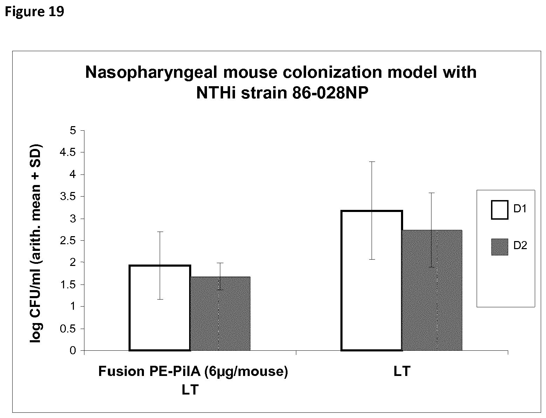

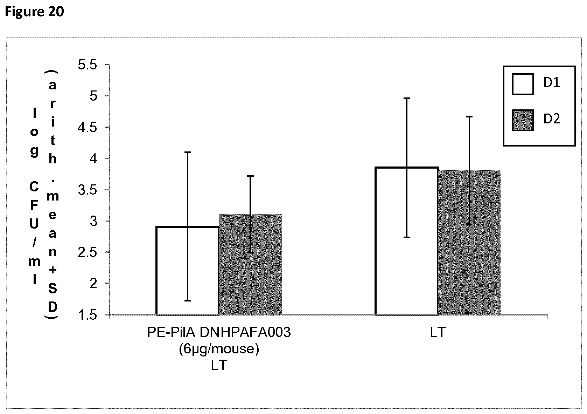

See Figure 19 for effect of PE-PilA fusion protein vaccination on NTHi strain 86-028NP bacterial clearance in mouse nasopharynx.
See Figure 20 for effect of PE-PilA fusion protein vaccination on NTHi strain 3224A bacterial clearance in mouse nasopharynx.

### Example 14: Murine nasopharyngeal colonization model. Immunization with PilA. Challenge with NTHi strain 3219C.

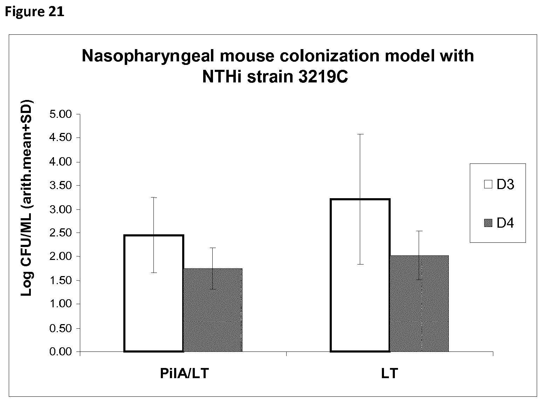

Female OF1 mice (20 mice/group) were immunized intranasally at days 0 and 14 with 3µg PilA (from vector 16790) formulated with LT and at day 28 with 3 µg PilA in PBS. Control mice were vaccinated with LT alone. Mice were subsequently challenged intranasally with 5 x 10⁶ CFU of NTHi strain 3219C. Bacterial colonies were counted in nasal cavities removed 3 and 4 days after the challenge. D3 = day 3. D4 = day 4.
See Figure 21 for effect of PilA vaccination on bacterial clearance in mouse nasopharynx.

### Example 15: Murine nasopharyngeal colonization model. Immunization with PE. Challenge with NTHi strain 3224A.

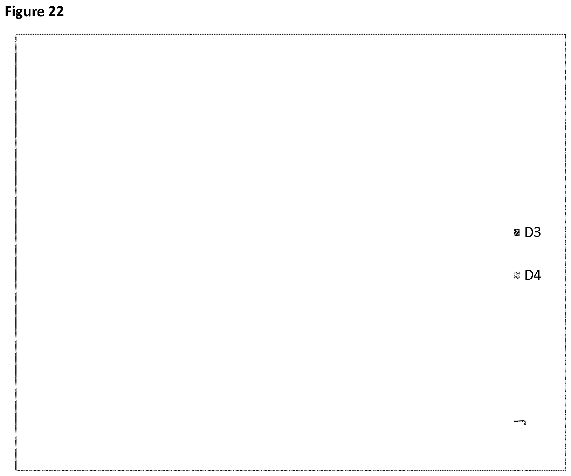

Balb/c female mice (20 mice/group) were immunized intranasally at days 0 and 14 with 3µg PE (from vector pRIT16762) formulated with LT and at day 28 with 3 µg PE in PBS. Control mice were vaccinated with LT alone. Mice were subsequently challenged intranasally with 5 x 10⁶ CFU of NTHi strain 3224A. Bacterial colonies were counted in nasal cavities removed 3 and 4 days after the challenge. 10 mice were examined on day 3 (D3). 10 mice were examined on day 4 (D4). PE vaccination increased significantly the clearance of NTHi in the naso-pharynx at day 4 post challenge (Figure 22), using on the Dunn test for statistical analysis.

See Figure 22 for effect of PE vaccination on bacterial clearance in the nasopharynx of mice.

### Example 16: Vibronectin binding. Inhibition of vibronectin binding by LVL317 & LVL735 PE-PilA fusion protein.

The ability of PE in the purified LVL317 PE-PilA fusion protein construct to bind to vitronectin was evaluated. Microtiter plates (POLYSORP™, Nunc, Thermo Fisher Scientific) were coated with PE (from vector pRIT16762) or with purified LVL317 PE-PilA fusion protein (10 µg/ml). Plates were washed four times with NaCl 150mM-polysorbate 20, 0.05% (for example, TWEEN™ 20) and blocked for one to two hours with PBS-BSA 1 %. After four washings, vitronectin (Vitronectin from human plasma, SIGMA-ALDRICH^{®}) was added (10 µg/ml), two fold diluted (12 dilutions), and the plates were incubated for 1h at room temperature. The plates were then washed 4 times with NaCl 150mM-polysorbate 20, 0.05% (for example TWEEN™ 20) After washings, the bound vitronectin was detected using peroxydase sheep anti-human vitronectin (US Biological) followed by the addition of ortho-phenylene diamine/H₂O₂ substrate. The color developed is directly proportional to the amount of antibody fixed to the vitronectin.

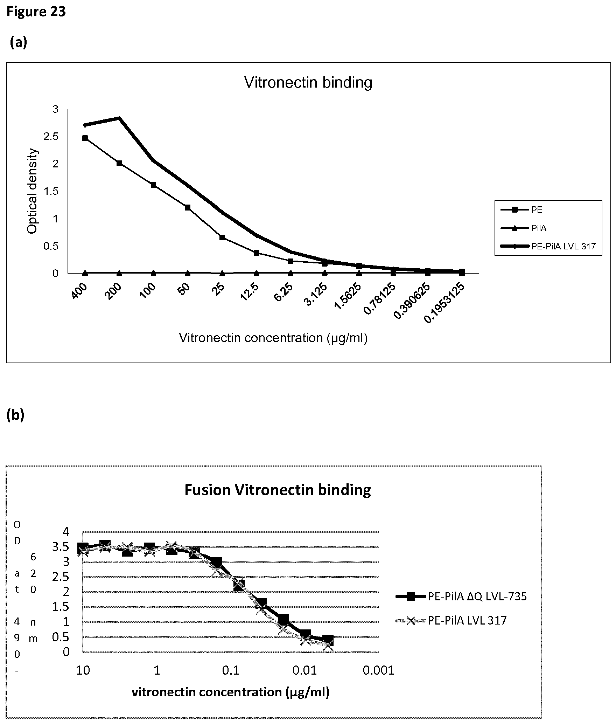

See Figure 23 for (a) LVL317 PE-PilA fusion protein bound to vitronectin. PilA = PilA from NTHi strain 86-028NP (as described for pRIT16790); PE = Protein E (as described for pRIT16762) and (b) LVL317 and LVL735 PE-PilA fusion protein bound to vitronectin.

### Example 17: Vibronectin binding. Inhibition of vibronectin binding by antibodies directed against the LVL291 PE-PilA fusion protein.

Microtiter plates (POLYSORP™, Nunc, Thermo Fisher Scientific) were coated with PE (from vector pRIT16762) or with purified PE-PilA fusion protein (10 µg/ml). Plates were washed four times with NaCl 150mM-polysorbate 20, 0.05% (for example, TWEEN™ 20) and blocked for two hours with PBS-BSA 1%. After washings, vitronectin (Vitronectin from human plasma, SIGMA-ALDRICH^{®}) was added at 50µg/ml and purified antibodies anti-PE-PilA (produced and purified in house) were two-fold serially diluted and incubated for 1h at room temperature. The plates were then washed 4 times with NaCl 150mM-polysorbate 20, 0.05% (for example, TWEEN™ 20). After four washings, the bound vitronectin was detected using peroxydase sheep anti-Vitronectin (US Biological) followed by the addition of ortho-phenylene diamine/H₂O₂ substrate. The color developed is directly proportional to the amount of antibody fixed to the vitronectin.

Inhibition of vitronectin binding to PE by polyclonal antibodies directed against PE-PilA was observed.

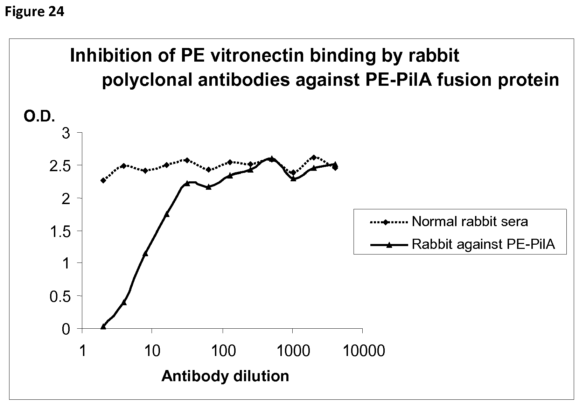

See Figure 24 for inhibition of vitronectin binding by polyclonal antibodies against PE-PilA fusion protein.

### Example 18: Antigenicity of LVL291 PE-PilA fusion protein. ELISA.

Purified LVL291 PE-PilA fusion protein was validated in an antigenicity test with monovalent proteins as control. The fusion protein was tested in a sandwich ELISA developed with polyclonal antibodies (rabbit and guinea pig) generated against the PE gene fragment coding for amino acids 22 to 160 of SEQ ID NO: 4 (as described for pRIT16711) or against PilA from NTHi strain 86-028NP (from vector pRIT16790).

PilA or PE was added at 100 ng/ml and serially two fold diluted. After 30 minutes incubation and after washing, the bound antigen was detected by a rabbit polyclonal serum obtained after immunisation with PE or PilA. The bound antibodies were detected using a peroxydase anti-rabbit lg (Jackson ImmunoResearch Laboratories, Inc.) followed by the addition of ortho-phenylene-diamine/H₂O₂ substrate. The color developed is directly proportional to the amount of antigen present. Absorbance readings were measured using a spectrophotometer for microtiter plates. The antigenicity of the samples was determined by comparison to the curve of the full length PE or full length PilA reference antigen and is expressed in ug/ml. The reference represented 100% of antigenicity.

As observed in the Table 6: Antigenicity was observed with the purified LVL291 PE-PilA fusion protein compared to the monovalent PE and PilA antigens.

**Table 6 : Relative antigenicity obtained with purified LVL291 PE-PilA fusion protein in the antigenicity test.**

| | **PE relative antigenicity (%)** |
|---|---|
| Protein E as Reference | 100 |
| PE-PilA | 130-148 |
| | |

| | **PE relative antigenicity (%)** |
|---|---|
| PilA as Reference | 100 |
| PE-PilA | 120-152 |

### Example 19: Immunogenicity of LVL735 PE-PilA fusion protein.

Female Balb/c mice (n = 34) were immunized by the intramuscular route at days 0, 14 and 28 with 50 µl of vaccine formulation containing 1, 0.2 or 0.04 µg of PE-PilA fusion protein LVL317 or LVL735 formulated within AS01_{E} or AlPO₄ (aluminium phosphate). The antibody responses to PE and PilA were determined in individual sera collected at day 42 and the IgG level against PE and PilA was measured and expressed in µg /ml.

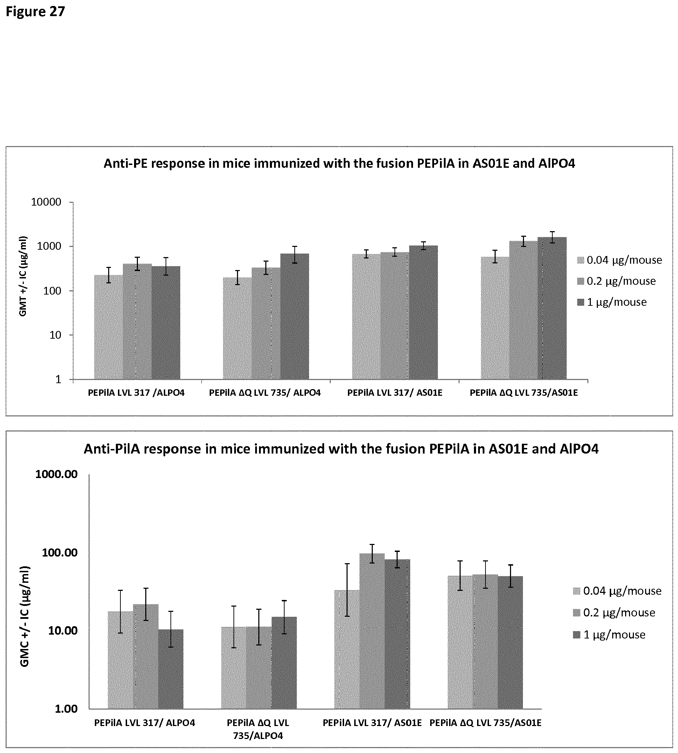

See Figure 27 for PE and PilA antibody response to LVL317 and LVL735. GMC= geometric mean concentration. GMT = geometric means titer. IC = confidence intervals.

### Example 20: Protective efficacy of the LVL735 and LVL317 fusion proteins in a mouse model of Non-typeable Haemophilus influenzae nasopharyngeal colonization.

Female Balb/c mice were intranasally immunized at days 0 and 14 with 10 µl of vaccine formulation containing 5.8 µg of LVL735 or LVL317 admixed with 0.5 µg of *E. coli* labile toxin (LT). A booster dose of 5.8 µg of non-adjuvanted LVL735 or LVL317 was administered at day 28. Control mice were vaccinated with LT alone at days 0 and 14, and PBS at day 28. Animals were intranasally challenged with 5 x 10⁶ cfu of NTHi 3224A strain at day 42. Bacterial colonies were counted in nasal cavities removed 1 and 2 days after the challenge (n = 10/time-point).
Nasal cavities are homogenized in medium and a bacterial quantification is performed. Results are well expressed in CFU/ml.

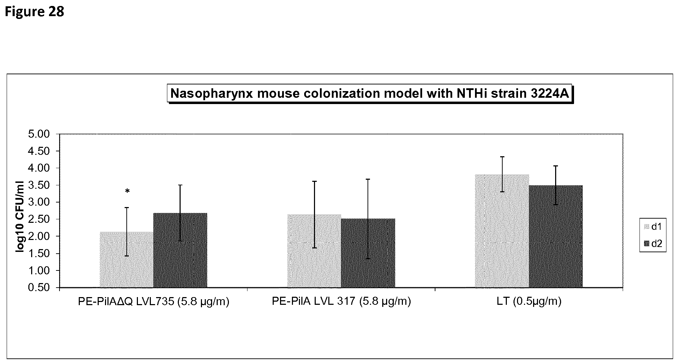

See Figure 28 for the effect of LVL735 and LVL317 vaccination on bacterial clearance in a mouse model of non-typeable *Haemophilus influenzae* nasopharyngeal colonization.

### Example 21: Formulation of multivalent vaccines comprising a PE-PilA fusion protein

3 vaccines were designed:
**10V**: A ten valent (10V) vaccine containing the following ten S. *pneumoniae* capsular saccharide conjugates: capsular saccharide from serotype 1 conjugated to protein D (1-PD), capsular saccharide from serotype 4 conjugated to protein D (4-PD), capsular saccharide from serotype 5 conjugated to protein D (5-PD), capsular saccharide from serotype 6B conjugated to protein D (6B-PD), capsular saccharide from serotype 7F conjugated to protein D (7F-PD), capsular saccharide from serotype 9V conjugated to protein D (9V-PD), capsular saccharide from serotype 14 conjugated to protein D (14-PD), capsular saccharide from serotype 23F conjugated to protein D (23F-PD), capsular saccharide from serotype 18C conjugated to tetanus toxoid (18C-TT) and capsular saccharide from serotype 19F conjugated to Diphtheria Toxin (19F-DT).
**12V**: A twelve valent (12V) vaccine containing the same ten *S*. *pneumoniae* capsular saccharide conjugates as 10V with an additional two *S*. *pneumoniae* saccharide conjugates, 19A conjugated to CRM197 (19ACRM) and 6A conjugated to CRM197 (6ACRM).
**12V+ proteins (12V+prot):** A vaccine containing the same twelve *S*. *pneumoniae* capsular saccharide conjugates as 12V with the addition of PhtD, dPly and PE-PilA fusion protein.

**Preparation of dPly:** Pneumococcal pneumolysin was prepared and detoxified as described in WO2004/081515 and WO2006/32499 using formaldehyde detoxification.

### Expression and purification of PhtD:

**EXPRESSION OF PhtD**: The PhtD protein is a member of the pneumococcal histidine-triad (Pht) protein family characterized by the presence of histidine-triads. PhtD is a 838 aa- molecule and carries 5 histidine triads (see Medlmmune WO00/37105 SEQ ID NO: 4 for amino acid sequence and SEQ ID NO: 5 for DNA sequence). PhtD also contains a proline-rich region in the middle (amino acid position 348-380). PhtD has a 20 aa-N-terminal signal sequence. Preparation and purification of PhtD is described in WO2007/071710 (see, for example, Example 1b). The sequence of amino acids 21-838 of SEQ ID NO:4 from WO00/37105 corresponds to SEQ ID NO:220.
SEQ ID NO:220

### Expression of protein D

Protein D was expressed as described in WO2007/071710

### Expression and purification of CRM197 E. coli:

In order to improve the yield of process for the production of CRM197, alternative expression modes were evaluated with a target of a 10-fold increase of process yield. The selected construction was expressed in *Escherichia coli* strain (B834 (DE3)) as a fusion between Flgl signal sequence from *E*. *coli* (19aa) and CRM197 (537aa). The signal sequence is cleaved upon transport to the periplasm. The CRM197 is extracted by osmotic shock before being purified. The purification process is similar to the one disclosed in WO2006/100108 except that an additional chromatographic step (Phenyl Sepharose) was added between the Q-Sepharose-XL and hydroxyapatite steps and the last chromatographic step on the octyl-Sepharose 4FF was removed.

### Preparation of conjugates

It is well known in the art how to make purified pneumococcal polysaccharides. For the purposes of these examples the polysaccharides were made essentially as described in EP072513 or by closely-related methods. Before conjugation the polysaccharides may be sized by microfluidisation as described below.

The activation and coupling conditions are specific for each polysaccharide. These are given in Table 1. Sized polysaccharide (except for 6B and 23F) was dissolved in NaCl 2M, NaCl 0.2M or in water for injection (WFI). The optimal polysaccharide concentration was evaluated for all the serotypes. All serotypes except serotype 18C were conjugated directly to the carrier protein as detailed below.

From a 100 mg/ml stock solution in acetonitrile or acetonitrile/water (50%/50%) solution, CDAP (1-cyano-4-dimethylaminopyridinium tetrafluoroborate) (CDAP/PS ratio 0.5-1.5 mg/mg PS) was added to the polysaccharide solution. 1.5 minute later, 0.2M-0.3M NaOH was added to obtain the specific activation pH. The activation of the polysaccharide was performed at this pH for 3 minutes at 25 °C. Purified protein (protein D, CRM197 or DT) (the quantity depends on the initial PS/carrier protein ratio) was added to the activated polysaccharide and the coupling reaction was performed at the specific pH for up to 2 hour (depending upon serotype) under pH regulation. In order to quench un-reacted cyanate ester groups, a 2M glycine solution was then added to the mixture. The pH was adjusted to the quenching pH (pH 9.0). The solution was stirred for 30 minutes at 25 °C and then incubatedovernight at 2-8 °C with continuous slow stirring.

### Preparation of 18C:

18C was linked to the carrier protein via a linker - Adipic acid dihydrazide (ADH)
Polysaccharide serotype 18C was microfluidized before conjugation.

### Derivatization of tetanus toxoid with EDAC (2-Ethyl-3-(3-dimethylaminopropyl) carbodiimide hydrochloride)

For derivatization of the tetanus toxoid, purified TT was diluted at 25 mg/ml in 0.2M NaCl and the ADH spacer was added in order to reach a final concentration of 0.2M. When the dissolution of the spacer was complete, the pH was adjusted to 6.2. EDAC (1-ethyl-3-(3-dimethyl-aminopropyl) carbodiimide) was then added to reach a final concentration of 0.02M and the mixture was stirred for 1 hour under pH regulation. The reaction of condensation was stopped by increasing pH up to 9.0 for at least 30 minutes at 25°C.

Derivatized TT was then diafiltrated (10 kDa CO membrane) in order to remove residual ADH and EDAC reagent.

TT_{AH} (Tetanus toxoid conjugated to an ADH linker) bulk was finally sterile filtered until coupling step and stored at -70°C.

### Chemical coupling of TT_{AH} to PS 18C

Details of the conjugation parameters can be found in Table 1.

2 grams of microfluidized PS were diluted at the defined concentration in water and adjusted to 2M NaCl by NaCl powder addition.

CDAP solution (100 mg/ml freshly prepared in 50/50 v/v acetonitrile/WFI) was added to reach the appropriate CDAP/PS ratio.

The pH was raised up to the activation pH 9.0 by the addition of 0.3M NaOH and was stabilised at this pH until addition of TT_{AH}.

After 3 minutes, derivatized TT_{AH} (20 mg/ml in 0.2 M NaCl) was added to reach a ratio TT_{AH} /PS of 2; the pH was regulated to the coupling pH 9.0. The solution was left one hour under pH regulation.

For quenching, a 2M glycine solution, was added to the mixture PS/TT_{AH}/CDAP.

The pH was adjusted to the quenching pH (pH 9.0).

The solution was stirred for 30 min at 25 °C, and then overnight at 2-8°C with continuous slow stirring.

### Specific activation/coupling/quenching conditions of S. pneumoniae capsular saccharide-Protein D/TT/DT/PhtD/Ply conjugates

Where "**µfluid**" appears in a row header, it indicates that the saccharide was sized by microfluidisation before conjugation. Sizes of saccharides following microfluidisation are given in table 2.

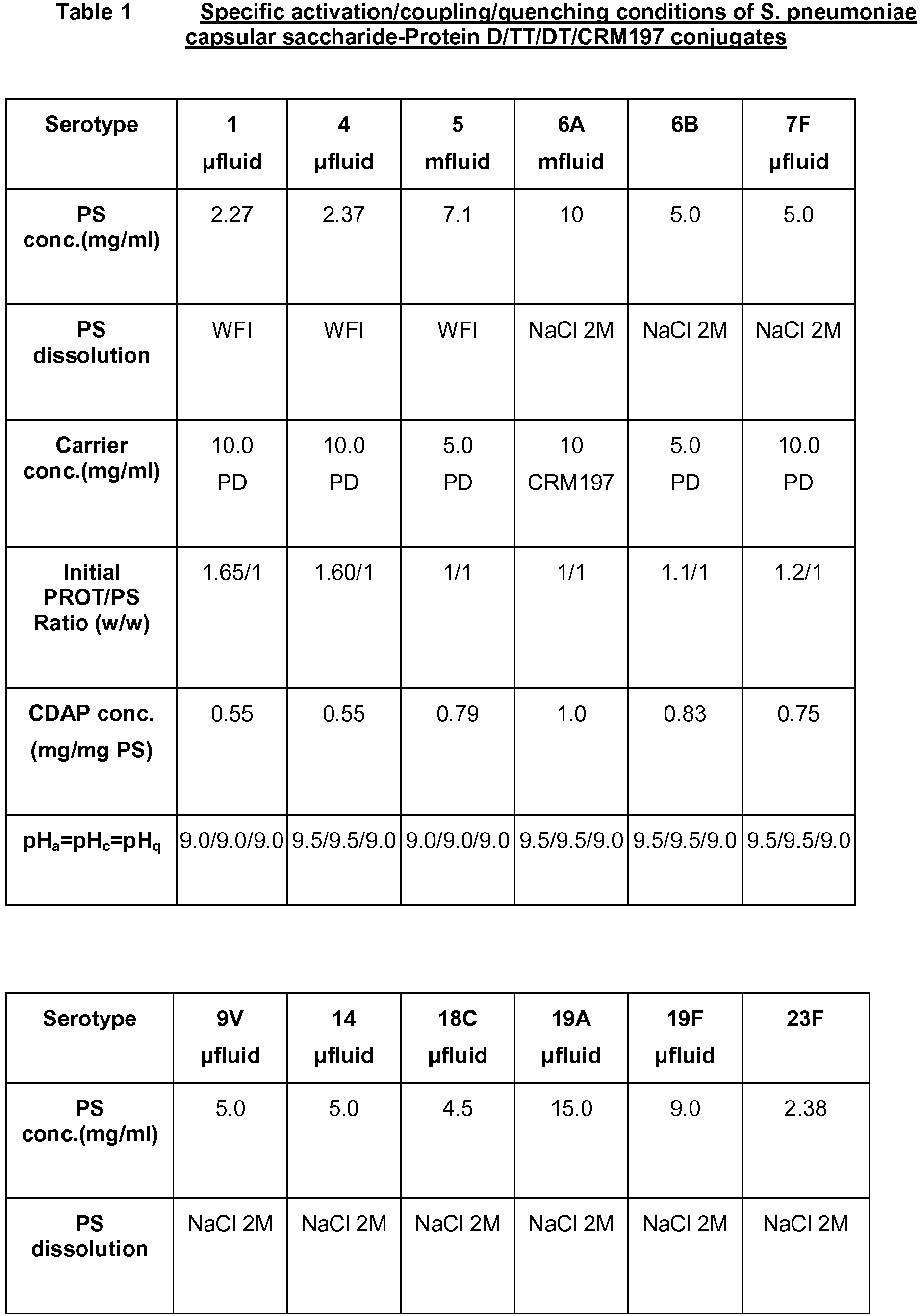

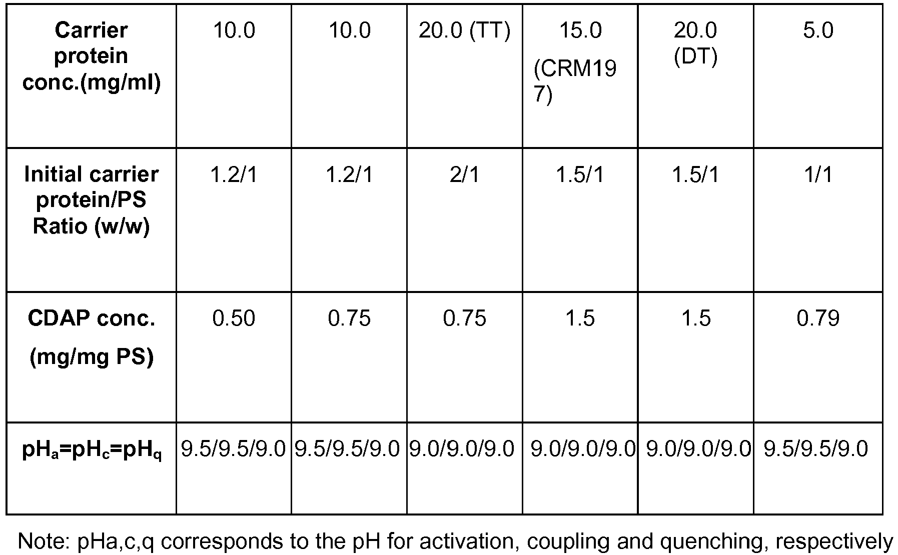

### Purification of the conjugates:

The conjugates were purified by gel filtration using a Sephacryl S400HR gel filtration column equilibrated with 0.15M NaCl (except S500HR was used as buffer for 18C and 20mM acetate containing 1.15MNaCl pH6.2 was used for 19A) to remove small molecules (including DMAP) and unconjugated saccharide and protein. Based on the different molecular sizes of the reaction components, PS-PD, PS-TT, PS-CRM197 or PS-DT conjugates are eluted first, followed by free PS, then by free protein carrier and finally DMAP and other salts (NaCl, glycine).

Fractions containing conjugates are detected by UV_{280 nm}. Fractions are pooled according to their Kd, sterile filtered (0.22µm) and stored at +2-8°C. The PS/Protein ratios in the conjugate preparations were determined.

### Formulation of the vaccines

The 10V vaccine contains *S*. *pneumoniae* capsular saccharide serotype 1, 4, 5, 6B, 7F, 9V, 14, 18C, 19F and 23F conjugates adsorbed onto aluminium phosphate together at a human dose of 1, 3, 1, 1, 1, 1, 1, 3, 3, 1 µg (the saccharides were individually adsorbed to aluminium phosphate, they were then mixed together and the level of aluminium phosphate adjusted to 500µg).

The 12V vaccine was made in the same way as the 10V vaccine with additional serotypes 19A and 6A conjugates at doses of 2µg adsorbed onto aluminium phosphate added.

The 12V + proteins vaccine was made in the same way as the 12V vaccine except proteins PhtD, dPly and PE-PilA were added. The PE-PilA was produced as described herein. The 12 conjugates and the proteins were mixed together using the dosages described for 12V above and the proteins at dosages of 30µg each (note that this refers to 30µg of PE-PilA, not 30µg of PE and 30µg of PilA).

### Example 22: Comparison of the immunogenicity of 10V, 12V and 12V+proteins vaccines in mice

### Description of the anti-pneumococcal polysaccharide PS (polysaccharide) ELISA

Microplates were coated for 2 hours at 37°C with capsular polysaccharide (CPS) (100 µl per well of 2.5 µg/ml of PS1 and PS3, 5 µg/ml of PS4, 5, 6A, 6B, 7F, 9V or 14; 10 µg/ml of PS19A and 23F or 40 µg/ml of PS18C and PS19F in PBS). The plates were washed three times with NaCl 150 mM (0.9%) -Polysorbate 20 0.05%. Sera was diluted (1/2 for 6A and 6B and 1/10 for the other serotypes) in PBS-Polysorbate20 0.05% containing CPS (1 mg CPS/ml of non diluted serum except or 6A and 6B which was at 2.5mg/ml) V/V and incubated for 1 hour at 37°C in order to neutralize antibodies directed to the CPS. The sera from mice immunised as described in the section entitled 'immunogenicity of three vaccine formulations in mice' or a reference (an internal reference calibrated with Chrompure mouse IgG) was added to the microwells and serially diluted 100 µl (two-fold dilution step) in PBS-Polysorbate20 0.05%. The plates were incubated under agitation for 30 minutes at room temperature. The plates were washed as above and anti-mouse IgG antibodies conjugated to peroxidase (100 µl per well) was added, the plates were incubated for 30 minutes at room temperature with shaking. After washing, the substrate (4 mg of OPDA (ortho phenylen-diamine) in 10 ml of citrate 0.1M pH 4.5-4.6 and 5 µl of H₂O₂) was added to each well (100 µl) and the plates incubated for 15 minutes in the dark. The reaction was stopped by addition of HCl 1N (50µl). The absorbance was read at 490nm or 620nm for the reference using a spectrophotometer. The color developed is directly proportional to the amount of antibody present in the serum.

### Description of the ELISA to measure PD, PE and PilA antibodies

Plates were coated overnight at 4°C with 100 µl per well of 2 µg/ml of PD (1 mg/ml), 2 µg/ml of PE (1500 µg/ml), 2 µg/ml of PilA (3660 µg/ml) in carbonate buffer pH 9.6. The plates were washed four times with NaCl 0.9% Polysorbate 20 0.05%. For PE and PilA ELISA, the plates were saturated for 30 min at room temperature (with shaking) with PBS-BSA1%. After washing, sera from mice immunised as described in the section entitled 'immunogenicity of three vaccine formulations in mice' or a reference (an internal reference calibrated with Chrompure mouse IgG) was added to microwells and serially diluted 100 µl (two-fold dilution step) in PBS Polysorbate 20 0.05% (for the PD assay) and PBS Polysorbate 20 0.05% BSA 0.1 % (for the PE and PilA assay). The plates were then incubated for 30 min at room temperature with agitation. After washing, the plates were incubated with an anti-mouse IgG antibody conjugated to peroxidaseperoxidase (100 µl per well) at room temperature for 30 minutes with shaking. The plates were then washed as above and the substrate conjugate (4 mg of OPDA (ortho phenylen-diamine) in 10 ml of citrate 0.1M pH 4.5-4.6 and 5 µl of H₂O₂) as added to each well (100 µl) for 15 min in darkness. The reaction was stopped by addition of HCl 1N 50 µl and the absorbance was read at 490 nm (620 nm for the reference).

### Description of the ELISAto measure PhtD and dPly antibodies

Plates were coated for 2 hours at 37°C with 100 µl per well of 1 µg/ml of PhtD (1021 µg/ml) or 4µg/ml of Ply (367µg/ml). The plates were then washed three times with NaCl 0.09% Polysorbate 0.05%. After washing, sera from mice immunised as described in the section entitled 'immunogenicity of three vaccine formulations in mice' or a reference (an internal reference calibrated with Chrompure mouse IgG) (was added to microwells and serially diluted 100 µl (two-fold dilution step) in PBS Polysorbate 20 0.05%. The plates were incubated for 30 min at room temperature with agitation. After washing, the plates were incubated with an anti-mouse IgG antibody conjugated to peroxidase (100 µl per well) at room temperature for 30 minutes with shaking. The plates were washed as above and the substrate conjugate (4 mg of OPDA in 10 ml of citrate 0.1 M ph 4.5 and 5 µl of H₂O₂) was added to each well (100 µl) for 15 min in darkness. The reaction was stopped by addition of HCl 1N 50 µl and the absorbance was read at 490 nm (620 nm for the reference filter).

### Description of opsonophagocytosis assay (OPA)

Serum samples were heated for 45 min at 56 °C to inactivate any remaining endogenous complement. Twenty-five microlitres aliquots of each 1:2 diluted serum sample were two-fold serially diluted in 25 µl OPA buffer (HBSS (Hank's Balanced Salt Solution)-14.4% inactivated FCS (Foetal Calf Serum)) per well of a 96-well round bottom microtitre plate. Subsequently, 25 µl of a mixture of activated HL-60 cells (1 × 107 cells/ml), freshly thawed pneumococcal working seed and freshly thawed baby rabbit complement in an e.g. 4/2/1 ratio (v/v/v) (except for serotypes 1, 6B and 6A for which the ratio was 4/2/2) were added to the diluted sera to yield a final volume of 50 µl. The assay plate was incubated for 2 h at 37 °C with orbital shaking (210 rpm) to promote the phagocytic process. The reaction was stopped by laying the microplate on ice for at least 1 min, the plate is kept on ice until use. A 20 µl aliquot of each well of the plate was then transferred into the corresponding well of a 96-well flat bottom microplate and 50 µl of Todd-Hewitt Broth-0.9% agar was added to each well. After overnight incubation at 37 °C and 5% CO₂, pneumococcal colonies which appeared in the agar were counted using an automated image analysis system (KS 400, Zeiss, Oberkochen, Germany). Eight wells without serum sample were used as bacterial controls to determine the number of pneumococci per well. The mean number of CFU of the control wells was determined and used for the calculation of the killing activity for each serum sample. The OPA titre for the serum samples was determined by the reciprocal dilution of serum able to facilitate 50% killing of the pneumococci. The opsonophagocytic titre was calculated by using a 4-parameter curve fit analysis.

### Immunogenicity of three vaccine formulations in mice.

2 groups of 27 female Balb/c mice distributed in 2 experiments were immunized by intramuscular (IM) injections on days 0, 14 and 28 with 1/10 human dose of different formulation including Proteins alone (PhtD, dPly and PEPilA), Prevnar 13 (_{TM} a commercially available Streptococcal vaccine - results not presented) 10V, 12V (DSP2A017) and 12V+ proteins (DSP2A012) GMP lots. Mice received in a different leg (mimicking co-administration at different sites in infants in clinical trials) 1/10^{th} human dose of *Infanrix Hexa* (_{TM} a vaccine comprising diphtheria toxoid, tetanus toxoid, pertussis toxoid, filamentous haemmagglutinin, pertactin, hepatitis B surface antigen, inactivated polio virus types 1, 2 and 3 and *Haemophilus influenzae* b saccharide (PRP)).

Anti-IgG levels and opsonophagocytosis titers were determined respectively in individual and pooled sera collected at day 42.

The potential of the 12V +proteins vaccine to induce IgG antibody titers and opsonic activity was evaluated and compared to that of the 12V and 10V vaccines.

Sera from mice injected with the different formulations were tested in ELISA (as described above) against the polysaccharide serotypes and proteins and in OPA against the 12 polysaccharide serotypes.

The 12V + proteins vaccine induced similar response to the 12V for most serotypes.

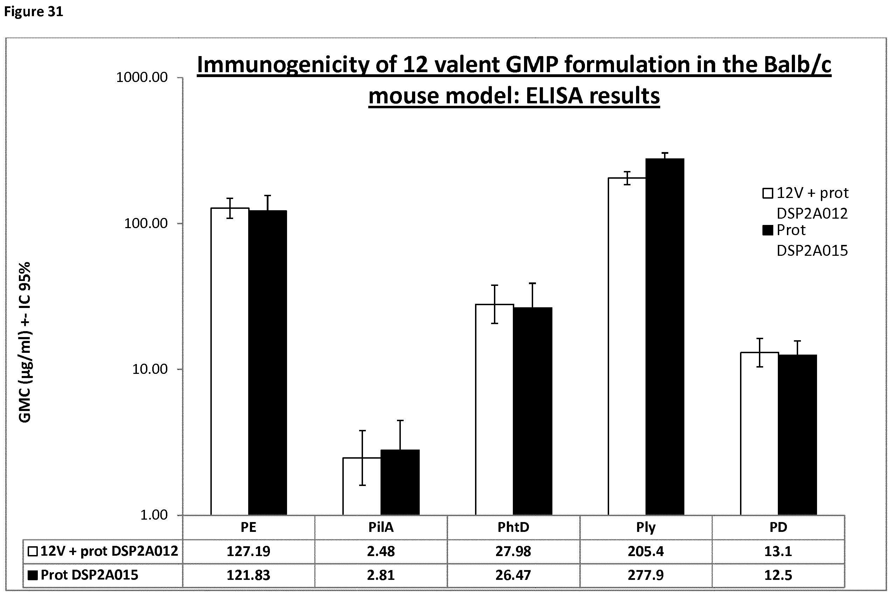

The results in Figure 31 demonstrated that there was no stastical difference between the PE, PilA, PhtD, Ply and PD antibody responses measured for the the 12V+ formulation and a formulation that did not comprises the *S*. *pneumoniae* saccharide conjugates.

### Example 23: Comparison of the immunogenicity of 10V, 12V and 12V+protein vaccines in guinea pigs

### Description of ELISA anti-pneumococcal polyssacharide PS

Microplates were coated for 2 hours at 37°C with 100 µl per well of 2.5 µg/ml of PS1, 5 µg/ml of PS4, 5, 6A, 6B, 7F, 9V or 14; 10 µg/ml of PS19A and 23F, 40 µg/ml of PS18C and PS19F or Affinipure Goat anti-guinea pig IgG(2.4mg/ml) diluted to 2µg/ml for the reference wells in PBS. The plates were washed three times with NaCl 150 mM (0.9%)-Polysorbate20 0.05%. Pooled serum from each group was diluted (1/2 for PS 6A and 6B and 1/10 for all the other serotypes) in PBS-Polysorbate20 0.05% containing CPS (1 mg CPS/ml of non diluted serum except for 6A and 6B at 2.5mg/ml) V/V and incubated for 1 hour at 37°C in order to neutralize antibodies directed to CPS. The serum from guinea pigs immunised as described in the section entitled 'immunogenicity of three vaccine formulations in guinea pigs' was added to the microwells and serially diluted 100 µl (two-fold dilution step) in PBS-Polysorbate20 0.05% or a reference (Chrompure guinea pig IgG (11mg/ml) diluted to 0.25µg/ml in PBS-polysorbate20 0.05%) was added. The plates were incubated under agitation for 30 minutes at room temperature. The plates were washed as above and anti-guinea pig IgG antibodies conjugated to peroxidase (100 µl per well) were added and the plates incubated for 30 minutes at room temperature with shaking. After washing, the substrate (4 mg of OPDA in 10 ml of citrate 0.1 M pH 4.5-4.6 and 5 µl of H₂O₂) was added to each well (100 µl) and incubated for 15 minutes in the dark. The reaction was stopped by addition of HCl 1 N. Absorbance was read at 490nm (620nm for the reference) using a spectrophotometer. The color developed is directly proportional to the amount of antibody present in the serum.

### Description of the ELISA to measure anti PD, PE, and PilA, antibodies

Plates were coated for 2 hours at 37°C with 100 µl per well of 2 µg/ml of PD (1 mg/ml), 2 µg/ml of PE (1500 µg/ml), or 2 µg/ml of PilA (3660 µg/ml) in carbonate buffer pH 9.6 in PBS or Affinipure Goat anti-guinea pig IgG (2.4mg/ml) diluted to 2µg/m for the reference wells in PBS. The plates were washed 4 times with NaCl 0.9% Polysorbate 20 0.05%. For PE and PilA ELISAs (this step was not carried out for the PD and Ply ELISAs), the plates were saturated 30 min at room temperature with PBS-BSA1%. After washing, sera from guinea pigs immunised as described in the section entitled 'immunogenicity of three formulations in guinea pigs' or a reference serum sample (an internal reference calibrated with Chrompure guinea pig IgG) was added to microwells and serially diluted 100 µl (two-fold dilution step) in PBS Polysorbate 20 0.05% (for the PD ELISA) and PBS Polysorbate 20 0.05% BSA 0.1 % for the PE and PilA ELISAs. The plates were incubated for 30 min at room temperature. After washing, the plates were incubated with an anti-guinea pig IgG antibody conjugated to peroxydase (100 µl per well) at room temperature for 30 minutes with shaking. Plates were washed as above and the substrate conjugate (4 mg of OPDA in 10 ml of citrate 0.1 M pH 4.5-4.6 and 5 µl of H₂O₂) was added to each well (100 µl) for 15 min in darkness. The reaction was stopped by addition of HCl 1 N 50 µl and the absorbance is read at 490 nm (620 nm for the reference filter).

### Description of the ELISAto measure PhtD and dPly antibodies

Plates were coated for 2 hours at 37°C with 100 µl per well of 1 µg/ml of PhtD (1021 µg/ml) or 2µg/ml Ply (376µg/ml) in PBS. The plates were then washed 4 times with NaCl 0.9% Polysorbate 20 0.05%. After washing, sera from guinea pigs immunised as described in the section entitled 'immunogenicity of three vaccine formulations in guinea pigs' or a reference (an internal reference calibrated with Chrompure guinea pig IgG) was added to microwells and serially diluted 100 µl (two-fold dilution step) in PBS Polysorbate 20 0.05%. The plates were incubated for 30 min at room temperature with agitation. After washing, the plates were incubated with an anti-guinea pig IgG antibody conjugated to peroxydase (100 µl per well) at room temperature for 30 minutes with shaking. Plates were washed as above and the substrate conjugate (4 mg of OPDA in 10 ml of citrate 0.1 M pH 4.5-4.6 and 5 µl of H₂O₂) was added to each well (100 µl) for 15 min in darkness. The reaction was stopped by addition of HCl 1N 50 µl and the absorbance was read at 490 nm (620 nm for the reference filter).

### Opsonophagocytosis assay

Serum samples were heated for 45 min at 56 °C to inactivate any remaining endogenous complement. Twenty-five microlitres aliquots of each 1:2 diluted serum sample was two-fold serially diluted in 25 µl OPA buffer (HBSS (Hank's Balanced Salt Solution)-14.4% inactivated FCS (foetal calf serum)) per well of a 96-well round bottom microtitre plate. Subsequently, 25 µl of a mixture of activated HL-60 cells (1 × 107 cells/ml), freshly thawed pneumococcal working seed and freshly thawed baby rabbit complement in an e.g. 4/2/1 ratio (v/v/v) (except for serotypes 1,6B and 6A for which the ratio was 4/2/2) was added to the diluted sera to yield a final volume of 50 µl. The assay plate was incubated for 2 h at 37 °C with orbital shaking (210 rpm) to promote the phagocytic process. The reaction was stopped by laying the microplate on ice for at least 1 min (the plate should be kept on ice until further use. A 20 µl aliquot of each well of the plate was then transferred into the corresponding well of a 96-well flat bottom microplate and 50 µl of Todd-Hewitt Broth-0.9% agar was added to each well. After overnight incubation at 37 °C and 5% CO2, pneumococcal colonies appearing in the agar were counted using an automated image analysis system (KS 400, Zeiss, Oberkochen, Germany). Eight wells without serum sample were used as bacterial controls to determine the number of pneumococci per well. The mean number of CFU of the control wells was determined and used for the calculation of the killing activity for each serum sample. The OPA titre for the serum samples was determined by the reciprocal dilution of serum able to facilitate 50% killing of the pneumococci. The opsonophagocytic titre was calculated by using a 4-parameter curve fit analysis.

### Immunogenicity of three formulations in guinea pigs

2 groups of 17 guinea pigs distributed in 2 experiments were immunized by intramuscular (IM) injections on days 0, 14 and 28 with % human dose of different formulation including Proteins alone (PhtD, dPly and PEPilA), Prevnar 13 (_{TM} a commercially available Streptococcal vaccine - results not presented) 10V, 12V (DSP2A017) and 12V+ proteins (DSP2A012) GMP lots. Guinea pigs received in a different leg (mimicking co-administration at different sites in infants in clinical trials) ¼ of human dose of *Infanrix Hexa (*(_{TM} a vaccine comprising diphtheria toxoid, tetanus toxoid, pertussis toxoid, filamentous haemmagglutinin, pertactin, hepatitis B surface antigen, inactivated polio virus types 1, 2 and 3 and *Haemophilus influenzae* b saccharide (PRP). Anti-IgG levels and opsonophagocytosis titers were determined respectively in individual and pooled sera collected at days 42.

The IgG antibody titers and opsonic activity was evaluated and compared between the 12V+proteins, 12V and 10V vaccines.

Sera from guinea pigs injected with the different formulations were tested in ELISA against the polysaccharide serotypes and proteins and in OPA against the 12 serotypes in the formulation. The 12V + proteins induced similar responses to the 12V formulation.

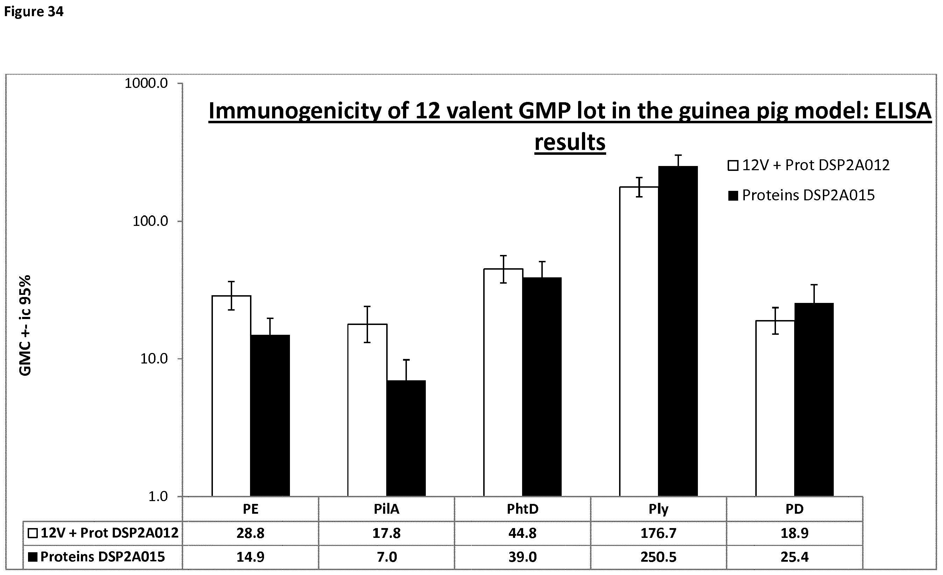

The results in Figure 34 demonstrated that there is no negative impact on the immunogenicity of the 12 valent conjugates when they are combined with PEPilA.

The results obtained with the 12V+proteins GMP formulation demonstrated immunogenicity of the 10V polysaccharides as well as the proteins and support the clinical evaluation of this formulation.

## Claims

1. An immunogenic composition comprising *Streptococcus pneumoniae* capsular saccharide conjugates and a protein component comprising PiIA or an immunogenic fragment of PiIA from *Haemophilus influenzae,* wherein the number of *Streptococcus pneumoniae* saccharide serotypes is less than or equal to 23.

2. The immunogenic composition of claim 1 wherein the protein component comprises PiIA from *Haemophilus influenzae.*

3. The immunogenic composition of claim 1 wherein the protein component comprises Protein E or an immunogenic fragment of Protein E and PiIA or an immunogenic fragment of PiIA from *Haemophilus influenzae.*

4. The immunogenic composition of claim 2 wherein the protein component comprises Protein E and PiIA from *Haemophilus influenzae.*

5. The immunogenic composition of any one of claims 3-4 wherein Protein E or the immunogenic fragment of Protein E is a polypeptide comprising a sequence having at least 75%, 77%, 80%, 85%, 90%, 95%, 97%, 99% or 100% identity, over the entire length, to SEQ ID NO. 4.

6. The immunogenic composition of claim 3, 4 or 5 wherein the immunogenic fragment of Protein E is a polypeptide comprising an immunogenic fragment of at least 7, 10, 15, 20, 25, 30, 50, 75 or 100 contiguous amino acids of SEQ ID NO. 4.

7. The immunogenic composition of any one of claims 1-6 wherein PiIA or the immunogenic fragment of PiIA is a polypeptide comprising a sequence having at least 80%, 85%, 90%, 95%, 97% or 100% identity, over the entire length, to SEQ ID NO. 58.

8. The immunogenic composition of any one of claims 1-7 wherein PiIA or the immunogenic fragment of PiIA is a polypeptide comprising an immunogenic fragment of at least 7, 10, 15, 20, 25, 30 or 50 contiguous amino acids of SEQ ID NO. 58.

9. The immunogenic composition of any preceding claim comprising Protein E or an immunogenic fragment of protein E covalently linked to PiIA or an immunogenic fragment of PiIA to form a fusion protein.

10. The immunogenic composition of claim 9 wherein the fusion protein has formula I:
(X) m - (R1)n - A - (Y) o - B - (Z)p (formula I)
wherein:
X is a signal peptide or MHHHHHH (SEQ ID NO. 2);
m is 0;
R1 is an amino acid;
n is 0;
o is 0 or 1;
Z is GGHHHHHH (SEQ ID NO. 3);
p is 0 or 1;
A is an immunogenic fragment of Protein E, wherein Protein E is selected from any one of SEQ ID NO. 4 - SEQ ID NO. 57;
Y is GG;
and B is an immunogenic fragment of PiIA, wherein PiIA is selected from any one of SEQ ID NO. 58-SEQ ID NO. 121.

11. The immunogenic composition of claim 9 or 10 wherein the fusion protein is SEQ ID NO.194.

12. The immunogenic composition of any one of claims 9-10 wherein the fusion protein is at least 95%, 98% or 99% identical to any of SEQ ID NO. 136, SEQ ID NO. 138, SEQ ID NO. 140, SEQ ID NO. 142, SEQ ID NO. 144, SEQ ID NO. 146, SEQ ID NO. 148, SEQ ID NO. 150, SEQ ID NO. 182, SEQ ID NO. 184, SEQ ID NO. 186, SEQ ID NO. 188, SEQ ID NO. 190, SEQ ID NO. 192, SEQ ID NO. 194, SEQ ID NO. 196, SEQ ID NO. 198, SEQ ID NO. 200, SEQ ID NO. 202 or SEQ ID NO. 204, optionally wherein the signal peptide has been removed.

13. The immunogenic composition of claim 9 or claim 10 wherein fusion protein is SEQ ID NO. 148 and the signal peptide has been removed (SEQ ID NO. 177) or wherein the fusion protein is SEQ ID NO. 194 and the signal peptide has been removed (SEQ ID NO. 219).

14. The immunogenic composition of any preceding claim wherein the *Streptococcus pneumoniae* capsular saccharide conjugates comprise a conjugated serotype 1 saccharide, a conjugated serotype 4 saccharide, a conjugated serotype 5 saccharide, a conjugated serotype 6B saccharide, a conjugated serotype 7F saccharide,a conjugated serotype 9V saccharide, a conjugated serotype 14 saccharide, a conjugated serotype 18C saccharide, a conjugated serotype 19F saccharide and a conjugated serotype 23F saccharide.

15. The immunogenic composition of any preceding claim wherein the *Streptococcus pneumoniae* capsular saccharide conjugates further comprise a conjugated serotype 6A saccharide and a conjugated serotype 19A saccharide.

16. The immunogenic composition of any preceding claim wherein the *Streptococcus pneumoniae* capsular saccharide conjugates further comprise a conjugated serotype 33F saccharide and a conjugated serotype 22F saccharide.

17. The immunogenic composition of any preceding claim wherein the *Streptococcus pneumoniae* capsular saccharide conjugate are conjugated to a carrier protein independently selected from the group consisting of tetanus toxoid (TT), fragment C of TT, diphtheria toxoid, CRM197 (cross reacting material 197), Pneumolysin, protein D (from *H.influenzae*), PhtD (Poly Histidine Triad D), PhtDE (a fusion between Pneumococal Histidine Triad D and E) and N19.

18. The immunogenic composition of any one of claims 1-17 wherein the *Streptococcus pneumoniae* capsular saccharide conjugates comprise a serotype 1 saccharide conjugated to protein D, a serotype 4 saccharide conjugated to protein D, a serotype 5 saccharide conjugated to protein D, a serotype 6B saccharide conjugated to protein D, a serotype 7F saccharide conjugated to protein D, a serotype 9V saccharide conjugated to protein D, a serotype 14 saccharide conjugated to protein D, a serotype 23F saccharide conjugated to protein D, a serotype 18C saccharide conjugated to tetanus toxoid and a serotype 19F saccharide conjugated to diphtheria toxoid.

19. The immunogenic composition of any preceding claim wherein the immunogenic composition further comprises at least one unconjugated or conjugated *Streptococcus pneumoniae* protein selected from the group consisting of Poly Histidine Triad family (PhtX), detoxified pneumolysin (dPly), Choline Binding Protein Family (CbpX), CbpX truncates, LytX (autolytic enzymes) family, LytX truncates, CbpX truncate-LytX truncate chimeric proteins, PcpA (Pneumococcal Choline Binding Protein A), PspA (Pneumococcal Surface Protein A), PsaA (Pneumococcal Surface Adhesin A), Sp128 *(Streptococcus pneumoniae* 128), Sp101 (*Streptococcus pneumoniae* 101), Sp130(*Streptococcus pneumoniae* 130), SP125(*Streptococcus pneumoniae* 125) and SP133(*Streptococcus pneumoniae* 133).

20. A vaccine comprising the immunogenic composition of any preceding claim and a pharmaceutically acceptable excipient.

21. An immunogenic composition according to any one of claims 1-19 or a vaccine according to claim 20 for use in the treatment or prevention of diseases caused by *Streptococcus pneumoniae* infection or an *Haemophilus influenzae* infection, wherein the diseases comprise at least one disease selected from the group consisting of pneumonia, invasive pneumococcal disease (IPD), exacerbations of chronic obstructive pulmonary disease (COPD), otitis media, meningitis, bacteraemia, and conjunctivitis.

## Patentansprüche

1. Immunogene Zusammensetzung, umfassend *Streptococcus pneumoniae*-Kapselsaccharid-Konjugate und eine Proteinkomponente, umfassend PilA oder ein immunogenes Fragment von PilA von *Haemophilus influenzae,* wobei die Anzahl an *Streptococcus pneumoniae*-Saccharid-Serotypen weniger als oder gleich 23 ist.

2. Immunogene Zusammensetzung gemäß Anspruch 1, wobei die Proteinkomponente PilA von *Haemophilus influenzae* umfasst.

3. Immunogene Zusammensetzung gemäß Anspruch 1, wobei die Proteinkomponente Protein E oder ein immunogenes Fragment von Protein E und PilA oder ein immunogenes Fragment von PilA von *Haemophilus influenzae* umfasst.

4. Immunogene Zusammensetzung gemäß Anspruch 2, wobei die Proteinkomponente Protein E und PilA von *Haemophilus influenzae* umfasst.

5. Immunogene Zusammensetzung gemäß irgendeinem der Ansprüche 3-4, wobei Protein E oder das immunogene Fragment von Protein E ein Polypeptid ist, umfassend eine Sequenz, die über die gesamte Länge wenigstens 75%, 77%, 80%, 85%, 90%, 95%, 97%, 99% oder 100% Identität zu SEQ ID NO: 4 hat.

6. Immunogene Zusammensetzung gemäß Anspruch 3, 4 oder 5, wobei das immunogene Fragment von Protein E ein Polypeptid ist, umfassend ein immunogenes Fragment von wenigstens 7, 10, 15, 20, 25, 30, 50, 75 oder 100 zusammenhängenden Aminosäuren von SEQ ID NO: 4.

7. Immunogene Zusammensetzung gemäß irgendeinem der Ansprüche 1-6, wobei PilA oder das immunogene Fragment von PilA ein Polypeptid ist, umfassend eine Sequenz, die über die gesamte Länge wenigstens 80%, 85%, 90%, 95%, 97% oder 100% Identität zu SEQ ID NO: 58 hat.

8. Immunogene Zusammensetzung gemäß irgendeinem der Ansprüche 1-7, wobei PilA oder das immunogene Fragment von PilA ein Polypeptid ist, umfassend ein immunogenes Fragment von wenigstens 7, 10, 15, 20, 25, 30 oder 50 zusammenhängenden Aminosäuren von SEQ ID NO: 58.

9. Immunogene Zusammensetzung gemäß irgendeinem der vorangegangenen Ansprüche, umfassend Protein E oder ein immunogenes Fragment von Protein E, welches mit PilA oder einem immunogenen Fragment von PilA kovalent verknüpft ist, um ein Fusionsprotein zu bilden.

10. Immunogene Zusammensetzung gemäß Anspruch 9, wobei das Fusionsprotein Formel I hat:
(X)m - (R1)n - A - (Y)o - B - (Z)p (Formel I),
worin:
X ein Signalpeptid oder MHHHHHH (SEQ ID NO: 2) ist;
m 0 ist;
R1 eine Aminosäure ist;
n 0 ist;
o 0 oder 1 ist;
Z GGHHHHHH (SEQ ID NO: 3) ist;
p 0 oder 1 ist;
A ein immunogenes Fragment von Protein E ist, wobei Protein E aus irgendeiner von SEQ ID NO: 4 - SEQ ID NO: 57 ausgewählt ist;
Y GG ist;
und B ein immunogenes Fragment von PilA ist, wobei PilA aus irgendeiner von SEQ ID NO: 58 - SEQ ID NO: 121 ausgewählt ist.

11. Immunogene Zusammensetzung gemäß Anspruch 9 oder 10, wobei das Fusionsprotein SEQ ID NO: 194 ist.

12. Immunogene Zusammensetzung gemäß irgendeinem der Ansprüche 9-10, wobei das Fusionsprotein zu wenigstens 95%, 98% oder 99% identisch ist zu irgendeiner von SEQ ID NO: 136, SEQ ID NO: 138, SEQ ID NO: 140, SEQ ID NO: 142, SEQ ID NO: 144, SEQ ID NO: 146, SEQ ID NO: 148, SEQ ID NO: 150, SEQ ID NO: 182, SEQ ID NO: 184, SEQ ID NO: 186, SEQ ID NO: 188, SEQ ID NO: 190, SEQ ID NO: 192, SEQ ID NO: 194, SEQ ID NO: 196, SEQ ID NO: 198, SEQ ID NO: 200, SEQ ID NO: 202 oder SEQ ID NO: 204, wobei optional das Signalpeptid entfernt wurde.

13. Immunogene Zusammensetzung gemäß Anspruch 9 oder Anspruch 10, wobei das Fusionsprotein SEQ ID NO: 148 ist und das Signalpeptid entfernt wurde (SEQ ID NO: 177), oder wobei das Fusionsprotein SEQ ID NO: 194 ist und das Signalpeptid entfernt wurde (SEQ ID NO: 219).

14. Immunogene Zusammensetzung gemäß irgendeinem der vorangegangenen Ansprüche, wobei die *Streptococcus pneumoniae*-Kapselsaccharid-Konjugate umfassen: ein konjugiertes Serotyp 1-Saccharid, ein konjugiertes Serotyp 4-Saccharid, ein konjugiertes Serotyp 5-Saccharid, ein konjugiertes Serotyp 6B-Saccharid, ein konjugiertes Serotyp 7F-Saccharid, ein konjugiertes Serotyp 9V-Saccharid, ein konjugiertes Serotyp 14-Saccharid, ein konjugiertes Serotyp 18C-Saccharid, ein konjugiertes Serotyp 19F-Saccharid und ein konjugiertes Serotyp 23F-Saccharid.

15. Immunogene Zusammensetzung gemäß irgendeinem der vorangegangenen Ansprüche, wobei die *Streptococcus pneumoniae*-Kapselsaccharid-Konjugate weiterhin ein konjugiertes Serotyp 6A-Saccharid und ein konjugiertes Serotyp 19A-Saccharid umfassen.

16. Immunogene Zusammensetzung gemäß irgendeinem der vorangegangenen Ansprüche, wobei die *Streptococcus pneumoniae*-Kapselsaccharid-Konjugate weiterhin ein konjugiertes Serotyp 33F-Saccharid und ein konjugiertes Serotyp 22F-Saccharid umfassen.

17. Immunogene Zusammensetzung gemäß irgendeinem der vorangegangenen Ansprüche, wobei die *Streptococcus pneumoniae*-Kapselsaccharid-Konjugate an ein Trägerprotein konjugiert sind, unabhängig ausgewählt aus der Gruppe, bestehend aus Tetanustoxoid (TT), Fragment C von TT, Diphtherietoxoid, CRM197 (kreuzreagierendem Material 197), Pneumolysin, Protein D (von *H*. *influenzae*)*,* PhtD (Poly-Histidin-Triade D), PhtDE (einer Fusion zwischen Pneumokokken-Histidin-Triade D und E) und N19.

18. Immunogene Zusammensetzung gemäß irgendeinem der Ansprüche 1-17, wobei die *Streptococcus pneumoniae-*Kapselsaccharid-Konjugate umfassen: ein Serotyp 1-Saccharid, konjugiert an Protein D, ein Serotyp 4-Saccharid, konjugiert an Protein D, ein Serotyp 5-Saccharid, konjugiert an Protein D, ein Serotyp 6B-Saccharid, konjugiert an Protein D, ein Serotyp 7F-Saccharid, konjugiert an Protein D, ein Serotyp 9V-Saccharid, konjugiert an Protein D, ein Serotyp 14-Saccharid, konjugiert an Protein D, ein Serotyp 23F-Saccharid, konjugiert an Protein D, ein Serotyp 18C-Saccharid, konjugiert an Tetanustoxoid, und ein Serotyp 19F-Saccharid, konjugiert an Diphtherietoxoid.

19. Immunogene Zusammensetzung gemäß irgendeinem der vorangegangenen Ansprüche, wobei die immunogene Zusammensetzung weiterhin umfasst: wenigstens ein unkonjugiertes oder konjugiertes *Streptococcus pneumoniae*-Protein*,* ausgewählt aus der Gruppe, bestehend aus Poly-Histidin-Triaden-Familie (PhtX), entgiftetem Pneumolysin (dPly), Cholin-Bindungsprotein-Familie (CbpX), CbpX-Trunkierungen, LytX- (autolytische Enzym-) Familie, LytX-Trunkierungen, chimäre Proteine mit CbpX-Trunkierung-LytX-Trunkierung, PcpA (Pneumokokken-Cholin-Bindungsprotein A), PspA (Pneumokokken-Oberflächenprotein A), PsaA (Pneumokokken-Oberflächenadhäsin A), Sp128 (*Streptococcus pneumoniae* 128), Sp101 (*Streptococcus pneumoniae* 101), Sp130 (*Streptococcus pneumoniae* 130), SP125 (*Streptococcus pneumoniae* 125) und SP133 (*Streptococcus pneumoniae* 133).

20. Impfstoff, umfassend die immunogene Zusammensetzung gemäß irgendeinem der vorangegangenen Ansprüche und einen pharmazeutisch akzeptablen Hilfsstoff.

21. Immunogene Zusammensetzung gemäß irgendeinem der Ansprüche 1-19 oder Impfstoff gemäß Anspruch 20 zur Verwendung bei der Behandlung oder Prävention von Erkrankungen, verursacht durch *Streptococcus pneumoniae-*Infektion oder eine *Haemophilus influenzae*-Infektion, wobei die Erkrankungen wenigstens eine Erkrankung umfassen, ausgewählt aus der Gruppe, bestehend aus Pneumonie, invasiver Pneumokokken-Erkrankung (IPD), Exazerbationen von chronisch-obstruktiver Lungenerkrankung (COPD), Otitis media, Meningitis, Bakteriämie und Konjunktivitis.

## Revendications

1. Composition immunogène comprenant des conjugués saccharides capsulaires de *Streptococcus pneumoniae* et un constituant protéique comprenant PilA ou un fragment immunogène de PilA de *Haemophilus influenzae,* dans laquelle le nombre de sérotypes de saccharides *Streptococcus pneumoniae* est inférieur ou égal à 23.

2. Composition immunogène selon la revendication 1 dans laquelle le constituant protéique comprend PilA de *Haemophilus influenzae.*

3. Composition immunogène selon la revendication 1 dans laquelle le constituant protéique comprend la protéine E ou un fragment immunogène de protéine E et PilA ou un fragment immunogène de PilA de *Haemophilus influenzae.*

4. Composition immunogène selon la revendication 2 dans laquelle le constituant protéique comprend la protéine E et PilA de *Haemophilus influenzae.*

5. Composition immunogène selon l'une quelconque des revendications 3 à 4 dans laquelle la protéine E ou le fragment immunogène de protéine E est un polypeptide comprenant une séquence ayant au moins 75 %, 77 %, 80 %, 85 %, 90 %, 95 %, 97 %, 99 % ou 100 % d'identité, sur toute la longueur, avec la SEQ ID N° 4.

6. Composition immunogène selon la revendication 3, 4 ou 5 dans laquelle le fragment immunogène de protéine E est un polypeptide comprenant un fragment immunogène d'au moins 7, 10, 15, 20, 25, 30, 50, 75 ou 100 acides aminés contigus de SEQ ID N° 4.

7. Composition immunogène selon l'une quelconque des revendications 1 à 6 dans laquelle PilA ou le fragment immunogène de PilA est un polypeptide comprenant une séquence ayant au moins 80 %, 85 %, 90 %, 95 %, 97 % ou 100 % d'identité, sur toute la longueur, avec la SEQ ID N° 58.

8. Composition immunogène selon l'une quelconque des revendications 1 à 7 dans laquelle PilA ou le fragment immunogène de PilA est un polypeptide comprenant un fragment immunogène d'au moins 7, 10, 15, 20, 25, 30 ou 50 acides aminés contigus de SEQ ID N° 58.

9. Composition immunogène selon l'une quelconque des revendications précédentes comprenant la protéine E ou un fragment immunogène de protéine E lié par covalence à PilA ou à un fragment immunogène de PilA pour former une protéine hybride.

10. Composition immunogène selon la revendication 9 dans laquelle la protéine hybride répond à la formule I :
(X)m - (R1)n - A - (Y)o - B - (Z)p (formule I)
dans laquelle :
X représente un peptide signal ou MHHHHHH (SEQ ID N° 2) ;
m vaut 0 ;
R1 représente un acide aminé ;
n vaut 0 ;
o vaut 0 ou 1 ;
Z représente GGHHHHHH (SEQ ID N° 3) ;
p vaut 0 ou 1 ;
A représente un fragment immunogène de protéine E, la protéine E étant choisie parmi l'une quelconque de SEQ ID N° 4 à SEQ ID N° 57 ;
Y représente GG ;
et B représente un fragment immunogène de PilA, PilA étant choisie parmi l'une quelconque de SEQ ID N° 58 à SEQ ID N° 121.

11. Composition immunogène selon la revendication 9 ou 10 dans laquelle la protéine hybride est SEQ ID N° 194.

12. Composition immunogène selon l'une quelconque des revendications 9 à 10 dans laquelle la protéine hybride est au moins 95 %, 98 % ou 99 % identique à l'une quelconque de SEQ ID N° 136, SEQ ID N° 138, SEQ ID N° 140, SEQ ID N° 142, SEQ ID N° 144, SEQ ID N° 146, SEQ ID N° 148, SEQ ID N° 150, SEQ ID N° 182, SEQ ID N° 184, SEQ ID N° 186, SEQ ID N° 188, SEQ ID N° 190, SEQ ID N° 192, SEQ ID N° 194, SEQ ID N° 196, SEQ ID N° 198, SEQ ID N° 200, SEQ ID N° 202 ou SEQ ID N° 204, facultativement dans laquelle le peptide signal a été retiré.

13. Composition immunogène selon la revendication 9 ou la revendication 10 dans laquelle la protéine hybride est SEQ ID N° 148 et le peptide signal a été retiré (SEQ ID N° 177) ou dans laquelle la protéine hybride est SEQ ID N° 194 et le peptide signal a été retiré (SEQ ID N° 219).

14. Composition immunogène selon l'une quelconque des revendications précédentes dans laquelle les conjugués saccharides capsulaires de *Streptococcus pneumoniae* comprennent un conjugué saccharide de sérotype 1, un conjugué saccharide de sérotype 4, un conjugué saccharide de sérotype 5, un conjugué saccharide de sérotype 6B, un conjugué saccharide de sérotype 7F, un conjugué saccharide de sérotype 9V, un conjugué saccharide de sérotype 14, un conjugué saccharide de sérotype 18C, un conjugué saccharide de sérotype 19F et un conjugué saccharide de sérotype 23F.

15. Composition immunogène selon l'une quelconque des revendications précédentes dans laquelle les conjugués saccharides capsulaires de *Streptococcus pneumoniae* comprennent en outre un conjugué saccharide de sérotype 6A et un conjugué saccharide de sérotype 19A.

16. Composition immunogène selon l'une quelconque des revendications précédentes dans laquelle les conjugués saccharides capsulaires de *Streptococcus pneumoniae* comprennent en outre un conjugué saccharide de sérotype 33F et un conjugué saccharide de sérotype 22F.

17. Composition immunogène selon l'une quelconque des revendications précédentes dans laquelle les conjugués saccharides capsulaires de *Streptococcus pneumoniae* sont conjugués à une protéine porteuse indépendamment choisie dans le groupe constitué de l'anatoxine tétanique (TT), d'un fragment C de TT, de l'anatoxine diphtérique, de CRM197 (matériau à réaction croisée 197), de la Pneumolysine, de la protéine D (de *H. influenzae*), de la PhtD (triade D de polyhistidine), de la PhtDE (une fusion entre les triades D et E d'histidine pneumococcique) et de N19.

18. Composition immunogène selon l'une quelconque des revendications 1 à 17 dans laquelle les conjugués saccharides capsulaires *Streptococcus pneumoniae* comprennent un saccharide de sérotype 1 conjugué à la protéine D, un saccharide de sérotype 4 conjugué à la protéine D, un saccharide de sérotype 5 conjugué à la protéine D, un saccharide de sérotype 6B conjugué à la protéine D, un saccharide de sérotype 7F conjugué à la protéine D, un saccharide de sérotype 9V conjugué à la protéine D, un saccharide de sérotype 14 conjugué à la protéine D, un saccharide de sérotype 23F conjugué à la protéine D, un saccharide de sérotype 18C conjugué à l'anatoxine tétanique et un saccharide de sérotype 19F conjugué à l'anatoxine diphtérique.

19. Composition immunogène selon l'une quelconque des revendications précédentes dans laquelle la composition immunogène comprend en outre au moins une protéine de *Streptococcus pneumoniae* non conjuguée ou conjuguée choisie dans le groupe constitué de la famille des triades de polyhistidine (PhtX), de la pneumolysine détoxifiée (dPly), de la famille des protéines de liaison de la choline (CbpX), des variantes tronquées de CbpX, de la famille des LytX (enzymes autolytiques), des variantes tronquées de LytX, des protéines chimériques variante tronquée de CbpX-variante tronquée de LytX, de PcpA (protéine de liaison A de la choline pneumococcique), de PspA (protéine de surface pneumococcique A), de PsaA (adhésine de surface pneumococcique A), de Sp128 (*Streptococcus pneumoniae* 128), de Sp101 (*Streptococcus pneumoniae* 101), de Sp130 (*Streptococcus pneumoniae* 130), de SP125 (*Streptococcus pneumoniae* 125) et de SP133 (*Streptococcus pneumoniae* 133).

20. Vaccin comprenant la composition immunogène selon l'une quelconque des revendications précédentes et un excipient pharmaceutiquement acceptable.

21. Composition immunogène selon l'une quelconque des revendications 1 à 19 ou vaccin selon la revendication 20 pour utilisation dans le traitement ou la prévention de maladies causées par une infection à *Streptococcus pneumoniae* ou une infection à *Haemophilus influenzae,* dans laquelle les maladies comprennent au moins une maladie choisie dans le groupe constitué de la pneumonie, de la maladie pneumococcique invasive (IPD), des exacerbations de la bronchopneumopathie chronique obstructive (COPD), de l'otite moyenne, de la méningite, de la bactériémie et de la conjonctivite.


### SEQUENCE LISTING

<110> GlaxoSmithKline Biologicals s.a.
<120> Immunogenic composition
<130> LJW/VB65265
<150> 1218660.7 <151> 2012-10-17
<160> 220
<170> FastSEQ for Windows Version 4.0
<210> 1
   <211> 25
   <212> PRT
   <213> Haemophilus influenzae
<400> 1
   Pro Lys Arg Tyr Ala Arg Ser Val Arg Gln Tyr Lys Ile Leu Asn Cys
    1               5                  10                  15
   Ala Asn Tyr His Leu Thr Gln Val Arg
               20                  25
<210> 2
   <211> 7
   <212> PRT
   <213> Artificial Sequence
<220>
   <223> synthetic construct
<400> 2
   Met His His His His His His
    1               5
<210> 3
   <211> 8
   <212> PRT
   <213> Artificial Sequence
<220>
   <223> synthetic construct
<400> 3
   Gly Gly His His His His His His
    1               5
<210> 4
   <211> 160
   <212> PRT
   <213> Haemophilus influenzae
<400> 4
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
<210> 5
   <211> 160
   <212> PRT
   <213> Haemophilus influenzae
<400> 5
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
<210> 6
   <211> 160
   <212> PRT
   <213> Haemophilus influenzae
<400> 6
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Arg Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Ile Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 7
   <211> 160
   <212> PRT
   <213> Haemophilus influenzae
<400> 7
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
<210> 8
   <211> 160
   <212> PRT
   <213> Haemophilus influenzae
<400> 8
   Met Lys Lys Ile Ile Leu Thr Leu Ser Leu Gly Leu Leu Thr Ala Cys
    1              5                   10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 9
   <211> 160
   <212> PRT
   <213> Haemophilus influenzae
<400> 9
   Met Lys Lys Ile Ile Leu Thr Leu Ser Leu Gly Leu Leu Thr Ala Cys
    1              5                   10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 10
   <211> 160
   <212> PRT
   <213> Haemophilus influenzae
<400> 10
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Met Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 11
   <211> 160
   <212> PRT
   <213> Haemophilus influenzae
<400> 11
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
<210> 12
   <211> 159
   <212> PRT
   <213> Haemophilus influenzae
<400> 12
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Met Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Val Asp Asn Gln Glu Pro Gln Ile
       50                  55                  60
   Val His Phe Asp Ala Val Val Asn Leu Asp Lys Gly Leu Tyr Val Tyr
   65                  70                  75                  80
   Pro Glu Pro Lys Arg Tyr Ala Arg Ser Val Arg Gln Tyr Lys Ile Leu
                   85                  90                  95
   Asn Cys Ala Asn Tyr His Leu Thr Gln Val Arg Thr Asp Phe Tyr Asp
               100                 105                 110
   Glu Phe Trp Gly Gln Gly Leu Arg Ala Ala Pro Lys Lys Gln Lys Lys
           115                 120                 125
   His Thr Leu Ser Leu Thr Pro Asp Thr Thr Leu Tyr Asn Ala Ala Gln
       130                 135                 140
   Ile Ile Cys Ala Asn Tyr Gly Lys Ala Phe Ser Val Asp Lys Lys
   145                 150                 155
<210> 13
   <211> 160
   <212> PRT
   <213> Haemophilus influenzae
<400> 13
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Thr Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 14
   <211> 160
   <212> PRT
   <213> Haemophilus influenzae
<400> 14
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 15
   <211> 194
   <212> PRT
   <213> Haemophilus influenzae
<400> 15
   Met Lys Lys Ile Ile Leu Thr Leu Ser Leu Gly Leu Leu Thr Ala Cys
    1               5                  10                  15
   Ser Ala Gln Thr Gln Lys Ala Glu Gln Asn Asp Val Lys Leu Thr Pro
               20                  25                  30
   Pro Thr Asp Val Gln Ser Gly Tyr Val Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Ile Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Asn
   145                 150                 155                 160
   Lys Lys Ile Cys Thr Leu Ile Ser Leu Asn Phe Ile Gln Leu Leu Gly
                   165                 170                 175
   Cys Arg Glu Tyr Ser Ile Phe Leu Gln Leu Leu Leu Phe Tyr Cys Trp
               180                 185                 190
   His Phe
<210> 16
   <211> 195
   <212> PRT
   <213> Haemophilus influenzae
<400> 16
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
   Ile Lys Lys Ile Cys Thr Leu Ile Ser Leu Asn Phe Ile Gln Leu Leu
                   165                 170                 175
   Gly Cys Arg Glu Tyr Ser Ile Phe Leu Gln Leu Leu Leu Phe Tyr Cys
               180                 185                 190
   Trp His Phe
           195
<210> 17
   <211> 160
   <212> PRT
   <213> Haemophilus influenzae
<400> 17
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Met Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 18
   <211> 160
   <212> PRT
   <213> Haemophilus influenzae
<400> 18
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 19
   <211> 160
   <212> PRT
   <213> Haemophilus influenzae
<400> 19
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
<210> 20
   <211> 160
   <212> PRT
   <213> Haemophilus influenzae
<400> 20
   Met Lys Lys Ile Ile Leu Thr Leu Ser Leu Gly Leu Leu Thr Ala Cys
    1               5                  10                  15
   Ser Ala Gln Thr Gln Lys Val Glu Gln Asn Asp Val Lys Leu Thr Ala
               20                  25                  30
   Pro Thr Asp Val Arg Ser Gly Phe Val Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Leu Val Asp Lys Lys
   145                 150                 155                 160
<210> 21
   <211> 160
   <212> PRT
   <213> Haemophilus influenzae
<400> 21
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
<210> 22
   <211> 160
   <212> PRT
   <213> Haemophilus influenzae
<400> 22
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Ile Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 23
   <211> 160
   <212> PRT
   <213> Haemophilus influenzae
<400> 23
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
<210> 24
   <211> 160
   <212> PRT
   <213> Haemophilus influenzae
<400> 24
   Met Lys Lys Ile Ile Leu Thr Leu Ser Leu Gly Leu Leu Thr Ala Cys
   1 5 10 15
   Ser Ala Gln Thr Gln Lys Val Glu Gln Asn Asp Val Lys Leu Thr Pro
   20 25 30
   Pro Thr Asp Val Arg Ser Gly Tyr Val Arg Leu Val Lys Asn Val Asn
   35 40 45
   Tyr Tyr Ile Asp Ser Glu Ser Ile Trp Val Asp Asn Gln Glu Pro Gln
   50 55 60
   Ile Val His Phe Asp Ala Val Val Asn Leu Asp Lys Gly Leu Tyr Val
   65 70 75 80
   Tyr Pro Glu Pro Lys Arg Tyr Ala Arg Ser Val Arg Gln Tyr Lys Ile
   85 90 95
   Leu Asn Cys Ala Asn Tyr His Leu Thr Gln Val Arg Thr Asp Phe Tyr
   100 105 110
   Asp Glu Phe Trp Gly Gln Gly Leu Arg Ala Ala Pro Lys Lys Gln Lys
   115 120 125
   Lys His Thr Leu Ser Leu Thr Pro Asp Thr Thr Leu Tyr Asn Ala Ala
   130 135 140
   Gln Ile Ile Cys Ala Asn Tyr Gly Lys Ala Phe Ser Val Asp Lys Lys
   145 150 155 160
<210> 25
   <211> 160
   <212> PRT
   <213> Haemophilus influenzae
<400> 25
   Met Lys Lys Ile Ile Leu Thr Leu Ser Leu Gly Leu Leu Thr Ala Cys
   1 5 10 15
   Ser Ala Gln Ile Gln Lys Ala Lys Gln Asn Asp Val Lys Leu Ala Pro
   20 25 30
   Pro Thr Asp Val Arg Ser Gly Tyr Ile Arg Leu Val Lys Asn Val Asn
   35 40 45
   Tyr Tyr Ile Asp Ser Glu Ser Ile Trp Val Asp Asn Gln Glu Pro Gln
   50 55 60
   Ile Val His Phe Asp Ala Val Val Asn Leu Asp Lys Gly Leu Tyr Val
   65 70 75 80
   Tyr Pro Glu Pro Lys Arg Tyr Ala Arg Ser Val Arg Gln Tyr Lys Ile
   85 90 95
   Leu Asn Cys Ala Asn Tyr His Leu Thr Gln Val Arg Thr Asp Phe Tyr
   100 105 110
   Asp Glu Phe Trp Gly Gln Gly Leu Arg Ala Ala Pro Lys Lys Gln Lys
   115 120 125
   Lys His Thr Leu Ser Leu Thr Pro Asp Thr Thr Leu Tyr Asn Ala Ala
   130 135 140
   Gln Ile Ile Cys Ala Asn Tyr Gly Glu Ala Phe Ser Val Asp Lys Lys
   145 150 155 160
<210> 26
   <211> 160
   <212> PRT
   <213> Haemophilus influenzae
<400> 26
   Met Lys Lys Ile Ile Leu Thr Leu Ser Leu Gly Leu Leu Thr Ala Cys
   1 5 10 15
   Ser Ala Gln Thr Gln Lys Val Glu Gln Asn Asp Val Lys Leu Thr Ala
   20 25 30
   Pro Thr Asp Val Arg Ser Gly Tyr Val Arg Leu Val Lys Asn Ala Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 27
   <211> 160
   <212> PRT
   <213> Haemophilus influenzae
<400> 27
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Ile Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 28
   <211> 160
   <212> PRT
   <213> Haemophilus influenzae
<400> 28
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 29
   <211> 160
   <212> PRT
   <213> Haemophilus influenzae
<400> 29
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Thr Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 30
   <211> 160
   <212> PRT
   <213> Haemophilus influenzae
<400> 30
   Met Lys Lys Ile Ile Leu Thr Leu Ser Leu Gly Leu Leu Thr Ala Cys
   1 5 10 15
   Ser Ala Gln Ile Gln Lys Ala Glu Gln Asn Asp Val Lys Leu Ala Pro
   20 25 30
   Pro Thr Asp Val Arg Ser Gly Tyr Ile Arg Leu Val Lys Asn Val Asn
   35 40 45
   Tyr Tyr Ile Asp Ser Glu Ser Ile Trp Val Asp Asn Gln Glu Pro Gln
   50 55 60
   Ile Val His Phe Asp Ala Val Val Asn Leu Asp Lys Gly Leu Tyr Val
   65 70 75 80
   Tyr Pro Glu Pro Lys Arg Tyr Ala Arg Ser Val Arg Gln Tyr Lys Ile
   85 90 95
   Leu Asn Cys Ala Asn Tyr His Leu Thr Gln Val Arg Thr Asp Phe Tyr
   100 105 110
   Asp Glu Phe Trp Gly Gln Gly Leu Arg Ala Ala Pro Lys Lys Gln Lys
   115 120 125
   Lys His Thr Leu Ser Leu Thr Pro Asp Thr Thr Leu Tyr Asn Ala Ala
   130 135 140
   Gln Ile Ile Cys Ala Asn Tyr Gly Lys Ala Phe Ser Val Asp Lys Lys
   145 150 155 160
<210> 31
   <211> 160
   <212> PRT
   <213> Haemophilus influenzae
<400> 31
   Met Lys Lys Ile Ile Leu Thr Leu Ser Leu Gly Leu Leu Thr Ala Cys
   1 5 10 15
   Ser Ala Gln Ile Gln Lys Ala Lys Gln Asn Asp Val Lys Leu Ala Pro
   20 25 30
   Pro Thr Asp Val Arg Ser Gly Tyr Ile Arg Leu Val Lys Asn Val Asn
   35 40 45
   Tyr Tyr Ile Asp Ser Glu Ser Ile Trp Val Asp Asn Gln Glu Pro Gln
   50 55 60
   Ile Val His Phe Asp Ala Val Val Asn Leu Asp Lys Gly Leu Tyr Val
   65 70 75 80
   Tyr Pro Glu Pro Lys Arg Tyr Ala Arg Ser Val Arg Gln Tyr Lys Ile
   85 90 95
   Leu Asn Cys Ala Asn Tyr His Leu Thr Gln Val Arg Thr Asp Phe Tyr
   100 105 110
   Asp Glu Phe Trp Gly Gln Gly Leu Arg Ala Ala Pro Lys Lys Gln Lys
   115 120 125
   Lys His Thr Leu Ser Leu Thr Pro Asp Thr Thr Leu Tyr Asn Ala Ala
   130 135 140
   Gln Ile Ile Cys Ala Asn Tyr Gly Glu Ala Phe Ser Val Asp Lys Lys
   145 150 155 160
<210> 32
   <211> 160
   <212> PRT
   <213> Haemophilus influenzae
<400> 32
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 33
   <211> 160
   <212> PRT
   <213> Haemophilus influenzae
<400> 33
   Met Lys Lys Ile Ile Leu Thr Leu Ser Leu Gly Leu Leu Thr Ala Cys
    1               5                  10                  15
   Ser Ala Gln Thr Gln Lys Val Glu Gln Asn Asp Val Lys Leu Thr Pro
               20                  25                  30
   Pro Thr Asp Val Arg Ser Gly Tyr Val Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 34
   <211> 160
   <212> PRT
   <213> Haemophilus influenzae
<400> 34
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 35
   <211> 160
   <212> PRT
   <213> Haemophilus influenzae
<400> 35
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
<210> 36
   <211> 160
   <212> PRT
   <213> Haemophilus influenzae
<400> 36
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
<210> 37
   <211> 160
   <212> PRT
   <213> Haemophilus influenzae
<400> 37
   Met Lys Lys Ile Ile Leu Thr Leu Ser Leu Gly Leu Leu Thr Ala Cys
    1               5                  10                  15
   Ser Ala Gln Thr Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Ile Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 38
   <211> 160
   <212> PRT
   <213> Haemophilus influenzae
<400> 38
   Met Lys Lys Ile Ile Leu Thr Leu Ser Leu Gly Leu Leu Thr Ala Cys
    1               5                  10                  15
   Ser Ala Gln Thr Gln Lys Val Glu Gln Asn Asp Val Lys Leu Thr Ala
               20                  25                  30
   Pro Ala Asp Val Arg Ser Gly Tyr Val Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 39
   <211> 159
   <212> PRT
   <213> Haemophilus influenzae
<400> 39
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Tyr Lys Ile Leu
                   85                  90                  95
   Asn Cys Ala Asn Tyr His Leu Thr Gln Val Arg Thr Asp Phe Tyr Asp
               100                 105                 110
   Glu Phe Trp Gly Gln Gly Leu Arg Ala Ala Pro Lys Lys Gln Lys Lys
           115                 120                 125
   His Thr Leu Ser Leu Thr Pro Asp Thr Thr Leu Tyr Asn Ala Ala Gln
       130                 135                 140
   Ile Ile Cys Ala Asn Tyr Gly Glu Ala Phe Ser Val Asp Lys Lys
   145                 150                 155
<210> 40
   <211> 159
   <212> PRT
   <213> Haemophilus influenzae
<400> 40
   Met Lys Lys Ile Ile Leu Thr Leu Ser Leu Gly Leu Leu Thr Ala Cys
   1               5                   10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Tyr Lys Ile Leu
                   85                  90                  95
   Asn Cys Ala Asn Tyr His Leu Thr Gln Val Arg Thr Asp Phe Tyr Asp
               100                 105                 110
   Glu Phe Trp Gly Gln Gly Leu Arg Ala Ala Pro Lys Lys Gln Lys Lys
           115                 120                 125
   His Thr Leu Ser Leu Thr Pro Asp Thr Thr Leu Tyr Asn Ala Ala Gln
       130                 135                 140
   Ile Ile Cys Ala Asn Tyr Gly Glu Ala Phe Ser Val Asp Lys Lys
   145                 150                 155
<210> 41
   <211> 160
   <212> PRT
   <213> Haemophilus influenzae
<400> 41
   Met Lys Lys Ile Ile Leu Thr Leu Ser Leu Gly Leu Leu Thr Ala Cys
   1               5                   10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 42
   <211> 160
   <212> PRT
   <213> Haemophilus influenzae
<400> 42
   Met Lys Lys Ile Ile Leu Thr Leu Ser Leu Gly Leu Leu Thr Ala Cys
   1 5 10 15
   Ser Ala Gln Ile Gln Lys Ala Glu Gln Asn Asp Val Lys Leu Ala Pro
   20 25 30
   Pro Thr Asp Val Arg Ser Gly Tyr Ile Arg Leu Val Lys Asn Val Asn
   35 40 45
   Tyr Tyr Ile Asp Ser Glu Ser Ile Trp Val Asp Asn Gln Glu Pro Gln
   50 55 60
   Ile Val His Phe Asp Ala Val Val Asn Leu Asp Lys Gly Leu Tyr Val
   65 70 75 80
   Tyr Pro Glu Pro Lys Arg Tyr Ala Arg Ser Val Arg Gln Tyr Lys Ile
   85 90 95
   Leu Asn Cys Ala Asn Tyr His Leu Thr Gln Val Arg Thr Asp Phe Tyr
   100 105 110
   Asp Glu Phe Trp Gly Gln Gly Leu Arg Ala Ala Pro Lys Lys Gln Lys
   115 120 125
   Lys His Thr Leu Ser Leu Thr Pro Asp Thr Thr Leu Tyr Asn Ala Ala
   130 135 140
   Gln Ile Ile Cys Ala Asn Tyr Gly Lys Ala Phe Ser Val Asp Lys Lys
   145 150 155 160
<210> 43
   <211> 160
   <212> PRT
   <213> Haemophilus influenzae
<400> 43
   Met Lys Lys Ile Ile Leu Thr Leu Ser Leu Gly Leu Leu Thr Ala Cys
   1 5 10 15
   Ser Ala Gln Ile Gln Lys Ala Glu Gln Asn Asp Val Lys Leu Ala Pro
   20 25 30
   Pro Thr Asp Val Arg Ser Gly Tyr Ile Arg Leu Val Lys Asn Val Asn
   35 40 45
   Tyr Tyr Ile Asp Ser Glu Ser Ile Trp Val Asp Asn Gln Glu Pro Gln
   50 55 60
   Ile Val His Phe Asp Ala Val Val Asn Leu Asp Lys Gly Leu Tyr Val
   65 70 75 80
   Tyr Pro Glu Pro Lys Arg Tyr Ala Arg Ser Val Arg Gln Tyr Lys Ile
   85 90 95
   Leu Asn Cys Ala Asn Tyr His Leu Thr Gln Val Arg Thr Asp Phe Tyr
   100 105 110
   Asp Glu Phe Trp Gly Gln Gly Leu Arg Ala Ala Pro Lys Lys Gln Lys
   115 120 125
   Lys His Thr Leu Ser Leu Thr Pro Asp Thr Thr Leu Tyr Asn Ala Ala
   130 135 140
   Gln Ile Ile Cys Ala Asn Tyr Gly Lys Ala Phe Ser Val Asp Lys Lys
   145 150 155 160
<210> 44
   <211> 160
   <212> PRT
   <213> Haemophilus influenzae
<400> 44
   Met Lys Lys Ile Ile Leu Thr Leu Ser Leu Gly Leu Leu Thr Ala Cys
   1 5 10 15
   Ser Ala Gln Ile Gln Lys Ala Glu Gln Asn Asp Val Lys Leu Ala Pro
   20 25 30
   Pro Thr Asp Val Arg Ser Gly Tyr Ile Arg Leu Val Lys Asn Val Asn
   35 40 45
   Tyr Tyr Ile Asp Ser Glu Ser Ile Trp Val Asp Asn Gln Glu Pro Gln
   50 55 60
   Ile Val His Phe Asp Ala Val Val Asn Leu Asp Lys Gly Leu Tyr Val
   65 70 75 80
   Tyr Pro Glu Pro Lys Arg Tyr Ala Arg Ser Val Arg Gln Tyr Lys Ile
   85 90 95
   Leu Asn Cys Ala Asn Tyr His Leu Thr Gln Val Arg Thr Asp Phe Tyr
   100 105 110
   Asp Glu Phe Trp Gly Gln Gly Leu Arg Ala Ala Pro Lys Lys Gln Lys
   115 120 125
   Lys His Thr Leu Ser Leu Thr Pro Asp Thr Thr Leu Tyr Asn Ala Ala
   130 135 140
   Gln Ile Ile Cys Ala Asn Tyr Gly Lys Ala Phe Ser Val Asp Lys Lys
   145 150 155 160
<210> 45
   <211> 160
   <212> PRT
   <213> Haemophilus influenzae
<400> 45
   Met Lys Lys Ile Ile Leu Thr Leu Ser Leu Gly Leu Leu Thr Ala Cys
   1 5 10 15
   Ser Ala Gln Ile Gln Lys Ala Glu Gln Asn Asp Val Lys Leu Ala Pro
   20 25 30
   Pro Thr Asp Val Arg Ser Gly Tyr Ile Arg Leu Val Lys Asn Val Asn
   35 40 45
   Tyr Tyr Ile Asp Ser Glu Ser Ile Trp Val Asp Asn Gln Glu Pro Gln
   50 55 60
   Ile Val His Phe Asp Thr Val Val Asn Leu Asp Lys Gly Leu Tyr Val
   65 70 75 80
   Tyr Pro Glu Pro Lys Arg Tyr Ala Arg Ser Val Arg Gln Tyr Lys Ile
   85 90 95
   Leu Asn Cys Ala Asn Tyr His Leu Thr Gln Val Arg Thr Asp Phe Tyr
   100 105 110
   Asp Glu Phe Trp Gly Gln Ser Leu Arg Ala Ala Pro Lys Lys Gln Lys
   115 120 125
   Lys His Thr Leu Ser Leu Thr Pro Asp Thr Thr Leu Tyr Asn Ala Ala
   130 135 140
   Gln Ile Ile Cys Ala Asn Tyr Gly Lys Ala Phe Ser Val Asp Lys Lys
   145 150 155 160
<210> 46
   <211> 160
   <212> PRT
   <213> Haemophilus influenzae
<400> 46
   Met Lys Lys Ile Ile Leu Thr Leu Ser Leu Gly Leu Leu Thr Ala Cys
    1               5                  10                  15
   Ser Ala Gln Thr Gln Lys Val Glu Gln Asn Asp Val Lys Leu Thr Pro
               20                  25                  30
   Pro Thr Asp Val Arg Ser Gly Tyr Val Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Val Tyr Asn Ala Ala
       130                 135                 140
   Gln Ile Ile Cys Ala Asn Tyr Gly Lys Ala Phe Ser Val Asp Lys Lys
   145                 150                 155                 160
<210> 47
   <211> 160
   <212> PRT
   <213> Haemophilus influenzae
<400> 47
   Met Lys Lys Ile Ile Leu Thr Leu Ser Leu Gly Leu Leu Thr Ala Cys
    1               5                  10                  15
   Ser Ala Gln Thr Gln Lys Val Glu Gln Asn Asp Val Lys Leu Thr Pro
               20                  25                  30
   Pro Thr Asp Val Arg Ser Gly Tyr Val Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Val Tyr Asn Ala Ala
       130                 135                 140
   Gln Ile Ile Cys Ala Asn Tyr Gly Lys Ala Phe Ser Val Asp Lys Lys
   145                 150                 155                 160
<210> 48
   <211> 160
   <212> PRT
   <213> Haemophilus influenzae
<400> 48
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
<210> 49
   <211> 160
   <212> PRT
   <213> Haemophilus influenzae
<400> 49
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Thr Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 50
   <211> 160
   <212> PRT
   <213> Haemophilus influenzae
<400> 50
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Thr Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 51
   <211> 160
   <212> PRT
   <213> Haemophilus influenzae
<400> 51
   Met Lys Lys Ile Ile Leu Thr Leu Ser Leu Gly Leu Leu Thr Ala Cys
    1               5                  10                  15
   Ser Ala Gln Thr Gln Lys Ala Glu Gln Asn Asp Val Lys Leu Thr Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 52
   <211> 160
   <212> PRT
   <213> Haemophilus influenzae
<400> 52
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 53
   <211> 160
   <212> PRT
   <213> Haemophilus influenzae
<400> 53
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Thr Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 54
   <211> 160
   <212> PRT
   <213> Haemophilus influenzae
<400> 54
   Met Lys Lys Ile Ile Leu Thr Leu Ser Leu Gly Leu Leu Thr Ala Cys
    1               5                  10                  15
   Ser Ala Gln Thr Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Ile Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 55
   <211> 160
   <212> PRT
   <213> Haemophilus influenzae
<400> 55
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
<210> 56
   <211> 160
   <212> PRT
   <213> Haemophilus influenzae
<400> 56
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 57
   <211> 159
   <212> PRT
   <213> Haemophilus influenzae
<400> 57
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Met Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Val Asp Asn Gln Glu Pro Gln Ile
       50                  55                  60
   Val His Phe Asp Ala Val Val Asn Leu Asp Lys Gly Leu Tyr Val Tyr
   65                  70                  75                  80
   Pro Glu Pro Lys Arg Tyr Ala Arg Ser Val Arg Gln Tyr Lys Ile Leu
                   85                  90                  95
   Asn Cys Ala Asn Tyr His Leu Thr Gln Val Arg Thr Asp Phe Tyr Asp
               100                 105                 110
   Glu Phe Trp Gly Gln Gly Leu Arg Ala Ala Pro Lys Lys Gln Lys Lys
           115                 120                 125
   His Thr Leu Ser Leu Thr Pro Asp Thr Thr Leu Tyr Asn Ala Ala Gln
       130                 135                 140
   Ile Ile Cys Ala Asn Tyr Gly Lys Ala Phe Ser Val Asp Lys Lys
   145                 150                 155
<210> 58
   <211> 149
   <212> PRT
   <213> Haemophilus influenzae
<400> 58
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 59
   <211> 149
   <212> PRT
   <213> Haemophilus influenzae
<400> 59
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Lys Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Ala Gly Asn Gly Thr Leu Asp Gly Met Ser
               100                 105                 110
   Tyr Thr Leu Thr Ala Glu Gly Asp Ser Ala Lys Gly Val Thr Trp Lys
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 60
   <211> 149
   <212> PRT
   <213> Haemophilus influenzae
<400> 60
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 61
   <211> 149
   <212> PRT
   <213> Haemophilus influenzae
<400> 61
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Lys Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Ser Cys Ser Gly Gly Ser Asn Gly Ile Ala
   65                  70                  75                  80
   Ala Asp Ile Thr Thr Ala Lys Gly Tyr Val Ala Ser Val Ile Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Ala Gly Asn Ala Ala Ala Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 62
   <211> 149
   <212> PRT
   <213> Haemophilus influenzae
<400> 62
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 63
   <211> 149
   <212> PRT
   <213> Haemophilus influenzae
<400> 63
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ser Asp Val Glu Leu Cys Val Tyr
       50                  55                  60
   Ser Thr Gly Lys Pro Ser Thr Cys Ser Gly Gly Ser Asn Gly Ile Ala
   65                  70                  75                  80
   Ala Asp Ile Thr Thr Val Lys Gly Tyr Val Lys Ser Val Thr Thr Ser
                   85                  90                  95
   Asn Gly Ala Ile Thr Val Ala Gly Asn Gly Thr Leu Asp Gly Met Ser
               100                 105                 110
   Tyr Thr Leu Thr Ala Glu Gly Asp Ser Ala Lys Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 64
   <211> 149
   <212> PRT
   <213> Haemophilus influenzae
<400> 64
   Met Lys Leu Thr Thr Gln Gln Thr Leu Lys Lys Gly Phe Thr Leu Ile
    1              5                   10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 65
   <211> 149
   <212> PRT
   <213> Haemophilus influenzae
<400> 65
   Met Lys Leu Thr Thr Gln Gln Thr Leu Lys Lys Gly Phe Thr Leu Ile
    1              5                   10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 66
   <211> 149
   <212> PRT
   <213> Haemophilus influenzae
<400> 66
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 67
   <211> 149
   <212> PRT
   <213> Haemophilus influenzae
<400> 67
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Lys Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Ile Thr Asn Cys Met Gly Gly Lys Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Thr Gly Asn Ala Ala Ala Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Ile Thr Gln
   145
<210> 68
   <211> 149
   <212> PRT
   <213> Haemophilus influenzae
<400> 68
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ser Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Lys Gly Asn Ala Thr Ala Gly Val Thr Trp Thr
           115                 120                 125
   Thr Thr Cys Lys Gly Thr Asp Ala Ser Leu Phe Pro Ala Asn Phe Cys
       130                 135                 140
   Arg Ser Val Thr Lys
   145
<210> 69
   <211> 149
   <212> PRT
   <213> Haemophilus influenzae
<400> 69
   Met Lys Leu Thr Thr Leu Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Ser Cys Thr Gly Gly Lys Asn Gly Ile Ala
   65                  70                  75                  80
   Ala Asp Ile Lys Thr Ala Lys Gly Tyr Val Ala Ser Val Ile Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Lys Gly Asn Ala Ala Ala Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Lys
   145
<210> 70
   <211> 149
   <212> PRT
   <213> Haemophilus influenzae
<400> 70
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Ser Cys Ser Gly Gly Ser Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Ser Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 71
   <211> 149
   <212> PRT
   <213> Haemophilus influenzae
<400> 71
   Met Lys Leu Thr Thr Leu Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Ser Cys Thr Gly Gly Lys Asn Gly Ile Ala
   65                  70                  75                  80
   Ala Asp Ile Lys Thr Ala Lys Gly Tyr Val Ala Ser Val Ile Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Lys Gly Asn Ala Ala Ala Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Lys
   145
<210> 72
   <211> 149
   <212> PRT
   <213> Haemophilus influenzae
<400> 72
   Met Lys Leu Thr Thr Leu Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Ser Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 73
   <211> 149
   <212> PRT
   <213> Haemophilus influenzae
<400> 73
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ser Val Ser Glu Leu
           35                  40                  45
   Leu Gln Ala Ser Ala Pro Tyr Lys Ser Asp Val Glu Leu Cys Val Tyr
       50                  55                  60
   Ser Thr Gly Lys Pro Ser Thr Cys Ser Gly Gly Ser Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Lys Gly Asn Ala Thr Ala Gly Val Thr Trp Thr
           115                 120                 125
   Thr Thr Cys Lys Gly Thr Asp Ala Ser Leu Phe Pro Ala Asn Phe Cys
       130                 135                 140
   Arg Ser Val Thr Lys
   145
<210> 74
   <211> 149
   <212> PRT
   <213> Haemophilus influenzae
<400> 74
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 75
   <211> 149
   <212> PRT
   <213> Haemophilus influenzae
<400> 75
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Glu Ala Ser Leu Phe Pro Ala Asn Phe Cys
       130                 135                 140
   Gly Ser Val Thr Gln
   145
<210> 76
   <211> 149
   <212> PRT
   <213> Haemophilus influenzae
<400> 76
   Met Lys Leu Thr Thr Gln Gln Thr Leu Lys Lys Gly Phe Thr Leu Ile
   1               5                   10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ser Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Lys Gly Asn Ala Thr Ala Gly Val Thr Trp Thr
           115                 120                 125
   Thr Thr Cys Lys Gly Thr Asp Ala Ser Leu Phe Pro Ala Asn Phe Cys
       130                 135                 140
   Arg Ser Val Thr Lys
   145
<210> 77
   <211> 149
   <212> PRT
   <213> Haemophilus influenzae
<400> 77
   Met Lys Leu Thr Thr Leu Gln Thr Leu Lys Lys Gly Phe Thr Leu Ile
   1               5                   10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Ser Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 78
   <211> 149
   <212> PRT
   <213> Haemophilus influenzae
<400> 78
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Ser Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Lys Gly Asn Ala Thr Ala Gly Val Thr Trp Thr
           115                 120                 125
   Thr Thr Cys Lys Gly Thr Asp Ala Ser Leu Phe Pro Ala Asn Phe Cys
       130                 135                 140
   Arg Ser Val Thr Lys
   145
<210> 79
   <211> 149
   <212> PRT
   <213> Haemophilus influenzae
<400> 79
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Glu Ala Ser Leu Phe Pro Ala Asn Phe Cys
       130                 135                 140
   Gly Ser Val Thr Gln
   145
<210> 80
   <211> 149
   <212> PRT
   <213> Haemophilus influenzae
<400> 80
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
   35 40 45
   Leu Gln Ala Ser Ala Pro Tyr Lys Ala Asp Val Glu Leu Cys Val Tyr
   50 55 60
   Ser Thr Asn Glu Thr Thr Asn Cys Thr Gly Gly Lys Asn Gly Ile Ala
   65 70 75 80
   Ala Asp Ile Thr Thr Ala Lys Gly Tyr Val Ala Ser Val Lys Thr Gln
   85 90 95
   Ser Gly Gly Ile Thr Val Lys Gly Asp Gly Thr Leu Ala Asn Met Glu
   100 105 110
   Tyr Ile Leu Gln Ala Thr Gly Asn Ala Ala Thr Gly Val Thr Trp Thr
   115 120 125
   Thr Thr Cys Lys Gly Thr Glu Ala Ser Leu Phe Pro Ala Asn Phe Cys
   130 135 140
   Gly Ser Val Thr Gln
   145
<210> 81
   <211> 149
   <212> PRT
   <213> Haemophilus influenzae
<400> 81
   Met Lys Leu Thr Thr Gln Gln Thr Leu Lys Lys Gly Phe Thr Leu Ile
   1 5 10 15
   Glu Leu Met Ile Val Ile Ala Ile Ile Ala Ile Leu Ala Thr Ile Ala
   20 25 30
   Ile Pro Ser Tyr Gln Asn Tyr Thr Lys Lys Ala Ala Val Ser Glu Leu
   35 40 45
   Leu Gln Ala Ser Ala Pro Tyr Lys Ser Asp Val Glu Leu Cys Val Tyr
   50 55 60
   Ser Thr Gly Lys Pro Ser Thr Cys Ser Gly Gly Ser Asn Gly Ile Ala
   65 70 75 80
   Ala Asp Ile Thr Thr Ala Lys Gly Tyr Val Lys Ser Val Thr Thr Ser
   85 90 95
   Asn Gly Ala Ile Thr Val Ala Gly Asn Gly Thr Leu Asp Gly Met Ser
   100 105 110
   Tyr Thr Leu Thr Ala Glu Gly Asp Ser Ala Lys Gly Val Thr Trp Lys
   115 120 125
   Thr Thr Cys Lys Gly Thr Asp Ala Ser Leu Phe Pro Ala Asn Phe Cys
   130 135 140
   Gly Ser Val Thr Lys
   145
<210> 82
   <211> 149
   <212> PRT
   <213> Haemophilus influenzae
<400> 82
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Ala Thr Lys Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Ser Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 83
   <211> 149
   <212> PRT
   <213> Haemophilus influenzae
<400> 83
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Ala Thr Lys Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Ser Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 84
   <211> 149
   <212> PRT
   <213> Haemophilus influenzae
<400> 84
   Met Lys Leu Thr Thr Gln Gln Thr Leu Lys Lys Gly Phe Thr Leu Ile
   1 5 10 15
   Glu Leu Met Ile Val Ile Ala Ile Ile Ala Ile Leu Ala Thr Ile Ala
   20 25 30
   Ile Pro Ser Tyr Gln Asn Tyr Thr Lys Lys Ala Ala Val Ser Glu Leu
   35 40 45
   Leu Gln Ala Ser Ala Pro Tyr Lys Ala Asp Val Glu Leu Cys Val Tyr
   50 55 60
   Ser Thr Asn Glu Thr Thr Asn Cys Thr Gly Gly Lys Asn Gly Ile Ala
   65 70 75 80
   Ala Asp Ile Thr Thr Ala Lys Gly Tyr Val Lys Ser Val Thr Thr Ser
   85 90 95
   Asn Gly Ala Ile Thr Val Lys Gly Asp Gly Thr Leu Ala Asn Met Glu
   100 105 110
   Tyr Ile Leu Gln Ala Thr Gly Asn Ala Ala Thr Gly Val Thr Trp Thr
   115 120 125
   Thr Thr Cys Lys Gly Thr Asp Ala Ser Leu Phe Pro Ala Asn Phe Cys
   130 135 140
   Gly Ser Val Thr Gln
   145
<210> 85
   <211> 149
   <212> PRT
   <213> Haemophilus influenzae
<400> 85
   Met Lys Leu Thr Thr Gln Gln Thr Leu Lys Lys Gly Phe Thr Leu Ile
   1 5 10 15
   Glu Leu Met Ile Val Ile Ala Ile Ile Ala Ile Leu Ala Thr Ile Ala
   20 25 30
   Ile Pro Ser Tyr Gln Asn Tyr Thr Lys Lys Ala Ala Ile Ser Glu Leu
   35 40 45
   Leu Gln Ala Ser Ala Pro Tyr Lys Ser Asp Val Glu Leu Cys Val Tyr
   50 55 60
   Ser Thr Gly Lys Pro Ser Thr Cys Ser Gly Gly Ser Asn Gly Ile Ala
   65 70 75 80
   Ala Asp Ile Thr Thr Ala Lys Gly Tyr Val Ala Ser Val Lys Thr Gln
   85 90 95
   Ser Gly Gly Ile Thr Val Lys Gly Asn Gly Thr Leu Ala Asn Met Glu
   100 105 110
   Tyr Ile Leu Gln Ala Lys Gly Asn Ala Thr Ala Gly Val Thr Trp Thr
   115 120 125
   Thr Thr Cys Lys Gly Thr Asp Ala Ser Leu Phe Pro Ala Asn Phe Cys
   130 135 140
   Arg Ser Val Thr Lys
   145
<210> 86
   <211> 149
   <212> PRT
   <213> Haemophilus influenzae
<400> 86
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Glu Ala Ser Leu Phe Pro Ala Asn Phe Cys
       130                 135                 140
   Gly Ser Val Thr Gln
   145
<210> 87
   <211> 149
   <212> PRT
   <213> Haemophilus influenzae
<400> 87
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 88
   <211> 149
   <212> PRT
   <213> Haemophilus influenzae
<400> 88
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ser Asp Val Glu Leu Cys Val Tyr
       50                  55                  60
   Ser Thr Gly Lys Pro Ser Thr Cys Ser Gly Gly Ser Asn Gly Ile Ala
   65                  70                  75                  80
   Ala Asp Ile Thr Thr Val Lys Gly Tyr Val Lys Ser Val Thr Thr Ser
                   85                  90                  95
   Asn Gly Ala Ile Thr Val Ala Gly Asn Gly Thr Leu Asp Gly Met Ser
               100                 105                 110
   Tyr Thr Leu Thr Ala Glu Gly Asp Ser Ala Lys Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 89
   <211> 149
   <212> PRT
   <213> Haemophilus influenzae
<400> 89
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 90
   <211> 149
   <212> PRT
   <213> Haemophilus influenzae
<400> 90
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 91
   <211> 149
   <212> PRT
   <213> Haemophilus influenzae
<400> 91
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Ser Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Ala Gly Asn Gly Thr Leu Asp Arg Met Ser
               100                 105                 110
   Tyr Thr Leu Thr Ala Glu Gly Asp Ser Ala Lys Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 92
   <211> 149
   <212> PRT
   <213> Haemophilus influenzae
<400> 92
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Ser Cys Ser Gly Gly Ser Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Ser Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 93
   <211> 149
   <212> PRT
   <213> Haemophilus influenzae
<400> 93
   Met Lys Leu Thr Thr Gln Thr Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ser Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Ala Ser Val Ile Thr Gln
                   85                  90                  95
   Ser Gly Gly Ile Thr Val Lys Gly Asn Gly Thr Leu Thr Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Ile Thr Gln
   145
<210> 94
   <211> 149
   <212> PRT
   <213> Haemophilus influenzae
<400> 94
   Met Lys Leu Thr Thr Leu Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Asn Asn Gly Ile Ala
   65                  70                  75                  80
   Ala Asp Ile Lys Thr Ala Lys Gly Tyr Val Ala Ser Val Lys Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 95
   <211> 149
   <212> PRT
   <213> Haemophilus influenzae
<400> 95
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Ser Cys Ser Gly Gly Ser Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Ser Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 96
   <211> 149
   <212> PRT
   <213> Haemophilus influenzae
<400> 96
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Ala Thr Lys Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Ser Gly Asn Ala Ala Thr Gly Val Thr Trp Thr
           115                 120                 125
   Thr Thr Cys Lys Gly Thr Asp Thr Ser Leu Phe Pro Ala Asn Phe Cys
       130                 135                 140
   Gly Ser Val Thr Gln
   145
<210> 97
   <211> 149
   <212> PRT
   <213> Haemophilus influenzae
<400> 97
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 98
   <211> 149
   <212> PRT
   <213> Haemophilus influenzae
<400> 98
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
   35 40 45
   Leu Gln Ala Ser Ala Pro Tyr Lys Ala Asp Val Glu Leu Cys Val Tyr
   50 55 60
   Ser Thr Asn Glu Thr Thr Asn Cys Thr Gly Gly Lys Asn Gly Ile Ala
   65 70 75 80
   Ala Asp Ile Thr Thr Ala Lys Gly Tyr Val Ala Ser Val Lys Thr Gln
   85 90 95
   Ser Gly Gly Ile Thr Val Lys Gly Asn Gly Thr Leu Ala Asn Met Glu
   100 105 110
   Tyr Ile Leu Gln Ala Thr Gly Asn Ala Ala Thr Gly Val Thr Trp Thr
   115 120 125
   Thr Thr Cys Lys Gly Thr Asp Ala Ser Leu Phe Pro Ala Asn Phe Cys
   130 135 140
   Gly Ser Val Thr Gln
   145
<210> 99
   <211> 149
   <212> PRT
   <213> Haemophilus influenzae
<400> 99
   Met Lys Leu Thr Thr Gln Gln Thr Leu Lys Lys Gly Phe Thr Leu Ile
   1 5 10 15
   Glu Leu Met Ile Val Ile Ala Ile Ile Ala Ile Leu Ala Thr Ile Ala
   20 25 30
   Ile Pro Ser Tyr Gln Asn Tyr Thr Lys Lys Ala Ala Val Ser Glu Leu
   35 40 45
   Leu Gln Ala Ser Ala Pro Tyr Lys Ala Asp Val Glu Leu Cys Val Tyr
   50 55 60
   Ser Thr Gly Lys Pro Ser Thr Cys Ser Gly Gly Ser Asn Gly Ile Ala
   65 70 75 80
   Ala Asp Ile Thr Thr Ala Lys Gly Tyr Val Ala Ser Val Lys Thr Gln
   85 90 95
   Ser Gly Gly Ile Thr Val Lys Gly Asn Gly Thr Leu Ala Asn Met Glu
   100 105 110
   Tyr Ile Leu Gln Ala Lys Gly Asn Ala Thr Ala Gly Val Thr Trp Thr
   115 120 125
   Thr Thr Cys Lys Gly Thr Asp Ala Ser Leu Phe Pro Ala Asn Phe Cys
   130 135 140
   Arg Ser Val Thr Lys
   145
<210> 100
   <211> 149
   <212> PRT
   <213> Haemophilus influenzae
<400> 100
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Ser Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Lys Gly Asn Ala Thr Ala Gly Val Thr Trp Thr
           115                 120                 125
   Thr Thr Cys Lys Gly Thr Asp Ala Ser Leu Phe Pro Ala Asn Phe Cys
       130                 135                 140
   Arg Ser Val Thr Lys
   145
<210> 101
   <211> 149
   <212> PRT
   <213> Haemophilus influenzae
<400> 101
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 102
   <211> 149
   <212> PRT
   <213> Haemophilus influenzae
<400> 102
   Met Lys Leu Thr Thr Gln Gln Thr Leu Lys Lys Gly Phe Thr Leu Ile
   1 5 10 15
   Glu Leu Met Ile Val Ile Ala Ile Ile Ala Ile Leu Ala Thr Ile Ala
   20 25 30
   Ile Pro Ser Tyr Gln Asn Tyr Thr Lys Lys Ala Ala Val Ser Glu Leu
   35 40 45
   Leu Gln Ala Ser Ala Pro Tyr Lys Ala Asp Val Glu Leu Cys Val Tyr
   50 55 60
   Ser Thr Gly Lys Pro Ser Thr Cys Ser Gly Gly Ser Asn Gly Ile Ala
   65 70 75 80
   Ala Asp Ile Thr Thr Ala Lys Gly Tyr Val Ala Ser Val Lys Thr Gln
   85 90 95
   Ser Gly Gly Ile Thr Val Lys Gly Asn Gly Thr Leu Ala Asn Met Glu
   100 105 110
   Tyr Ile Leu Gln Ala Lys Gly Asn Ala Thr Ala Gly Val Thr Trp Thr
   115 120 125
   Thr Thr Cys Lys Gly Thr Asp Ala Ser Leu Phe Pro Ala Asn Phe Cys
   130 135 140
   Arg Ser Val Thr Lys
   145
<210> 103
   <211> 149
   <212> PRT
   <213> Haemophilus influenzae
<400> 103
   Met Lys Leu Thr Thr Gln Gln Thr Leu Lys Lys Gly Phe Thr Leu Ile
   1 5 10 15
   Glu Leu Met Ile Val Ile Ala Ile Ile Ala Ile Leu Ala Thr Ile Ala
   20 25 30
   Ile Pro Ser Tyr Gln Asn Tyr Thr Lys Lys Ala Ala Val Ser Glu Leu
   35 40 45
   Leu Gln Ala Ser Ala Pro Tyr Lys Ala Asp Val Glu Leu Cys Val Tyr
   50 55 60
   Ser Thr Asn Glu Ala Thr Lys Cys Thr Gly Gly Lys Asn Gly Ile Ala
   65 70 75 80
   Ala Asp Ile Thr Thr Ala Lys Gly Tyr Val Lys Ser Val Thr Thr Ser
   85 90 95
   Asn Gly Ala Ile Thr Val Lys Gly Asp Gly Thr Leu Ala Asn Met Glu
   100 105 110
   Tyr Ile Leu Gln Ala Ser Gly Asn Ala Ala Thr Gly Val Thr Trp Thr
   115 120 125
   Thr Thr Cys Lys Gly Thr Asp Ala Ser Leu Phe Pro Ala Asn Phe Cys
   130 135 140
   Gly Ser Val Thr Gln
   145
<210> 104
   <211> 149
   <212> PRT
   <213> Haemophilus influenzae
<400> 104
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 105
   <211> 149
   <212> PRT
   <213> Haemophilus influenzae
<400> 105
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 106
   <211> 149
   <212> PRT
   <213> Haemophilus influenzae
<220>
   <221> VARIANT
   <222> (1)...(149)
   <223> Xaa = glutamine or leucine
<400> 106
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Xaa Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 107
   <211> 149
   <212> PRT
   <213> Haemophilus influenzae
<400> 107
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 108
   <211> 149
   <212> PRT
   <213> Haemophilus influenzae
<400> 108
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 109
   <211> 149
   <212> PRT
   <213> Haemophilus influenzae
<400> 109
   Met Lys Leu Thr Thr Leu Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Asn Asn Gly Ile Ala
   65                  70                  75                  80
   Ala Asp Ile Lys Thr Ala Lys Gly Tyr Val Ala Ser Val Lys Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 110
   <211> 149
   <212> PRT
   <213> Haemophilus influenzae
<400> 110
   Met Lys Leu Thr Thr Leu Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Asn Asn Gly Ile Ala
   65                  70                  75                  80
   Ala Asp Ile Lys Thr Ala Lys Gly Tyr Val Ala Ser Val Lys Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 111
   <211> 149
   <212> PRT
   <213> Haemophilus influenzae
<400> 111
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Ser Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Ala Gly Asn Gly Thr Leu Asp Gly Met Ser
               100                 105                 110
   Tyr Thr Leu Thr Ala Glu Gly Asp Ser Ala Lys Gly Val Thr Trp Lys
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 112
   <211> 149
   <212> PRT
   <213> Haemophilus influenzae
<400> 112
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Ser Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Ala Gly Asn Gly Thr Leu Asp Gly Met Ser
               100                 105                 110
   Tyr Thr Leu Thr Ala Glu Gly Asp Ser Ala Lys Gly Val Thr Trp Lys
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 113
   <211> 149
   <212> PRT
   <213> Haemophilus influenzae
<400> 113
   Met Lys Leu Thr Thr Leu Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Asn Asn Gly Ile Ala
   65                  70                  75                  80
   Ala Asp Ile Lys Thr Ala Lys Gly Tyr Val Ala Ser Val Lys Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 114
   <211> 149
   <212> PRT
   <213> Haemophilus influenzae
<400> 114
   Met Lys Leu Thr Thr Leu Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Asn Asn Gly Ile Ala
   65                  70                  75                  80
   Ala Asp Ile Lys Thr Ala Lys Gly Tyr Val Ala Ser Val Lys Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 115
   <211> 149
   <212> PRT
   <213> Haemophilus influenzae
<400> 115
   Met Lys Leu Thr Thr Leu Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Asn Asn Gly Ile Ala
   65                  70                  75                  80
   Ala Asp Ile Lys Thr Ala Lys Gly Tyr Val Ala Ser Val Lys Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 116
   <211> 149
   <212> PRT
   <213> Haemophilus influenzae
<400> 116
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Thr Thr Asn Cys Thr Gly Gly Lys Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Glu Ala Ser Leu Phe Pro Ala Asn Phe Cys
       130                 135                 140
   Gly Ser Val Thr Gln
   145
<210> 117
   <211> 149
   <212> PRT
   <213> Haemophilus influenzae
<400> 117
   Met Lys Leu Thr Thr Leu Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Asn Asn Gly Ile Ala
   65                  70                  75                  80
   Ala Asp Ile Lys Thr Ala Lys Gly Tyr Val Ala Ser Val Lys Thr Gln
                   85                  90                  95
   Ser Gly Gly Ile Thr Val Lys Gly Asp Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 118
   <211> 149
   <212> PRT
   <213> Haemophilus influenzae
<400> 118
   Met Lys Leu Thr Thr Gln Gln Thr Leu Lys Lys Gly Phe Thr Leu Ile
   1               5                   10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Ser Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Lys Gly Asn Ala Thr Ala Gly Val Thr Trp Thr
           115                 120                 125
   Thr Thr Cys Lys Gly Thr Asp Ala Ser Leu Phe Pro Ala Asn Phe Cys
       130                 135                 140
   Arg Ser Val Thr Lys
   145
<210> 119
   <211> 149
   <212> PRT
   <213> Haemophilus influenzae
<400> 119
   Met Lys Leu Thr Thr Gln Gln Thr Leu Lys Lys Gly Phe Thr Leu Ile
   1               5                   10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Asn Glu Ala Thr Lys Cys Thr Gly Gly Lys Asn Gly Ile Ala
   65                  70                  75                  80
   Ala Asp Ile Thr Thr Ala Lys Gly Tyr Val Lys Ser Val Thr Thr Ser
                   85                  90                  95
   Asn Gly Ala Ile Thr Val Lys Gly Asp Gly Thr Leu Ala Asn Met Glu
               100                 105                 110
   Tyr Ile Leu Gln Ala Ser Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 120
   <211> 149
   <212> PRT
   <213> Haemophilus influenzae
<400> 120
   Met Lys Leu Thr Thr Leu Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ala Val Ser Glu Leu
           35                  40                  45
   Leu Gln Ala Ser Ala Pro Tyr Lys Ala Asp Val Glu Leu Cys Val Tyr
       50                  55                  60
   Ser Thr Gly Lys Pro Ser Thr Cys Ser Gly Gly Ser Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Thr Gly Asn Ala Ala Thr Gly Val Thr Trp Thr
           115                 120                 125
   Thr Thr Cys Lys Gly Thr Asp Ala Ser Leu Phe Pro Ala Asn Phe Cys
       130                 135                 140
   Gly Ser Val Thr Gln
   145
<210> 121
   <211> 149
   <212> PRT
   <213> Haemophilus influenzae
<400> 121
   Met Lys Leu Thr Thr Gln Gln Thr Leu Lys Lys Gly Phe Thr Leu Ile
    1               5                  10                  15
   Glu Leu Met Ile Val Ile Ala Ile Ile Ala Ile Leu Ala Thr Ile Ala
               20                  25                  30
   Ile Pro Ser Tyr Gln Asn Tyr Thr Lys Lys Ala Ser Val Ser Glu Leu
           35                  40                  45
   Leu Gln Ala Ser Ala Pro Tyr Lys Ser Asp Val Glu Leu Cys Val Tyr
       50                  55                  60
   Ser Thr Gly Lys Pro Ser Thr Cys Ser Gly Gly Ser Asn Gly Ile Ala
   65                  70                  75                  80
   Ala Asp Ile Thr Thr Ala Lys Gly Tyr Val Ala Ser Val Lys Thr Gln
                   85                  90                  95
   Ser Gly Gly Ile Thr Val Lys Gly Asn Gly Thr Leu Ala Asn Met Glu
               100                 105                 110
   Tyr Ile Leu Gln Ala Lys Gly Asn Ala Thr Ala Gly Val Thr Trp Thr
           115                 120                 125
   Thr Thr Cys Lys Gly Thr Asp Ala Ser Leu Phe Pro Ala Asn Phe Cys
       130                 135                 140
   Arg Ser Val Thr Lys
   145
<210> 122
   <211> 144
   <212> PRT
   <213> Haemophilus influenzae
<400> 122
   Ser Ala Gln Ile Gln Lys Ala Glu Gln Asn Asp Val Lys Leu Ala Pro
   1 5 10 15
   Pro Thr Asp Val Arg Ser Gly Tyr Ile Arg Leu Val Lys Asn Val Asn
   20 25 30
   Tyr Tyr Ile Asp Ser Glu Ser Ile Trp Val Asp Asn Gln Glu Pro Gln
   35 40 45
   Ile Val His Phe Asp Ala Val Val Asn Leu Asp Lys Gly Leu Tyr Val
   50 55 60
   Tyr Pro Glu Pro Lys Arg Tyr Ala Arg Ser Val Arg Gln Tyr Lys Ile
   65 70 75 80
   Leu Asn Cys Ala Asn Tyr His Leu Thr Gln Val Arg Thr Asp Phe Tyr
   85 90 95
   Asp Glu Phe Trp Gly Gln Gly Leu Arg Ala Ala Pro Lys Lys Gln Lys
   100 105 110
   Lys His Thr Leu Ser Leu Thr Pro Asp Thr Thr Leu Tyr Asn Ala Ala
   115 120 125
   Gln Ile Ile Cys Ala Asn Tyr Gly Glu Ala Phe Ser Val Asp Lys Lys
   130 135 140
<210> 123
   <211> 143
   <212> PRT
   <213> Haemophilus influenzae
<400> 123
   Ala Gln Ile Gln Lys Ala Glu Gln Asn Asp Val Lys Leu Ala Pro Pro
   1 5 10 15
   Thr Asp Val Arg Ser Gly Tyr Ile Arg Leu Val Lys Asn Val Asn Tyr
   20 25 30
   Tyr Ile Asp Ser Glu Ser Ile Trp Val Asp Asn Gln Glu Pro Gln Ile
   35 40 45
   Val His Phe Asp Ala Val Val Asn Leu Asp Lys Gly Leu Tyr Val Tyr
   50 55 60
   Pro Glu Pro Lys Arg Tyr Ala Arg Ser Val Arg Gln Tyr Lys Ile Leu
   65 70 75 80
   Asn Cys Ala Asn Tyr His Leu Thr Gln Val Arg Thr Asp Phe Tyr Asp
   85 90 95
   Glu Phe Trp Gly Gln Gly Leu Arg Ala Ala Pro Lys Lys Gln Lys Lys
   100 105 110
   His Thr Leu Ser Leu Thr Pro Asp Thr Thr Leu Tyr Asn Ala Ala Gln
   115 120 125
   Ile Ile Cys Ala Asn Tyr Gly Glu Ala Phe Ser Val Asp Lys Lys
   130 135 140
<210> 124
   <211> 142
   <212> PRT
   <213> Haemophilus influenzae
<400> 124
   Gln Ile Gln Lys Ala Glu Gln Asn Asp Val Lys Leu Ala Pro Pro Thr
    1               5                  10                  15
   Asp Val Arg Ser Gly Tyr Ile Arg Leu Val Lys Asn Val Asn Tyr Tyr
               20                  25                  30
   Ile Asp Ser Glu Ser Ile Trp Val Asp Asn Gln Glu Pro Gln Ile Val
           35                  40                  45
   His Phe Asp Ala Val Val Asn Leu Asp Lys Gly Leu Tyr Val Tyr Pro
       50                  55                  60
   Glu Pro Lys Arg Tyr Ala Arg Ser Val Arg Gln Tyr Lys Ile Leu Asn
   65                  70                  75                  80
   Cys Ala Asn Tyr His Leu Thr Gln Val Arg Thr Asp Phe Tyr Asp Glu
                   85                  90                  95
   Phe Trp Gly Gln Gly Leu Arg Ala Ala Pro Lys Lys Gln Lys Lys His
               100                 105                 110
   Thr Leu Ser Leu Thr Pro Asp Thr Thr Leu Tyr Asn Ala Ala Gln Ile
           115                 120                 125
   Ile Cys Ala Asn Tyr Gly Glu Ala Phe Ser Val Asp Lys Lys
       130                 135                 140
<210> 125
   <211> 141
   <212> PRT
   <213> Haemophilus influenzae
<400> 125
   Ile Gln Lys Ala Glu Gln Asn Asp Val Lys Leu Ala Pro Pro Thr Asp
    1               5                  10                  15
   Val Arg Ser Gly Tyr Ile Arg Leu Val Lys Asn Val Asn Tyr Tyr Ile
               20                  25                  30
   Asp Ser Glu Ser Ile Trp Val Asp Asn Gln Glu Pro Gln Ile Val His
           35                  40                  45
   Phe Asp Ala Val Val Asn Leu Asp Lys Gly Leu Tyr Val Tyr Pro Glu
       50                  55                  60
   Pro Lys Arg Tyr Ala Arg Ser Val Arg Gln Tyr Lys Ile Leu Asn Cys
   65                  70                  75                  80
   Ala Asn Tyr His Leu Thr Gln Val Arg Thr Asp Phe Tyr Asp Glu Phe
                   85                  90                  95
   Trp Gly Gln Gly Leu Arg Ala Ala Pro Lys Lys Gln Lys Lys His Thr
               100                 105                 110
   Leu Ser Leu Thr Pro Asp Thr Thr Leu Tyr Asn Ala Ala Gln Ile Ile
           115                 120                 125
   Cys Ala Asn Tyr Gly Glu Ala Phe Ser Val Asp Lys Lys
       130                 135                 140
<210> 126
   <211> 139
   <212> PRT
   <213> Haemophilus influenzae
<400> 126
   Lys Ala Glu Gln Asn Asp Val Lys Leu Ala Pro Pro Thr Asp Val Arg
    1               5                  10                  15
   Ser Gly Tyr Ile Arg Leu Val Lys Asn Val Asn Tyr Tyr Ile Asp Ser
               20                  25                  30
   Glu Ser Ile Trp Val Asp Asn Gln Glu Pro Gln Ile Val His Phe Asp
           35                  40                  45
   Ala Val Val Asn Leu Asp Lys Gly Leu Tyr Val Tyr Pro Glu Pro Lys
       50                  55                  60
   Arg Tyr Ala Arg Ser Val Arg Gln Tyr Lys Ile Leu Asn Cys Ala Asn
   65                  70                  75                  80
   Tyr His Leu Thr Gln Val Arg Thr Asp Phe Tyr Asp Glu Phe Trp Gly
                   85                  90                  95
   Gln Gly Leu Arg Ala Ala Pro Lys Lys Gln Lys Lys His Thr Leu Ser
               100                 105                 110
   Leu Thr Pro Asp Thr Thr Leu Tyr Asn Ala Ala Gln Ile Ile Cys Ala
           115                 120                 125
   Asn Tyr Gly Glu Ala Phe Ser Val Asp Lys Lys
       130                 135
<210> 127
   <211> 110
   <212> PRT
   <213> Haemophilus influenzae
<400> 127
   Thr Lys Lys Ala Ala Val Ser Glu Leu Leu Gln Ala Ser Ala Pro Tyr
    1               5                  10                  15
   Lys Ala Asp Val Glu Leu Cys Val Tyr Ser Thr Asn Glu Thr Thr Asn
               20                  25                  30
   Cys Thr Gly Gly Lys Asn Gly Ile Ala Ala Asp Ile Thr Thr Ala Lys
           35                  40                  45
   Gly Tyr Val Lys Ser Val Thr Thr Ser Asn Gly Ala Ile Thr Val Lys
       50                  55                  60
   Gly Asp Gly Thr Leu Ala Asn Met Glu Tyr Ile Leu Gln Ala Thr Gly
   65                  70                  75                  80
   Asn Ala Ala Thr Gly Val Thr Trp Thr Thr Thr Cys Lys Gly Thr Asp
                   85                  90                  95
   Ala Ser Leu Phe Pro Ala Asn Phe Cys Gly Ser Val Thr Gln
               100                 105                 110
<210> 128
   <211> 25
   <212> PRT
   <213> Haemophilus influenzae
<400> 128
   Pro Lys Arg Tyr Ala Arg Ser Val Arg Gln Tyr Lys Ile Leu Asn Cys
    1               5                  10                  15
   Ala Asn Tyr His Leu Thr Gln Val Arg
               20                  25
<210> 129
   <211> 66
   <212> DNA
   <213> Artificial Sequence
<220>
   <223> pelB signal peptide
<400> 129
   atgaaatacc tgctgccgac cgctgctgct ggtctgctgc tcctcgctgc ccagccggcg 60
   atggcc 66
<210> 130
   <211> 22
   <212> PRT
   <213> Artificial Sequence
<220>
   <223> pelB signal peptide
<400> 130
   Met Lys Tyr Leu Leu Pro Thr Ala Ala Ala Gly Leu Leu Leu Leu Ala
   1 5 10 15
   Ala Gln Pro Ala Met Ala
   20
<210> 131
   <211> 57
   <212> DNA
   <213> Artificial Sequence
<220>
   <223> FlgI signal peptide
<400> 131
   atgattaaat ttctctctgc attaattctt ctactggtca cgacggcggc tcaggct 57
<210> 132
   <211> 19
   <212> PRT
   <213> Artificial Sequence
<220>
   <223> FlgI signal peptide
<400> 132
   Met Ile Lys Phe Leu Ser Ala Leu Ile Leu Leu Leu Val Thr Thr Ala
   1 5 10 15
   Ala Gln Ala
<210> 133
   <211> 69
   <212> DNA
   <213> Artificial Sequence
<220>
   <223> NadA signal peptide
<400> 133
   atgaaacact ttccatccaa agtactgacc acagccatcc ttgccacttt ctgtagcggc 60
   gcactggca                                                         69
<210> 134
   <211> 23
   <212> PRT
   <213> Artificial Sequence
<220>
   <223> NadA signal peptide
<400> 134
   Met Lys His Phe Pro Ser Lys Val Leu Thr Thr Ala Ile Leu Ala Thr
    1               5                  10                  15
   Phe Cys Ser Gly Ala Leu Ala
               20
<210> 135
   <211> 852
   <212> DNA
   <213> Artificial Sequence
<220>
   <223> LVL312
<400> 135
   atgattaaat ttctctctgc attaattctt ctactggtca cgacggcggc tcaggctgag 60
   actaaaaaag cagcggtatc tgaattactg caagcgtcag cgccttataa ggctgatgtg 120
   gaattatgtg tatatagcac aaatgaaaca acaaactgta cgggtggaaa aaatggtatt 180
   gcagcagata taaccacagc aaaaggctat gtaaaatcag tgacaacaag caacggtgca 240
   ataacagtaa aaggggatgg cacattggca aatatggaat atattttgca agctacaggt 300
   aatgctgcaa caggtgtaac ttggacaaca acttgcaaag gaacggatgc ctctttattt 360
   ccagcaaatt tttgcggaag tgtcacacaa ggcggcgcgc agattcagaa ggctgaacaa 420
   aatgatgtga agctggcacc gccgactgat gtacgaagcg gatatatacg tttggtaaag 480
   aatgtgaatt attacatcga tagtgaatcg atctgggtgg ataaccaaga gccacaaatt 540
   gtacattttg atgcagtggt gaatttagat aagggattgt atgtttatcc tgagcctaaa 600
   cgttatgcac gttctgttcg tcagtataag atcttgaatt gtgcaaatta tcatttaact 660
   caagtacgaa ctgatttcta tgatgaattt tggggacagg gtttgcgggc agcacctaaa 720
   aagcaaaaga aacatacgtt aagtttaaca cctgatacaa cgctttataa tgctgctcag 780
   attatttgtg cgaactatgg tgaagcattt tcagttgata aaaaaggcgg ccaccaccac 840
   caccaccact aa                                                     852
<210> 136
   <211> 283
   <212> PRT
   <213> Artificial Sequence
<220>
   <223> LVL312
<400> 136
   Met Ile Lys Phe Leu Ser Ala Leu Ile Leu Leu Leu Val Thr Thr Ala
   1 5 10 15
   Ala Gln Ala Glu Thr Lys Lys Ala Ala Val Ser Glu Leu Leu Gln Ala
   20 25 30
   Ser Ala Pro Tyr Lys Ala Asp Val Glu Leu Cys Val Tyr Ser Thr Asn
   35 40 45
   Glu Thr Thr Asn Cys Thr Gly Gly Lys Asn Gly Ile Ala Ala Asp Ile
   50 55 60
   Thr Thr Ala Lys Gly Tyr Val Lys Ser Val Thr Thr Ser Asn Gly Ala
   65 70 75 80
   Ile Thr Val Lys Gly Asp Gly Thr Leu Ala Asn Met Glu Tyr Ile Leu
   85 90 95
   Gln Ala Thr Gly Asn Ala Ala Thr Gly Val Thr Trp Thr Thr Thr Cys
   100 105 110
   Lys Gly Thr Asp Ala Ser Leu Phe Pro Ala Asn Phe Cys Gly Ser Val
   115 120 125
   Thr Gln Gly Gly Ala Gln Ile Gln Lys Ala Glu Gln Asn Asp Val Lys
   130 135 140
   Leu Ala Pro Pro Thr Asp Val Arg Ser Gly Tyr Ile Arg Leu Val Lys
   145 150 155 160
   Asn Val Asn Tyr Tyr Ile Asp Ser Glu Ser Ile Trp Val Asp Asn Gln
   165 170 175
   Glu Pro Gln Ile Val His Phe Asp Ala Val Val Asn Leu Asp Lys Gly
   180 185 190
   Leu Tyr Val Tyr Pro Glu Pro Lys Arg Tyr Ala Arg Ser Val Arg Gln
   195 200 205
   Tyr Lys Ile Leu Asn Cys Ala Asn Tyr His Leu Thr Gln Val Arg Thr
   210 215 220
   Asp Phe Tyr Asp Glu Phe Trp Gly Gln Gly Leu Arg Ala Ala Pro Lys
   225 230 235 240
   Lys Gln Lys Lys His Thr Leu Ser Leu Thr Pro Asp Thr Thr Leu Tyr
   245 250 255
   Asn Ala Ala Gln Ile Ile Cys Ala Asn Tyr Gly Glu Ala Phe Ser Val
   260 265 270
   Asp Lys Lys Gly Gly His His His His His His
   275 280
<210> 137
   <211> 855
   <212> DNA
   <213> Artificial Sequence
<220>
   <223> LVL291
<400> 137
   atgaaatacc tgctgccgac cgctgctgct ggtctgctgc tcctcgctgc ccagccggcg 60
   atggcccaga ttcagaaggc tgaacaaaat gatgtgaagc tggcaccgcc gactgatgta 120
   cgaagcggat atatacgttt ggtaaagaat gtgaattatt acatcgatag tgaatcgatc 180
   tgggtggata accaagagcc acaaattgta cattttgatg cagtggtgaa tttagataag 240
   ggattgtatg tttatcctga gcctaaacgt tatgcacgtt ctgttcgtca gtataagatc 300
   ttgaattgtg caaattatca tttaactcaa gtacgaactg atttctatga tgaattttgg 360
   ggacagggtt tgcgggcagc acctaaaaag caaaagaaac atacgttaag tttaacacct 420
   gatacaacgc tttataatgc tgctcagatt atttgtgcga actatggtga agcattttca 480
   gttgataaaa aaggcggcac taaaaaagca gcggtatctg aattactgca agcgtcagcg 540
   ccttataagg ctgatgtgga attatgtgta tatagcacaa atgaaacaac aaactgtacg 600
   ggtggaaaaa atggtattgc agcagatata accacagcaa aaggctatgt aaaatcagtg 660
   acaacaagca acggtgcaat aacagtaaaa ggggatggca cattggcaaa tatggaatat 720
   attttgcaag ctacaggtaa tgctgcaaca ggtgtaactt ggacaacaac ttgcaaagga 780
   acggatgcct ctttatttcc agcaaatttt tgcggaagtg tcacacaagg cggccaccac 840
   caccaccacc actaa                                                  855
<210> 138
   <211> 284
   <212> PRT
   <213> Artificial Sequence
<220>
   <223> LVL291
<400> 138
   Met Lys Tyr Leu Leu Pro Thr Ala Ala Ala Gly Leu Leu Leu Leu Ala
    1               5                  10                  15
   Ala Gln Pro Ala Met Ala Gln Ile Gln Lys Ala Glu Gln Asn Asp Val
               20                  25                  30
   Lys Leu Ala Pro Pro Thr Asp Val Arg Ser Gly Tyr Ile Arg Leu Val
           35                  40                  45
   Lys Asn Val Asn Tyr Tyr Ile Asp Ser Glu Ser Ile Trp Val Asp Asn
       50                  55                  60
   Gln Glu Pro Gln Ile Val His Phe Asp Ala Val Val Asn Leu Asp Lys
   65                  70                  75                  80
   Gly Leu Tyr Val Tyr Pro Glu Pro Lys Arg Tyr Ala Arg Ser Val Arg
                   85                  90                  95
   Gln Tyr Lys Ile Leu Asn Cys Ala Asn Tyr His Leu Thr Gln Val Arg
               100                 105                 110
   Thr Asp Phe Tyr Asp Glu Phe Trp Gly Gln Gly Leu Arg Ala Ala Pro
           115                 120                 125
   Lys Lys Gln Lys Lys His Thr Leu Ser Leu Thr Pro Asp Thr Thr Leu
       130                 135                 140
   Tyr Asn Ala Ala Gln Ile Ile Cys Ala Asn Tyr Gly Glu Ala Phe Ser
   145                 150                 155                 160
   Val Asp Lys Lys Gly Gly Thr Lys Lys Ala Ala Val Ser Glu Leu Leu
                   165                 170                 175
   Gln Ala Ser Ala Pro Tyr Lys Ala Asp Val Glu Leu Cys Val Tyr Ser
               180                 185                 190
   Thr Asn Glu Thr Thr Asn Cys Thr Gly Gly Lys Asn Gly Ile Ala Ala
           195                 200                 205
   Asp Ile Thr Thr Ala Lys Gly Tyr Val Lys Ser Val Thr Thr Ser Asn
       210                 215                 220
   Gly Ala Ile Thr Val Lys Gly Asp Gly Thr Leu Ala Asn Met Glu Tyr
   225                 230                 235                 240
   Ile Leu Gln Ala Thr Gly Asn Ala Ala Thr Gly Val Thr Trp Thr Thr
                   245                 250                 255
   Thr Cys Lys Gly Thr Asp Ala Ser Leu Phe Pro Ala Asn Phe Cys Gly
               260                 265                 270
   Ser Val Thr Gln Gly Gly His His His His His His
           275                 280
<210> 139
   <211> 852
   <212> DNA
   <213> Artificial Sequence
<220>
   <223> LVL268
<400> 139
   atgaaatacc tgctgccgac cgctgctgct ggtctgctgc tcctcgctgc ccagccggcg 60
   atggccgata ttcagaaggc tgaacaaaat gatgtgaagc tggcaccgcc gactgatgta 120
   cgaagcggat atatacgttt ggtaaagaat gtgaattatt acatcgatag tgaatcgatc 180
   tgggtggata accaagagcc acaaattgta cattttgatg cagtggtgaa tttagataag 240
   ggattgtatg tttatcctga gcctaaacgt tatgcacgtt ctgttcgtca gtataagatc 300
   ttgaattgtg caaattatca tttaactcaa gtacgaactg atttctatga tgaattttgg 360
   ggacagggtt tgcgggcagc acctaaaaag caaaagaaac atacgttaag tttaacacct 420
   gatacaacgc tttataatgc tgctcagatt atttgtgcga actatggtga agcattttca 480
   gttgataaaa aaggcggcac taaaaaagca gcggtatctg aattactgca agcgtcagcg 540
   ccttataagg ctgatgtgga attatgtgta tatagcacaa atgaaacaac aaactgtacg 600
   ggtggaaaaa atggtattgc agcagatata accacagcaa aaggctatgt aaaatcagtg 660
   acaacaagca acggtgcaat aacagtaaaa ggggatggca cattggcaaa tatggaatat 720
   attttgcaag ctacaggtaa tgctgcaaca ggtgtaactt ggacaacaac ttgcaaagga 780
   acggatgcct ctttatttcc agcaaatttt tgcggaagtg tcacacaagg cggccaccac 840
   caccaccacc ac                                                     852
<210> 140
   <211> 284
   <212> PRT
   <213> Artificial Sequence
<220>
   <223> LVL268
<400> 140
   Met Lys Tyr Leu Leu Pro Thr Ala Ala Ala Gly Leu Leu Leu Leu Ala
   1               5                   10                  15
   Ala Gln Pro Ala Met Ala Asp Ile Gln Lys Ala Glu Gln Asn Asp Val
               20                  25                  30
   Lys Leu Ala Pro Pro Thr Asp Val Arg Ser Gly Tyr Ile Arg Leu Val
           35                  40                  45
   Lys Asn Val Asn Tyr Tyr Ile Asp Ser Glu Ser Ile Trp Val Asp Asn
       50                  55                  60
   Gln Glu Pro Gln Ile Val His Phe Asp Ala Val Val Asn Leu Asp Lys
   65                  70                  75                  80
   Gly Leu Tyr Val Tyr Pro Glu Pro Lys Arg Tyr Ala Arg Ser Val Arg
                   85                  90                  95
   Gln Tyr Lys Ile Leu Asn Cys Ala Asn Tyr His Leu Thr Gln Val Arg
               100                 105                 110
   Thr Asp Phe Tyr Asp Glu Phe Trp Gly Gln Gly Leu Arg Ala Ala Pro
           115                 120                 125
   Lys Lys Gln Lys Lys His Thr Leu Ser Leu Thr Pro Asp Thr Thr Leu
       130                 135                 140
   Tyr Asn Ala Ala Gln Ile Ile Cys Ala Asn Tyr Gly Glu Ala Phe Ser
   145                 150                 155                 160
   Val Asp Lys Lys Gly Gly Thr Lys Lys Ala Ala Val Ser Glu Leu Leu
                   165                 170                 175
   Gln Ala Ser Ala Pro Tyr Lys Ala Asp Val Glu Leu Cys Val Tyr Ser
               180                 185                 190
   Thr Asn Glu Thr Thr Asn Cys Thr Gly Gly Lys Asn Gly Ile Ala Ala
           195                 200                 205
   Asp Ile Thr Thr Ala Lys Gly Tyr Val Lys Ser Val Thr Thr Ser Asn
       210                 215                 220
   Gly Ala Ile Thr Val Lys Gly Asp Gly Thr Leu Ala Asn Met Glu Tyr
   225                 230                 235                 240
   Ile Leu Gln Ala Thr Gly Asn Ala Ala Thr Gly Val Thr Trp Thr Thr
                   245                 250                 255
   Thr Cys Lys Gly Thr Asp Ala Ser Leu Phe Pro Ala Asn Phe Cys Gly
               260                 265                 270
   Ser Val Thr Gln Gly Gly His His His His His His
           275                 280
<210> 141
   <211> 867
   <212> DNA
   <213> Artificial Sequence
<220>
   <223> LVL269
<400> 141
   atgaaacact ttccatccaa agtactgacc acagccatcc ttgccacttt ctgtagcggc 60
   gcactggcag ccacaaacga cgacgataag gctgaacaaa atgatgtgaa gctggcaccg 120
   ccgactgatg tacgaagcgg atatatacgt ttggtaaaga atgtgaatta ttacatcgat 180
   agtgaatcga tctgggtgga taaccaagag ccacaaattg tacattttga tgcagtggtg 240
   aatttagata agggattgta tgtttatcct gagcctaaac gttatgcacg ttctgttcgt 300
   cagtataaga tcttgaattg tgcaaattat catttaactc aagtacgaac tgatttctat 360
   gatgaatttt ggggacaggg tttgcgggca gcacctaaaa agcaaaagaa acatacgtta 420
   agtttaacac ctgatacaac gctttataat gctgctcaga ttatttgtgc gaactatggt 480
   gaagcatttt cagttgataa aaaaggcggc actaaaaaag cagcggtatc tgaattactg 540
   caagcgtcag cgccttataa ggctgatgtg gaattatgtg tatatagcac aaatgaaaca 600
   acaaactgta cgggtggaaa aaatggtatt gcagcagata taaccacagc aaaaggctat 660
   gtaaaatcag tgacaacaag caacggtgca ataacagtaa aaggggatgg cacattggca 720
   aatatggaat atattttgca agctacaggt aatgctgcaa caggtgtaac ttggacaaca 780
   acttgcaaag gaacggatgc ctctttattt ccagcaaatt tttgcggaag tgtcacacaa 840
   ggcggccacc accaccacca ccactaa                                     867
<210> 142
   <211> 288
   <212> PRT
   <213> Artificial Sequence
<220>
   <223> LVL269
<400> 142
   Met Lys His Phe Pro Ser Lys Val Leu Thr Thr Ala Ile Leu Ala Thr
    1               5                  10                  15
   Phe Cys Ser Gly Ala Leu Ala Ala Thr Asn Asp Asp Asp Lys Ala Glu
               20                  25                  30
   Gln Asn Asp Val Lys Leu Ala Pro Pro Thr Asp Val Arg Ser Gly Tyr
           35                  40                  45
   Ile Arg Leu Val Lys Asn Val Asn Tyr Tyr Ile Asp Ser Glu Ser Ile
       50                  55                  60
   Trp Val Asp Asn Gln Glu Pro Gln Ile Val His Phe Asp Ala Val Val
   65                  70                  75                  80
   Asn Leu Asp Lys Gly Leu Tyr Val Tyr Pro Glu Pro Lys Arg Tyr Ala
                   85                  90                  95
   Arg Ser Val Arg Gln Tyr Lys Ile Leu Asn Cys Ala Asn Tyr His Leu
               100                 105                 110
   Thr Gln Val Arg Thr Asp Phe Tyr Asp Glu Phe Trp Gly Gln Gly Leu
           115                 120                 125
   Arg Ala Ala Pro Lys Lys Gln Lys Lys His Thr Leu Ser Leu Thr Pro
       130                 135                 140
   Asp Thr Thr Leu Tyr Asn Ala Ala Gln Ile Ile Cys Ala Asn Tyr Gly
   145                 150                 155                 160
   Glu Ala Phe Ser Val Asp Lys Lys Gly Gly Thr Lys Lys Ala Ala Val
                   165                 170                 175
   Ser Glu Leu Leu Gln Ala Ser Ala Pro Tyr Lys Ala Asp Val Glu Leu
               180                 185                 190
   Cys Val Tyr Ser Thr Asn Glu Thr Thr Asn Cys Thr Gly Gly Lys Asn
           195                 200                 205
   Gly Ile Ala Ala Asp Ile Thr Thr Ala Lys Gly Tyr Val Lys Ser Val
       210                 215                 220
   Thr Thr Ser Asn Gly Ala Ile Thr Val Lys Gly Asp Gly Thr Leu Ala
   225                 230                 235                 240
   Asn Met Glu Tyr Ile Leu Gln Ala Thr Gly Asn Ala Ala Thr Gly Val
                   245                 250                 255
   Thr Trp Thr Thr Thr Cys Lys Gly Thr Asp Ala Ser Leu Phe Pro Ala
               260                 265                 270
   Asn Phe Cys Gly Ser Val Thr Gln Gly Gly His His His His His His
           275                 280                 285
<210> 143
   <211> 792
   <212> DNA
   <213> Artificial Sequence
<220>
   <223> LVL270
<400> 143
   atgcaccacc accaccacca cagcgcgcag attcagaagg ctgaacaaaa tgatgtgaag 60
   ctggcaccgc cgactgatgt acgaagcgga tatatacgtt tggtaaagaa tgtgaattat 120
   tacatcgata gtgaatcgat ctgggtggat aaccaagagc cacaaattgt acattttgat 180
   gcagtggtga atttagataa gggattgtat gtttatcctg agcctaaacg ttatgcacgt 240
   tctgttcgtc agtataagat cttgaattgt gcaaattatc atttaactca agtacgaact 300
   gatttctatg atgaatttgg gggacagggt ttgcgggcag cacctaaaaa gcaaaagaaa 360
   catacgttaa gtttaacacc tgatacaacg ctttataatg ctgctcagat tatttgtgcg 420
   aactatggtg aagcattttc agttgataaa aaaggcggca ctaaaaaagc agcggtatct 480
   gaattactgc aagcgtcagc gccttataag gctgatgtgg aattatgtgt atatagcaca 540
   aatgaaacaa caaactgtac gggtggaaaa aatggtattg cagcagatat aaccacagca 600
   aaaggctatg taaaatcagt gacaacaagc aacggtgcaa taacagtaaa aggggatggc 660
   acattggcaa atatggaata tattttgcaa gctacaggta atgctgcaac aggtgtaact 720
   tggacaacaa cttgcaaagg aacggatgcc tctttatttc cagcaaattt ttgcggaagt 780
   gtcacacaat aa                                                      792
<210> 144
   <211> 263
   <212> PRT
   <213> Artificial Sequence
<220>
   <223> LVL270
<400> 144
   Met His His His His His His Ser Ala Gln Ile Gln Lys Ala Glu Gln
   1               5                   10                  15
   Asn Asp Val Lys Leu Ala Pro Pro Thr Asp Val Arg Ser Gly Tyr Ile
               20                  25                  30
   Arg Leu Val Lys Asn Val Asn Tyr Tyr Ile Asp Ser Glu Ser Ile Trp
           35                  40                  45
   Val Asp Asn Gln Glu Pro Gln Ile Val His Phe Asp Ala Val Val Asn
       50                  55                  60
   Leu Asp Lys Gly Leu Tyr Val Tyr Pro Glu Pro Lys Arg Tyr Ala Arg
   65                  70                  75                  80
   Ser Val Arg Gln Tyr Lys Ile Leu Asn Cys Ala Asn Tyr His Leu Thr
                   85                  90                  95
   Gln Val Arg Thr Asp Phe Tyr Asp Glu Phe Trp Gly Gln Gly Leu Arg
               100                 105                 110
   Ala Ala Pro Lys Lys Gln Lys Lys His Thr Leu Ser Leu Thr Pro Asp
           115                 120                 125
   Thr Thr Leu Tyr Asn Ala Ala Gln Ile Ile Cys Ala Asn Tyr Gly Glu
       130                 135                 140
   Ala Phe Ser Val Asp Lys Lys Gly Gly Thr Lys Lys Ala Ala Val Ser
   145                 150                 155                 160
   Glu Leu Leu Gln Ala Ser Ala Pro Tyr Lys Ala Asp Val Glu Leu Cys
                   165                 170                 175
   Val Tyr Ser Thr Asn Glu Thr Thr Asn Cys Thr Gly Gly Lys Asn Gly
               180                 185                 190
   Ile Ala Ala Asp Ile Thr Thr Ala Lys Gly Tyr Val Lys Ser Val Thr
           195                 200                 205
   Thr Ser Asn Gly Ala Ile Thr Val Lys Gly Asp Gly Thr Leu Ala Asn
       210                 215                 220
   Met Glu Tyr Ile Leu Gln Ala Thr Gly Asn Ala Ala Thr Gly Val Thr
   225                 230                 235                 240
   Trp Thr Thr Thr Cys Lys Gly Thr Asp Ala Ser Leu Phe Pro Ala Asn
                   245                 250                 255
   Phe Cys Gly Ser Val Thr Gln
               260
<210> 145
   <211> 852
   <212> DNA
   <213> Artificial Sequence
<220>
   <223> LVL315
<400> 145
   atgaaatacc tgctgccgac cgctgctgct ggtctgctgc tcctcgctgc ccagccggcg 60
   atggccatgg ataaggctga acaaaatgat gtgaagctgg caccgccgac tgatgtacga 120
   agcggatata tacgtttggt aaagaatgtg aattattaca tcgatagtga atcgatctgg 180
   gtggataacc aagagccaca aattgtacat tttgatgcag tggtgaattt agataaggga 240
   ttgtatgttt atcctgagcc taaacgttat gcacgttctg ttcgtcagta taagatcttg 300
   aattgtgcaa attatcattt aactcaagta cgaactgatt tctatgatga attttgggga 360
   cagggtttgc gggcagcacc taaaaagcaa aagaaacata cgttaagttt aacacctgat 420
   acaacgcttt ataatgctgc tcagattatt tgtgcgaact atggtgaagc attttcagtt 480
   gataaaaaag gcggcactaa aaaagcagcg gtatctgaat tactgcaagc gtcagcgcct 540
   tataaggctg atgtggaatt atgtgtatat agcacaaatg aaacaacaaa ctgtacgggt 600
   ggaaaaaatg gtattgcagc agatataacc acagcaaaag gctatgtaaa atcagtgaca 660
   acaagcaacg gtgcaataac agtaaaaggg gatggcacat tggcaaatat ggaatatatt 720
   ttgcaagcta caggtaatgc tgcaacaggt gtaacttgga caacaacttg caaaggaacg 780
   gatgcctctt tatttccagc aaatttttgc ggaagtgtca cacaaggcgg ccaccaccac 840
   caccaccact aa                                                      852
<210> 146
   <211> 283
   <212> PRT
   <213> Artificial Sequence
<220>
   <223> LVL315
<400> 146
   Met Lys Tyr Leu Leu Pro Thr Ala Ala Ala Gly Leu Leu Leu Leu Ala
    1               5                  10                  15
   Ala Gln Pro Ala Met Ala Met Asp Lys Ala Glu Gln Asn Asp Val Lys
               20                  25                  30
   Leu Ala Pro Pro Thr Asp Val Arg Ser Gly Tyr Ile Arg Leu Val Lys
           35                  40                  45
   Asn Val Asn Tyr Tyr Ile Asp Ser Glu Ser Ile Trp Val Asp Asn Gln
       50                  55                  60
   Glu Pro Gln Ile Val His Phe Asp Ala Val Val Asn Leu Asp Lys Gly
   65                  70                  75                  80
   Leu Tyr Val Tyr Pro Glu Pro Lys Arg Tyr Ala Arg Ser Val Arg Gln
                   85                  90                  95
   Tyr Lys Ile Leu Asn Cys Ala Asn Tyr His Leu Thr Gln Val Arg Thr
               100                 105                 110
   Asp Phe Tyr Asp Glu Phe Trp Gly Gln Gly Leu Arg Ala Ala Pro Lys
           115                 120                 125
   Lys Gln Lys Lys His Thr Leu Ser Leu Thr Pro Asp Thr Thr Leu Tyr
       130                 135                 140
   Asn Ala Ala Gln Ile Ile Cys Ala Asn Tyr Gly Glu Ala Phe Ser Val
   145                 150                 155                 160
   Asp Lys Lys Gly Gly Thr Lys Lys Ala Ala Val Ser Glu Leu Leu Gln
                   165                 170                 175
   Ala Ser Ala Pro Tyr Lys Ala Asp Val Glu Leu Cys Val Tyr Ser Thr
               180                 185                 190
   Asn Glu Thr Thr Asn Cys Thr Gly Gly Lys Asn Gly Ile Ala Ala Asp
           195                 200                 205
   Ile Thr Thr Ala Lys Gly Tyr Val Lys Ser Val Thr Thr Ser Asn Gly
       210                 215                 220
   Ala Ile Thr Val Lys Gly Asp Gly Thr Leu Ala Asn Met Glu Tyr Ile
   225                 230                 235                 240
   Leu Gln Ala Thr Gly Asn Ala Ala Thr Gly Val Thr Trp Thr Thr Thr
                   245                 250                 255
   Cys Lys Gly Thr Asp Ala Ser Leu Phe Pro Ala Asn Phe Cys Gly Ser
               260                 265                 270
   Val Thr Gln Gly Gly His His His His His His
           275                 280
<210> 147
   <211> 831
   <212> DNA
   <213> Artificial Sequence
<220>
   <223> LVL317
<400> 147
   atgaaatacc tgctgccgac cgctgctgct ggtctgctgc tcctcgctgc ccagccggcg 60
   atggcccaga ttcagaaggc tgaacaaaat gatgtgaagc tggcaccgcc gactgatgta 120
   cgaagcggat atatacgttt ggtaaagaat gtgaattatt acatcgatag tgaatcgatc 180
   tgggtggata accaagagcc acaaattgta cattttgatg cagtggtgaa tttagataag 240
   ggattgtatg tttatcctga gcctaaacgt tatgcacgtt ctgttcgtca gtataagatc 300
   ttgaattgtg caaattatca tttaactcaa gtacgaactg atttctatga tgaattttgg 360
   ggacagggtt tgcgggcagc acctaaaaag caaaagaaac atacgttaag tttaacacct 420
   gatacaacgc tttataatgc tgctcagatt atttgtgcga actatggtga agcattttca 480
   gttgataaaa aaggcggcac taaaaaagca gcggtatctg aattactgca agcgtcagcg 540
   ccttataagg ctgatgtgga attatgtgta tatagcacaa atgaaacaac aaactgtacg 600
   ggtggaaaaa atggtattgc agcagatata accacagcaa aaggctatgt aaaatcagtg 660
   acaacaagca acggtgcaat aacagtaaaa ggggatggca cattggcaaa tatggaatat 720
   attttgcaag ctacaggtaa tgctgcaaca ggtgtaactt ggacaacaac ttgcaaagga 780
   acggatgcct ctttatttcc agcaaatttt tgcggaagtg tcacacaata a          831
<210> 148
   <211> 276
   <212> PRT
   <213> Artificial Sequence
<220>
   <223> LVL317
<400> 148
   Met Lys Tyr Leu Leu Pro Thr Ala Ala Ala Gly Leu Leu Leu Leu Ala
    1               5                  10                  15
   Ala Gln Pro Ala Met Ala Gln Ile Gln Lys Ala Glu Gln Asn Asp Val
               20                  25                  30
   Lys Leu Ala Pro Pro Thr Asp Val Arg Ser Gly Tyr Ile Arg Leu Val
           35                  40                  45
   Lys Asn Val Asn Tyr Tyr Ile Asp Ser Glu Ser Ile Trp Val Asp Asn
       50                  55                  60
   Gln Glu Pro Gln Ile Val His Phe Asp Ala Val Val Asn Leu Asp Lys
   65                  70                  75                  80
   Gly Leu Tyr Val Tyr Pro Glu Pro Lys Arg Tyr Ala Arg Ser Val Arg
                   85                  90                  95
   Gln Tyr Lys Ile Leu Asn Cys Ala Asn Tyr His Leu Thr Gln Val Arg
                   100                 105                 110
   Thr Asp Phe Tyr Asp Glu Phe Trp Gly Gln Gly Leu Arg Ala Ala Pro
               115                 120                 125
   Lys Lys Gln Lys Lys His Thr Leu Ser Leu Thr Pro Asp Thr Thr Leu
       130                 135                 140
   Tyr Asn Ala Ala Gln Ile Ile Cys Ala Asn Tyr Gly Glu Ala Phe Ser
   145                 150                 155                 160
   Val Asp Lys Lys Gly Gly Thr Lys Lys Ala Ala Val Ser Glu Leu Leu
                   165                 170                 175
   Gln Ala Ser Ala Pro Tyr Lys Ala Asp Val Glu Leu Cys Val Tyr Ser
               180                 185                 190
   Thr Asn Glu Thr Thr Asn Cys Thr Gly Gly Lys Asn Gly Ile Ala Ala
           195                 200                 205
   Asp Ile Thr Thr Ala Lys Gly Tyr Val Lys Ser Val Thr Thr Ser Asn
       210                 215                 220
   Gly Ala Ile Thr Val Lys Gly Asp Gly Thr Leu Ala Asn Met Glu Tyr
   225                 230                 235                 240
   Ile Leu Gln Ala Thr Gly Asn Ala Ala Thr Gly Val Thr Trp Thr Thr
                   245                 250                 255
   Thr Cys Lys Gly Thr Asp Ala Ser Leu Phe Pro Ala Asn Phe Cys Gly
               260                 265                 270
   Ser Val Thr Gln
           275
<210> 149
   <211> 828
   <212> DNA
   <213> Artificial Sequence
<220>
   <223> LVL318
<400> 149
   atgaaatacc tgctgccgac cgctgctgct ggtctgctgc tcctcgctgc ccagccggcg 60
   atggccatgg ataaggctga acaaaatgat gtgaagctgg caccgccgac tgatgtacga 120
   agcggatata tacgtttggt aaagaatgtg aattattaca tcgatagtga atcgatctgg 180
   gtggataacc aagagccaca aattgtacat tttgatgcag tggtgaattt agataaggga 240
   ttgtatgttt atcctgagcc taaacgttat gcacgttctg ttcgtcagta taagatcttg 300
   aattgtgcaa attatcattt aactcaagta cgaactgatt tctatgatga attttgggga 360
   cagggtttgc gggcagcacc taaaaagcaa aagaaacata cgttaagttt aacacctgat 420
   acaacgcttt ataatgctgc tcagattatt tgtgcgaact atggtgaagc attttcagtt 480
   gataaaaaag gcggcactaa aaaagcagcg gtatctgaat tactgcaagc gtcagcgcct 540
   tataaggctg atgtggaatt atgtgtatat agcacaaatg aaacaacaaa ctgtacgggt 600
   ggaaaaaatg gtattgcagc agatataacc acagcaaaag gctatgtaaa atcagtgaca 660
   acaagcaacg gtgcaataac agtaaaaggg gatggcacat tggcaaatat ggaatatatt 720
   ttgcaagcta caggtaatgc tgcaacaggt gtaacttgga caacaacttg caaaggaacg 780
   gatgcctctt tatttccagc aaatttttgc ggaagtgtca cacaataa             828
<210> 150
   <211> 275
   <212> PRT
   <213> Artificial Sequence
<220>
   <223> LVL318
<400> 150
   Met Lys Tyr Leu Leu Pro Thr Ala Ala Ala Gly Leu Leu Leu Leu Ala
    1               5                  10                  15
   Ala Gln Pro Ala Met Ala Met Asp Lys Ala Glu Gln Asn Asp Val Lys
               20                  25                  30
   Leu Ala Pro Pro Thr Asp Val Arg Ser Gly Tyr Ile Arg Leu Val Lys
           35                  40                  45
   Asn Val Asn Tyr Tyr Ile Asp Ser Glu Ser Ile Trp Val Asp Asn Gln
       50                  55                  60
   Glu Pro Gln Ile Val His Phe Asp Ala Val Val Asn Leu Asp Lys Gly
   65                  70                  75                  80
   Leu Tyr Val Tyr Pro Glu Pro Lys Arg Tyr Ala Arg Ser Val Arg Gln
                   85                  90                  95
   Tyr Lys Ile Leu Asn Cys Ala Asn Tyr His Leu Thr Gln Val Arg Thr
               100                 105                 110
   Asp Phe Tyr Asp Glu Phe Trp Gly Gln Gly Leu Arg Ala Ala Pro Lys
           115                 120                 125
   Lys Gln Lys Lys His Thr Leu Ser Leu Thr Pro Asp Thr Thr Leu Tyr
       130                 135                 140
   Asn Ala Ala Gln Ile Ile Cys Ala Asn Tyr Gly Glu Ala Phe Ser Val
   145                 150                 155                 160
   Asp Lys Lys Gly Gly Thr Lys Lys Ala Ala Val Ser Glu Leu Leu Gln
                   165                 170                 175
   Ala Ser Ala Pro Tyr Lys Ala Asp Val Glu Leu Cys Val Tyr Ser Thr
               180                 185                 190
   Asn Glu Thr Thr Asn Cys Thr Gly Gly Lys Asn Gly Ile Ala Ala Asp
           195                 200                 205
   Ile Thr Thr Ala Lys Gly Tyr Val Lys Ser Val Thr Thr Ser Asn Gly
       210                 215                 220
   Ala Ile Thr Val Lys Gly Asp Gly Thr Leu Ala Asn Met Glu Tyr Ile
   225                 230                 235                 240
   Leu Gln Ala Thr Gly Asn Ala Ala Thr Gly Val Thr Trp Thr Thr Thr
                   245                 250                 255
   Cys Lys Gly Thr Asp Ala Ser Leu Phe Pro Ala Asn Phe Cys Gly Ser
               260                 265                 270
   Val Thr Gln
           275
<210> 151
   <211> 483
   <212> DNA
   <213> Haemophilus influenzae
<400> 151
   atgaaaaaaa ttattttaac attatcactt gggttactta ccgcttgttc tgctcaaatc 60
   caaaaggctg aacaaaatga tgtgaagctg gcaccgccga ctgatgtacg aagcggatat 120
   atacgtttgg taaagaatgt gaattattac atcgatagtg aatcgatctg ggtggataac 180
   caagagccac aaattgtaca ttttgatgct gtggtgaatt tagatagggg attgtatgtt 240
   tatcctgagc ctaaacgtta tgcacgttct gttcgtcagt ataagatttt gaattgtgca 300
   aattatcatt taactcaaat acgaactgat ttctatgatg aattttgggg acagggtttg 360
   cgggcagcac ctaaaaagca aaagaaacat acgttaagtt taacacctga tacaacgctt 420
   tataatgctg ctcagattat ttgtgcaaat tatggtaaag cattttcagt tgataaaaaa 480
   taa                                                               483
<210> 152
   <211> 160
   <212> PRT
   <213> Haemophilus influenzae
<400> 152
   Met Lys Lys Ile Ile Leu Thr Leu Ser Leu Gly Leu Leu Thr Ala Cys
    1               5                  10                  15
   Ser Ala Gln Ile Gln Lys Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Arg Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Ile Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Lys Ala Phe Ser Val Asp Lys Lys
   145                 150                 155                 160
<210> 153
   <211> 480
   <212> DNA
   <213> Haemophilus influenzae
<400> 153
   atgaaaaaaa ttattttaac attatcactt gggttactta ctgcctgttc tgctcaaatc 60
   caaaaggcta aacaaaatga tgtgaagctg gcaccgccga ctgatgtacg aagcggatat 120
   atacgtttgg taaagaatgt gaattattac atcgatagtg aatcgatctg ggtggataac 180
   caagagccac aaattgtaca ttttgatgca gtggtgaatt tagataaggg attgtatgtt 240
   tatcctgagc ctaaacgtta tgcacgttct gttcgtcagt ataagatctt gaattgtgca 300
   aattatcatt taactcaagt acgaactgat ttctatgatg aattttgggg acagggtttg 360
   cgggcagcac ctaaaaagca aaagaaacat acgttaagtt taacacctga tacaacgctt 420
   tataatgctg ctcagattat ttgtgcgaac tatggtgaag cattttcagt tgataaaaaa 480
<210> 154
   <211> 160
   <212> PRT
   <213> Haemophilus influenzae
<400> 154
   Met Lys Lys Ile Ile Leu Thr Leu Ser Leu Gly Leu Leu Thr Ala Cys
    1               5                  10                  15
   Ser Ala Gln Ile Gln Lys Ala Lys Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
<210> 155
   <211> 91
   <212> DNA
   <213> Artificial Sequence
<220>
   <223> primer
<400> 155
   cacacacata tgattaaatt tctctctgca ttaattcttc tactggtcac gacggcggct 60
   caggctgaga ctaaaaaagc agcggtatct g                                91
<210> 156
   <211> 66
   <212> DNA
   <213> Artificial Sequence
<220>
   <223> primer
<400> 156
   tgtgtgaagc ttttagtggt ggtggtggtg gtggccgcct tgtgtgacac ttccgcaaaa 60
   atttgc                                                            66
<210> 157
   <211> 58
   <212> DNA
   <213> Artificial Sequence
<220>
   <223> primer
<400> 157
   tttgcggaag tgtcacacaa ggcggcgcgc agattcagaa ggctgaacaa aatgatgt 58
<210> 158
   <211> 58
   <212> DNA
   <213> Artificial Sequence
<220>
   <223> primer
<400> 158
   acatcatttt gttcagcctt ctgaatctgc gcgccgcctt gtgtgacact tccgcaaa 58
<210> 159
   <211> 59
   <212> DNA
   <213> Artificial Sequence
<220>
   <223> primer
<400> 159
   tgtgtgaagc ttttagtggt ggtggtggtg gtggccgcct tttttatcaa ctgaaaatg 59
<210> 160
   <211> 65
   <212> DNA
   <213> Artificial Sequence
<220>
   <223> primer
<400> 160
   cacacacata tgcaccacca ccaccaccac agcgcgcaga ttcagaaggc tgaacaaaat 60
   gatgt 65
<210> 161
   <211> 46
   <212> DNA
   <213> Artificial Sequence
<220>
   <223> primer
<400> 161
   cattttcagt tgataaaaaa ggcggcacta aaaaagcagc ggtatc 46
<210> 162
   <211> 46
   <212> DNA
   <213> Artificial Sequence
<220>
   <223> primer
<400> 162
   gataccgctg cttttttagt gccgcctttt ttatcaactg aaaatg 46
<210> 163
   <211> 35
   <212> DNA
   <213> Artificial Sequence
<220>
   <223> primer
<400> 163
   tgtgtgaagc ttttattgtg tgacacttcc gcaaa 35
<210> 164
   <211> 104
   <212> DNA
   <213> Artificial Sequence
<220>
   <223> primer
<400> 164
   cacacacata tgaaatacct gctgccgacc gctgctgctg gtctgctgct cctcgctgcc 60
   cagccggcga tggcccagat tcagaaggct gaacaaaatg atgt 104
<210> 165
   <211> 49
   <212> DNA
   <213> Artificial Sequence
<220>
   <223> primer
<400> 165
   gcattttcag ttgataaaaa aggcggcact aaaaaagcag cggtatctg 49
<210> 166
   <211> 49
   <212> DNA
   <213> Artificial Sequence
<220>
   <223> primer
<400> 166
   cagataccgc tgctttttta gtgccgcctt ttttatcaac tgaaaatgc 49
<210> 167
   <211> 104
   <212> DNA
   <213> Artificial Sequence
<220>
   <223> primer
<400> 167
   cacacacata tgaaatacct gctgccgacc gctgctgctg gtctgctgct cctcgctgcc 60
   cagccggcga tggccgatat tcagaaggct gaacaaaatg atgt                 104
<210> 168
   <211> 115
   <212> DNA
   <213> Artificial Sequence
<220>
   <223> primer
<400> 168
   cacacacata tgaaacactt tccatccaaa gtactgacca cagccatcct tgccactttc 60
   tgtagcggcg cactggcagc cacaaacgac gacgataagg ctgaacaaaa tgatg      115
<210> 169
   <211> 35
   <212> DNA
   <213> Artificial Sequence
<220>
   <223> primer
<400> 169
   gccggcgatg gccatggata aggctgaaca aaatg 35
<210> 170
   <211> 35
   <212> DNA
   <213> Artificial Sequence
<220>
   <223> primer
<400> 170
   cattttgttc agccttatcc atggccatcg ccggc 35
<210> 171
   <211> 34
   <212> DNA
   <213> Artificial Sequence
<220>
   <223> primer
<400> 171
   ggaagtgtca cacaataagg cggccaccac cacc 34
<210> 172
   <211> 34
   <212> DNA
   <213> Artificial Sequence
<220>
   <223> primer
<400> 172
   ggtggtggtg gccgccttat tgtgtgacac ttcc 34
<210> 173
   <211> 54
   <212> DNA
   <213> Artificial Sequence
<220>
   <223> primer
<400> 173
   gaattccata tgcaccatca ccatcaccat actaaaaaag cagcggtatc tgaa 54
<210> 174
   <211> 32
   <212> DNA
   <213> Artificial Sequence
<220>
   <223> primer
<400> 174
   gcgccgctcg agtcattgtg tgacacttcc gc 32
<210> 175
   <211> 43
   <212> DNA
   <213> Artificial Sequence
<220>
   <223> primer
<400> 175
   gcccagccgg cgatggccca gatccagaag gctgaacaaa atg 43
<210> 176
   <211> 43
   <212> DNA
   <213> Artificial Sequence
<220>
   <223> primer
<400> 176
   cattttgttc agccttctgg atctgggcca tcgccggctg ggc 43
<210> 177
   <211> 254
   <212> PRT
   <213> Artificial Sequence
<220>
   <223> Fusion protein
<400> 177
   Gln Ile Gln Lys Ala Glu Gln Asn Asp Val Lys Leu Ala Pro Pro Thr
    1               5                  10                  15
   Asp Val Arg Ser Gly Tyr Ile Arg Leu Val Lys Asn Val Asn Tyr Tyr
               20                  25                  30
   Ile Asp Ser Glu Ser Ile Trp Val Asp Asn Gln Glu Pro Gln Ile Val
           35                  40                  45
   His Phe Asp Ala Val Val Asn Leu Asp Lys Gly Leu Tyr Val Tyr Pro
       50                  55                  60
   Glu Pro Lys Arg Tyr Ala Arg Ser Val Arg Gln Tyr Lys Ile Leu Asn
   65                  70                  75                  80
   Cys Ala Asn Tyr His Leu Thr Gln Val Arg Thr Asp Phe Tyr Asp Glu
                   85                  90                  95
   Phe Trp Gly Gln Gly Leu Arg Ala Ala Pro Lys Lys Gln Lys Lys His
               100                 105                 110
   Thr Leu Ser Leu Thr Pro Asp Thr Thr Leu Tyr Asn Ala Ala Gln Ile
           115                 120                 125
   Ile Cys Ala Asn Tyr Gly Glu Ala Phe Ser Val Asp Lys Lys Gly Gly
       130                 135                 140
   Thr Lys Lys Ala Ala Val Ser Glu Leu Leu Gln Ala Ser Ala Pro Tyr
   145                 150                 155                 160
   Lys Ala Asp Val Glu Leu Cys Val Tyr Ser Thr Asn Glu Thr Thr Asn
                   165                 170                 175
   Cys Thr Gly Gly Lys Asn Gly Ile Ala Ala Asp Ile Thr Thr Ala Lys
               180                 185                 190
   Gly Tyr Val Lys Ser Val Thr Thr Ser Asn Gly Ala Ile Thr Val Lys
           195                 200                 205
   Gly Asp Gly Thr Leu Ala Asn Met Glu Tyr Ile Leu Gln Ala Thr Gly
       210                 215                 220
   Asn Ala Ala Thr Gly Val Thr Trp Thr Thr Thr Cys Lys Gly Thr Asp
   225                 230                 235                 240
   Ala Ser Leu Phe Pro Ala Asn Phe Cys Gly Ser Val Thr Gln
                   245                 250
<210> 178
   <211> 6
   <212> PRT
   <213> Artificial Sequence
<220>
   <223> Synthetic construct
<400> 178
   Ala Thr Asn Asp Asp Asp
    1               5
<210> 179
   <211> 138
   <212> PRT
   <213> Haemophilus influenzae
<400> 179
   Ala Glu Gln Asn Asp Val Lys Leu Ala Pro Pro Thr Asp Val Arg Ser
    1               5                  10                  15
   Gly Tyr Ile Arg Leu Val Lys Asn Val Asn Tyr Tyr Ile Asp Ser Glu
               20                  25                  30
   Ser Ile Trp Val Asp Asn Gln Glu Pro Gln Ile Val His Phe Asp Ala
           35                  40                  45
   Val Val Asn Leu Asp Lys Gly Leu Tyr Val Tyr Pro Glu Pro Lys Arg
       50                  55                  60
   Tyr Ala Arg Ser Val Arg Gln Tyr Lys Ile Leu Asn Cys Ala Asn Tyr
   65                  70                  75                  80
   His Leu Thr Gln Val Arg Thr Asp Phe Tyr Asp Glu Phe Trp Gly Gln
                   85                  90                  95
   Gly Leu Arg Ala Ala Pro Lys Lys Gln Lys Lys His Thr Leu Ser Leu
               100                 105                 110
   Thr Pro Asp Thr Thr Leu Tyr Asn Ala Ala Gln Ile Ile Cys Ala Asn
           115                 120                 125
   Tyr Gly Glu Ala Phe Ser Val Asp Lys Lys
       130                 135
<210> 180
   <211> 137
   <212> PRT
   <213> Haemophilus influenzae
<400> 180
   Glu Gln Asn Asp Val Lys Leu Ala Pro Pro Thr Asp Val Arg Ser Gly
    1               5                  10                  15
   Tyr Ile Arg Leu Val Lys Asn Val Asn Tyr Tyr Ile Asp Ser Glu Ser
               20                  25                  30
   Ile Trp Val Asp Asn Gln Glu Pro Gln Ile Val His Phe Asp Ala Val
           35                  40                  45
   Val Asn Leu Asp Lys Gly Leu Tyr Val Tyr Pro Glu Pro Lys Arg Tyr
       50                  55                  60
   Ala Arg Ser Val Arg Gln Tyr Lys Ile Leu Asn Cys Ala Asn Tyr His
   65                  70                  75                  80
   Leu Thr Gln Val Arg Thr Asp Phe Tyr Asp Glu Phe Trp Gly Gln Gly
                   85                  90                  95
   Leu Arg Ala Ala Pro Lys Lys Gln Lys Lys His Thr Leu Ser Leu Thr
               100                 105                 110
   Pro Asp Thr Thr Leu Tyr Asn Ala Ala Gln Ile Ile Cys Ala Asn Tyr
           115                 120                 125
   Gly Glu Ala Phe Ser Val Asp Lys Lys
       130                 135
<210> 181
   <211> 849
   <212> DNA
   <213> Artificial Sequence
<220>
   <223> LVL702
<400> 181
   atgaaatacc tgctgccgac cgctgctgct ggtctgctgc tcctcgctgc ccagccggcg 60
   atggccattc agaaggctga acaaaatgat gtgaagctgg caccgccgac tgatgtacga 120
   agcggatata tacgtttggt aaagaatgtg aattattaca tcgatagtga atcgatctgg 180
   gtggataacc aagagccaca aattgtacat tttgatgcag tggtgaattt agataaggga 240
   ttgtatgttt atcctgagcc taaacgttat gcacgttctg ttcgtcagta taagatcttg 300
   aattgtgcaa attatcattt aactcaagta cgaactgatt tctatgatga attttgggga 360
   cagggtttgc gggcagcacc taaaaagcaa aagaaacata cgttaagttt aacacctgat 420
   acaacgcttt ataatgctgc tcagattatt tgtgcgaact atggtgaagc attttcagtt 480
   gataaaaaag gcggcactaa aaaagcagcg gtatctgaat tactgcaagc gtcagcgcct 540
   tataaggctg atgtggaatt atgtgtatat agcacaaatg aaacaacaaa ctgtacgggt 600
   ggaaaaaatg gtattgcagc agatataacc acagcaaaag gctatgtaaa atcagtgaca 660
   acaagcaacg gtgcaataac agtaaaaggg gatggcacat tggcaaatat ggaatatatt 720
   ttgcaagcta caggtaatgc tgcaacaggt gtaacttgga caacaacttg caaaggaacg 780
   gatgcctctt tatttccagc aaatttttgc ggaagtgtca cacaaggcgg ccaccaccac 840
   caccaccac                                                         849
<210> 182
   <211> 283
   <212> PRT
   <213> Artificial Sequence
<220>
   <223> LVL702
<400> 182
   Met Lys Tyr Leu Leu Pro Thr Ala Ala Ala Gly Leu Leu Leu Leu Ala
    1               5                  10                  15
   Ala Gln Pro Ala Met Ala Ile Gln Lys Ala Glu Gln Asn Asp Val Lys
               20                  25                  30
   Leu Ala Pro Pro Thr Asp Val Arg Ser Gly Tyr Ile Arg Leu Val Lys
           35                  40                  45
   Asn Val Asn Tyr Tyr Ile Asp Ser Glu Ser Ile Trp Val Asp Asn Gln
       50                  55                  60
   Glu Pro Gln Ile Val His Phe Asp Ala Val Val Asn Leu Asp Lys Gly
   65                  70                  75                  80
   Leu Tyr Val Tyr Pro Glu Pro Lys Arg Tyr Ala Arg Ser Val Arg Gln
                   85                  90                  95
   Tyr Lys Ile Leu Asn Cys Ala Asn Tyr His Leu Thr Gln Val Arg Thr
               100                 105                 110
   Asp Phe Tyr Asp Glu Phe Trp Gly Gln Gly Leu Arg Ala Ala Pro Lys
           115                 120                 125
   Lys Gln Lys Lys His Thr Leu Ser Leu Thr Pro Asp Thr Thr Leu Tyr
       130                 135                 140
   Asn Ala Ala Gln Ile Ile Cys Ala Asn Tyr Gly Glu Ala Phe Ser Val
   145                 150                 155                 160
   Asp Lys Lys Gly Gly Thr Lys Lys Ala Ala Val Ser Glu Leu Leu Gln
                   165                 170                 175
   Ala Ser Ala Pro Tyr Lys Ala Asp Val Glu Leu Cys Val Tyr Ser Thr
               180                 185                 190
   Asn Glu Thr Thr Asn Cys Thr Gly Gly Lys Asn Gly Ile Ala Ala Asp
           195                 200                 205
   Ile Thr Thr Ala Lys Gly Tyr Val Lys Ser Val Thr Thr Ser Asn Gly
       210                 215                 220
   Ala Ile Thr Val Lys Gly Asp Gly Thr Leu Ala Asn Met Glu Tyr Ile
   225                 230                 235                 240
   Leu Gln Ala Thr Gly Asn Ala Ala Thr Gly Val Thr Trp Thr Thr Thr
                   245                 250                 255
   Cys Lys Gly Thr Asp Ala Ser Leu Phe Pro Ala Asn Phe Cys Gly Ser
               260                 265                 270
   Val Thr Gln Gly Gly His His His His His His
           275                 280
<210> 183
   <211> 858
   <212> DNA
   <213> Artificial Sequence
<220>
   <223> LVL736
<400> 183
   atgaaatacc tgctgccgac cgctgctgct ggtctgctgc tcctcgctgc ccagccggcg 60
   atggccagcg cccagattca gaaggctgaa caaaatgatg tgaagctggc accgccgact 120
   gatgtacgaa gcggatatat acgtttggta aagaatgtga attattacat cgatagtgaa 180
   tcgatctggg tggataacca agagccacaa attgtacatt ttgatgcagt ggtgaattta 240
   gataagggat tgtatgttta tcctgagcct aaacgttatg cacgttctgt tcgtcagtat 300
   aagatcttga attgtgcaaa ttatcattta actcaagtac gaactgattt ctatgatgaa 360
   ttttggggac agggtttgcg ggcagcacct aaaaagcaaa agaaacatac gttaagttta 420
   acacctgata caacgcttta taatgctgct cagattattt gtgcgaacta tggtgaagca 480
   ttttcagttg ataaaaaagg cggcactaaa aaagcagcgg tatctgaatt actgcaagcg 540
   tcagcgcctt ataaggctga tgtggaatta tgtgtatata gcacaaatga aacaacaaac 600
   tgtacgggtg gaaaaaatgg tattgcagca gatataacca cagcaaaagg ctatgtaaaa 660
   tcagtgacaa caagcaacgg tgcaataaca gtaaaagggg atggcacatt ggcaaatatg 720
   gaatatattt tgcaagctac aggtaatgct gcaacaggtg taacttggac aacaacttgc 780
   aaaggaacgg atgcctcttt atttccagca aatttttgcg gaagtgtcac acaaggcggc 840
   caccaccacc accaccac                                                858
<210> 184
   <211> 286
   <212> PRT
   <213> Artificial Sequence
<220>
   <223> LVL736
<400> 184
   Met Lys Tyr Leu Leu Pro Thr Ala Ala Ala Gly Leu Leu Leu Leu Ala
    1               5                  10                  15
   Ala Gln Pro Ala Met Ala Ser Ala Gln Ile Gln Lys Ala Glu Gln Asn
               20                  25                  30
   Asp Val Lys Leu Ala Pro Pro Thr Asp Val Arg Ser Gly Tyr Ile Arg
           35                  40                  45
   Leu Val Lys Asn Val Asn Tyr Tyr Ile Asp Ser Glu Ser Ile Trp Val
       50                  55                  60
   Asp Asn Gln Glu Pro Gln Ile Val His Phe Asp Ala Val Val Asn Leu
   65                  70                  75                  80
   Asp Lys Gly Leu Tyr Val Tyr Pro Glu Pro Lys Arg Tyr Ala Arg Ser
                   85                  90                  95
   Val Arg Gln Tyr Lys Ile Leu Asn Cys Ala Asn Tyr His Leu Thr Gln
               100                 105                 110
   Val Arg Thr Asp Phe Tyr Asp Glu Phe Trp Gly Gln Gly Leu Arg Ala
           115                 120                 125
   Ala Pro Lys Lys Gln Lys Lys His Thr Leu Ser Leu Thr Pro Asp Thr
       130                 135                 140
   Thr Leu Tyr Asn Ala Ala Gln Ile Ile Cys Ala Asn Tyr Gly Glu Ala
   145                 150                 155                 160
   Phe Ser Val Asp Lys Lys Gly Gly Thr Lys Lys Ala Ala Val Ser Glu
                   165                 170                 175
   Leu Leu Gln Ala Ser Ala Pro Tyr Lys Ala Asp Val Glu Leu Cys Val
               180                 185                 190
   Tyr Ser Thr Asn Glu Thr Thr Asn Cys Thr Gly Gly Lys Asn Gly Ile
           195                 200                 205
   Ala Ala Asp Ile Thr Thr Ala Lys Gly Tyr Val Lys Ser Val Thr Thr
       210                 215                 220
   Ser Asn Gly Ala Ile Thr Val Lys Gly Asp Gly Thr Leu Ala Asn Met
   225                 230                 235                 240
   Glu Tyr Ile Leu Gln Ala Thr Gly Asn Ala Ala Thr Gly Val Thr Trp
                   245                 250                 255
   Thr Thr Thr Cys Lys Gly Thr Asp Ala Ser Leu Phe Pro Ala Asn Phe
               260                 265                 270
   Cys Gly Ser Val Thr Gln Gly Gly His His His His His His
           275                 280                 285
<210> 185
   <211> 855
   <212> DNA
   <213> Artificial Sequence
<220>
   <223> LVL737
<400> 185
   atgaaatacc tgctgccgac cgctgctgct ggtctgctgc tcctcgctgc ccagccggcg 60
   atggccgccc agattcagaa ggctgaacaa aatgatgtga agctggcacc gccgactgat 120
   gtacgaagcg gatatatacg tttggtaaag aatgtgaatt attacatcga tagtgaatcg 180
   atctgggtgg ataaccaaga gccacaaatt gtacattttg atgcagtggt gaatttagat 240
   aagggattgt atgtttatcc tgagcctaaa cgttatgcac gttctgttcg tcagtataag 300
   atcttgaatt gtgcaaatta tcatttaact caagtacgaa ctgatttcta tgatgaattt 360
   tggggacagg gtttgcgggc agcacctaaa aagcaaaaga aacatacgtt aagtttaaca 420
   cctgatacaa cgctttataa tgctgctcag attatttgtg cgaactatgg tgaagcattt 480
   tcagttgata aaaaaggcgg cactaaaaaa gcagcggtat ctgaattact gcaagcgtca 540
   gcgccttata aggctgatgt ggaattatgt gtatatagca caaatgaaac aacaaactgt 600
   acgggtggaa aaaatggtat tgcagcagat ataaccacag caaaaggcta tgtaaaatca 660
   gtgacaacaa gcaacggtgc aataacagta aaaggggatg gcacattggc aaatatggaa 720
   tatattttgc aagctacagg taatgctgca acaggtgtaa cttggacaac aacttgcaaa 780
   ggaacggatg cctctttatt tccagcaaat ttttgcggaa gtgtcacaca aggcggccac 840
   caccaccacc accac                                                  855
<210> 186
   <211> 285
   <212> PRT
   <213> Artificial Sequence
<220>
   <223> LVL737
<400> 186
   Met Lys Tyr Leu Leu Pro Thr Ala Ala Ala Gly Leu Leu Leu Leu Ala
    1               5                  10                  15
   Ala Gln Pro Ala Met Ala Ala Gln Ile Gln Lys Ala Glu Gln Asn Asp
               20                  25                  30
   Val Lys Leu Ala Pro Pro Thr Asp Val Arg Ser Gly Tyr Ile Arg Leu
           35                  40                  45
   Val Lys Asn Val Asn Tyr Tyr Ile Asp Ser Glu Ser Ile Trp Val Asp
       50                  55                  60
   Asn Gln Glu Pro Gln Ile Val His Phe Asp Ala Val Val Asn Leu Asp
   65                  70                  75                  80
   Lys Gly Leu Tyr Val Tyr Pro Glu Pro Lys Arg Tyr Ala Arg Ser Val
                   85                  90                  95
   Arg Gln Tyr Lys Ile Leu Asn Cys Ala Asn Tyr His Leu Thr Gln Val
               100                 105                 110
   Arg Thr Asp Phe Tyr Asp Glu Phe Trp Gly Gln Gly Leu Arg Ala Ala
           115                 120                 125
   Pro Lys Lys Gln Lys Lys His Thr Leu Ser Leu Thr Pro Asp Thr Thr
       130                 135                 140
   Leu Tyr Asn Ala Ala Gln Ile Ile Cys Ala Asn Tyr Gly Glu Ala Phe
   145                 150                 155                 160
   Ser Val Asp Lys Lys Gly Gly Thr Lys Lys Ala Ala Val Ser Glu Leu
                   165                 170                 175
   Leu Gln Ala Ser Ala Pro Tyr Lys Ala Asp Val Glu Leu Cys Val Tyr
               180                 185                 190
   Ser Thr Asn Glu Thr Thr Asn Cys Thr Gly Gly Lys Asn Gly Ile Ala
           195                 200                 205
   Ala Asp Ile Thr Thr Ala Lys Gly Tyr Val Lys Ser Val Thr Thr Ser
       210                 215                 220
   Asn Gly Ala Ile Thr Val Lys Gly Asp Gly Thr Leu Ala Asn Met Glu
   225                 230                 235                 240
   Tyr Ile Leu Gln Ala Thr Gly Asn Ala Ala Thr Gly Val Thr Trp Thr
                   245                 250                 255
   Thr Thr Cys Lys Gly Thr Asp Ala Ser Leu Phe Pro Ala Asn Phe Cys
               260                 265                 270
   Gly Ser Val Thr Gln Gly Gly His His His His His His
           275                 280                 285
<210> 187
   <211> 843
   <212> DNA
   <213> Artificial Sequence
<220>
   <223> LVL738
<400> 187
   atgaaatacc tgctgccgac cgctgctgct ggtctgctgc tcctcgctgc ccagccggcg 60
   atggccaagg ctgaacaaaa tgatgtgaag ctggcaccgc cgactgatgt acgaagcgga 120
   tatatacgtt tggtaaagaa tgtgaattat tacatcgata gtgaatcgat ctgggtggat 180
   aaccaagagc cacaaattgt acattttgat gcagtggtga atttagataa gggattgtat 240
   gtttatcctg agcctaaacg ttatgcacgt tctgttcgtc agtataagat cttgaattgt 300
   gcaaattatc atttaactca agtacgaact gatttctatg atgaattttg gggacagggt 360
   ttgcgggcag cacctaaaaa gcaaaagaaa catacgttaa gtttaacacc tgatacaacg 420
   ctttataatg ctgctcagat tatttgtgcg aactatggtg aagcattttc agttgataaa 480
   aaaggcggca ctaaaaaagc agcggtatct gaattactgc aagcgtcagc gccttataag 540
   gctgatgtgg aattatgtgt atatagcaca aatgaaacaa caaactgtac gggtggaaaa 600
   aatggtattg cagcagatat aaccacagca aaaggctatg taaaatcagt gacaacaagc 660
   aacggtgcaa taacagtaaa aggggatggc acattggcaa atatggaata tattttgcaa 720
   gctacaggta atgctgcaac aggtgtaact tggacaacaa cttgcaaagg aacggatgcc 780
   tctttatttc cagcaaattt ttgcggaagt gtcacacaag gcggccacca ccaccaccac 840
   cac                                                               843
<210> 188
   <211> 281
   <212> PRT
   <213> Artificial Sequence
<220>
   <223> LVL738
<400> 188
   Met Lys Tyr Leu Leu Pro Thr Ala Ala Ala Gly Leu Leu Leu Leu Ala
    1               5                  10                  15
   Ala Gln Pro Ala Met Ala Lys Ala Glu Gln Asn Asp Val Lys Leu Ala
               20                  25                  30
   Pro Pro Thr Asp Val Arg Ser Gly Tyr Ile Arg Leu Val Lys Asn Val
           35                  40                  45
   Asn Tyr Tyr Ile Asp Ser Glu Ser Ile Trp Val Asp Asn Gln Glu Pro
       50                  55                  60
   Gln Ile Val His Phe Asp Ala Val Val Asn Leu Asp Lys Gly Leu Tyr
   65                  70                  75                  80
   Val Tyr Pro Glu Pro Lys Arg Tyr Ala Arg Ser Val Arg Gln Tyr Lys
                   85                  90                  95
   Ile Leu Asn Cys Ala Asn Tyr His Leu Thr Gln Val Arg Thr Asp Phe
               100                 105                 110
   Tyr Asp Glu Phe Trp Gly Gln Gly Leu Arg Ala Ala Pro Lys Lys Gln
           115                 120                 125
   Lys Lys His Thr Leu Ser Leu Thr Pro Asp Thr Thr Leu Tyr Asn Ala
       130                 135                 140
   Ala Gln Ile Ile Cys Ala Asn Tyr Gly Glu Ala Phe Ser Val Asp Lys
   145                 150                 155                 160
   Lys Gly Gly Thr Lys Lys Ala Ala Val Ser Glu Leu Leu Gln Ala Ser
                   165                 170                 175
   Ala Pro Tyr Lys Ala Asp Val Glu Leu Cys Val Tyr Ser Thr Asn Glu
               180                 185                 190
   Thr Thr Asn Cys Thr Gly Gly Lys Asn Gly Ile Ala Ala Asp Ile Thr
           195                 200                 205
   Thr Ala Lys Gly Tyr Val Lys Ser Val Thr Thr Ser Asn Gly Ala Ile
       210                 215                 220
   Thr Val Lys Gly Asp Gly Thr Leu Ala Asn Met Glu Tyr Ile Leu Gln
   225                 230                 235                 240
   Ala Thr Gly Asn Ala Ala Thr Gly Val Thr Trp Thr Thr Thr Cys Lys
                   245                 250                 255
   Gly Thr Asp Ala Ser Leu Phe Pro Ala Asn Phe Cys Gly Ser Val Thr
               260                 265                 270
   Gln Gly Gly His His His His His His
           275                 280
<210> 189
   <211> 840
   <212> DNA
   <213> Artificial Sequence
<220>
   <223> LVL739
<400> 189
   atgaaatacc tgctgccgac cgctgctgct ggtctgctgc tcctcgctgc ccagccggcg 60
   atggccgctg aacaaaatga tgtgaagctg gcaccgccga ctgatgtacg aagcggatat 120
   atacgtttgg taaagaatgt gaattattac atcgatagtg aatcgatctg ggtggataac 180
   caagagccac aaattgtaca ttttgatgca gtggtgaatt tagataaggg attgtatgtt 240
   tatcctgagc ctaaacgtta tgcacgttct gttcgtcagt ataagatctt gaattgtgca 300
   aattatcatt taactcaagt acgaactgat ttctatgatg aattttgggg acagggtttg 360
   cgggcagcac ctaaaaagca aaagaaacat acgttaagtt taacacctga tacaacgctt 420
   tataatgctg ctcagattat ttgtgcgaac tatggtgaag cattttcagt tgataaaaaa 480
   ggcggcacta aaaaagcagc ggtatctgaa ttactgcaag cgtcagcgcc ttataaggct 540
   gatgtggaat tatgtgtata tagcacaaat gaaacaacaa actgtacggg tggaaaaaat 600
   ggtattgcag cagatataac cacagcaaaa ggctatgtaa aatcagtgac aacaagcaac 660
   ggtgcaataa cagtaaaagg ggatggcaca ttggcaaata tggaatatat tttgcaagct 720
   acaggtaatg ctgcaacagg tgtaacttgg acaacaactt gcaaaggaac ggatgcctct 780
   ttatttccag caaatttttg cggaagtgtc acacaaggcg gccaccacca ccaccaccac 840
<210> 190
   <211> 280
   <212> PRT
   <213> Artificial Sequence
<220>
   <223> LVL739
<400> 190
   Met Lys Tyr Leu Leu Pro Thr Ala Ala Ala Gly Leu Leu Leu Leu Ala
    1               5                  10                  15
   Ala Gln Pro Ala Met Ala Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
   Gly Gly Thr Lys Lys Ala Ala Val Ser Glu Leu Leu Gln Ala Ser Ala
                   165                 170                 175
   Pro Tyr Lys Ala Asp Val Glu Leu Cys Val Tyr Ser Thr Asn Glu Thr
               180                 185                 190
   Thr Asn Cys Thr Gly Gly Lys Asn Gly Ile Ala Ala Asp Ile Thr Thr
           195                 200                 205
   Ala Lys Gly Tyr Val Lys Ser Val Thr Thr Ser Asn Gly Ala Ile Thr
       210                 215                 220
   Val Lys Gly Asp Gly Thr Leu Ala Asn Met Glu Tyr Ile Leu Gln Ala
   225                 230                 235                 240
   Thr Gly Asn Ala Ala Thr Gly Val Thr Trp Thr Thr Thr Cys Lys Gly
                   245                 250                 255
   Thr Asp Ala Ser Leu Phe Pro Ala Asn Phe Cys Gly Ser Val Thr Gln
               260                 265                 270
   Gly Gly His His His His His His
           275                 280
<210> 191
   <211> 837
   <212> DNA
   <213> Artificial Sequence
<220>
   <223> LVL740
<400> 191
   atgaaatacc tgctgccgac cgctgctgct ggtctgctgc tcctcgctgc ccagccggcg 60
   atggccgaac aaaatgatgt gaagctggca ccgccgactg atgtacgaag cggatatata 120
   cgtttggtaa agaatgtgaa ttattacatc gatagtgaat cgatctgggt ggataaccaa 180
   gagccacaaa ttgtacattt tgatgcagtg gtgaatttag ataagggatt gtatgtttat 240
   cctgagccta aacgttatgc acgttctgtt cgtcagtata agatcttgaa ttgtgcaaat 300
   tatcatttaa ctcaagtacg aactgatttc tatgatgaat tttggggaca gggtttgcgg 360
   gcagcaccta aaaagcaaaa gaaacatacg ttaagtttaa cacctgatac aacgctttat 420
   aatgctgctc agattatttg tgcgaactat ggtgaagcat tttcagttga taaaaaaggc 480
   ggcactaaaa aagcagcggt atctgaatta ctgcaagcgt cagcgcctta taaggctgat 540
   gtggaattat gtgtatatag cacaaatgaa acaacaaact gtacgggtgg aaaaaatggt 600
   attgcagcag atataaccac agcaaaaggc tatgtaaaat cagtgacaac aagcaacggt 660
   gcaataacag taaaagggga tggcacattg gcaaatatgg aatatatttt gcaagctaca 720
   ggtaatgctg caacaggtgt aacttggaca acaacttgca aaggaacgga tgcctcttta 780
   tttccagcaa atttttgcgg aagtgtcaca caaggcggcc accaccacca ccaccac    837
<210> 192
   <211> 279
   <212> PRT
   <213> Artificial Sequence
<220>
   <223> LVL740
<400> 192
   Met Lys Tyr Leu Leu Pro Thr Ala Ala Ala Gly Leu Leu Leu Leu Ala
   1               5                   10                  15
   Ala Gln Pro Ala Met Ala Glu Gln Asn Asp Val Lys Leu Ala Pro Pro
               20                  25                  30
   Thr Asp Val Arg Ser Gly Tyr Ile Arg Leu Val Lys Asn Val Asn Tyr
           35                  40                  45
   Tyr Ile Asp Ser Glu Ser Ile Trp Val Asp Asn Gln Glu Pro Gln Ile
       50                  55                  60
   Val His Phe Asp Ala Val Val Asn Leu Asp Lys Gly Leu Tyr Val Tyr
   65                  70                  75                  80
   Pro Glu Pro Lys Arg Tyr Ala Arg Ser Val Arg Gln Tyr Lys Ile Leu
                   85                  90                  95
   Asn Cys Ala Asn Tyr His Leu Thr Gln Val Arg Thr Asp Phe Tyr Asp
               100                 105                 110
   Glu Phe Trp Gly Gln Gly Leu Arg Ala Ala Pro Lys Lys Gln Lys Lys
           115                 120                 125
   His Thr Leu Ser Leu Thr Pro Asp Thr Thr Leu Tyr Asn Ala Ala Gln
       130                 135                 140
   Ile Ile Cys Ala Asn Tyr Gly Glu Ala Phe Ser Val Asp Lys Lys Gly
   145                 150                 155                 160
   Gly Thr Lys Lys Ala Ala Val Ser Glu Leu Leu Gln Ala Ser Ala Pro
                   165                 170                 175
   Tyr Lys Ala Asp Val Glu Leu Cys Val Tyr Ser Thr Asn Glu Thr Thr
               180                 185                 190
   Asn Cys Thr Gly Gly Lys Asn Gly Ile Ala Ala Asp Ile Thr Thr Ala
           195                 200                 205
   Lys Gly Tyr Val Lys Ser Val Thr Thr Ser Asn Gly Ala Ile Thr Val
       210                 215                 220
   Lys Gly Asp Gly Thr Leu Ala Asn Met Glu Tyr Ile Leu Gln Ala Thr
   225                 230                 235                 240
   Gly Asn Ala Ala Thr Gly Val Thr Trp Thr Thr Thr Cys Lys Gly Thr
                   245                 250                 255
   Asp Ala Ser Leu Phe Pro Ala Asn Phe Cys Gly Ser Val Thr Gln Gly
               260                 265                 270
   Gly His His His His His His
           275
<210> 193
   <211> 825
   <212> DNA
   <213> Artificial Sequence
<220>
   <223> LVL735
<400> 193
   atgaaatacc tgctgccgac cgctgctgct ggtctgctgc tcctcgctgc ccagccggcg 60
   atggccattc agaaggctga acaaaatgat gtgaagctgg caccgccgac tgatgtacga 120
   agcggatata tacgtttggt aaagaatgtg aattattaca tcgatagtga atcgatctgg 180
   gtggataacc aagagccaca aattgtacat tttgatgcag tggtgaattt agataaggga 240
   ttgtatgttt atcctgagcc taaacgttat gcacgttctg ttcgtcagta taagatcttg 300
   aattgtgcaa attatcattt aactcaagta cgaactgatt tctatgatga attttgggga 360
   cagggtttgc gggcagcacc taaaaagcaa aagaaacata cgttaagttt aacacctgat 420
   acaacgcttt ataatgctgc tcagattatt tgtgcgaact atggtgaagc attttcagtt 480
   gataaaaaag gcggcactaa aaaagcagcg gtatctgaat tactgcaagc gtcagcgcct 540
   tataaggctg atgtggaatt atgtgtatat agcacaaatg aaacaacaaa ctgtacgggt 600
   ggaaaaaatg gtattgcagc agatataacc acagcaaaag gctatgtaaa atcagtgaca 660
   acaagcaacg gtgcaataac agtaaaaggg gatggcacat tggcaaatat ggaatatatt 720
   ttgcaagcta caggtaatgc tgcaacaggt gtaacttgga caacaacttg caaaggaacg 780
   gatgcctctt tatttccagc aaatttttgc ggaagtgtca cacaa                 825
<210> 194
   <211> 275
   <212> PRT
   <213> Artificial Sequence
<220>
   <223> LVL735
<400> 194
   Met Lys Tyr Leu Leu Pro Thr Ala Ala Ala Gly Leu Leu Leu Leu Ala
    1               5                  10                  15
   Ala Gln Pro Ala Met Ala Ile Gln Lys Ala Glu Gln Asn Asp Val Lys
               20                  25                  30
   Leu Ala Pro Pro Thr Asp Val Arg Ser Gly Tyr Ile Arg Leu Val Lys
           35                  40                  45
   Asn Val Asn Tyr Tyr Ile Asp Ser Glu Ser Ile Trp Val Asp Asn Gln
       50                  55                  60
   Glu Pro Gln Ile Val His Phe Asp Ala Val Val Asn Leu Asp Lys Gly
   65                  70                  75                  80
   Leu Tyr Val Tyr Pro Glu Pro Lys Arg Tyr Ala Arg Ser Val Arg Gln
                   85                  90                  95
   Tyr Lys Ile Leu Asn Cys Ala Asn Tyr His Leu Thr Gln Val Arg Thr
               100                 105                 110
   Asp Phe Tyr Asp Glu Phe Trp Gly Gln Gly Leu Arg Ala Ala Pro Lys
           115                 120                 125
   Lys Gln Lys Lys His Thr Leu Ser Leu Thr Pro Asp Thr Thr Leu Tyr
       130                 135                 140
   Asn Ala Ala Gln Ile Ile Cys Ala Asn Tyr Gly Glu Ala Phe Ser Val
   145                 150                 155                 160
   Asp Lys Lys Gly Gly Thr Lys Lys Ala Ala Val Ser Glu Leu Leu Gln
                   165                 170                 175
   Ala Ser Ala Pro Tyr Lys Ala Asp Val Glu Leu Cys Val Tyr Ser Thr
               180                 185                 190
   Asn Glu Thr Thr Asn Cys Thr Gly Gly Lys Asn Gly Ile Ala Ala Asp
           195                 200                 205
   Ile Thr Thr Ala Lys Gly Tyr Val Lys Ser Val Thr Thr Ser Asn Gly
       210                 215                 220
   Ala Ile Thr Val Lys Gly Asp Gly Thr Leu Ala Asn Met Glu Tyr Ile
   225                 230                 235                 240
   Leu Gln Ala Thr Gly Asn Ala Ala Thr Gly Val Thr Trp Thr Thr Thr
                   245                 250                 255
   Cys Lys Gly Thr Asp Ala Ser Leu Phe Pro Ala Asn Phe Cys Gly Ser
               260                 265                 270
   Val Thr Gln
           275
<210> 195
   <211> 834
   <212> DNA
   <213> Artificial Sequence
<220>
   <223> LVL778
<400> 195
   atgaaatacc tgctgccgac cgctgctgct ggtctgctgc tcctcgctgc ccagccggcg 60
   atggccagcg cccagattca gaaggctgaa caaaatgatg tgaagctggc accgccgact 120
   gatgtacgaa gcggatatat acgtttggta aagaatgtga attattacat cgatagtgaa 180
   tcgatctggg tggataacca agagccacaa attgtacatt ttgatgcagt ggtgaattta 240
   gataagggat tgtatgttta tcctgagcct aaacgttatg cacgttctgt tcgtcagtat 300
   aagatcttga attgtgcaaa ttatcattta actcaagtac gaactgattt ctatgatgaa 360
   ttttggggac agggtttgcg ggcagcacct aaaaagcaaa agaaacatac gttaagttta 420
   acacctgata caacgcttta taatgctgct cagattattt gtgcgaacta tggtgaagca 480
   ttttcagttg ataaaaaagg cggcactaaa aaagcagcgg tatctgaatt actgcaagcg 540
   tcagcgcctt ataaggctga tgtggaatta tgtgtatata gcacaaatga aacaacaaac 600
   tgtacgggtg gaaaaaatgg tattgcagca gatataacca cagcaaaagg ctatgtaaaa 660
   tcagtgacaa caagcaacgg tgcaataaca gtaaaagggg atggcacatt ggcaaatatg 720
   gaatatattt tgcaagctac aggtaatgct gcaacaggtg taacttggac aacaacttgc 780
   aaaggaacgg atgcctcttt atttccagca aatttttgcg gaagtgtcac acaa       834
<210> 196
   <211> 278
   <212> PRT
   <213> Artificial Sequence
<220>
   <223> LVL778
<400> 196
   Met Lys Tyr Leu Leu Pro Thr Ala Ala Ala Gly Leu Leu Leu Leu Ala
    1               5                  10                  15
   Ala Gln Pro Ala Met Ala Ser Ala Gln Ile Gln Lys Ala Glu Gln Asn
               20                  25                  30
   Asp Val Lys Leu Ala Pro Pro Thr Asp Val Arg Ser Gly Tyr Ile Arg
           35                  40                  45
   Leu Val Lys Asn Val Asn Tyr Tyr Ile Asp Ser Glu Ser Ile Trp Val
       50                  55                  60
   Asp Asn Gln Glu Pro Gln Ile Val His Phe Asp Ala Val Val Asn Leu
   65                  70                  75                  80
   Asp Lys Gly Leu Tyr Val Tyr Pro Glu Pro Lys Arg Tyr Ala Arg Ser
                   85                  90                  95
   Val Arg Gln Tyr Lys Ile Leu Asn Cys Ala Asn Tyr His Leu Thr Gln
               100                 105                 110
   Val Arg Thr Asp Phe Tyr Asp Glu Phe Trp Gly Gln Gly Leu Arg Ala
           115                 120                 125
   Ala Pro Lys Lys Gln Lys Lys His Thr Leu Ser Leu Thr Pro Asp Thr
       130                 135                 140
   Thr Leu Tyr Asn Ala Ala Gln Ile Ile Cys Ala Asn Tyr Gly Glu Ala
   145                 150                 155                 160
   Phe Ser Val Asp Lys Lys Gly Gly Thr Lys Lys Ala Ala Val Ser Glu
                   165                 170                 175
   Leu Leu Gln Ala Ser Ala Pro Tyr Lys Ala Asp Val Glu Leu Cys Val
               180                 185                 190
   Tyr Ser Thr Asn Glu Thr Thr Asn Cys Thr Gly Gly Lys Asn Gly Ile
           195                 200                 205
   Ala Ala Asp Ile Thr Thr Ala Lys Gly Tyr Val Lys Ser Val Thr Thr
       210                 215                 220
   Ser Asn Gly Ala Ile Thr Val Lys Gly Asp Gly Thr Leu Ala Asn Met
   225                 230                 235                 240
   Glu Tyr Ile Leu Gln Ala Thr Gly Asn Ala Ala Thr Gly Val Thr Trp
                   245                 250                 255
   Thr Thr Thr Cys Lys Gly Thr Asp Ala Ser Leu Phe Pro Ala Asn Phe
               260                 265                 270
   Cys Gly Ser Val Thr Gln
           275
<210> 197
   <211> 831
   <212> DNA
   <213> Artificial Sequence
<220>
   <223> LVL779
<400> 197
   atgaaatacc tgctgccgac cgctgctgct ggtctgctgc tcctcgctgc ccagccggcg 60
   atggccgccc agattcagaa ggctgaacaa aatgatgtga agctggcacc gccgactgat 120
   gtacgaagcg gatatatacg tttggtaaag aatgtgaatt attacatcga tagtgaatcg 180
   atctgggtgg ataaccaaga gccacaaatt gtacattttg atgcagtggt gaatttagat 240
   aagggattgt atgtttatcc tgagcctaaa cgttatgcac gttctgttcg tcagtataag 300
   atcttgaatt gtgcaaatta tcatttaact caagtacgaa ctgatttcta tgatgaattt 360
   tggggacagg gtttgcgggc agcacctaaa aagcaaaaga aacatacgtt aagtttaaca 420
   cctgatacaa cgctttataa tgctgctcag attatttgtg cgaactatgg tgaagcattt 480
   tcagttgata aaaaaggcgg cactaaaaaa gcagcggtat ctgaattact gcaagcgtca 540
   gcgccttata aggctgatgt ggaattatgt gtatatagca caaatgaaac aacaaactgt 600
   acgggtggaa aaaatggtat tgcagcagat ataaccacag caaaaggcta tgtaaaatca 660
   gtgacaacaa gcaacggtgc aataacagta aaaggggatg gcacattggc aaatatggaa 720
   tatattttgc aagctacagg taatgctgca acaggtgtaa cttggacaac aacttgcaaa 780
   ggaacggatg cctctttatt tccagcaaat ttttgcggaa gtgtcacaca a          831
<210> 198
   <211> 277
   <212> PRT
   <213> Artificial Sequence
<220>
   <223> LVL779
<400> 198
   Met Lys Tyr Leu Leu Pro Thr Ala Ala Ala Gly Leu Leu Leu Leu Ala
    1               5                  10                  15
   Ala Gln Pro Ala Met Ala Ala Gln Ile Gln Lys Ala Glu Gln Asn Asp
               20                  25                  30
   Val Lys Leu Ala Pro Pro Thr Asp Val Arg Ser Gly Tyr Ile Arg Leu
           35                  40                  45
   Val Lys Asn Val Asn Tyr Tyr Ile Asp Ser Glu Ser Ile Trp Val Asp
       50                  55                  60
   Asn Gln Glu Pro Gln Ile Val His Phe Asp Ala Val Val Asn Leu Asp
   65                  70                  75                  80
   Lys Gly Leu Tyr Val Tyr Pro Glu Pro Lys Arg Tyr Ala Arg Ser Val
                   85                  90                  95
   Arg Gln Tyr Lys Ile Leu Asn Cys Ala Asn Tyr His Leu Thr Gln Val
               100                 105                 110
   Arg Thr Asp Phe Tyr Asp Glu Phe Trp Gly Gln Gly Leu Arg Ala Ala
           115                 120                 125
   Pro Lys Lys Gln Lys Lys His Thr Leu Ser Leu Thr Pro Asp Thr Thr
       130                 135                 140
   Leu Tyr Asn Ala Ala Gln Ile Ile Cys Ala Asn Tyr Gly Glu Ala Phe
   145                 150                 155                 160
   Ser Val Asp Lys Lys Gly Gly Thr Lys Lys Ala Ala Val Ser Glu Leu
                   165                 170                 175
   Leu Gln Ala Ser Ala Pro Tyr Lys Ala Asp Val Glu Leu Cys Val Tyr
               180                 185                 190
   Ser Thr Asn Glu Thr Thr Asn Cys Thr Gly Gly Lys Asn Gly Ile Ala
           195                 200                 205
   Ala Asp Ile Thr Thr Ala Lys Gly Tyr Val Lys Ser Val Thr Thr Ser
       210                 215                 220
   Asn Gly Ala Ile Thr Val Lys Gly Asp Gly Thr Leu Ala Asn Met Glu
   225                 230                 235                 240
   Tyr Ile Leu Gln Ala Thr Gly Asn Ala Ala Thr Gly Val Thr Trp Thr
                   245                 250                 255
   Thr Thr Cys Lys Gly Thr Asp Ala Ser Leu Phe Pro Ala Asn Phe Cys
               260                 265                 270
   Gly Ser Val Thr Gln
           275
<210> 199
   <211> 819
   <212> DNA
   <213> Artificial Sequence
<220>
   <223> LVL780
<400> 199
   atgaaatacc tgctgccgac cgctgctgct ggtctgctgc tcctcgctgc ccagccggcg 60
   atggccaagg ctgaacaaaa tgatgtgaag ctggcaccgc cgactgatgt acgaagcgga 120
   tatatacgtt tggtaaagaa tgtgaattat tacatcgata gtgaatcgat ctgggtggat 180
   aaccaagagc cacaaattgt acattttgat gcagtggtga atttagataa gggattgtat 240
   gtttatcctg agcctaaacg ttatgcacgt tctgttcgtc agtataagat cttgaattgt 300
   gcaaattatc atttaactca agtacgaact gatttctatg atgaattttg gggacagggt 360
   ttgcgggcag cacctaaaaa gcaaaagaaa catacgttaa gtttaacacc tgatacaacg 420
   ctttataatg ctgctcagat tatttgtgcg aactatggtg aagcattttc agttgataaa 480
   aaaggcggca ctaaaaaagc agcggtatct gaattactgc aagcgtcagc gccttataag 540
   gctgatgtgg aattatgtgt atatagcaca aatgaaacaa caaactgtac gggtggaaaa 600
   aatggtattg cagcagatat aaccacagca aaaggctatg taaaatcagt gacaacaagc 660
   aacggtgcaa taacagtaaa aggggatggc acattggcaa atatggaata tattttgcaa 720
   gctacaggta atgctgcaac aggtgtaact tggacaacaa cttgcaaagg aacggatgcc 780
   tctttatttc cagcaaattt ttgcggaagt gtcacacaa                        819
<210> 200
   <211> 273
   <212> PRT
   <213> Artificial Sequence
<220>
   <223> LVL780
<400> 200
   Met Lys Tyr Leu Leu Pro Thr Ala Ala Ala Gly Leu Leu Leu Leu Ala
    1               5                  10                  15
   Ala Gln Pro Ala Met Ala Lys Ala Glu Gln Asn Asp Val Lys Leu Ala
               20                  25                  30
   Pro Pro Thr Asp Val Arg Ser Gly Tyr Ile Arg Leu Val Lys Asn Val
           35                  40                  45
   Asn Tyr Tyr Ile Asp Ser Glu Ser Ile Trp Val Asp Asn Gln Glu Pro
       50                  55                  60
   Gln Ile Val His Phe Asp Ala Val Val Asn Leu Asp Lys Gly Leu Tyr
   65                  70                  75                  80
   Val Tyr Pro Glu Pro Lys Arg Tyr Ala Arg Ser Val Arg Gln Tyr Lys
                   85                  90                  95
   Ile Leu Asn Cys Ala Asn Tyr His Leu Thr Gln Val Arg Thr Asp Phe
               100                 105                 110
   Tyr Asp Glu Phe Trp Gly Gln Gly Leu Arg Ala Ala Pro Lys Lys Gln
           115                 120                 125
   Lys Lys His Thr Leu Ser Leu Thr Pro Asp Thr Thr Leu Tyr Asn Ala
       130                 135                 140
   Ala Gln Ile Ile Cys Ala Asn Tyr Gly Glu Ala Phe Ser Val Asp Lys
   145                 150                 155                 160
   Lys Gly Gly Thr Lys Lys Ala Ala Val Ser Glu Leu Leu Gln Ala Ser
                   165                 170                 175
   Ala Pro Tyr Lys Ala Asp Val Glu Leu Cys Val Tyr Ser Thr Asn Glu
               180                 185                 190
   Thr Thr Asn Cys Thr Gly Gly Lys Asn Gly Ile Ala Ala Asp Ile Thr
           195                 200                 205
   Thr Ala Lys Gly Tyr Val Lys Ser Val Thr Thr Ser Asn Gly Ala Ile
       210                 215                 220
   Thr Val Lys Gly Asp Gly Thr Leu Ala Asn Met Glu Tyr Ile Leu Gln
   225                 230                 235                 240
   Ala Thr Gly Asn Ala Ala Thr Gly Val Thr Trp Thr Thr Thr Cys Lys
                   245                 250                 255
   Gly Thr Asp Ala Ser Leu Phe Pro Ala Asn Phe Cys Gly Ser Val Thr
               260                 265                 270
   Gln
<210> 201
   <211> 816
   <212> DNA
   <213> Artificial Sequence
<220>
   <223> LVL781
<400> 201
   atgaaatacc tgctgccgac cgctgctgct ggtctgctgc tcctcgctgc ccagccggcg 60
   atggccgctg aacaaaatga tgtgaagctg gcaccgccga ctgatgtacg aagcggatat 120
   atacgtttgg taaagaatgt gaattattac atcgatagtg aatcgatctg ggtggataac 180
   caagagccac aaattgtaca ttttgatgca gtggtgaatt tagataaggg attgtatgtt 240
   tatcctgagc ctaaacgtta tgcacgttct gttcgtcagt ataagatctt gaattgtgca 300
   aattatcatt taactcaagt acgaactgat ttctatgatg aattttgggg acagggtttg 360
   cggcagcacc ctaaaaagca aaagaaacat acgttaagtt taacacctga tacaacgctt 420
   tataatgctg ctcagattat ttgtgcgaac tatggtgaag cattttcagt tgataaaaaa 480
   ggcggcacta aaaaagcagc ggtatctgaa ttactgcaag cgtcagcgcc ttataaggct 540
   gatgtggaat tatgtgtata tagcacaaat gaaacaacaa actgtacggg tggaaaaaat 600
   ggtattgcag cagatataac cacagcaaaa ggctatgtaa aatcagtgac aacaagcaac 660
   ggtgcaataa cagtaaaagg ggatggcaca ttggcaaata tggaatatat tttgcaagct 720
   acaggtaatg ctgcaacagg tgtaacttgg acaacaactt gcaaaggaac ggatgcctct 780
   ttatttccag caaatttttg cggaagtgtc acacaa                           816
<210> 202
   <211> 272
   <212> PRT
   <213> Artificial Sequence
<220>
   <223> LVL781
<400> 202
   Met Lys Tyr Leu Leu Pro Thr Ala Ala Ala Gly Leu Leu Leu Leu Ala
    1               5                  10                  15
   Ala Gln Pro Ala Met Ala Ala Glu Gln Asn Asp Val Lys Leu Ala Pro
               20                  25                  30
   Pro Thr Asp Val Arg Ser Gly Tyr Ile Arg Leu Val Lys Asn Val Asn
           35                  40                  45
   Tyr Tyr Ile Asp Ser Glu Ser Ile Trp Val Asp Asn Gln Glu Pro Gln
       50                  55                  60
   Ile Val His Phe Asp Ala Val Val Asn Leu Asp Lys Gly Leu Tyr Val
   65                  70                  75                  80
   Tyr Pro Glu Pro Lys Arg Tyr Ala Arg Ser Val Arg Gln Tyr Lys Ile
                   85                  90                  95
   Leu Asn Cys Ala Asn Tyr His Leu Thr Gln Val Arg Thr Asp Phe Tyr
               100                 105                 110
   Asp Glu Phe Trp Gly Gln Gly Leu Arg Ala Ala Pro Lys Lys Gln Lys
           115                 120                 125
   Lys His Thr Leu Ser Leu Thr Pro Asp Thr Thr Leu Tyr Asn Ala Ala
       130                 135                 140
   Gln Ile Ile Cys Ala Asn Tyr Gly Glu Ala Phe Ser Val Asp Lys Lys
   145                 150                 155                 160
   Gly Gly Thr Lys Lys Ala Ala Val Ser Glu Leu Leu Gln Ala Ser Ala
                   165                 170                 175
   Pro Tyr Lys Ala Asp Val Glu Leu Cys Val Tyr Ser Thr Asn Glu Thr
               180                 185                 190
   Thr Asn Cys Thr Gly Gly Lys Asn Gly Ile Ala Ala Asp Ile Thr Thr
           195                 200                 205
   Ala Lys Gly Tyr Val Lys Ser Val Thr Thr Ser Asn Gly Ala Ile Thr
       210                 215                 220
   Val Lys Gly Asp Gly Thr Leu Ala Asn Met Glu Tyr Ile Leu Gln Ala
   225                 230                 235                 240
   Thr Gly Asn Ala Ala Thr Gly Val Thr Trp Thr Thr Thr Cys Lys Gly
                   245                 250                 255
   Thr Asp Ala Ser Leu Phe Pro Ala Asn Phe Cys Gly Ser Val Thr Gln
               260                 265                 270
<210> 203
   <211> 813
   <212> DNA
   <213> Artificial Sequence
<220>
   <223> LVL782
<400> 203
   atgaaatacc tgctgccgac cgctgctgct ggtctgctgc tcctcgctgc ccagccggcg 60
   atggccgaac aaaatgatgt gaagctggca ccgccgactg atgtacgaag cggatatata 120
   cgtttggtaa agaatgtgaa ttattacatc gatagtgaat cgatctgggt ggataaccaa 180
   gagccacaaa ttgtacattt tgatgcagtg gtgaatttag ataagggatt gtatgtttat 240
   cctgagccta aacgttatgc acgttctgtt cgtcagtata agatcttgaa ttgtgcaaat 300
   tatcatttaa ctcaagtacg aactgatttc tatgatgaat tttggggaca gggtttgcgg 360
   gcagcaccta aaaagcaaaa gaaacatacg ttaagtttaa cacctgatac aacgctttat 420
   aatgctgctc agattatttg tgcgaactat ggtgaagcat tttcagttga taaaaaaggc 480
   ggcactaaaa aagcagcggt atctgaatta ctgcaagcgt cagcgcctta taaggctgat 540
   gtggaattat gtgtatatag cacaaatgaa acaacaaact gtacgggtgg aaaaaatggt 600
   attgcagcag atataaccac agcaaaaggc tatgtaaaat cagtgacaac aagcaacggt 660
   gcaataacag taaaagggga tggcacattg gcaaatatgg aatatatttt gcaagctaca 720
   ggtaatgctg caacaggtgt aacttggaca acaacttgca aaggaacgga tgcctcttta 780
   tttccagcaa atttttgcgg aagtgtcaca caa                              813
<210> 204
   <211> 271
   <212> PRT
   <213> Artificial Sequence
<220>
   <223> LVL782
<400> 204
   Met Lys Tyr Leu Leu Pro Thr Ala Ala Ala Gly Leu Leu Leu Leu Ala
   1               5                   10                  15
   Ala Gln Pro Ala Met Ala Glu Gln Asn Asp Val Lys Leu Ala Pro Pro
               20                  25                  30
   Thr Asp Val Arg Ser Gly Tyr Ile Arg Leu Val Lys Asn Val Asn Tyr
           35                  40                  45
   Tyr Ile Asp Ser Glu Ser Ile Trp Val Asp Asn Gln Glu Pro Gln Ile
       50                  55                  60
   Val His Phe Asp Ala Val Val Asn Leu Asp Lys Gly Leu Tyr Val Tyr
   65                  70                  75                  80
   Pro Glu Pro Lys Arg Tyr Ala Arg Ser Val Arg Gln Tyr Lys Ile Leu
                   85                  90                  95
   Asn Cys Ala Asn Tyr His Leu Thr Gln Val Arg Thr Asp Phe Tyr Asp
               100                 105                 110
   Glu Phe Trp Gly Gln Gly Leu Arg Ala Ala Pro Lys Lys Gln Lys Lys
           115                 120                 125
   His Thr Leu Ser Leu Thr Pro Asp Thr Thr Leu Tyr Asn Ala Ala Gln
       130                 135                 140
   Ile Ile Cys Ala Asn Tyr Gly Glu Ala Phe Ser Val Asp Lys Lys Gly
   145                 150                 155                 160
   Gly Thr Lys Lys Ala Ala Val Ser Glu Leu Leu Gln Ala Ser Ala Pro
                   165                 170                 175
   Tyr Lys Ala Asp Val Glu Leu Cys Val Tyr Ser Thr Asn Glu Thr Thr
               180                 185                 190
   Asn Cys Thr Gly Gly Lys Asn Gly Ile Ala Ala Asp Ile Thr Thr Ala
           195                 200                 205
   Lys Gly Tyr Val Lys Ser Val Thr Thr Ser Asn Gly Ala Ile Thr Val
       210                 215                 220
   Lys Gly Asp Gly Thr Leu Ala Asn Met Glu Tyr Ile Leu Gln Ala Thr
   225                 230                 235                 240
   Gly Asn Ala Ala Thr Gly Val Thr Trp Thr Thr Thr Cys Lys Gly Thr
                   245                 250                 255
   Asp Ala Ser Leu Phe Pro Ala Asn Phe Cys Gly Ser Val Thr Gln
               260                 265                 270
<210> 205
   <211> 95
   <212> DNA
   <213> Artificial Sequence
<220>
   <223> primer
<400> 205
   gatatacata tgaaatacct gctgccgacc gctgctgctg gtctgctgct cctcgctgcc 60
   cagccggcga tggccattca gaaggctgaa caaaa                            95
<210> 206
   <211> 39
   <212> DNA
   <213> Artificial Sequence
<220>
   <223> primer
<400> 206
   ggccgcaagc ttttagtggt ggtggtggtg gtggccgcc 39
<210> 207
   <211> 30
   <212> DNA
   <213> Artificial Sequence
<220>
   <223> primer
<400> 207
   ggccgcaagc ttttattgtg tgacacttcc 30
<210> 208
   <211> 42
   <212> DNA
   <213> Artificial Sequence
<220>
   <223> primer
<400> 208
   gctgcccagc cggcgatggc caaggctgaa caaaatgatg tg 42
<210> 209
   <211> 42
   <212> DNA
   <213> Artificial Sequence
<220>
   <223> primer
<400> 209
   cacatcattt tgttcagcct tggccatcgc cggctgggca gc 42
<210> 210
   <211> 43
   <212> DNA
   <213> Artificial Sequence
<220>
   <223> primer
<400> 210
   gctgcccagc cggcgatggc cgctgaacaa aatgatgtga agc 43
<210> 211
   <211> 43
   <212> DNA
   <213> Artificial Sequence
<220>
   <223> primer
<400> 211
   gcttcacatc attttgttca gcggccatcg ccggctgggc agc 43
<210> 212
   <211> 43
   <212> DNA
   <213> Artificial Sequence
<220>
   <223> primer
<400> 212
   gctgcccagc cggcgatggc cgaacaaaat gatgtgaagc tgg 43
<210> 213
   <211> 43
   <212> DNA
   <213> Artificial Sequence
<220>
   <223> primer
<400> 213
   ccagcttcac atcattttgt tcggccatcg ccggctgggc agc 43
<210> 214
   <211> 43
   <212> DNA
   <213> Artificial Sequence
<220>
   <223> primer
<400> 214
   gctgcccagc cggcgatggc cgcccagatt cagaaggctg aac 43
<210> 215
   <211> 43
   <212> DNA
   <213> Artificial Sequence
<220>
   <223> primer
<400> 215
   gttcagcctt ctgaatctgg gcggccatcg ccggctgggc agc 43
<210> 216
   <211> 46
   <212> DNA
   <213> Artificial Sequence
<220>
   <223> primer
<400> 216
   gctgcccagc cggcgatggc cagcgcccag attcagaagg ctgaac 46
<210> 217
   <211> 46
   <212> DNA
   <213> Artificial Sequence
<220>
   <223> primer
<400> 217
   gttcagcctt ctgaatctgg gcgctggcca tcgccggctg ggcagc 46
<210> 218
   <211> 30
   <212> DNA
   <213> Artificial Sequence
<220>
   <223> primer
<400> 218
   cacacacata tgaaatacct gctgccgacc 30
<210> 219
   <211> 253
   <212> PRT
   <213> Artificial Sequence
<220>
   <223> FUSION PROTEIN
<400> 219
   Ile Gln Lys Ala Glu Gln Asn Asp Val Lys Leu Ala Pro Pro Thr Asp
    1               5                  10                  15
   Val Arg Ser Gly Tyr Ile Arg Leu Val Lys Asn Val Asn Tyr Tyr Ile
               20                  25                  30
   Asp Ser Glu Ser Ile Trp Val Asp Asn Gln Glu Pro Gln Ile Val His
           35                  40                  45
   Phe Asp Ala Val Val Asn Leu Asp Lys Gly Leu Tyr Val Tyr Pro Glu
       50                  55                  60
   Pro Lys Arg Tyr Ala Arg Ser Val Arg Gln Tyr Lys Ile Leu Asn Cys
   65                  70                  75                  80
   Ala Asn Tyr His Leu Thr Gln Val Arg Thr Asp Phe Tyr Asp Glu Phe
                   85                  90                  95
   Trp Gly Gln Gly Leu Arg Ala Ala Pro Lys Lys Gln Lys Lys His Thr
               100                 105                 110
   Leu Ser Leu Thr Pro Asp Thr Thr Leu Tyr Asn Ala Ala Gln Ile Ile
           115                 120                 125
   Cys Ala Asn Tyr Gly Glu Ala Phe Ser Val Asp Lys Lys Gly Gly Thr
       130                 135                 140
   Lys Lys Ala Ala Val Ser Glu Leu Leu Gln Ala Ser Ala Pro Tyr Lys
   145                 150                 155                 160
   Ala Asp Val Glu Leu Cys Val Tyr Ser Thr Asn Glu Thr Thr Asn Cys
                   165                 170                 175
   Thr Gly Gly Lys Asn Gly Ile Ala Ala Asp Ile Thr Thr Ala Lys Gly
               180                 185                 190
   Tyr Val Lys Ser Val Thr Thr Ser Asn Gly Ala Ile Thr Val Lys Gly
           195                 200                 205
   Asp Gly Thr Leu Ala Asn Met Glu Tyr Ile Leu Gln Ala Thr Gly Asn
       210                 215                 220
   Ala Ala Thr Gly Val Thr Trp Thr Thr Thr Cys Lys Gly Thr Asp Ala
   225                 230                 235                 240
   Ser Leu Phe Pro Ala Asn Phe Cys Gly Ser Val Thr Gln
                   245                 250
<210> 220
   <211> 818
   <212> PRT
   <213> Streptococcus pneumoniae
<400> 220
   Ser Tyr Glu Leu Gly Arg His Gln Ala Gly Gln Val Lys Lys Glu Ser
   1               5                   10                  15
   Asn Arg Val Ser Tyr Ile Asp Gly Asp Gln Ala Gly Gln Lys Ala Glu
               20                  25                  30
   Asn Leu Thr Pro Asp Glu Val Ser Lys Arg Glu Gly Ile Asn Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Ile Ile
   65                  70                  75                  80
   Ser Glu Glu Leu Leu Met Lys Asp Pro Asn Tyr Gln Leu Lys Asp Ser
                   85                  90                  95
   Asp Ile Val Asn Glu Ile Lys Gly Gly Tyr Val Ile Lys Val Asp Gly
               100                 105                 110
   Lys Tyr Tyr Val Tyr Leu Lys Asp Ala Ala His Ala Asp Asn Ile Arg
           115                 120                 125
   Thr Lys Glu Glu Ile Lys Arg Gln Lys Gln Glu His Ser His Asn His
       130                 135                 140
   Gly Gly Gly Ser Asn Asp Gln Ala Val Val Ala Ala Arg Ala Gln Gly
   145                 150                 155                 160
   Arg Tyr Thr Thr Asp Asp Gly Tyr Ile Phe Asn Ala Ser Asp Ile Ile
                   165                 170                 175
   Glu Asp Thr Gly Asp Ala Tyr Ile Val Pro His Gly Asp His Tyr His
               180                 185                 190
   Tyr Ile Pro Lys Asn Glu Leu Ser Ala Ser Glu Leu Ala Ala Ala Glu
           195                 200                 205
   Ala Tyr Trp Asn Gly Lys Gln Gly Ser Arg Pro Ser Ser Ser Ser Ser
       210                 215                 220
   Tyr Asn Ala Asn Pro Ala Gln Pro Arg Leu Ser Glu Asn His Asn Leu
   225                 230                 235                 240
   Thr Val Thr Pro Thr Tyr His Gln Asn Gln Gly Glu Asn Ile Ser Ser
                   245                 250                 255
   Leu Leu Arg Glu Leu Tyr Ala Lys Pro Leu Ser Glu Arg His Val Glu
               260                 265                 270
   Ser Asp Gly Leu Ile Phe Asp Pro Ala Gln Ile Thr Ser Arg Thr Ala
           275                 280                 285
   Arg Gly Val Ala Val Pro His Gly Asn His Tyr His Phe Ile Pro Tyr
       290                 295                 300
   Glu Gln Met Ser Glu Leu Glu Lys Arg Ile Ala Arg Ile Ile Pro Leu
   305                 310                 315                 320
   Arg Tyr Arg Ser Asn His Trp Val Pro Asp Ser Arg Pro Glu Gln Pro
                   325                 330                 335
   Ser Pro Gln Ser Thr Pro Glu Pro Ser Pro Ser Pro Gln Pro Ala Pro
   340 345 350
   Asn Pro Gln Pro Ala Pro Ser Asn Pro Ile Asp Glu Lys Leu Val Lys
   355 360 365
   Glu Ala Val Arg Lys Val Gly Asp Gly Tyr Val Phe Glu Glu Asn Gly
   370 375 380
   Val Ser Arg Tyr Ile Pro Ala Lys Asp Leu Ser Ala Glu Thr Ala Ala
   385 390 395 400
   Gly Ile Asp Ser Lys Leu Ala Lys Gln Glu Ser Leu Ser His Lys Leu
   405 410 415
   Gly Ala Lys Lys Thr Asp Leu Pro Ser Ser Asp Arg Glu Phe Tyr Asn
   420 425 430
   Lys Ala Tyr Asp Leu Leu Ala Arg Ile His Gln Asp Leu Leu Asp Asn
   435 440 445
   Lys Gly Arg Gln Val Asp Phe Glu Ala Leu Asp Asn Leu Leu Glu Arg
   450 455 460
   Leu Lys Asp Val Pro Ser Asp Lys Val Lys Leu Val Asp Asp Ile Leu
   465 470 475 480
   Ala Phe Leu Ala Pro Ile Arg His Pro Glu Arg Leu Gly Lys Pro Asn
   485 490 495
   Ala Gln Ile Thr Tyr Thr Asp Asp Glu Ile Gln Val Ala Lys Leu Ala
   500 505 510
   Gly Lys Tyr Thr Thr Glu Asp Gly Tyr Ile Phe Asp Pro Arg Asp Ile
   515 520 525
   Thr Ser Asp Glu Gly Asp Ala Tyr Val Thr Pro His Met Thr His Ser
   530 535 540
   His Trp Ile Lys Lys Asp Ser Leu Ser Glu Ala Glu Arg Ala Ala Ala
   545 550 555 560
   Gln Ala Tyr Ala Lys Glu Lys Gly Leu Thr Pro Pro Ser Thr Asp His
   565 570 575
   Gln Asp Ser Gly Asn Thr Glu Ala Lys Gly Ala Glu Ala Ile Tyr Asn
   580 585 590
   Arg Val Lys Ala Ala Lys Lys Val Pro Leu Asp Arg Met Pro Tyr Asn
   595 600 605
   Leu Gln Tyr Thr Val Glu Val Lys Asn Gly Ser Leu Ile Ile Pro His
   610 615 620
   Tyr Asp His Tyr His Asn Ile Lys Phe Glu Trp Phe Asp Glu Gly Leu
   625 630 635 640
   Tyr Glu Ala Pro Lys Gly Tyr Thr Leu Glu Asp Leu Leu Ala Thr Val
   645 650 655
   Lys Tyr Tyr Val Glu His Pro Asn Glu Arg Pro His Ser Asp Asn Gly
   660 665 670
   Phe Gly Asn Ala Ser Asp His Val Arg Lys Asn Lys Val Asp Gln Asp
   675 680 685
   Ser Lys Pro Asp Glu Asp Lys Glu His Asp Glu Val Ser Glu Pro Thr
   690 695 700
   His Pro Glu Ser Asp Glu Lys Glu Asn His Ala Gly Leu Asn Pro Ser
   705 710 715 720
   Ala Asp Asn Leu Tyr Lys Pro Ser Thr Asp Thr Glu Glu Thr Glu Glu
   725 730 735
   Glu Ala Glu Asp Thr Thr Asp Glu Ala Glu Ile Pro Gln Val Glu Asn
   740 745 750
   Ser Val Ile Asn Ala Lys Ile Ala Asp Ala Glu Ala Leu Leu Glu Lys
   755 760 765
   Val Thr Asp Pro Ser Ile Arg Gln Asn Ala Met Glu Thr Leu Thr Gly
   770 775 780
   Leu Lys Ser Ser Leu Leu Leu Gly Thr Lys Asp Asn Asn Thr Ile Ser
   785 790 795 800
   Ala Glu Val Asp Ser Leu Leu Ala Leu Leu Lys Glu Ser Gln Pro Ala
   805 810 815
   Pro Ile